# EUROPEAN PATENT SPECIFICATION

(11) **EP 3 590 523 B1**
(45) Date of publication and mention of the grant of the patent: **15.09.2021**
(21) Application number: 19184361.4
(22) Date of filing: 18.07.2016
(51) Int. Cl.: A61K 38/01, C07K 14/415, C12N 9/02, A61K 8/64, A61K 38/00, A61P 17/02, A61P 35/00, A61Q 19/08

(54) **GROWTH PROMOTING PEPTIDES AND USES THEREOF**
WACHSTUMSFÖRDERNDE PEPTIDE UND VERWENDUNGEN DAVON
PEPTIDES POUR PROMOUVOIR LA CROISSANCE ET LEURS UTILISATIONS

(30) Priority: 16.07.2015 EP 15177017
(43) Date of publication of application: 08.01.2020
(62) Divisional of application: 16751182.3
(73) Proprietor: Nuritas Limited, D02 RY95 Dublin 2 (IE)
(72) Inventor: Khaldi, Nora, Dublin D02RY95 (IE); Lopez, Cyril, Dublin D02RY95 (IE); Adelfio, Alessandro, Dublin D02RY95 (IE)
(74) Representative: Purdylucey Intellectual Property

(56) References cited:
- DATABASE UniProt [Online] 1 July 1989 (1989-07-01), "Seed linoleate 9S-lipoxygenase-3; EC=1.13.11.58; Lipoxygenase-3 from Pisum sativum", XP002794154, retrieved from EBI accession no. UNIPROT:P09918 Database accession no. P09918
- HUGHES ET AL: "characterization of authentic recombinant pea-seed lipoxygenases with distinct properties and reaction mechanisms", BIOCHEMICAL JOURNAL, PUBLISHED BY PORTLAND PRESS ON BEHALF OF THE BIOCHEMICAL SOCIETY, vol. 333, no. PART 01, 1 July 1998 (1998-07-01), pages 33-43, XP002107003, ISSN: 0264-6021

## Description

### Background to the Invention

The growing will of maintaining a youthful appearance is leading to more and more research of new dermatological procedures for treatment of skin aging, especially when people keeps living longer and healthier. With age, structural and functional changes are be observed in human skin (Calleja-Agius J. *et al.* 2013) and many factors are responsible for them like environmental factors such as UV radiation from sunlight or internal factors such as changes in hormones due menopause (Affinito P. *et al.* 1999).

The alterations of connective tissue in the dermis and epidermis, especially the reduction of the extracellular matrix is highly responsible for skin wrinkling and sagging since they induce important changes in its mechanical properties. Furthermore, metabolism and synthesis of the extra-cellular matrix are affected by the ageing process through enzymatic activities.

There are mainly four solutions to fight against ageing process at the moment:
*Diet and hormones management:* fighting the ageing process by managing our diet by using supplements. This method is still controverted at the moment. Some studies suggest that antioxidant supplements like Vitamin C or lipoic acid for example could have anti-ageing properties.

*Hormone treatment:* a risky and controverted solution for anti-ageing purpose. One have to be really careful with anything related to hormones since it can have a broad spectrum of adverse effects. This solution isn't widely used and most of the studies are still on the animal stage.

*Surgical anti-aging solutions:* they are very effective on the short term but they are costly and can require long period of healing. Like in every surgical operation, there are risks but also side effects. For example, during surgical anti-aging solutions like eyelifts and facelifts, where an incision is made at the hairline, there are some risks of bruising, swelling, drooping eyelids, and secondary infections can occur.

Recently, there has been an increasing enthusiasm on minimally invasive treatments and techniques designed to deal with problems like wrinkles, volume loss and other skin damages. The most common topical anti-ageing solutions are creams and serums. Their active ingredients can be divided in several families:
*Moisturiser* ingredients will keep the skin hydrated. It's mostly big polar molecules that will make bonds with water like glycosaminoglycan (hyaluronic acid for instance).

*Collagen and other extra-cellular matrix* related ingredients will contribute to maintain a well organised skin structure by stimulating biosynthesis if they can trigger the right receptors. *Cell proliferation* ingredients are important as well since they help to regenerate skin cells which will synthesise the component the skin needs like extra-cellular matrix and growth factors.

It is an object of the invention to provide an alternative minimally invasive treatment of skin ageing.

### Statements of Invention

The invention provides peptides, conjugates, compositions, methods and uses as set out in the claims.

The peptides of the invention are used in the topical cosmetic or pharmaceutical composition of this invention at cosmetically or pharmaceutically effective concentrations to achieve the desired effect; in a preferred form with regards to the total weight of the composition, between 0.00000001% (in weight) and 20% (in weight); preferably between 0.000001% (in weight) and 15% (in weight), more preferably between 0.0001% (in weight) and 10% (in weight) and even more preferably between 0.0001% (in weight) and 5% (in weight). Ideally, the peptides of the present invention are preferably used from about 0.00001% w/w to about 0.5% w/w [0.1 to 5000 ppm], and more preferably from 0.00005 w/w to about 0.05 w/w [0.5 to 500 ppm], and most preferably from about 0.0001 w/w to about 0.01 w/w of the composition [1 to 100 ppm]. Ideally, the peptides of the present invention are preferably used from about 0.0001% w/w to about 0.004% w/w of the composition.

For compositions of peptides of the invention, a typical daily dosage may be 0.2g to 100g. However, when administered as a food for special medicinal purpose, or medical food, the daily dosage may be 50-500g per day.

The dosage of compositions of the invention for use in food products and food supplements (i.e. comestible compositions) will be broadly in the 0.2-100 g/day range. In one embodiment, the daily dosage is 1-10 g/day, ideally about 3-8 g/day. In one embodiment, the daily dosage is 10-20 g/day. In one embodiment, the daily dosage is 20-30 g/day. In one embodiment, the daily dosage is 30-40 g/day. In one embodiment, the daily dosage is 10-100 g/day. In one embodiment, the daily dosage is about 5 g/day, ideally about 3-8 g/day. In one embodiment, the dosage is 2-1000 mg/day/kg body weight. In one embodiment, the dosage is 10-500 mg/day/kg body weight. In one embodiment, the dosage is 10-100 mg/day/kg body weight. In one embodiment, the dosage is 30-70 mg/day/kg body weight. The dosage of peptides of the invention for food supplements may be 0.00001mg-0.01mg per day or dose.

The invention also provides topical composition comprising a peptide of the invention. It will be appreciated that the topical composition may comprise a plurality of peptides, fragments and/or variants. In one embodiment, the topical composition comprises substantially all the peptides. In one embodiment, the topical composition comprises substantially all the variants. The topical composition of the invention may be presented in a formulation selected from the group comprising creams, multiple emulsions, anhydrous compositions, aqueous dispersions, oils, milks, balsams, foams, lotions, gels, cream gels, hydro-alcoholic solutions, hydro-glycolic solutions, cosmetic, personal care product, hydrogels, liniments, sera, soaps, dusting powder, paste, semi solid formulations, liniments, serums, shampoo, conditioner, ointments, any rinse off formulation, talc, mousses, powders, sprays, aerosols, solutions, suspensions, emulsions, syrups, elixirs, polysaccharide films, patches, gel patches, bandages, an adhesive system, water-in-oil emulsions, oil-in-water emulsions, and silicone emulsions.

In an embodiment of the current invention, the emulsion contains a lipid or oil. The emulsion may be, but is not limited to, oil-in-water, water-in-oil, water-in-oil-in-water and oil-in-water-in-silcone emulsions. The emulsion may contain a humectant. The emulsion may contain an anti-foaming agent, such as silicone. The emulsion may have any suitable viscosity. Emulsions may further contain an emulsifier and/or an anti-foaming agent. Methods of preparing an emulsion are known to a person skilled in the art.

The topical composition of the invention may be incorporated into a medical device for administration. Such a device can include but is not limited to a fabric, patch, bandage, gauge, sock, tight, underwear, dressing, glove, mask, adhesive patches, non-adhesive patches, occlusive patches and microelectric patches or suitable adhesive system. In such an embodiment, the device is in direct contact with the keratinous layer such as the skin, thus releasing the peptides of the invention. It will be understood that the topical composition may be incorporated in any suitable form as detailed herein. For example, the topical composition or peptides of the invention can be incorporated into the device or be present on the surface of the device or can be in a cream, gel or wax formulation or any suitable formulation defined herein and incorporated into the device or on the surface of the device.

The device may be adapted for adhesion or attachment to the skin.

In one embodiment the device is adapted to release a constant quantity of the composition or the peptides of the invention. It will be understood that the amount of the composition contained in the sustained release system will depend, for example, on where the composition is to be administered, the kinetics and duration of the release of the composition of the invention, as well as the nature of the condition, disorder and/or disease to be treated and/or cared for. The device may be such that the composition is released by biodegradation of the device, or by friction between the device and the body, due to bodily moisture, the skin's pH or body temperature.

In an embodiment of the invention the topical composition may further comprise at least one cosmetically or pharmaceutically acceptable excipient. Excipient may be used interchangeably with functional ingredient or additive. It will be understood that although the topical compositions of the current invention can be administered alone, they will generally be administered in admixture with a cosmetic or pharmaceutical excipient. Cosmetically or pharmaceutically acceptable excipient are well known in the art and any known excipient, may be used provided that it is suitable for topical administration and is dermatologically acceptable without undue toxicity, incompatibility and/or allergic reaction.

Preferably any excipient included is present in trace amounts. The amount of excipient included will depend on numerous factors, including the type of excipient used, the nature of the excipient, the component(s) of the topical composition, the amount of active or peptide in the topical composition and/or the intended use of the topical composition. The nature and amount of any excipient should not unacceptably alter the benefits of the peptides of this invention.

In an embodiment of the invention the excipient may be a suitable diluent, carrier, binder, lubricant, suspending agent, coating agent, preservative, stabilisers, dyes, vehicle, solubilising agent, base, emollient, emulsifying agent, fragrance, humectant, and/or surfactants.

Examples of suitable diluents include, but are not limited to, any diluent disclosed in disclosed in US2014120131 or US2004132667. Examples include ethanol, glycerol and water.

Examples of suitable carriers include, but are not limited to, lactose, starch, glucose, methyl cellulose, magnesium stearate, mannitol, sorbitol and any suitable carrier disclosed in US2014120131 or US2004132667.

Examples of suitable binders include, but are not limited to, starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol and any suitable binder disclosed in US2014120131 or US2004132667.

Examples of suitable lubricants include, but are not limited to, sodium oleate, sodium stearate, magnesium stearate, sodium benzoate, sodium acetate, and sodium chloride and any suitable lubricant disclosed in US2014120131 or US2004132667.

The carrier may be any suitable carried known in the art or disclosed in US2014120131 or US2004132667. In some embodiments, the carrier may include, but is not limited to, a liquid, such as water, oils or surfactants, including those of petroleum, animal, plant or synthetic origin, polymer, oil, such as peanut oil, mineral oil, castor oil, soybean oil, alcohol, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, or digitonin. It will be understood that the carrier will be dermatologically acceptable. Preferred carriers contain an emulsion such as oil-in-water, water-in-oil, water-in-oil-in-water and oil-in-water-in-silicone emulsions. Emulsions may further contain an emulsifier and/or an anti-foaming agent.

In an embodiment of the invention, the topical composition may further comprise one or more additional ingredients. The topical composition of the invention may be administered consecutively, simultaneously or sequentially with the one or more other additional agents. Such additional ingredients may be those of benefit to include in a topical composition, or of benefit depending on the intended use of the topical composition. The additional ingredient may be active or functional or both.

Examples of such additional ingredients include, but are not limited to, one or more of cleaning agents, conditioning agents, sunscreen, pigment, moisturiser, thickening agents, gelling agents, essential oil, astringents, pigments, anti-caking agent, anti-foaming agent, binders, additives, buffers, chelating agents, external analgesics, film formers or materials, bulking agents, polymers, opacifying agents, pH adjusters, propellants, reducing agents, sequestrants, skin bleaching and lightening agents, skin conditioning agents, aloe vera, healing agents, soothing agents, smoothing agents, pantothenic acid, treating agents, thickeners, vitamins. colourants, pharmaceuticals, antiseptic agents, antifoaming agents, buffering agents, astringents, polymers, pH adjuster, deodorant or any other dermatologically acceptable carrier or surfactant.

It is to be understood that additional ingredients listed may provide more than one benefit. The classification given herein is for clarity and convenience only and not intended to limit the additional ingredient to that particular application or category listed.

Any additional ingredients should be suitable for application to the skin without undue toxicity, incompatibility and/or allergic reaction.

In some embodiments, the additional ingredient has glucose transport activity or aids glucose transport activity. In some embodiments, the additional ingredient has anti-inflammatory activity or aids anti-inflammatory activity. In some embodiments, the additional ingredient has anti-aging activity or aids anti-aging activity. In some embodiments, the additional ingredient is for keratinous layer health and/or development, skin health and/or development, and/or muscle health, recovery and/or development. The active agent may be a pharmacological enhancer. Such active agents are known and available on the market. In such cases, the topical composition of the invention may be administered consecutively, simultaneously or sequentially with the one or more other active agents.

In some embodiments, the additional ingredient may be farnesol ([2E, 6E], -3, 7, 11,-trimethyl-2, 6, 10, dodecatrien-1-ol), phytantriol (3, 7, 11, 15, tetramethylhexadecane-1, 2, 3, -triol), desquamation actives, enzymes, enzyme inhibitors, enzyme activators, botanical extracts and marine extracts, anti-acne actives, anti-wrinkle or anti atrophy actives, antioxidant/radical scavengers, chelators, flavonoids, anti-inflammatory agents, anti-cellulite agents, topical anaesthetics, tanning actives, skin lightening agents, skin healing agents, bisabolol, antimicrobial or antifungal active, sunscreen actives, particulate material, conditioning agents, structuring agents, thickening agent,

The desquamation active may be any suitable agent that enhances the skin appearance or texture of the skin and is as disclosed in US2014120131 or US2004132667.

Examples of anti-acne actives are as disclosed in US2014120131 or US2004132667 and include, resorcinol, salicylic acid, erythromycin, zine, sulfur, benzoyl peroxides.

Examples of thickening agents are as disclosed in US2014120131 or US2004132667 and include carboxylic acid polymers, crosslinked polyacrylate polymers, polyacrylamide polymers, polysaccharides.

Examples of conditioning agents are as disclosed in US2014120131 or US2004132667 and include humectants, moisturiser or skin conditioner.

Examples of structuring agents are as disclosed in US2014120131 or US2004132667 and include any agent that provide rheological characteristics to the composition and contributes to the stability of the composition.

Any suitable antimicrobial or antifungal active may be used and examples are as disclosed in US2014120131 or US2004132667. Such actives are capable of destroying microbes, preventing growth or action of microbes. Examples include but are not limited to β-lactam drugs, quinolone drugs, tetracycline, erythromycin, streptomycin sulfate, salicylic acid, benzoyl peroxide.

Examples of a particulate material include metallic oxide. Examples of anti-cellulite agents include xanthine agents. Examples of tanning actives includes 1, 3-dihydroxy-2-propanone and those disclosed in US2014120131 or US2004132667. Examples of topical anaesthetics include benzocaine, lidocaine and bupivacaine and those disclosed in US2014120131 or US2004132667.

Examples of skin lightening agents include any agent known in the art such as kojic acid, ascorbic acid and those disclosed in US2014120131 or US2004132667.

Examples of sunscreen actives include any suitable organic or inorganic sunscreen active. Examples include metallic oxides, 2-ethylhexyl-p-methoxycinnamate and those disclosed in US2014120131 or US2004132667.

Examples of skin healing agents includes panthenoic acid as disclosed in US2014120131 or US2004132667.

Examples of anti-inflammatory agents include any agent that enhances the skin appearance, tone or colour and include but are not limited to corticosteroids, hydrocortisone, non-steroidal agents such as ibuprofen and aspirin and those disclosed in US2014120131 or US2004132667.

Examples of flavonoids includes flavanones, methoxy flavonones, unsubstituted chalcone and mixtures thereof and those disclosed in US2014120131 or US2004132667.

Examples of enzymes include lipases, proteases, catalase, super oxide-dismutase, amylase, peroxidase, glucuronidase, ceramidases, hyaluronidases. Examples of enzyme inhibitors include trypsine inhibitors, Bowmann Birk inhibitors, chymotrypsin inhibitors, botanical extracts, flavonoids, quercetin chalcone and those disclosed in US2014120131 or US2004132667 and mixtures thereof. Examples of enzyme activators include coenzyme A, Q10 (ubiquinone), glycyrrhizin, berberine, chrysin and those disclosed in US2014120131 or US2004132667 and mixtures thereof

Examples of anti-wrinkle or anti atrophy actives include sulfur containing D and L amino acids, particular, N-acyl derivatives such as N-acetyl-L-cysteine, hydroxyl acids, phytic acid, lipoic acid, lysophosphatidic acid, skin peel agents, vitamin B₃, retinoids and those disclosed in US2014120131 or US2004132667 and mixtures thereof.

The anti-oxidant/radical scavenger agent may be any agent that is useful for providing protection against UV radiation or other environmental agents which may cause skin damage such as those disclosed in US2014120131 or US2004132667. Examples of antioxidant/radical scavengers include ascorbic acid, its salts and derivatives (vitamin C), tocopherol its salts and derivatives (vitamin E), butylated hydroxyl benzoic acids and their salts, peroxides, gallic acids and alkyl esters, sorbic acid, lipoic acid, amines, lycine pidolate, arginine pilolate, nordihydroguaiaretic acid, bioflavonoids, curcumin, lysine, methionine, proline, superoxide dismutase, silymarin, tea extracts and mixtures thereof.

Examples of chelators include EDTA, NTA, hydoxamic acids, phytic acid, lactoferrin and those disclosed in US2014120131 or US2004132667 and mixtures thereof. A chelator means an agent capable of removing a metal ion by forming a complex so that the metal ion cannot participate in or catalyse chemical reactions. A chelator is useful for protection against UV radiation or other environmental agents that can cause skin damage.

It will be appreciated that a plurality of additional ingredients may be added. The amount of the additional ingredient may be from about 0.001% to about 50% weight of the composition, preferably, about 0.01% to about 20%, preferably about 0.1% to about 10%, about 0.5% to about 10%, about 1% to about 5%, preferably 2% weight of the composition. The amount of additional ingredient included will depend on numerous factors, including the type of additional ingredient used, the nature of the additional ingredient, the component(s) of the topical composition, the amount of active or peptide in the topical composition and/or the intended use of the topical composition. The nature and amount of any additional ingredient should not unacceptably alter the benefits of the peptides of this invention.

The topical composition may be alcohol free.

In some embodiments of the invention, the composition further comprises one or more additional active agents, in addition to the peptide of the invention (also known as the active of the composition). In addition, or alternatively, the composition may be administered with one or more other additional active agents. Typical said additional active agent is present in trace amounts only. In some embodiments, there may be no additional active agent present in the composition. The amount of additional active agent included will depend on numerous factors, including the type of additional active agent used, the nature of the additional active agent, the component(s) of the topical composition, the amount of active or peptide in the topical composition and/or the intended use of the topical composition. The nature and amount of any additional active agent should not unacceptably alter the benefits of the peptides of this invention.

It is to be understood that an ingredient that is considered to be an "active" ingredient in one product may be a "functional" or "excipient" ingredient in another and vice versa. It will also be appreciated that some ingredients play a dual role as both an active ingredient and as a functional or excipient ingredient.

Examples of the additional active agents include glucose transport promoting drugs, skin supplement, agent for treatment and/or care of the skin, anti-inflammatory agent, an anti-aging agent, a cellular growth promoting agent and pharmacological enhancers. Such agents are well known in the art and it will be appreciated that any suitable additional active agent may be used. Additional active agents for treatment and/or care of the skin may include collagen synthesis agents, retinoids, exfoliating agents, anti-cellulite agents, elastase inhibiting agents, melanin synthesis stimulating or inhibiting agents, self-tanning agents, antiaging agents, antimicrobial agents, antifungal agents, fungistatic agents, bactericidal agents, and healing agents. Active agents also include anti-inflammatory agents.

Any additional active agent should be suitable for application to the skin without undue toxicity, incompatibility and/or allergic reaction.

It will be understood that the classification given herein is for clarity and convenience only and not intended to limit the additional ingredient, excipient, or active to that particular application or category listed.

In a particularly preferred embodiment, the methods and uses of the invention involve administration of a peptide or composition of the invention in combination with one or more other active agents, for example, existing growth promoting drugs or pharmacological enhancers available on the market. In such cases, the compounds of the invention may be administered consecutively, simultaneously or sequentially with the one or more other active agents.

The effect of the current invention is accomplished by topical application or administration of the topical composition of the invention described herein to a person, animal or a patient in need of treatment or care. Topical delivery preferably means delivery to a keratinous layer such as the skin, hair and/or nails, but can also mean delivery to a body lumen lined with epithelial cells, for example the lungs or airways, the gastrointestinal tract, the buccal cavity. The effect may be confined to the surface of the skin or may be within the skin or a combination of both.

The topical composition of the invention is administered in a cosmetically or pharmaceutically effective amount. In other words, in an amount that is non-toxic but sufficient amount to provide the desired effect. It will be appreciated that a person skilled in the art would be capable of determining an appropriate dose of the topical compositions of the invention to administer without undue experimentation. Alternatively, a physician will determine the actual dose that is most suitable for a patient depending on the particular condition, disease or disorder to be treated or cared for and the age, body weight and/or health of the person. It will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. There can, of course, be individual instances where higher or lower dosage ranges are merited, and such are within the scope of this invention. For example, the composition may be administered at a dose of from 0.01 to 50 mg/kg body weight, such as from 0.1 to 30 mg/kg, more preferably from 0.1 to 20 mg/kg body weight, more preferably from 0.1 to 10 mg/kg body weight, preferably 0.1 to 5mg/kg body weight. In an exemplary embodiment, one or more doses of 10 to 300 mg/day or more preferably, 10 to 150 mg/day, will be administered to the patient. The amount and the frequency is as best suited to the purpose. The frequency of application or administration can vary greatly, depending on the needs of each subject, with a recommendation of an application or administration range from once a month to ten times a day, preferably from once a week to four times a day, more preferably from three times a week to three times a day, even more preferably once or twice a day.

In preferred embodiments, repeated use of the topical composition is provided.

The topical composition may be applied by, but not limited to, rubbing, or massaging into the keratinous tissue, skin or area of the body to be treated or cared for. In some embodiments, the composition is left on or not removed from the area of the body. In other embodiments, the composition is removed after a period of time, such as, but not limited to, from about 2 minutes to 60 minutes, from about 5 minutes to about 30 minutes, preferably from about 10 minutes to about 20 minutes. The composition may be removed immediately after application. In some embodiments of the current invention, the composition of the invention may be applied to an area to be treated by means to achieve a greater penetration of the composition and/or peptide of the invention, such as, but not limited to, iontophoresis, sonophoresis, electroporation, microelectric patches, mechanical pressure, osmotic pressure gradient, occlusive cure, microinjections or needle-free injections by means of pressure, such as injections by oxygen pressure, or any combination thereof.

The peptides of the invention are used in the topical cosmetic or pharmaceutical composition of this invention at cosmetically or pharmaceutically effective concentrations to achieve the desired effect; in a preferred form with regards to the total weight of the composition, between 0.00000001% (in weight) and 20% (in weight); preferably between 0.000001% (in weight) and 15% (in weight), more preferably between 0.0001% (in weight) and 10% (in weight) and even more preferably between 0.0001% (in weight) and 5% (in weight).

In some embodiments of the current invention, the composition may be delivered via any one of liposomes, mixed liposomes, oleosomes, niosomes, ethosomes, millicapsules, capsules, macrocapsules, nanocapsules, nanostructured lipid carriers, sponges, cyclodextrins, vesicles, micelles, mixed micelles of surfactants, surfactant-phospholipid mixed micelles, millispheres, spheres, lipospheres, particles, nanospheres, nanoparticles,milliparticles, solid nanopartciles as well as microemulsions including water-in-oil microemulsions with an internal structure of reverse micelle and nanoemulsions microspheres, microparticles.

A variety of methods are available for preparing liposomes. See, e.g., Szoka et al., Ann. Rev. Biophys. Bioeng. 9:467 (1980), U.S. Pat. Nos. 4,186,183, 4,217,344, 4,235,871, 4,261,975, 4,485,054, 4,501,728, 4,774,085, 4,837,028, 4,235,871, 4,261,975, 4,485,054, 4,501,728, 4,774,085, 4,837,028, 4,946,787, PCT Publication No. WO 91/17424, Deamer & Bangham, Biochim. Biophys. Acta 443:629-634 (1976); Fraley, et al., PNAS 76:3348-3352 (1979); Hope et al., Biochim. Biophys. Acta 812:55-65 (1985); Mayer et al., Biochim. Biophys. Acta 858:161-168 (1986); Williams et al., PNAS 85:242-246 (1988); Liposomes (Ostro (ed.), 1983, Chapter 1); Hope et al., Chem. Phys. Lip. 40:89 (1986); Gregoriadis, Liposome Technology (1984) and Lasic, Liposomes: from Physics to Applications (1993)). Suitable methods include, for example, sonication, extrusion, high pressure/homogenization, microfluidization, detergent dialysis, calcium-induced fusion of small liposome vehicles and ether fusion methods, all of which are well known in the art.

These delivery systems may be adapted to achieve a greater penetration of the compound and/or peptides of the invention. This may improve pharmacokinetic and pharmacodynamics properties. The delivery system may be a sustained release system wherein the compound or peptide of the invention is gradually released during a period of time and preferably with a constant release rate over a period of time. The delivery systems are prepared by methods known in the art. The amount of peptide contained in the sustained release system will depend on where the composition is to be delivered and the duration of the release as well as the type of the condition, disease and/or disorder to be treated or cared for.

The topical composition of the invention may be for human or animal usage in human and veterinary medicine.

The topical composition of the invention may be used for pharmaceutical, personal care and/or cosmetic uses.

The composition can be used to treat or care for any disease, disorder or condition of the skin, including but not limited to, psoriasis, dermatitis, allergic dermatitis, eczema, spongiosis, edema, skin cancer, ulcers, acne, scars, cellulitis, elastosis, keratosis, rosacea, varicose veins, inflammatory disorders.

The topical composition may be used to for treating or caring for visible signs of aging including but not limited to wrinkles, stretch marks and dark circles, dryness, fine lines, age spots, red blotches, sagging skin, and conditions caused by sun exposure including sunburn, stress, pollution and/diet. The topical composition may also be used for delaying, slowing or inhibiting the skins or the onset of aging. The composition may be administered by a medical device, such as a plaster or a patch as described herein.

The topical composition may be used to treat or care for a wound in a mammal. In another embodiment, the topical composition is for use in the treatment or prevention of a disease or condition characterised by damaged epithelial cells or tissue, and/or damaged dermal or epithelial cells or tissue. The disease may be but is not limited to cancer and trauma.

The topical composition may be used to treat or care for any muscle condition, to improve, muscle status in a mammal, to promote recovery of muscle, typically following exercise, to maintain or restore muscle health (for example lean tissue mass) in a mammal, to enhance physical performance, in treatment or prevention of a disease or condition characterised by lethargy or low energy levels.

The topical composition may be used to promote growth of a tissue, promote growth of epithelial tissue, promote growth of skin, promote growth of an organ, promote growth of an organism. The skin can have a normal pathology and/or an abnormal pathology.

The topical composition may also be used to treat or care for any inflammatory disorder.

A further aspect of the invention relates to a pharmaceutical composition comprising a peptide of the invention or a composition of peptides of the invention, admixed with one or more pharmaceutically acceptable diluents, excipients or carriers. Even though the peptides and compositions of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients, 2nd Edition, (1994), Edited by A Wade and PJ Weller. In particular, formulations for topical delivery are described in Topical drug delivery formulations edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985). Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of p-hydroxybenzoic acid. Antioxidants and suspending agents may be also used.

The peptide or composition of the invention may be adapted for topical, oral, rectal, parenteral, intramuscular, intraperitoneal, intra-arterial, intrabronchial, subcutaneous, intradermal, intravenous, nasal, vaginal, buccal or sublingual routes of administration. For oral administration, particular use is made of compressed tablets, pills, tablets, gellules, drops, and capsules. Preferably, these compositions contain from 1 to 250 mg and more preferably from 10-100 mg, of active ingredient per dose. Other forms of administration comprise solutions or emulsions which may be injected intravenously, intra-arterial, subcutaneously, intradermally, intraperitoneally or intramuscularly, and which are prepared from sterile or sterilisable solutions. The pharmaceutical compositions of the present invention may also be in form of suppositories, vaginal rings, pessaries, suspensions, emulsions, lotions, ointments, creams, gels, sprays, solutions or dusting powders. The composition of the invention may be formulated for topical delivery. Topical delivery generally means delivery to the skin, but can also mean delivery to a body lumen lined with epithelial cells, for example the lungs or airways, the gastrointestinal tract, the buccal cavity. In particular, formulations for topical delivery are described in Topical drug delivery formulations edited by David Osborne and Antonio Aman, Taylor & Francis, the complete contents of which are incorporated herein by reference. Compositions or formulations for delivery to the airways are described in O'Riordan et al (Respir Care, 2002, Nov. 47), EP2050437, WO2005023290, US2010098660, and US20070053845. Composition and formulations for delivering active agents to the iluem, especially the proximal iluem, include microparticles and microencapsulates where the active agent is encapsulated within a protecting matrix formed of polymer or dairy protein that is acid resistant but prone to dissolution in the more alkaline environment of the ileum. Examples of such delivery systems are described in EP1072600.2 and EP13171757.1. An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Injectable forms may contain between 10-1000 mg, preferably between 10-250 mg, of active ingredient per dose.

Compositions may be formulated in unit dosage form, i.e., in the form of discrete portions containing a unit dose, or a multiple or sub-unit of a unit dose.

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention. Depending upon the need, the agent may be administered at a dose of from 0.01 to 30 mg/kg body weight, such as from 0.1 to 10 mg/kg, more preferably from 0.1 to 1 mg/kg body weight. In an exemplary embodiment, one or more doses of 10 to 300 mg/day or more preferably, 10 to 150 mg/day, will be administered to the patient for the treatment of an inflammatory disorder.

In a particularly preferred embodiment, the methods and uses of the invention involve administration of a peptide or composition of the invention in combination with one or more other active agents, for example, existing anti-inflammatory drugs or pharmacological enhancers available on the market. In such cases, the compounds of the invention may be administered consecutively, simultaneously or sequentially with the one or more other active agents.

In one embodiment of the invention, the peptide of the invention may be administered in the form of a conjugate comprising the peptide, and may optionally include a linker, and a partner molecule, for example a protein such as an antibody molecule intended to increase the half-life of the conjugate in-vivo. In one embodiment, the peptide may be modified to substitute one or more amino acids with amino acids employed to attach partner molecules. For example, an amino acid may be substituted with a lysine residue for the purpose of conjugating a partner molecule such as a PEG molecule.

### Definitions

All publications, patents, patent applications and other references mentioned herein are hereby incorporated by reference in their entireties for all purposes as if each individual publication, patent or patent application were specifically and individually indicated to be incorporated by reference and the content thereof recited in full.

Where used herein and unless specifically indicated otherwise, the following terms are intended to have the following meanings in addition to any broader (or narrower) meanings the terms might enj oy in the art:
Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s) (for example, the reduction in accumulation of pathological levels of lysosomal enzymes). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an effective amount or a therapeutically effective amount of an agent defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

The term "human or animal" should be understood to means humans or mammalian or non-mammalian animals such as fish.

The term "composition" should be understood to mean a composition of matter made by the hand of man and not occurring in nature. Exemplary compositions include food compositions, beverage compositions, pharmaceutical compositions, nutritional supplement compositions, personal care compositions and healthcare compositions.

The term "peptide" used herein refers to a polymer composed of 5 to 50 amino acid monomers typically via peptide bond linkage. Peptides (including fragments and variants thereof) of and for use in the invention may be generated wholly or partly by chemical synthesis or by expression from nucleic acid. For example, the peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984). When necessary, any of the peptides employed in the invention can be chemically modified to increase their stability. A chemically modified peptide or a peptide analog includes any functional chemical equivalent of the peptide characterized by its increased stability and/or efficacy in vivo or in vitro in respect of the practice of the invention. The term peptide analog also refers to any amino acid derivative of a peptide as described herein. A peptide analog can be produced by procedures that include, but are not limited to, modifications to side chains, incorporation of unnatural amino acids and/or their derivatives during peptide synthesis and the use of crosslinkers and other methods that impose conformational constraint on the peptides or their analogs. Examples of side chain modifications include modification of amino groups, such as by reductive alkylation by reaction with an aldehyde followed by reduction with NaBH₄; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH₄. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. Peptide structure modification includes the generation of retro-inverso peptides comprising the reversed sequence encoded by D-amino acids.

The term "modified peptide" is used interchangeably with the term derivative of the peptide. The modified peptide includes a peptide which has been substituted with one or more groups as defined herein. The modification may be any modified that provides the peptides and or the composition of the invention with an increased ability to penetrate a cell. The modification may be any modification that increases the half-life of the composition or peptides of the invention. In one embodiment, the group is a protecting group. The protecting group may be an N-terminal protecting group, a C-terminal protecting group or a side-chain protecting group. The peptide may have one or more of these protecting groups. The person skilled in the art is aware of suitable techniques to react amino acids with these protecting groups. These groups can be added by preparation methods known in the art, for example the methods as outlined in paragraphs [0104] to [0107] of US2014120141. The groups may remain on the peptide or may be removed. The protecting group may be added during synthesis. In an embodiment of the invention the peptides may be substituted with a group selected from one or more straight chain or branched chain, long or short chain, saturated, or unsaturated, substituted with a hydroxyl, amino, amino acyl, sulfate or sulphide group or unsubstituted having from 1 to 29 carbon atoms. N-acyl derivatives include acyl groups derived from acetic acid, capric acid, lauric acid, myristic acid, octanoic acid, palmitic acid, stearic acid, behenic acid, linoleic acid, linolenic acid, lipoic acid, oleic acid, isosteric acid, elaidoic acid, 2-ethylhexaneic acid, coconut oil fatty acid, tallow fatty acid, hardened tallow fatty acid, palm kernel fatty acid, lanolin fatty acid or similar acids. These may be substituted or unsubstituted. When substituted they are preferably substituted with hydroxyl, or sulphur containing groups such as but not limited to SO₃H, SH, or S-S. In an embodiment of the current invention, the peptide is R₁-X- R₂. R₁ and/or R₂ groups respectively bound to the amino-terminal (N-terminal) and carboxyl-terminal (C-terminal) of the peptide sequence. In one embodiment, the peptide is R₁-X. Alternatively, the peptide is X- R₂. Preferably, R₁ is H, C₁₋₄ alkyl, acetyl, benzoyl or trifluoroacetyl; X is the peptide of the invention; R₂ is OH or NH₂. In an embodiment, R₁ is selected from the group formed by H, a non-cyclic substituted or unsubstituted aliphatic group, substituted or unsubstituted alicyclyl, substituted or unsubstituted heterocyclyl, substituted or unsubstituted heteroarylalkyl, substituted or unsubstituted aryl, substituted or unsubstituted aralkyl, Tert-butyloxycarbonyl, 9-fluorenylmethyloxycarbonyl (Fmoc) and R₅-CO-, wherein R₅ is selected from the group formed by H, a non-cyclic substituted or unsubstituted aliphatic group, substituted or unsubstituted alicyclyl, substituted or unsubstituted aryl, substituted or unsubstituted aralkyl, substituted or unsubstituted heterocyclyl and substituted or unsubstituted heteroarylalkyl; R₂ is selected from the group formed by -NR₃R₄, -OR₃ and -SR₃, wherein R₃ and R₄ are independently selected from the group formed by H, a non-cyclic substituted or unsubstituted aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl; and with the condition that R₁ and R₂ are not α-amino acids. In accordance with another preferred embodiment, R₂ is -NR₃R₄, -OR₃ or -SR₃ wherein R₃ and R₄ are independently selected from the group formed by H, substituted or unsubstituted C₁-C₂₄ alkyl, substituted or unsubstituted C₂-C₂₄ alkenyl, Tert-butyloxycarbonyl, 9-fluorenylmethyloxycarbonyl (Fmoc), substituted or unsubstituted C₂-C ₂₄ alkynyl, substituted or unsubstituted C₃-C₂₄ cycloalkyl, substituted or unsubstituted C₅-C ₂₄ cycloalkenyl, substituted or unsubstituted C₈-C₂₄ cycloalkynyl, substituted or unsubstituted C₆-C₃₀ aryl, substituted or unsubstituted C₇-C₂₄ aralkyl, substituted or unsubstituted heterocyclyl ring of 3-10 members, and substituted or unsubstituted heteroarylalkyl of 2 to 24 carbon atoms and 1 to 3 atoms other than carbon wherein the alkyl chain is of 1 to 6 carbon atoms. Optionally, R₃ and R₄ can be bound by a saturated or unsaturated carbon-carbon bond, forming a cycle with the nitrogen atom. More preferably R₂ is -NR₃R₄ or -OR₃, wherein R₃ and R₄ are independently selected from the group formed by H, substituted or unsubstituted C₁-C₂₄ alkyl, substituted or unsubstituted C₂-C₂₄ alkenyl, substituted or unsubstituted C₂-C₂₄ alkynyl, substituted or unsubstituted C₃-C₁₀ cycloalkyl, substituted or unsubstituted C₆-C₁₅ aryl and substituted or unsubstituted heterocyclyl of 3-10 members, substituted or unsubstituted heteroarylalkyl with a ring of 3 to 10 members and an alkyl chain of 1 to 6 carbon atoms. More preferably R₃ and R₄ are selected from the group formed by H, methyl, ethyl, hexyl, dodecyl, or hexadecyl. Even more preferably R₃ is H and R₄ is selected from the group formed by H, methyl, ethyl, hexyl, dodecyl, or hexadecyl. In accordance with an even more preferred embodiment, R₂ is selected from -OH and -NH₂.

In accordance with another embodiment of this invention R₁ is selected from the group formed by H, acetyl, lauroyl, myristoyl or palmitoyl, and R₂ is -NR₃R₄ or -OR₃ wherein R₃ and R₄ are independently selected from H, methyl, ethyl, hexyl, dodecyl and hexadecyl, preferably R₂ is -OH or -NH₂ More preferably, R₁ is acetyl or palmitoyl and R₂ is -NH₂. In a preferred embodiment, the acyl group is bound to the N-terminal end of at least one amino acid of the peptide. In an embodiment of the invention, the peptide is modified to comprise a side chain protecting group. The side chain protecting group may be one or more of the group comprising benzyl or benzyl based groups, t-butyl-based groups, benzyloxy-carbonyl (Z) group, and allyloxycarbonyl (alloc) protecting group. The side chain protecting group may be derived from an achiral amino acid such as achiral glycine. The use of an achiral amino acid helps to stabilise the resultant peptide and also facilitate the facile synthesis route of the present invention. Preferably, the peptide further comprises a modified C-terminus, preferably an amidated C-terminus. The achiral residue may be alpha-aminoisobutyric acid (methylalaine). It will be appreciated that the specific side chain protecting groups used will depend on the sequence of the peptide and the type of N-terminal protecting group used.

"Conjugate": In one embodiment of the invention the peptide is conjugated, linked or fused to a binding partner, for example one or more polyethylene glycol polymers or other compounds, such as molecular weight increasing compounds or lipophilic groups. The molecular weight increasing compound is any compound that will increase the molecular weight, typically by 10% to 90%, or 20% to 50% of the resulting conjugate and may have a molecular weight of between 200 and 20, 000, preferably between 500 and 10, 000. The molecular weight increasing compound may be PEG, any water-soluble(amphiphilic or hydrophilic) polymer moiety, homo or co-polymers of PEG, a monomethyl-subsitututed polymer of PEG (mPEG) and polyoxyethylene glycerol (POG), polyamino acids such as poly-lysine, poly-glutamic acid, poly-aspartic acid, particular those of L conformation, pharmacologically inactive proteins such as albumin, gelatin, a fatty acid, olysaccharide, a lipid amino acid and dextran. The polymer moiety may be straight chained or branched and it may have a molecular weight of 500 to 40000Da, 5000 to 10000 Da, 10000 to 5000, Da. The compound (binding partner) may be any suitable cell penetrating compound, such as tat peptide, penetratin, pep-1. The compound (binding partner) may be an antibody molecule. The compound (binding partner) may be a lipophilic moiety or a polymeric moiety. The lipophilic substituent and polymeric substituents are known in the art. The lipophilic substituent includes an acyl group, a sulphonyl group, an N atom, an O atom or an S atom which forms part of the ester, sulphonyl ester, thioester, amide or sulphonamide. The lipophilic moiety may include a hydrocarbon chain having 4 to 30 C atoms, preferably between 8 and 12 C atoms. It may be linear or branched, saturated or unsaturated. The hydrocarbon chain may be further substituted. It may be cycloalkane or heterocycloalkane. The peptide may be modified at the N-terminal, C-terminal or both. The polymer or compound (binding partner) is preferably linked to an amino, carboxyl or thio group and may be linked by N-termini or C-termini of side chains of any amino acid residue. The polymer or compound (binding partner) may be conjugated to the side chain of any suitable residue. The polymer or compound (binding partner) may be conjugated via a spacer. The spacer may be a natural or unnatural amino acid, succinic acid, lysyl, glutamyl, asparagyl, glycyl, beta-alanyl, gamma-amino butanoyl. The polymer or compound (binding partner) may be conjugated via an ester, a sulphonyl ester, a thioester, an amide, a carbamate, a urea, a sulphonamide. A person skilled in the art is aware of suitable means to prepare the described conjugate.

"Fragment" means a segment of a protein selected from SEQUENCE ID NO's: 1 to 14, the fragment typically being 7 to 37 contiguous amino acids in length, and generally having a charge of between -9 and +3; a c-terminal amino acid that typcially is not cysteine (C) or methionine (M); and an n-terminal amino acid that typcially is not cysteine (C), histidine (H), proline (P) or threonine (T). The charge of a peptide, fragment or region is determined using the method of Cameselle, J.C., Ribeiro, J.M., and Sillero, A. (1986). Derivation and use of a formula to calculate the net charge of acid-base compounds. Its application to amino acids, proteins and nucleotides. Biochem. Educ. 14, 131-136.

The term "natural" as applied to a peptide means a peptide that includes (a) a fragment of a plant protein, typically rice or pea protein, or variants of pea protein including lentil, sweet pea, or chick pea or variants of rice protein including oat, grass, corn, wild rice and bananas, or (b) a variant of the fragment of a plant protein, for example a fragment of a homolog of the plant protein. The peptides or fragments of the invention may be isolated from plant proteins or made synthetically using methods known to a person skilled in the art and described herein.

"C-terminal domain" as applied to a fragment means the first three amino acids at the c-terminus of the fragment.

"N-terminal domain" as applied to a fragment means the last three amino acids at the n-terminus of the fragment.

"Bioactive" as applied to a peptide or fragment means having a health promoting effect when administered a mammal, for example one or more of glucose transport promoting, anti-bacterial, anti-inflammatory, or cellular growth or proliferation promoting. In one embodiment, the term "bioactive" means cellular growth promoting.

"Growth promoting" or "growth promoting activity" as applied to a peptide or fragment means a peptide or fragment that is capable of increasing elastin production or cellular proliferation of human skin treated with a 20µM solution of peptide or fragment as described in the assay below.

"Glucose transport promoting" or "glucose transport promoting activity" as applied to a peptide or variant or fragment means a peptide, variant or fragment that is capable of increasing GLUT4 translocation into skeletal muscle compared with an untreated control when employed at a concentration of 2µM in the following *in-vitro* assay. L6-GLUT4myc cells were grown in 10% FBS and 2 µg/ml blasticidin. Cells were grown for 48-72 hours before being seeded in 24-well plates at 15,000 cells per well in 2% FBS and allowed to differentiate for 6 to 8 days prior to experimentation. L6-GLUT4myc cells were serum-starved for three hours prior to incubation with 100 nM of insulin for 30mins, or 200, 20, 2.0 and 0.2 µM of SP, and 2, 1, 0.5 and .25mg/ml of pepide/peptide composition for 3 hours respectively. A 3-hour incubation period was selected based on previous findings identifying that incubation with branch chain amino acid containing di-peptides for 3 hours increases glucose uptake in L6 myotubes 1. Treatments were staggered in order to determine GLUT4myc translocation at the same time point. The quantity of myc-tagged GLUT4 at the cell surface was measured by antibody-coupled colorimetric assay. Briefly, after incubation with either insulin for 30mins or synthetic peptide or peptide composition for 3 hours respectively, L6-GLUT4myc cells were fixed via incubation with 3% paraformaldehyde (PFA). A 0.1 M glycine solution was then added to quench PFA and cells were blocked with 5% goat serum. The myotube monolayer was exposed to anti-myc antibody and then incubated with peroxidase conjugated donkey anti-mouse IgG. 1mL of o-phenylenediamine dihydrochloride (OPD) reagent was added to each well and this reaction was stopped by adding 250µl/well of 3 M HCL. To determine GLUT4 translocation to cell surface, a measured aliquot of each condition was determined spectrophotometrically on a plate reader using absorbance at 492nm. Preferably the peptide or fragment is capable of increasing GLUT4 translocation compared with an untreated control by at least 50% (i.e a relative unit increase in GLUT4 translocation of 1% to 1.5%).

"Antibacterial" or "antibacterial activity" as applied to a peptide or fragment means a peptide or fragment that is capable of visibly inhibiting the growth of a bacteria in the following agar-plate based growth inhibition assay: Peptide stock=5mg/mL dissolved in DMSO. Bacterial inoculums were adjusted to McFarland 0.5 standard and MHA plates swabbed. Blank disks were placed in the plates and 10 µL of each compound (at 64 µg/mL - maximum concentration tested) added. Plates were incubated at 37°C for 16-18 hours. Appropriate controls (DMSO; Mueller-Hinton media alone; and two antibiotic discs - ciprofloxacin and tetracycline) were also performed.

"Anti-inflammatory" as applied to a peptide or fragment means a peptide or fragment that is capable of significantly reducing the secretion of TNFa by LPS-stimulated J774.2 macrophages (compared with untreated LPS-stimulated J774.2 macrophages) when the macrophages are treated with 100µM of the peptide or fragment. J774.2 macrophages were treated with 100µM of synthetic peptide for 24 hours and then stimulated with (A) LPS (10ng/ml) for five hours or (B) LPS (10ng/ml) for 5 hours followed by ATP (5mM) for one hour. Supernatant was collected and levels of TNFa were determined by ELISA.

"Enriched in peptides having a molecular weight of less than 10 KD" as applied to a composition of the invention means that the dry weight % of peptides in the composition having a molecular weight of less than 10 KD is greater than the dry weight % of polypeptide/protein in the composition having a molecular weight of 10 KD or greater.

"Homolog" of a reference protein should be understood to mean a protein from a different species of plant having at least 60%, 70%, 80%, 90%, 91%, 92%, 93%, 94%, 95%, 96%, 97%, 98%, 99% sequence homology with the reference protein. Thus, for example, homologs of pea protein P13918 include:
>gi|137584|sp|P08438.1|VCL_VICFA RecName: Full=Vicilin; Flags: Precursor [Vicia faba]
>gi|22057|emb|CAA68559.1|vicilin [Vicia faba var. minor] >gi|383931031|gb|AFH56916.1| vicilin **[Vicia faba]**
>gi|502105533|ref|XP_004492829.1|PREDICTED: vicilin-like isoform X1 **[Cicer arietinum] ChickPea**
>gi|29539109|emb|CAD87730.1|allergen Len c 1.0101 **[Lens culinaris] Lentil**

A "variant" of a growth promoting fragment shall be taken to mean a fragment having an amino acid sequence that is substantially identical to the reference growth promoting fragment, and which has growth promoting activity as defined above. Thus, for example, the term should be taken to include fragments that are altered in respect of one or more amino acid residues. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Generally, the variant will have at least 70% amino acid sequence homology, preferably at least 80% sequence homology, more preferably at least 90% sequence homology, and ideally at least 95%, 96%, 97%, 98% or 99% sequence homology with the reference growth promoting fragment.

In this specification, the term "sequence identity" should be understand to comprise both sequence identity and similarity, i.e. a variant (or homolog) that shares 70% sequence identity with a reference sequence is one in which any 70% of aligned residues of the variant (or homolog) are identical to or conservative substitutions of the corresponding residues in the reference sequence across the entire length of the sequence. Sequence identity is the amount of characters which match exactly between two different sequences. Hereby, gaps are not counted and the measurement is relational to the shorter of the two sequences. In terms of "sequence homology", the term should be understood to mean that a variant (or homolog) which shares a defined percent similarity or identity with a reference sequence when the percentage of aligned residues of the variant (or homolog) are either identical to, or conservative substitutions of, the corresponding residues in the reference sequence and where the variant (or homolog) shares the same function as the reference sequence. This alignment and the percent homology or sequence identity can be determined using software programs known in the art, for example, one alignment program is BLAST, using default parameters. Details of these programs can be found at the following Internet address: http://www.ncbi.nlm.nih.gov/blast/Blast.cgi.
Variants of SEQUENCE ID NO: 448 (QSFLLSGNQ)
Variants of SEQUENCE ID NO: 448 (QSFLLSGNQ) including variants having 1 or 2 conservative amino acid substitutions, 1, 2 to 3 non-conservative amino acid substitutions, 1-2 amino acid additions, 1, 2 or 3 amino acid deletions, are provided below:
   One conservative amino acid substitution:
      QSFILSGNE, ESFLLSGNQ, QSYLLSGNQ, QSFLLSGDQ (SEQ ID NO'S 418-421)
   Two conservative amino acid substitutions:
      QSYLLSGNE, ESFLLSGNE, ESYLLSGNQ, QSFLLSGDE, QSYLLSGDQ (SEQ ID NO'S 422 to 426)
   One non-conservative amino acid substitution
      QSFRLSGNQ, QSFLLSYNQ, QFFLLSGNQ, QSFLLSGAQ, QSFLLSGNP (SEQ ID NO'S 427 to 431)
   Two non-conservative amino acid substitution
      QSFRRSGNQ, QSFLLSYIQ, QFFLLSGNL, QSFLLSGAQ, QSFLLSGNP (SEQ ID NO'S 432 to 436)
   One or two amino acid additions
      QSFLLSGNQQ, QSFLLLSGNQ, AQSFGLLSGNQ, RQSFLLISGNQ, QSFLLSGNQK (SEQ ID NO'S 437 to 441)
   One, two or three amino acid deletions

The term "variant" also includes fragment of peptides of the invention. "Fragment of a peptide of the invention" or "peptide fragment" means a fragment of one of the peptides of the invention having at least 3, 4, 5, 6, 7, 8, 9, 10, 11, 12, 13, 14, 15, 16, 17, 18, 19, 20, 21 or 22 amino acids and that typically has a bioactivity, for example anti-inflammatory activity, anti-ageing activity, glucose transport promoting activity, or anti-bacterial activity. In one embodiment, the fragment consists of at least 30%, 40%, 50%, 60%, 70%, 80%, or 90% of the reference sequence. Thus, the invention also provides bioactive fragments of the peptides of the invention, and peptides comprising one or more of these fragments. In one embodiment, the fragments are bioactive. In one embodiment, the fragments are cellular growth or proliferation promoting fragments. Examples of fragments of peptides of the invention are provide in SEQUENCE ID NO'S 494 to 524.

"Ani-ageing" means inhibiting or slowing the appearance of ageing of a human skin and/or reversing the appearance of ageing. "Slowing or inhibiting ageing of the skin" means slowing or inhibiting the ageing process in the skin, and/or reversing the appearance of ageing.

"Disease or condition characterised by damaged dermal or epithelial cells or tissue" means any condition or disease that results in damaged dermal or epithelial tissue or cells or organs. One example is trauma which often results in damaged skin. Another example is an inflammatory skin condition such as psoriasis or excezma which often results in damaged skin. Another example is an inflammatory disorder of the lower intestines which can result in damaged epithelial cells/tissue lining the lower intestines. Another example is damaged epithelial cells/tissue lining the lower intestines caused by ingestion of a toxic or damaging substance, for example toxic chemicals or drugs. Another example is cancer, for example bowel cancer, which can result in damaged epithelial tissue in the bowel. Another condition is a peripheral inflammatory disorder such as atopic dermatitis which can result in damage to the skin in humans.

"Inflammatory disorder" means an immune-mediated inflammatory condition that affects humans and is generally characterised by dysregulated expression of one or more cytokines. Examples of inflammatory disorders include skin inflammatory disorders, inflammatory disorders of the joints, inflammatory disorders of the cardiovascular system, certain autoimmune diseases, lung and airway inflammatory disorders, intestinal inflammatory disorders. Examples of skin inflammatory disorders include dermatitis, for example atopic dermatitis and contact dermatitis, acne vulgaris, and psoriasis. Examples of inflammatory disorders of the joints include rheumatoid arthritis. Examples of inflammatory disorders of the cardiovascular system are cardiovascular disease and atherosclerosis. Examples of autoimmune diseases include Type 1 diabetes, Graves disease, Guillain-Barre disease, Lupus, Psoriatic arthritis, and Ulcerative colitis. Examples of lung and airway inflammatory disorders include asthma, cystic fibrosis, COPD, emphysema, and acute respiratory distress syndrome. Examples of intestinal inflammatory disorders include colitis and inflammatory bowel disease. Other inflammatory disorders include cancer, hay fever, periodontitis, allergies, hypersensitivity, ischemia, depression, systemic diseases, post infection inflammation and bronchitis. The invention also relates to a peptide or composition of the invention for use in treating an inflammatory disorder in a mammal.

"Metabolic disorder" should be understood to include pre-diabetes, diabetes; Type-1 diabetes; Type-2 diabetes; metabolic syndrome; obesity; diabetic dyslipidemia; hyperlipidemia; hypertension; hypertriglyceridemia; hyperfattyacidemia; hypercholerterolemia; hyperinsulinemia, and MODY. The invention also relates to a peptide or composition of the invention for use in treating a metabolic disorder in a mammal.

"Disease or condition characterised by damaged dermal or epithelial cells or tissue" means any condition or disease that results in damaged dermal or epithelial tissue or cells or organs. One example is trauma which often results in damaged skin. Another example is an inflammatory skin condition such as psoriasis or excezma which often results in damaged skin. Another example is an inflammatory disorder of the lower intestines which can result in damaged epithelial cells/tissue lining the lower intestines. Another example is damaged epithelial cells/tissue lining the lower intestines caused by ingestion of a toxic or damaging substance, for example toxic chemicals or drugs. Another example is cancer, for example bowel cancer, which can result in damaged epithelial tissue in the bowel. Another condition is a peripheral inflammatory disorder such as atopic dermatitis which can result in damage to the skin in humans.

"Disease or condition characterised by bacterial infection" means any condition or disease characterised having a pathology caused by growth of bacteria or by bacterial infection, including for example MRSA, salmonella, listeria, bacterial pneumonia, Staphylococcal food poisoning, bacterial memingitis. Specific examples are provided in https://en.wikipedia.org/wiki/List_of_infectious_diseases.

"Man-made" as applied to comestible products should be understood to mean made by a human being and not existing in nature.

"Maintaining or restoring gut health" means reducing and/or regulating the pro-inflammatory response in the gut and more specifically the epithelial cells. The healthy microbiome offers some protection against pathogenic viruses and bacteria, and their presence is needed to guide the development of our immune system. It has been shown that these bacteria can react to human signals of stress, sickness, or age which can be manifested by inflammation and as a consequence switch on their virulence genes and cause or contribute to disease. Having the ability to reduce and maintain at healthy levels the inflammatory response can help maintain the healthy bacteria. Digestive problems, which comprise the number one health problem in North America, appear to be occurring with more frequency in recent years. One way to maintain digestive health is to maintain proper inflammation and intestinal flora.

"Improving muscle status" means improving the muscle health, for example promoting skeletal muscle protein synthesis, skeletal glucose absorbtion, improving lean tissue mass in therapeutic or non-therapeutic context, promoting muscle recovery generally after activity exercise, or improving muscle performance. The methods or uses may be therapeutic or non-therapeutic. The term "improving lean tissue mass status" should be understood to mean increasing lean tissue mass, or inhibiting or preventing the rate of lean tissue mass degradation.

"Promoting muscle recovery" means causing an increase in absorbtion of glucose in skeletal muscle compared with untreated skeletal muscle.

"Disease or condition characterised by lethargy or low energy levels" means any condition or disease characterised by a feeling or tiredness or low energy. Examples include allergies, asthma, anaemia, cancer and its treatments, chronic pain, heart disease, infection, depression, eating disorders, grief, sleeping disorders, thyroid problems, medication side effects, alcohol use, or drug use.

"Maintaining or restoring muscle health" means helping retain or restore mammalian muscle health resulting from damage incurred during exercise. By promoting glucose transport in skeletal muscle the peptides promote recovery from exercise, and relieve muscle soreness/pain and injury connected with exercise. They can also be used to decrease and prevent muscle cramping, and to allow a faster recovery from muscle cramping. Cramping can result from physical stress, mental stress, and or Repetitive Strain Injury stress. By promoting glucose transport the peptides help reduce Myopathy of the muscle, and help prevent Sarcopenia in mammals, promote recovery from injuries during exercise, and relieve muscle soreness/pain and injury connected with exercise. The invention also relates to a peptide or composition of the invention for use in maintaining or restoring muscle health in a mammal.

In this specification, the term "substantially all" as applied to a list of peptides should be understood to mean at least 60%, 70%, 80%, 90% or 95% of the peptides.

"Man-made" as applied to comestible products should be understood to mean made by a human being and not existing in nature.

### Brief Description of the Figures

**Figures 1 to 100****:** Effect of synthetic peptides of the invention on proliferation of Human Dermal Fibroblasts (HDF).
**Figure 101****:** Effect of synthetic peptide of the invention (SEQ ID 42) on elastin synthesis of Human Dermal Fibroblasts (HDF).
**Figure 102****:** Effect of synthetic peptide of the invention (SEQ ID 42) on collagen synthesis of Human Dermal Fibroblasts (HDF).
**Figure 103****:** Effect of synthetic peptide of the invention (SEQ ID 701)on elastin synthesis of Human Dermal Fibroblasts (HDF).
**Figure 104****:** Effect of synthetic peptide of the invention (SEQ ID 701) on collagen synthesis of Human Dermal Fibroblasts (HDF).
**Figure 105****:** Effect of synthetic peptide of the invention (SEQ ID 246) on elastin synthesis of Human Dermal Fibroblasts (HDF).
**Figure 106****:** Effect of synthetic peptide of the invention (SEQ ID 246) on collagen synthesis of Human Dermal Fibroblasts (HDF).
**Figure 107****:** Effect of synthetic peptide of the invention (SEQ ID 284) on elastin synthesis of Human Dermal Fibroblasts (HDF).
**Figure 108****:** Effect of synthetic peptide of the invention (SEQ ID 245) on elastin synthesis of Human Dermal Fibroblasts (HDF).
**Figure 109****:** Effect of synthetic peptide of the invention (SEQ ID 245) on collagen synthesis of Human Dermal Fibroblasts (HDF).
**Figure 110****.** shows the integrity controls and viability controls for the assay system.

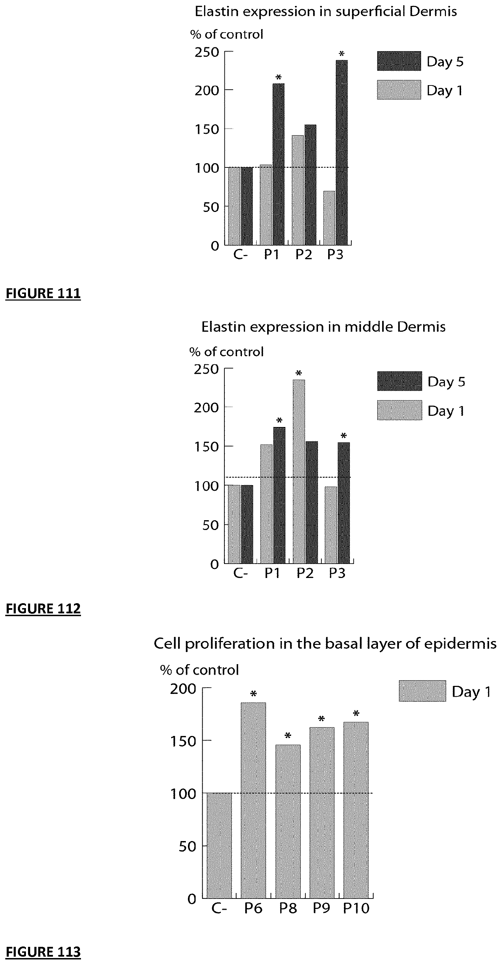

**Figure 111****.** % of elastin expression in superficial dermis compared to control (water or DMSO) for peptides PI, P2 and P3
   * shows significant increases of elastin expression in superficial AND middle dermis.
**Figure 112****.** % of elastin expression in middle dermis compared to control (water or DMSO) for peptides PI, P2 and P3.
   * shows significant increases of elastin expression in superficial AND middle dermis.
**Figure 113****.** % of cell proliferation in the basal layer of epidermis compared to control (water or DMSO) for peptides P6 and P8, and peptide compositions P9 and P10
   * shows significant increases.

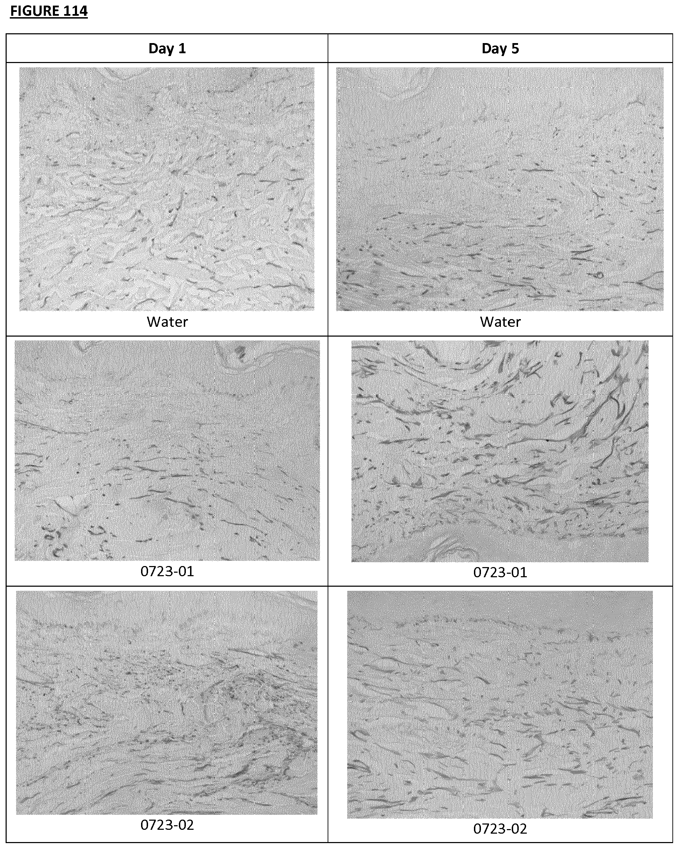

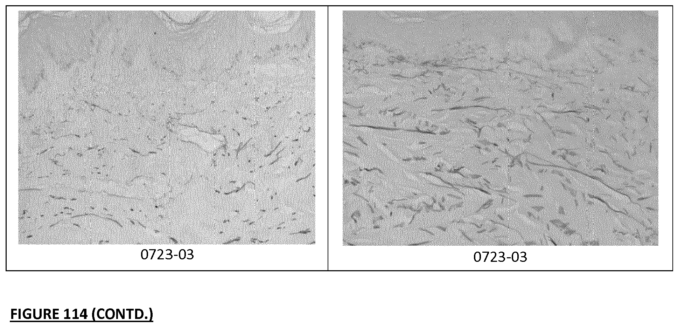

**Figure 114****.** Histological analysis of the elastic fibers (+catechin, x200)

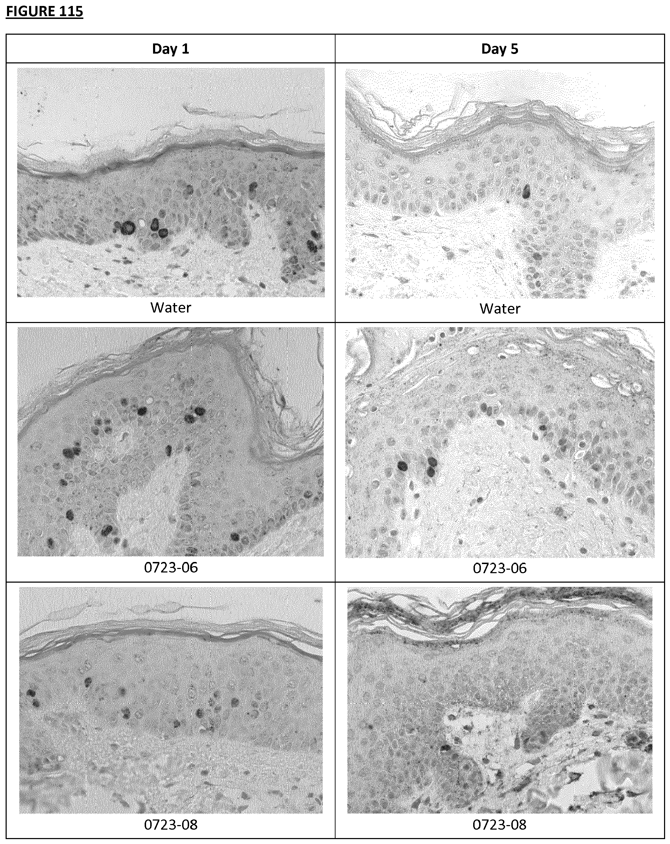

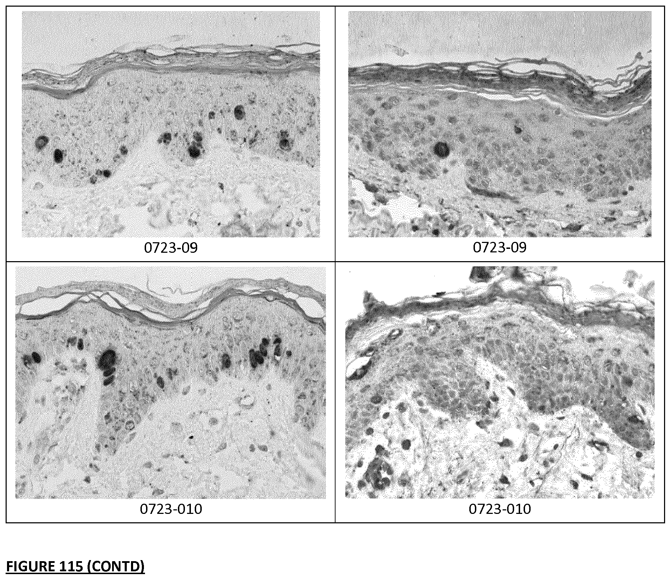

**Figure 115****.** Immunohistochemical evaluation of the mitotic index (Ki67, x400)

### Detailed Description of the Invention

### EXAMPLE 1 - CELL PROLIFERATION ASSAY

BrDu is incorporated into newly synthesised DNA strands of actively proliferating cells. Following partial denaturation of double stranded DNA, Brdu is detected immunochemically allowing the assessment of the population of cells which are synthesizing DNA.

Human Dermal Fibroblasts (HDF - Sigma 10605a) were seeded in a 96 well plate at 10,000 cells per well in DMEM containing 10% fetal calf serum (FCS), 1% Pen/strep, 1% L-glutamine and allowed to adhere for 24h.

Following the initial 24h incubation the cells were incubated with 5 µg/ml, 0.5 µg/ml or 0.05µg/ml synthetic peptide for 24h respectively.

After 18 h incubation with synthetic peptides 20 µl BrDu reagent was added to each well.

At 24h incubation the cell were fixed and the amount of 2-DG6P was measured using the BrdU Cell Proliferation Assay, all steps were carried out according to the manufacturer's instructions.

Results were calculated as a percentage of the untreated control. An increase in optical density reading indicates greatER incorporation of BrDu and increase cell proliferation.

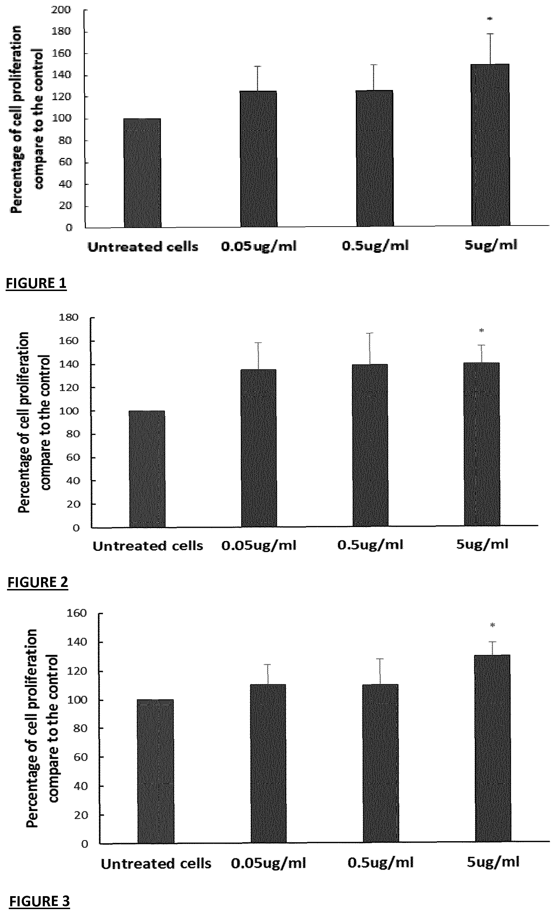

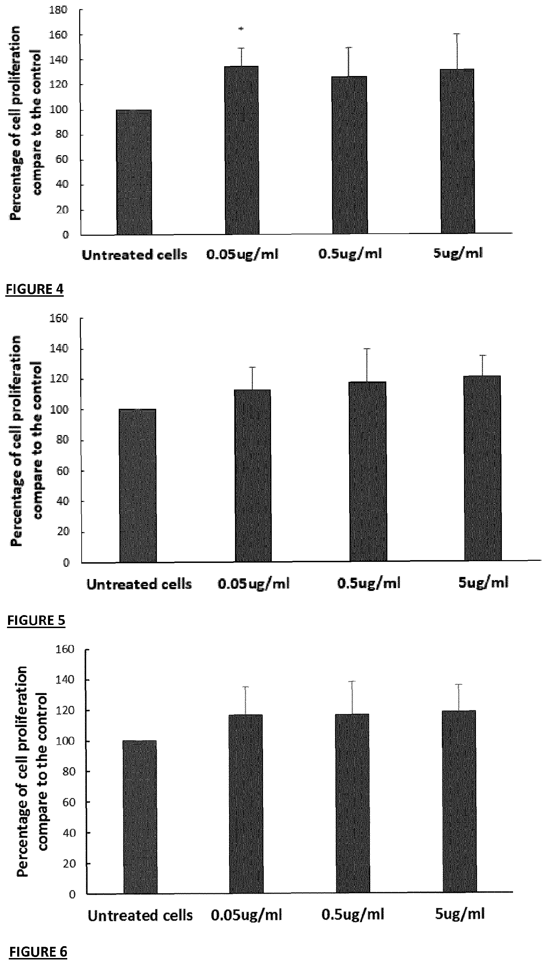

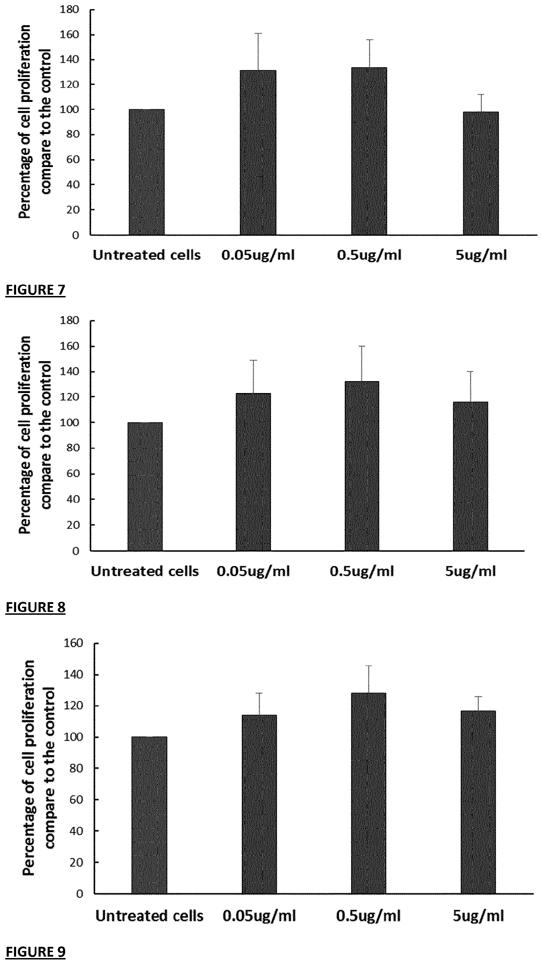

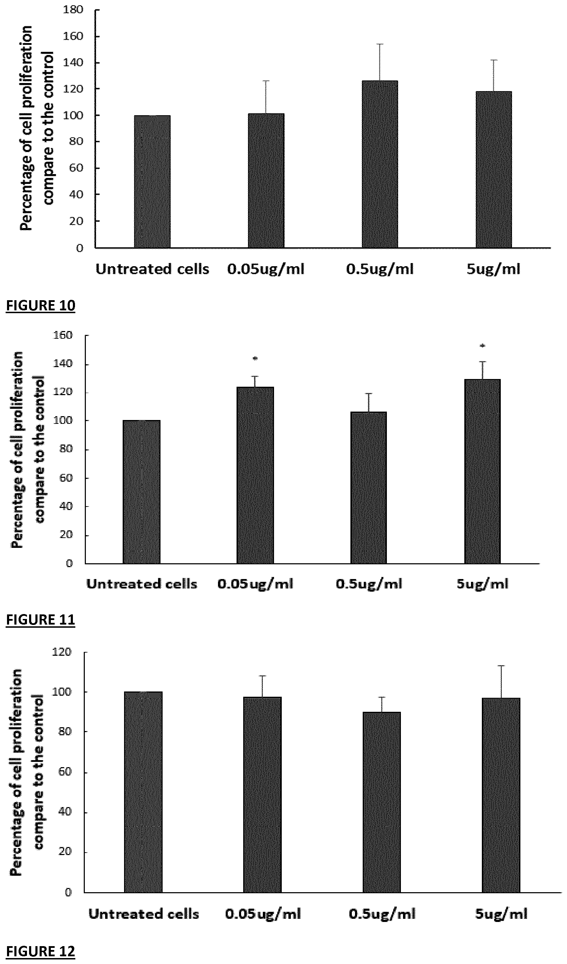

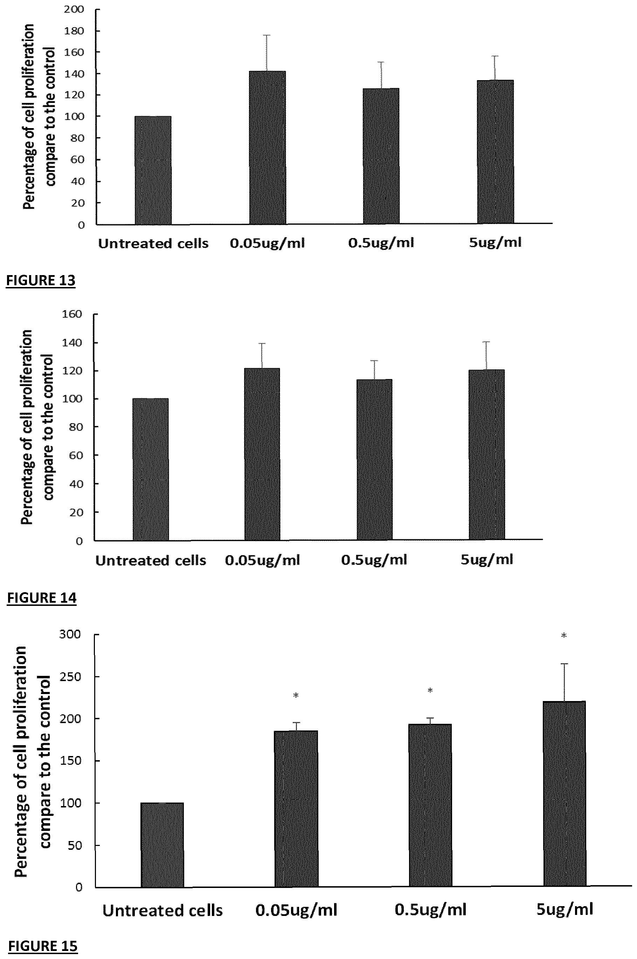

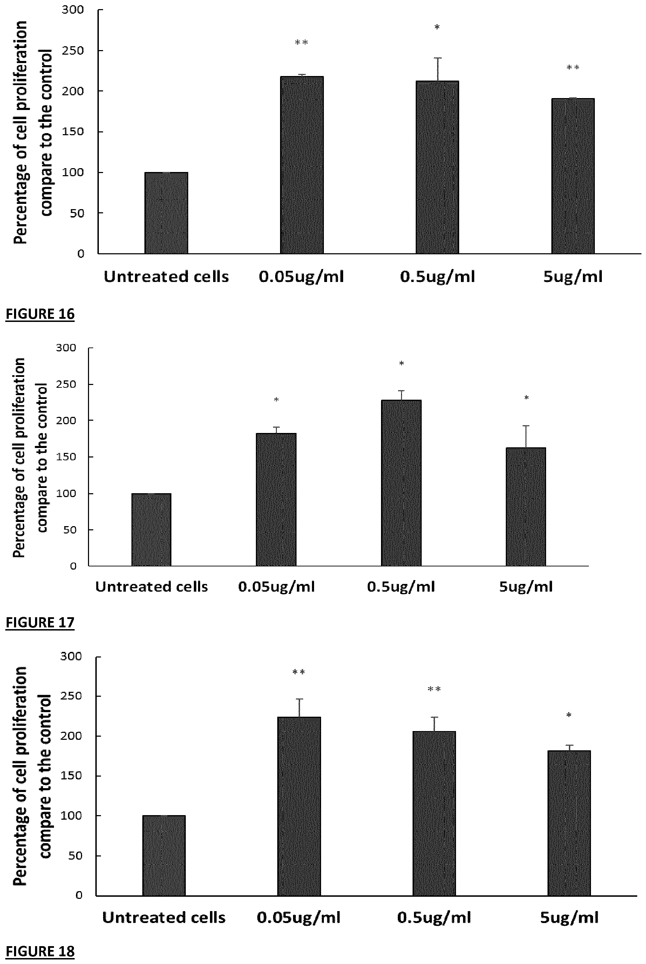

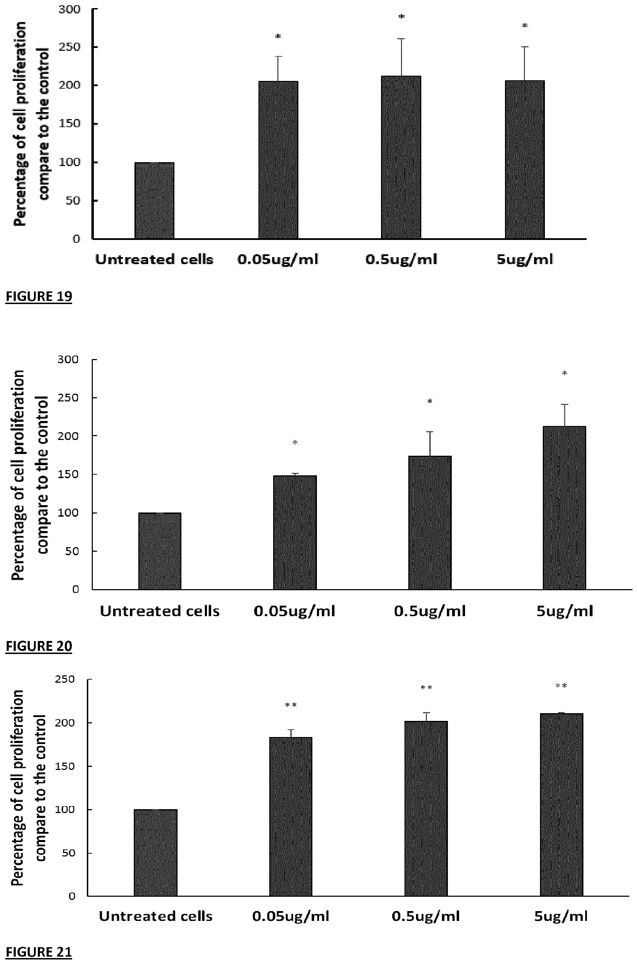

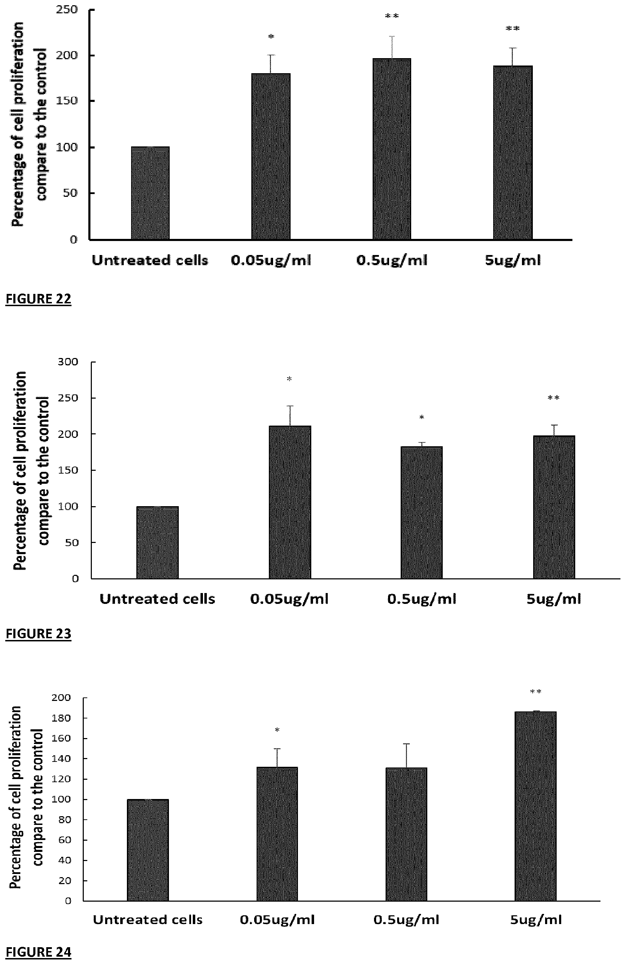

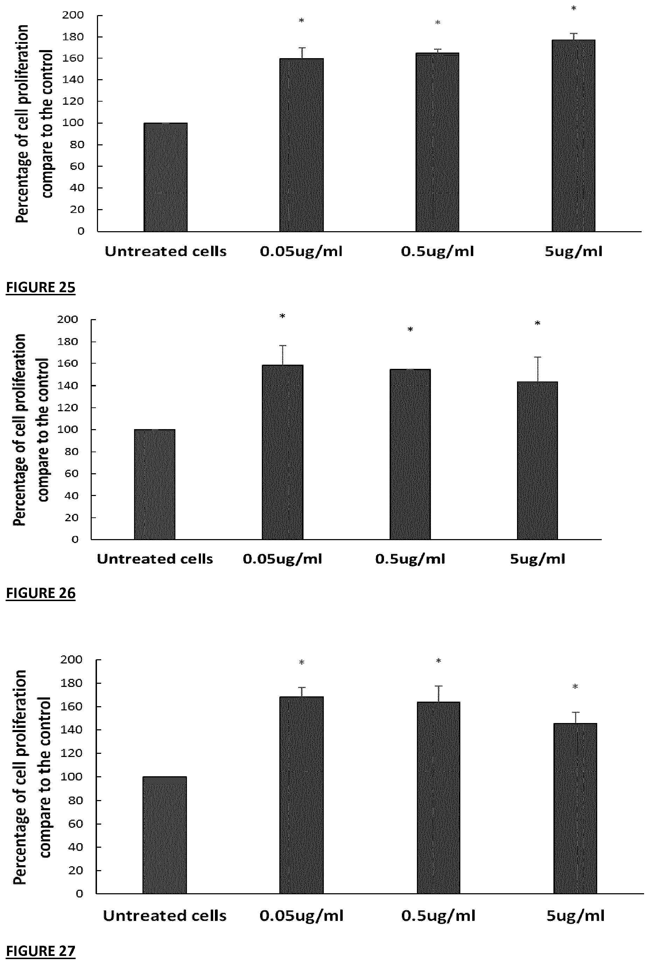

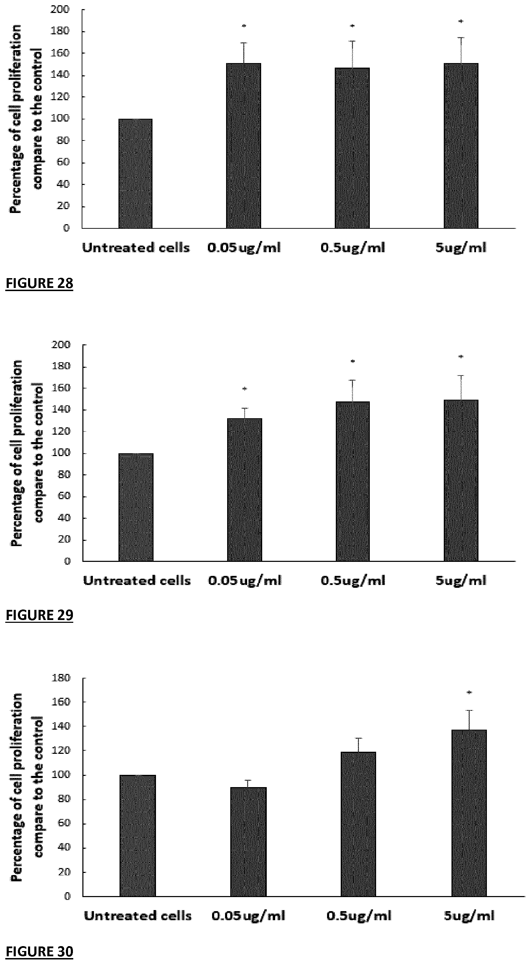

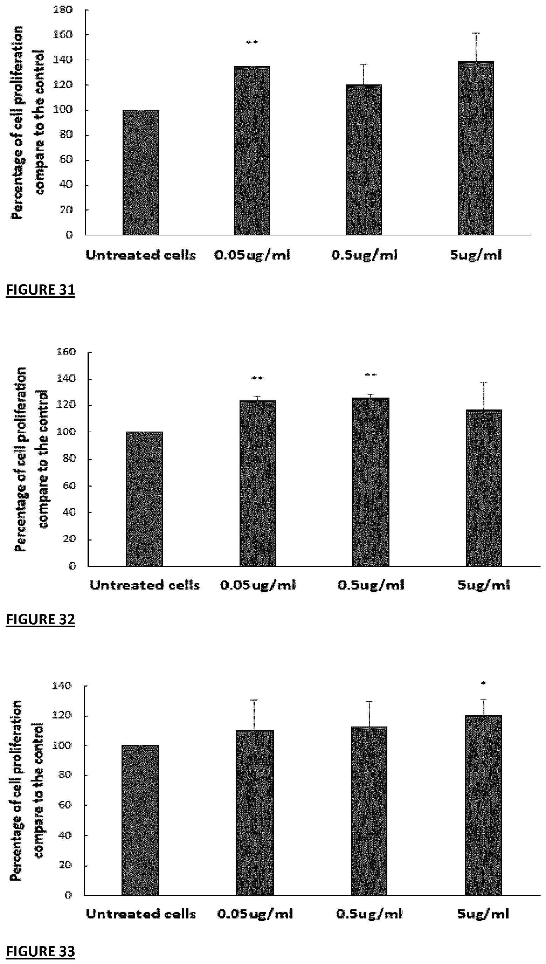

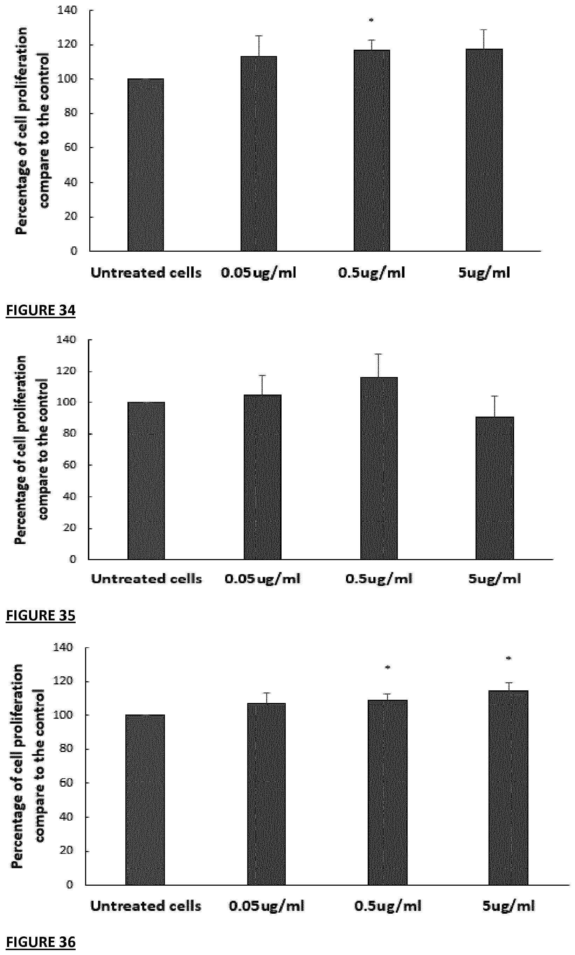

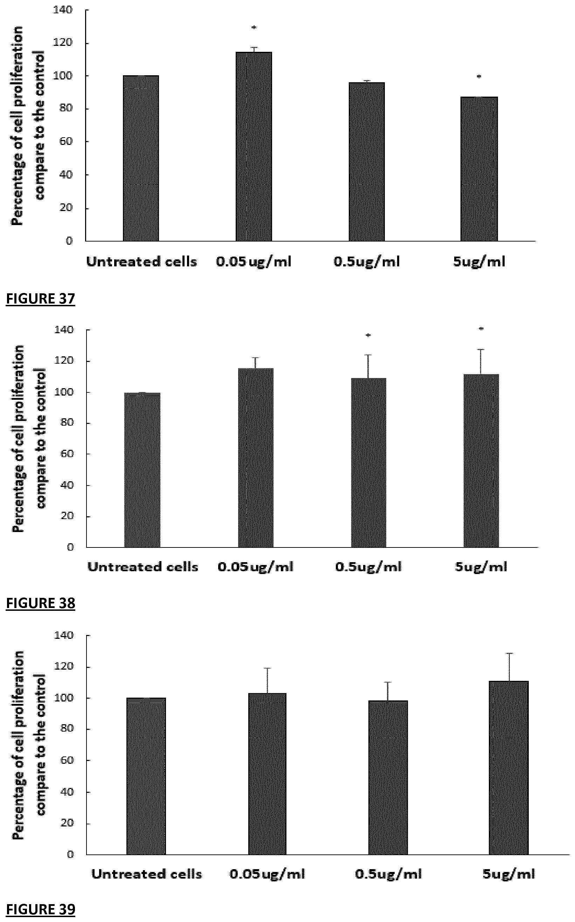

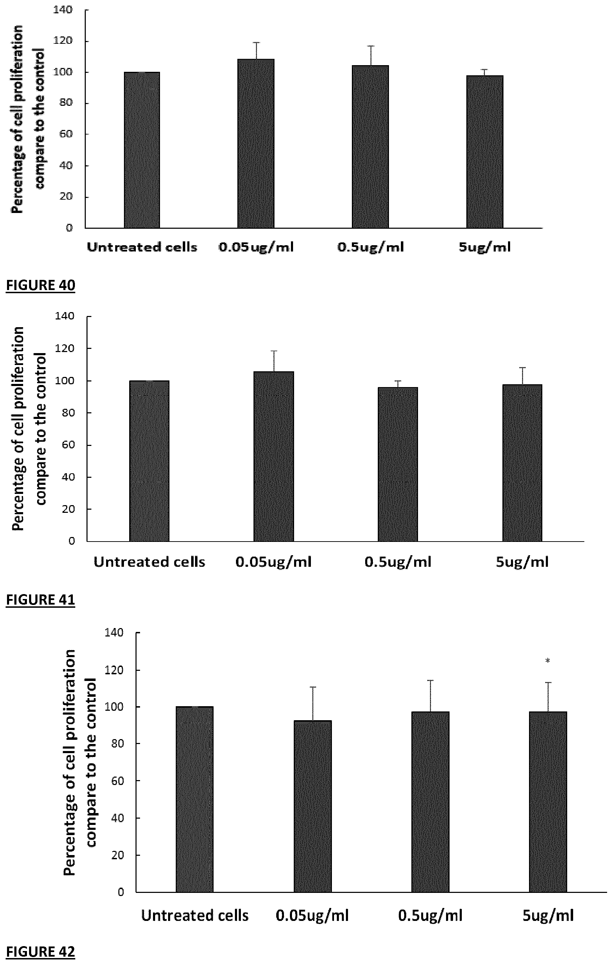

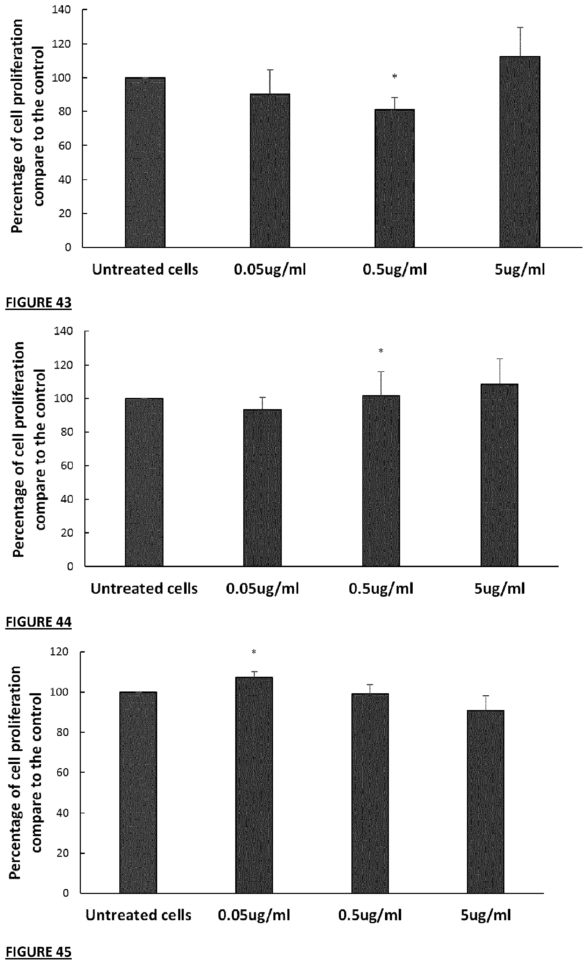

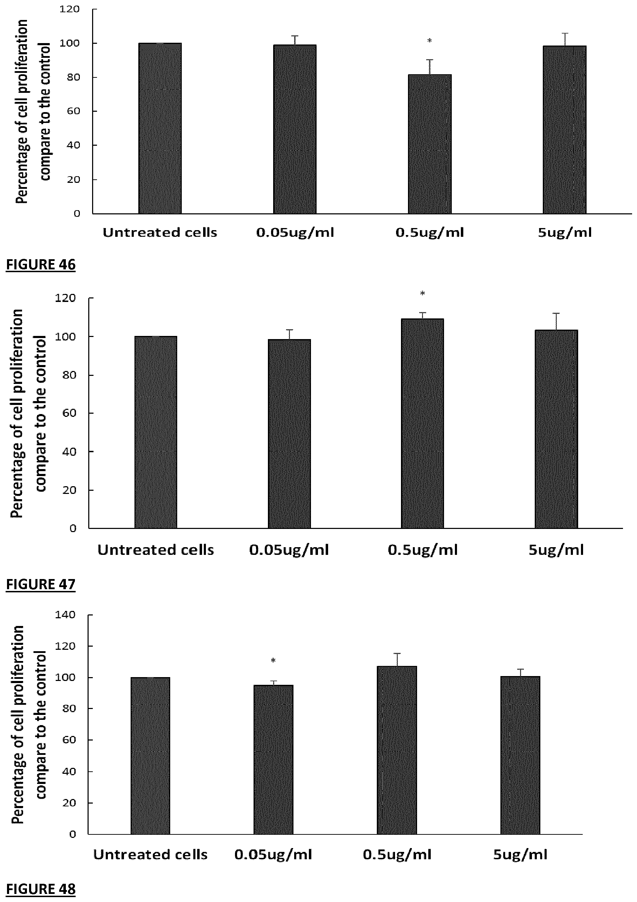

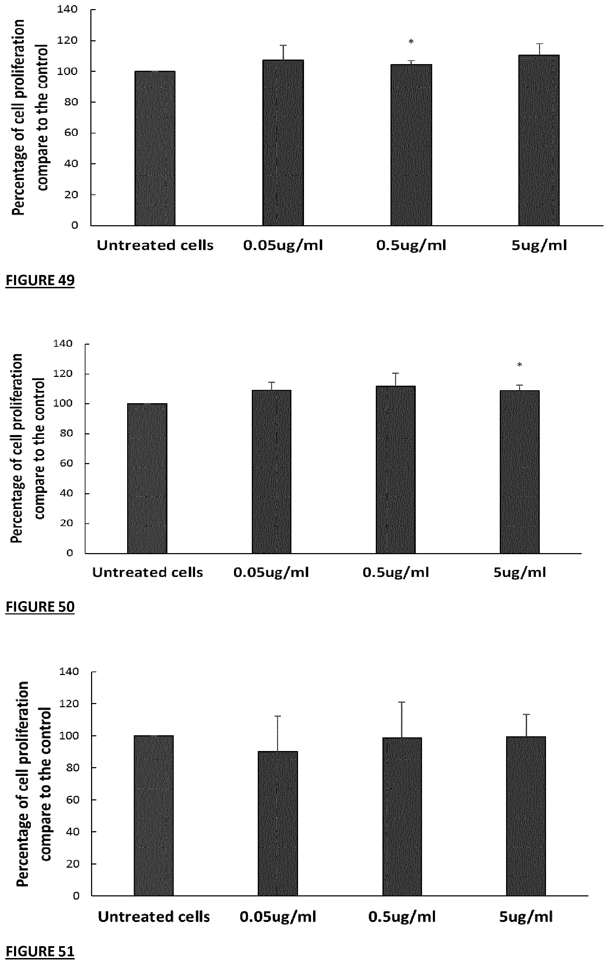

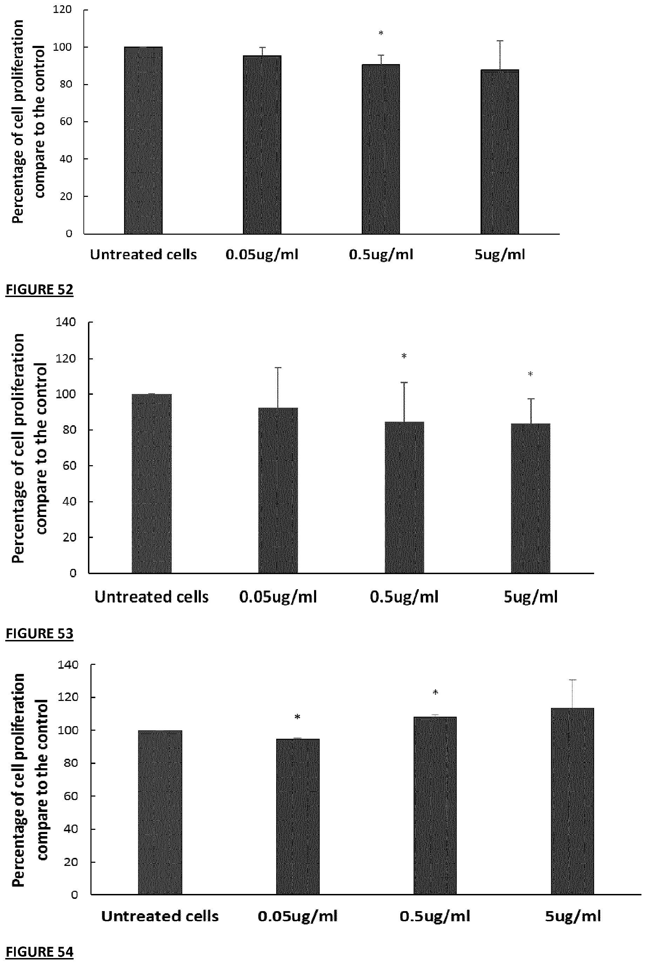

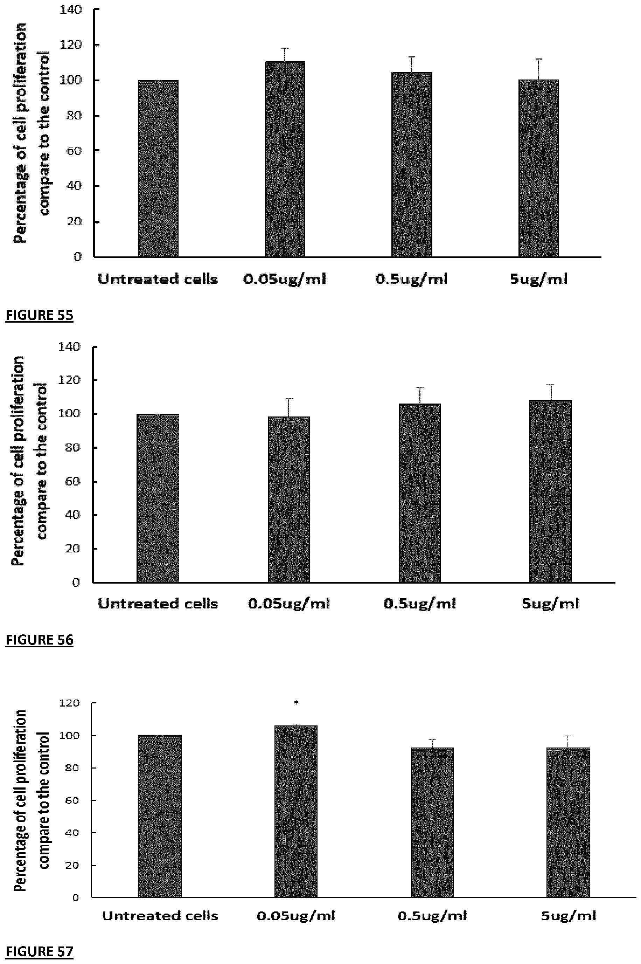

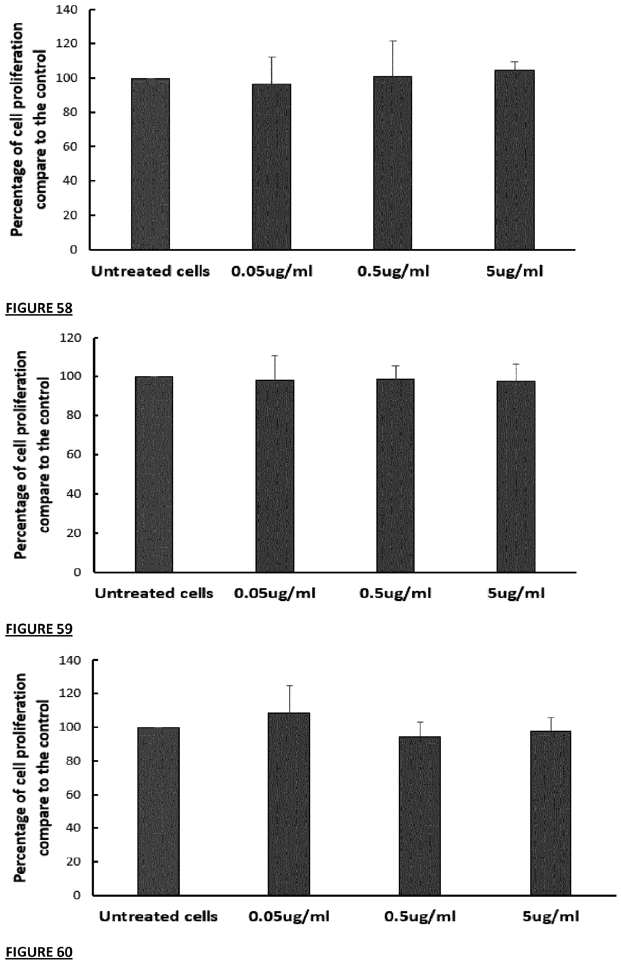

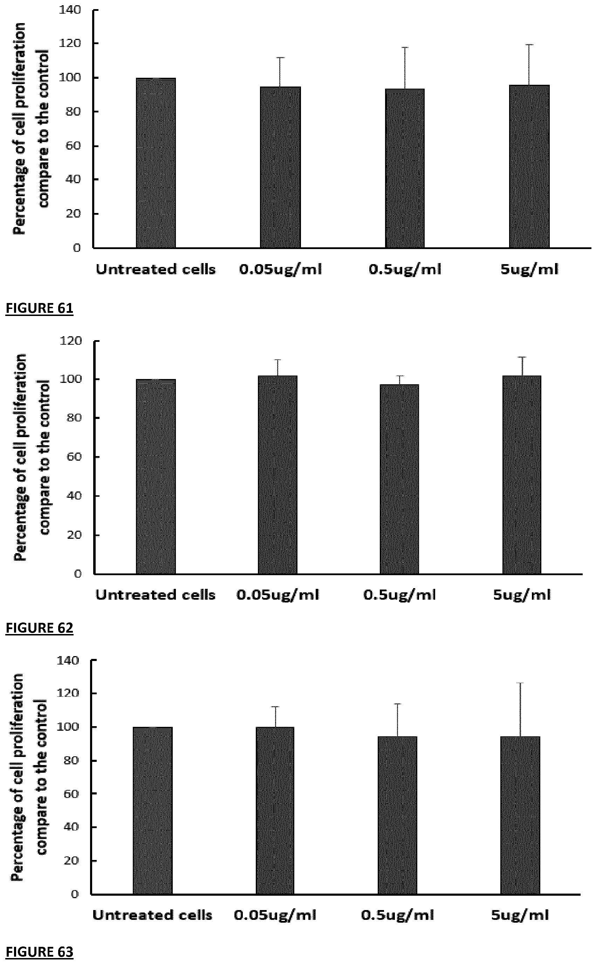

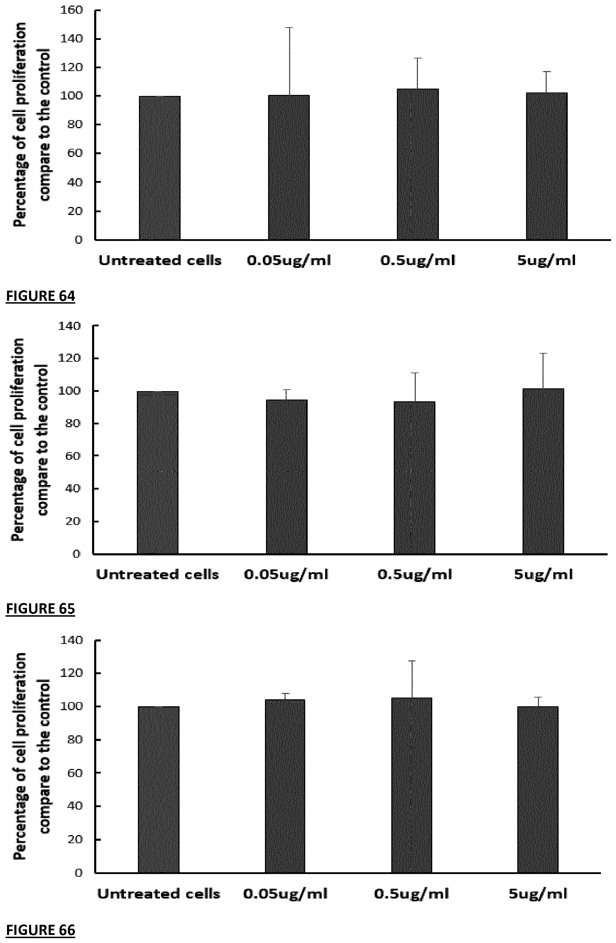

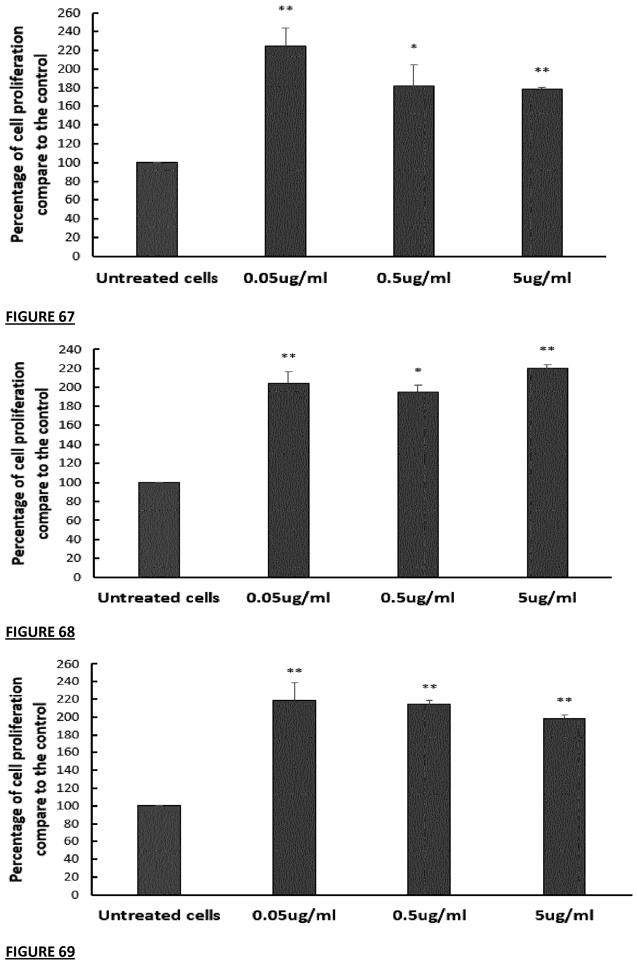

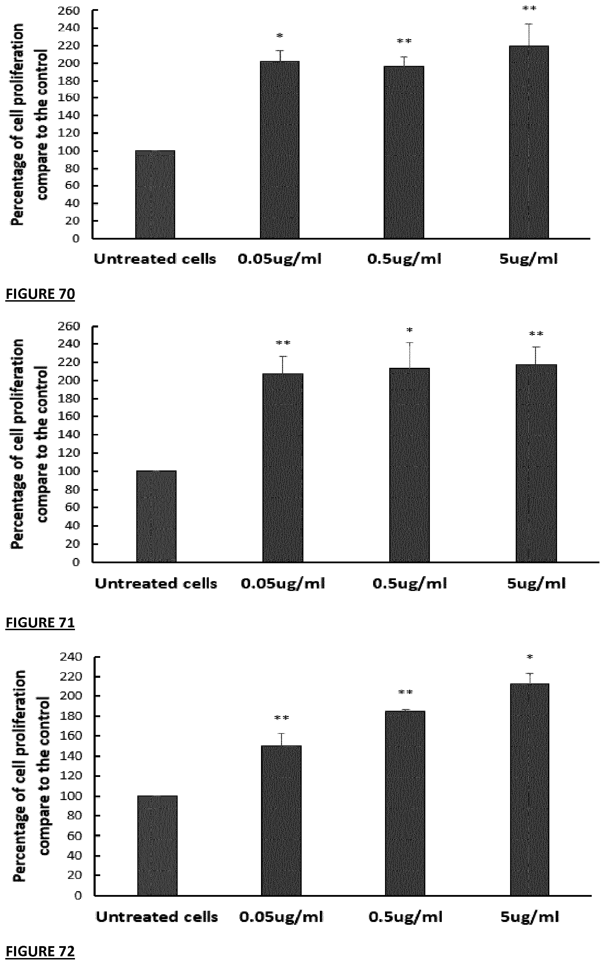

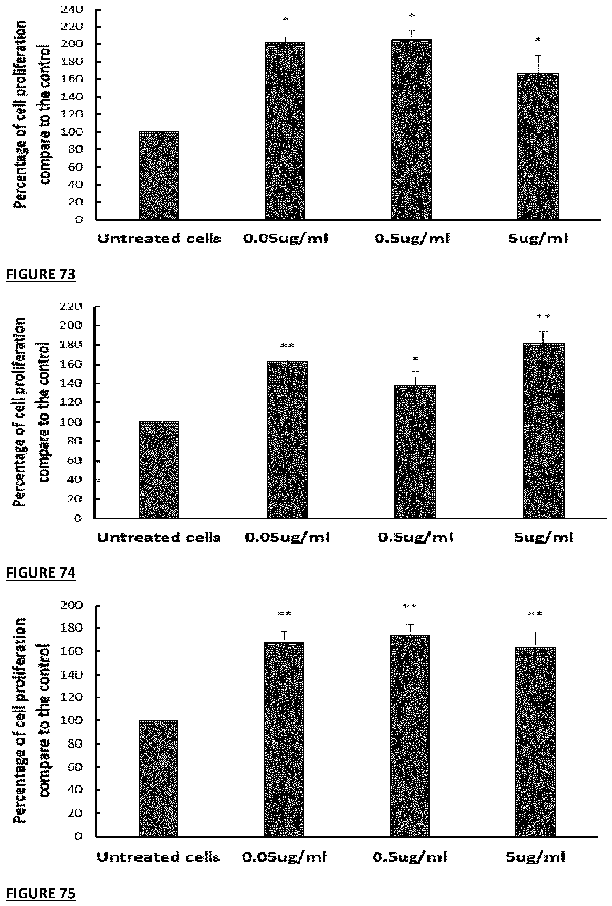

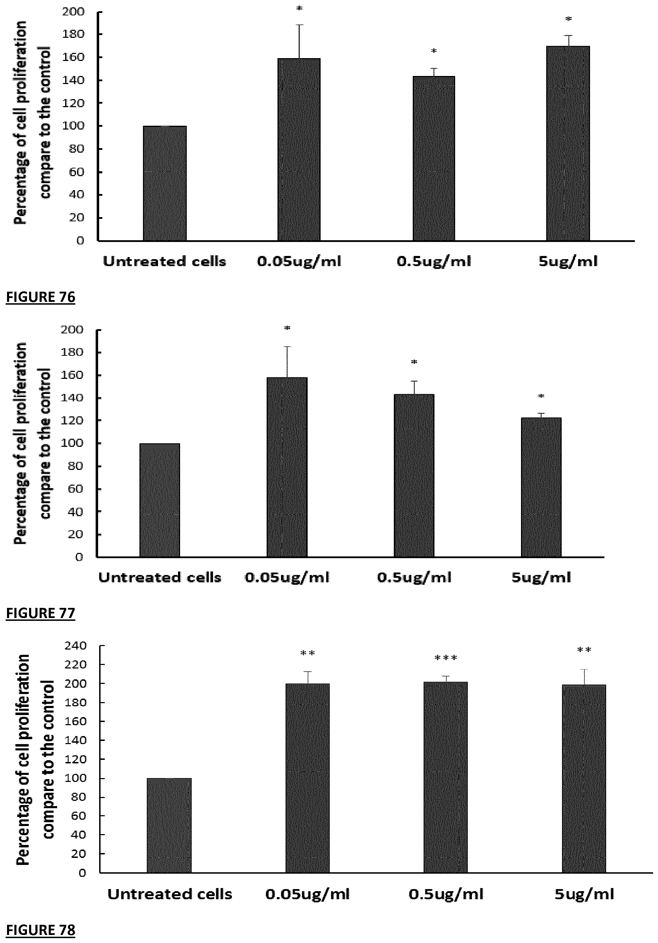

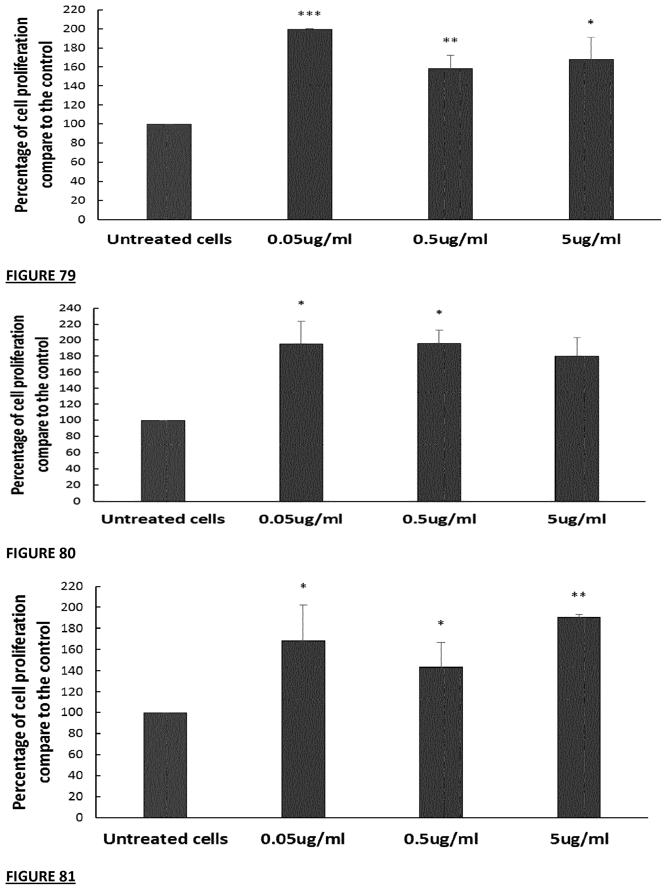

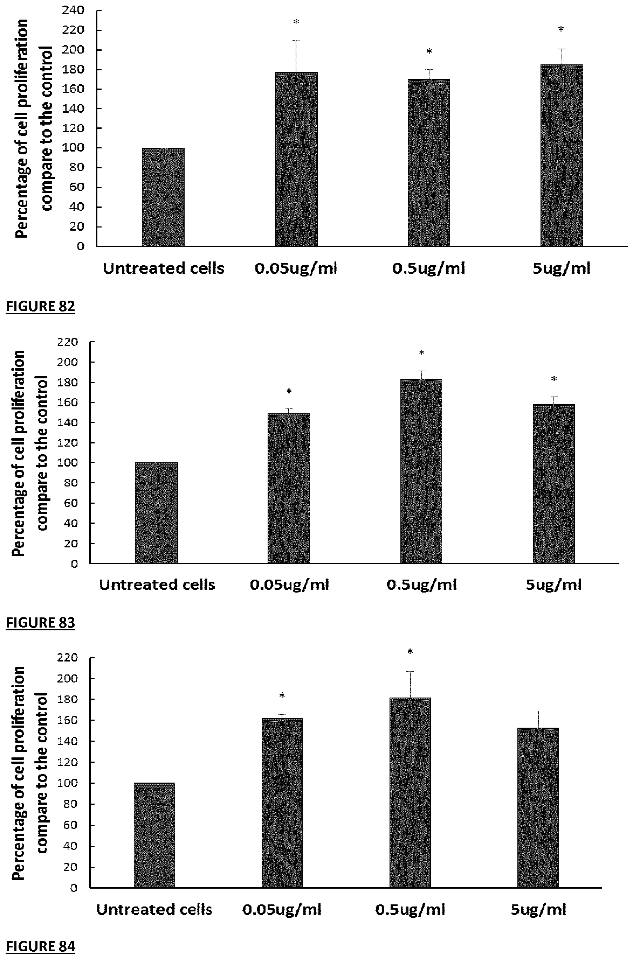

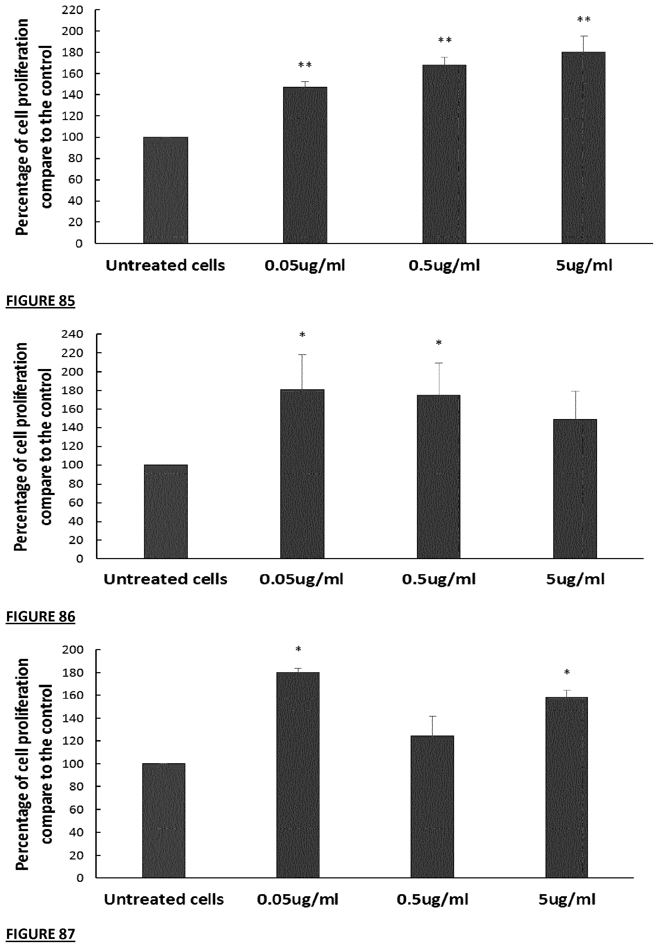

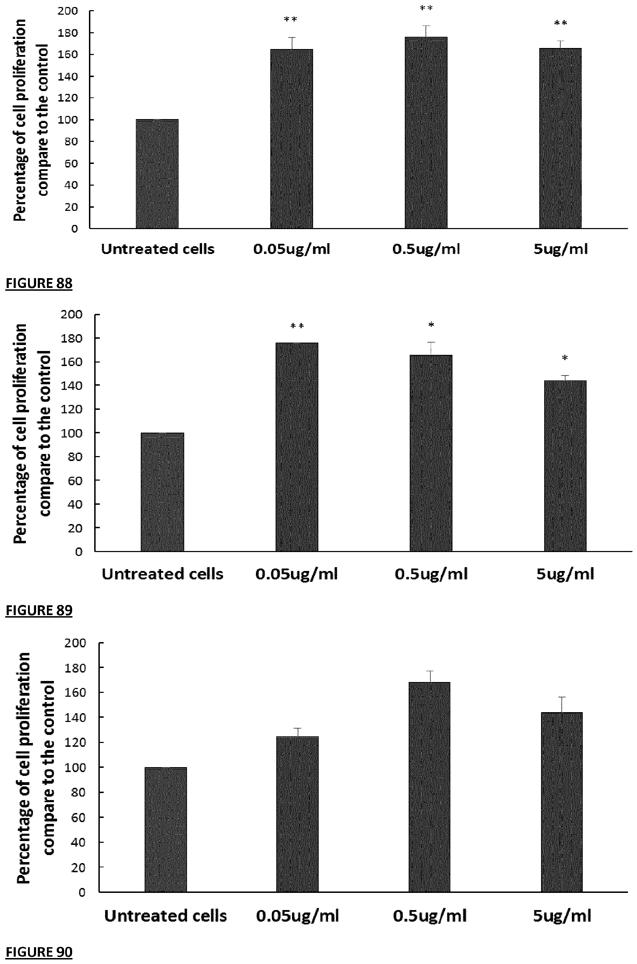

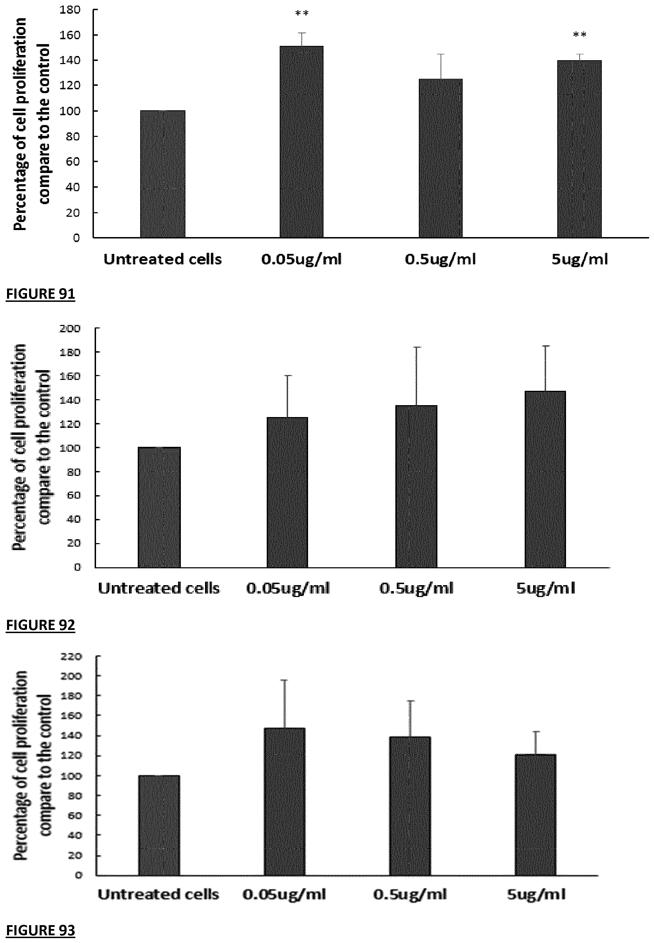

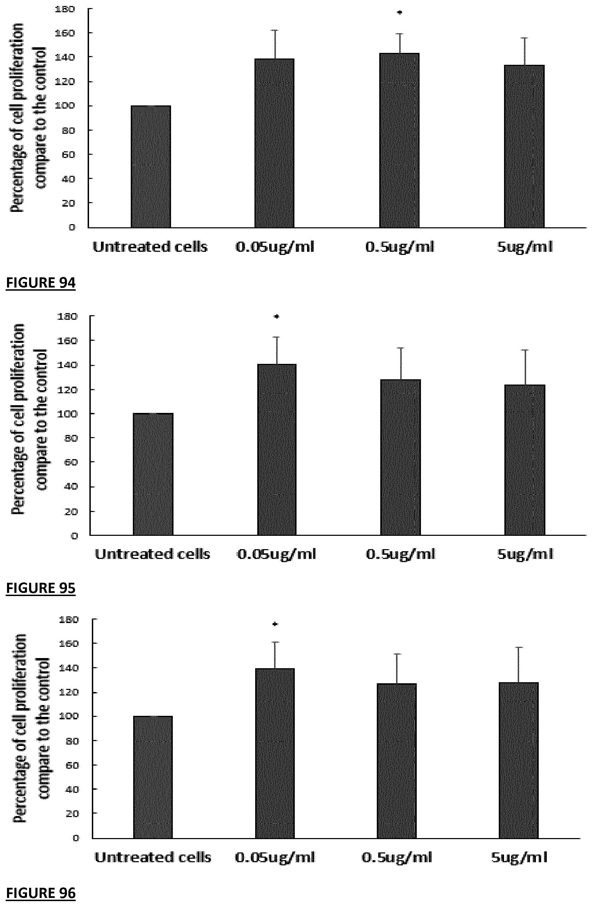

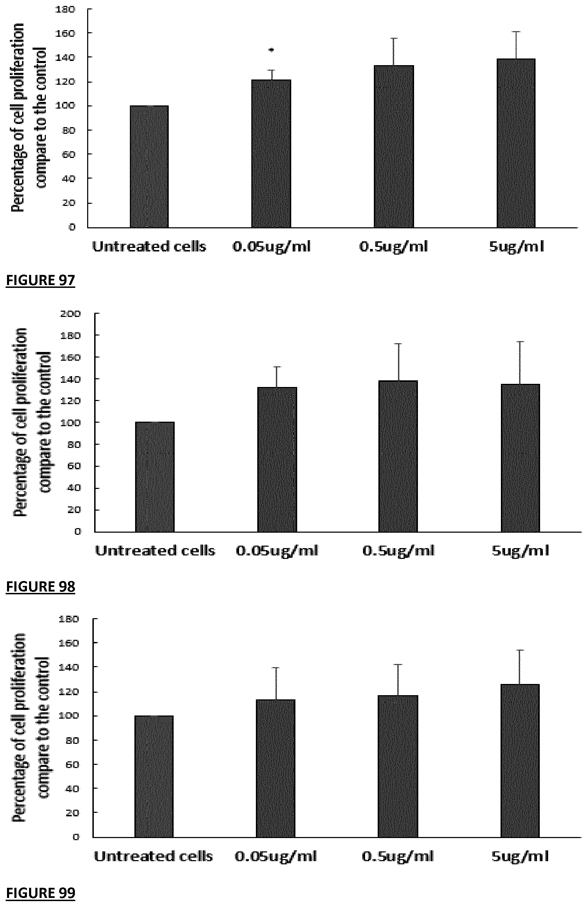

The results are shown in Figures 1-100 and Table 1 below.

**Table 1**

| **FIGURE NO** | **SEQ ID** | **INCREASE IN PROLIFERATION** | **FIGURE NO** | **SEQ ID** | **INCREASE IN PROLIFERATION** |
|---|---|---|---|---|---|
| 1 | 121 | 48% | 29 | 98 | 49% |
| 2 | 105 | 40% | 30 | 454 | 38% |
| 3 | 249 | 30% | 31 | 85 | 35% |
| 4 | 226 | 30% | 32 | 453 | 25% |
| 5 | 84 | 20% | 33 | 158 | 21% |
| 6 | 330 | 18% | 34 | 464 | 18% |
| 7 | 181 | 33% | 35 | 73 | 16% |
| 8 | 83 | 32% | 36 | 359 | 15% |
| 9 | 247 | 28% | 37 | 124 | 15% |
| 10 | 97 | 26% | 38 | 112 | 15% |
| 11 | 74 | 29% | 39 | 733 | |
| 12 | | | 40 | 728 | |
| 13 | 168 | | 41 | 727 | |
| 14 | 151 | | 42 | 730 | |
| 15 | 470 | 119% | 43 | 731 | |
| 16 | 257 | 118% | 44 | 148 | |
| 17 | 256 | 117% | 45 | 343 | |
| 18 | 457 | 114% | 46 | 345 | |
| 19 | 499 | 113% | 47 | 484 | |
| 20 | 253 | 112% | 48 | 729 | |
| 21 | 222 | 110% | 49 | 456 | |
| 22 | 272 | 97% | 50 | 494 | |
| 23 | 252 | 111% | 51 | 723 | |
| 24 | 248 | 86% | 52 | 722 | |
| 25 | 472 | 77% | 53 | 498 | |
| 26 | 365 | 58% | 54 | 475 | 13% |
| 27 | 502 | 68% | 55 | 718 | |
| 28 | 496 | 51% | 56 | 337 | 8% |
| 57 | 500 | 6% | 79 | 463 | 100% |
| 58 | 717 | | 80 | 465 | 96% |
| 59 | 297 | | 81 | 467 | 90% |
| 60 | 340 | | 82 | 461 | 85% |
| 61 | 719 | | 83 | 341 | 83% |
| 62 | 726 | | 84 | 468 | 82% |
| 63 | 725 | | 85 | 285 | 81% |
| 64 | 724 | | 86 | 496 | 81% |
| 65 | 720 | | 87 | 146 | 80% |
| 66 | 721 | | 88 | 478 | 76% |
| 67 | 503 | 125% | 89 | 452 | 76% |
| 68 | 474 | 121% | 90 | 495 | 68% |
| 69 | 504 | 119% | 91 | 403 | 51% |
| 70 | 114 | 119% | 92 | 455 | 47% |
| 71 | 505 | 118% | 93 | 270 | 47% |
| 72 | 482 | 113% | 94 | 501 | 43% |
| 73 | 479 | 106% | 95 | 473 | 41% |
| 74 | 477 | 81% | 96 | | 39% |
| 75 | 410 | 73% | 97 | 471 | 38% |
| 76 | 475 | 69% | 98 | 460 | 38% |
| 77 | 497 | 58% | 99 | 93 | 26% |
| 78 | 480 | 102% | 100 | 462 | 15% |

### EXAMPLE 2 - COLLAGEN PRODUCTION ASSAY

Hydroxyproline in tissue preparations is a direct measure of the amount of collagen present. FIRELISA Human Hydroxyproline ELISA kit assay is designed to measure hydroxyproline in tissue or peptide compositions..

Human Dermal Fibroblasts (HDF Sigma 10605a) were seeded in 24 well plates at 50,000 cells per well in DMEM containing 10% fetal calf serum (FCS), 1% Pen/strep, 1% L-glutamine and allowed to adhere for 24h.

Following the initial 24h incubation the cells were incubated with 5 µg/ml, 1 µg/ml or 0.1 µg/ml synthetic peptide for 96h respectively.

After treatment the cells were lysed using 4 freeze thaw cycles in liquid nitrogen. The lysed cells were centrifuged and 50 µl/ml of each supernatant was assayed using the FIRELISA Human Hydroxyproline ELISA kit. All steps were carried out according to the manufacturer's instructions.

Results were calculated as a percentage of the untreated control. An increase in optical density reading indicates an increase collagen content.

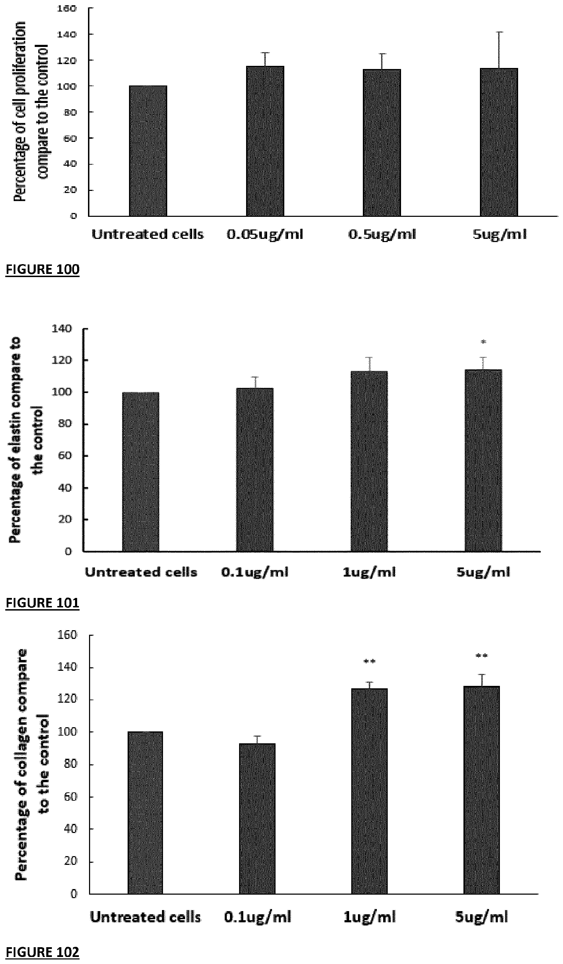

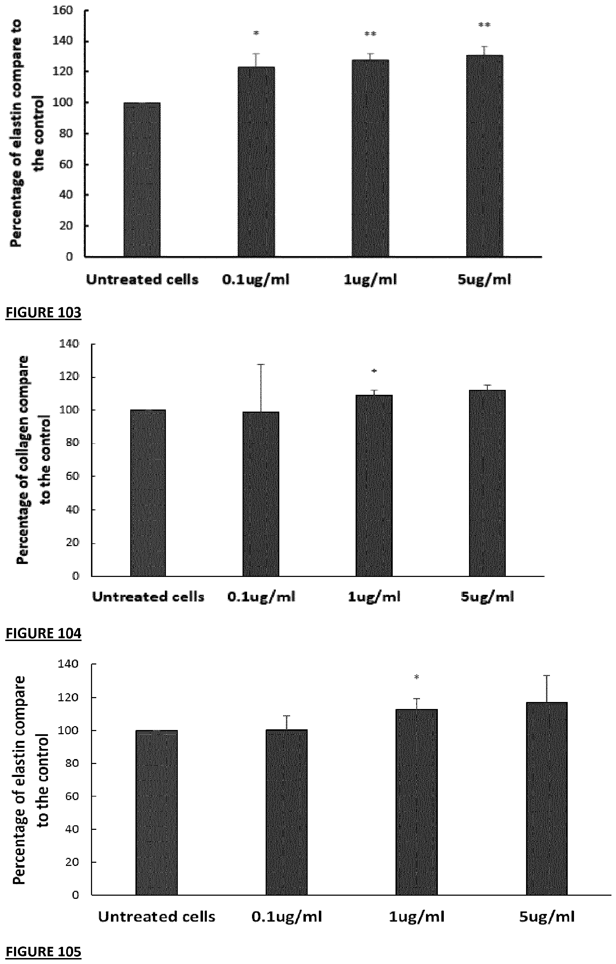

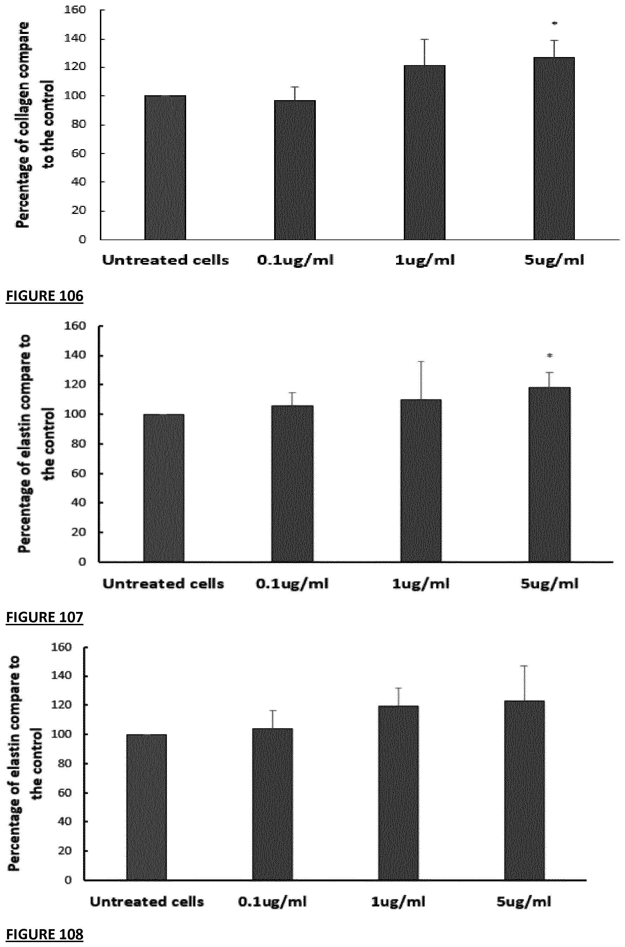

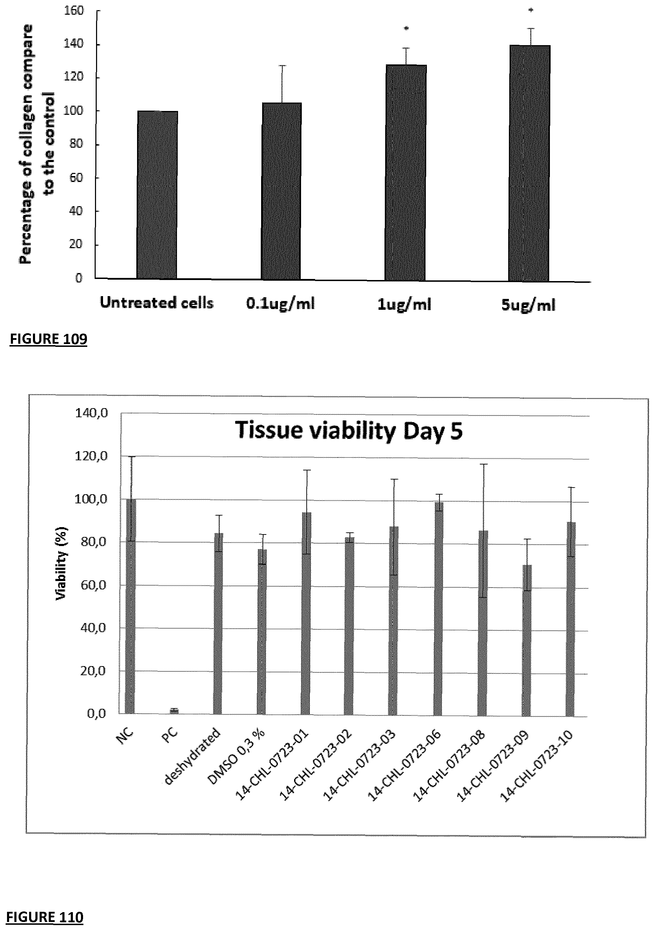

The results are shown in Figures 102, 104, 106 and 109

### EXAMPLE 3 - ELASTIN PRODUCTION ASSAY

Elastin is a highly elastic protein in connective tissue and allows many tissues in the body to resume their shape after stretching or contracting. FIRELISA Human Elastin ELISA kit assay is designed to measure Elastin in tissue or protein/peptide compositions.

Human Dermal Fibroblasts (HDF) were seeded in 24 well plates at 50,000 cells per well in DMEM containing 10% fetal calf serum (FCS), 1% Pen/strep, 1% L-glutamine and allowed to adhere for 24h.

Following the initial 24h incubation the cells were incubated with 5 µg/ml, 1 µg/ml or 0.1 µg/ml synthetic peptide for 96h respectively.

After treatment the cells were lysed using 4 freeze thaw cycles in liquid nitrogen. The lysed cells were centrifuged and 50 µl/ml of each supernatant was assayed using the FIRELISA Human Elastin ELISA kit. All steps were carried out according to the manufacturer's instructions.

Results were calculated as a percentage of the untreated control. An increase in optical density reading indicates an increase collagen content.

The results are shown in Figures 101, 103, 105, 107, 108 and 109.

### EXAMPLE 4 - ELASTIN AND CELL PROLIFERATION ASSAYS

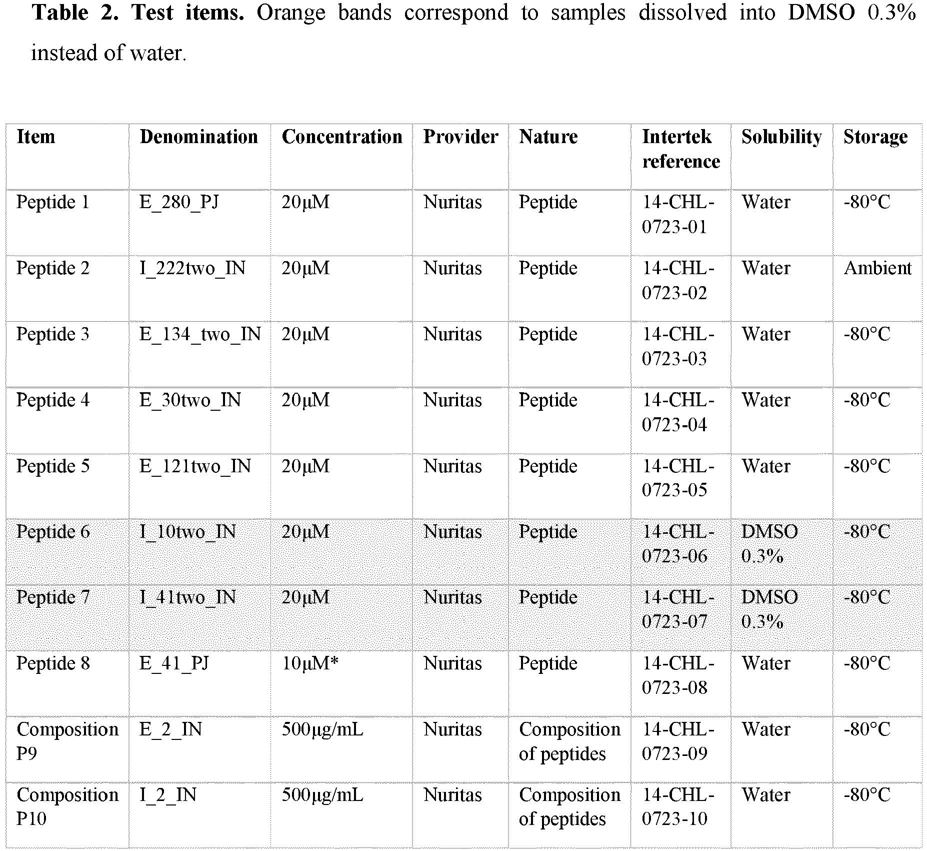

### Equipment

Incubator, Flow Laminar Chamber, Sterile Polished Plastic Rod, Pipettor, Maintenance medium, Plate 6 well, Plate 24 well.

### Reagents

MTT, PBS, SDS, Formaldehyde, Xylene, Ethanol absolute, Dulbecco's phosphate-buffered saline (DPBS), Metal Enhanced DAB substrate kit, ABC peroxidase staining kit, Citric acid, Sodium hydroxide 2N, Hydrogen peroxide 30%, Anti-Filaggrin, Anti-rabbit IgG-Biotin, Tween 20.

### Test system

Nature: Human skin tissue 5mm diameter
Batch number: EXP004050B009 and EXP004050B011
Provider: Laboratoire Biopredic International - 8-18 rue Jean Pecker - 35000 Rennes - France. Tel: +33 (0)2.99.14.36.14 - Fax: +33 (0)2.99.54.44.72.
Certificates of analysis are present in Annex 1.
Two batches are used for the assay. Batch EXP004050B005 is used for experiment day 1,
and Batch EXP004050B006 is used for experiment day 5.

### Maintenance medium

Maintenance Medium: Batch n°: MIL 218C
Provider: Laboratoire Biopredic International - 8-18 rue Jean Pecker - 35000 Rennes - France.

### Peptides Tested

P1: SEQ ID NO: 283
P2: SEQ ID NO: 246
P3: SEQ ID NO: 284
P4: RPYYSNAPQEIF
P5: VLLEQQEQEPQH
P6: SEQ ID NO: 245
P7: QQYGIAASPFLQSAA
P8: SEQ ID NO: 42

### Compositons tested

P9 (14-CHL-0723-09) is the Pea composition (SEQ ID Numbers:50, 85, 74, 140, 82, 136, 189, 77, 169, 149, 171, 178, 143, 127, 190, 141, 147, 133, 186, 125, 122, 119, 87, 90, 86, 89, 138, 129, 123, 120, 117, 113, 110, 121, 105, 98, 55, 161, 19, 317, 135, 130, 146, 177, 160, 170, 188, 83, 78, 36, 96, 159, 26, 330, 168, 148, 184, 151, 151, 165, 114, 284)
P10 (14-CHL-0723-010) is the Rice composition (SEQ ID Numbers: 245, 246, 263, 250, 257, 259, 276, 255, 251, 264, 256, 266, 274, 270, 269, 356, 245, 380, 262, 258, 356, 218, 252, 358, 271, 253, 344, 275, 272, 226, 224, 220, 248, 261, 265, 373, 375, 247, 249, 363, 273, 343, 273, 362)

### Application method

Skin explants were prepared from abdominal plastic surgery. Some explants were delipidated with alcohol to obtain a dehydrated skin.

These explants were maintained in maintenance medium supplied by the provider Bioprédic International for 5 days. Test items are applied twice per day with 5µL per explant.

At the end of the test, viabilities controls are realized with the MTT on two explants, the third explant is fixed in the formaldehyde 4% for histology and cell staining.

For each time of analysis (D1 and D5), histologies on delipidated explants, treated explants with test items, the DMSO 0.3% control and water control, are performed.

After receipt in the laboratory, each skin explant in the maintenance medium is delipidated with 5µL alcohol during 3 hours.

After 3 hours, all skin explants are treated two per day with test items, and they are incubated at 37°C +/- 2°C, 5% CO2 for 1 day or 5 days.

Integrity of the system is realized at day 1 and day 5 with a viability control with MTT.

### Immunostaining

Histology is realized by the laboratory *Gredeco* and the immunostaining to elastin and Ki67 are realized by the same laboratory. Immunostaining to filaggrin is realized by the laboratory *Intertek.*

The detection of elastin (rabbit monoclonal antibody, clone P15502, LSBio) is performed using an immunoperoxidase technique two layers (ABC kit, Vector Laboratories) and revealed by AEC (3-amino-9 -éthylcarbazole). The immunohistochemical staining intensity in the elastic fibers is evaluated using a semi-quantitative histological score.

Epithelial proliferation was analyzed by immunohistochemistry using anti-Ki67 antibody. Immunodetection was performed using an indirect immunoperoxidase technique three layers, amplified (DAKO kit) and revealed by AEC (3-Amino-9-ethylcarbazole). Counting the number of labeled cells (keratinocytes of the basal layer of the epidermis) is performed and provides the total number of basal cells to calculate the % of labeled cells.

The specific staining of filaggrin is performed with an immunoperoxidase staining (ABC kit, Fisher). The intensity of immunohistochemical marker in the epidermis is evaluated relative to the negative control of the solvent (Water or DMSO 0.3%).

### C: Results

### Viability control

The integrity control and the viability control are present in Figure 1. These controls do allow to validate the assay system. The viability is > 50% for test items, and they do not show a cytotoxicity according to the test.

### Immunostaining

### Elastin Expression

The elastic fibers of the dermis were revealed by staining with the catechin and morphometrically quantified by analysis by computer-assisted image. The percentage area taken up by elastic fibers in the dermis was calculated in the dermis and the average superficial dermis. Results are presents in Table 1, Figure 2 and Figure 3.

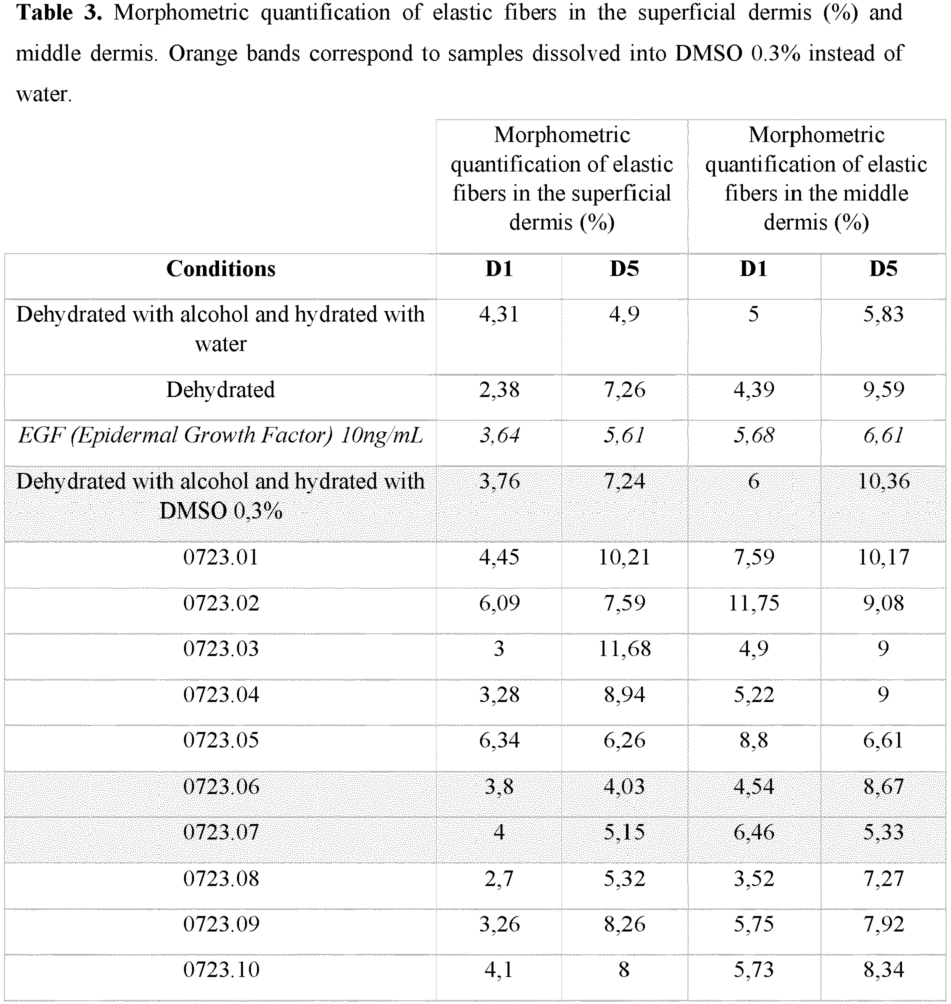

Under the experimental conditions of the study, 0723-1 and 0723-3 samples show an increase by twice of elastic fibers in the superficial dermis compared to control water (Figure), and an increase in the middle dermis compared to the water control at D5.

The 0723-2 sample shows an increase doubled in the middle dermis at day 1 compared to control water and an increase at day 5.

### Ki67 Expression

The results of the immunohistochemical analysis of Ki67 are reported in
**Table** and expressed as % of labelled at the basal layer of the epidermis. The Figure shows the percentage of Ki 67 cells compared to negative controls (water or DMSO). Immunohistochemical analysis of mitotic activity is shown in annex 4 with a reminder of the average for each analysed conditions.

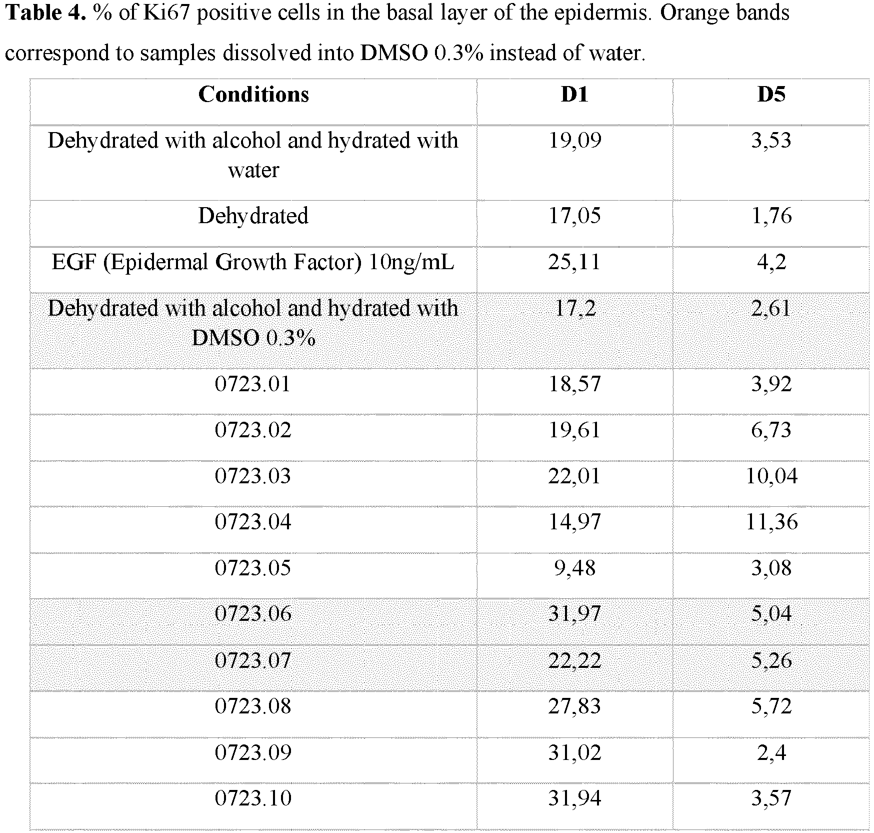

Under the experimental conditions of the study, test item 0723-06, 0723-08, 0723-09 and 0723-010 show an increase in the number of mitotic cells compared to EGF at day 1. A decrease in the mitotic index was observed on day 5 compared to day 1 for all analysed conditions.

The decrease in this cell staining on day 5 is caused by the model. Indeed, after approximately 3 days cell turnover is exhausted on this model.

### SEQUENCES

PROTEIN : P13918- 2-PEA
   PEPTIDE : QNYLSGFSKNILE [SEQ ID 15]
   PEPTIDE : TIKLPAGTIAYLVNRDDNEE [SEQ ID 16]
   PEPTIDE : LAIPVNRPGQLQSFL [SEQ ID 17]
   PEPTIDE : AIPVNRPGQLQ [SEQ ID 18]
   PEPTIDE : PAGHPVAVK [SEQ ID 19]
   PEPTIDE : VQNYKAKLSSGDVFVIPAG [SEQ ID 20]
   PEPTIDE : NNQRNFLAGDEDNVISQIQRPVKE [SEQ ID 21]
   PEPTIDE : INKQVQNYKAKLSSGDVFVIPAG [SEQ ID 22]
   PEPTIDE : LAIPVNRPGQ [SEQ ID 23]
   PEPTIDE : NFLAGDEDNVISQIQRPVKE [SEQ ID 24]
   PEPTIDE : DLAIPVNRPGQLQSF [SEQ ID 25]
   PEPTIDE : VIPAGHPVAVK [SEQ ID 26]
   PEPTIDE : DTIKLPAGTIAYLVNRDDNEE [SEQ ID 27]
   PEPTIDE : LAIPVNRPGQLQSF [SEQ ID 28]
   PEPTIDE : KQVQNYKAKLSSGDVFVIPAG [SEQ ID 29]
   PEPTIDE : RGDTIKLPAGTIAYLVNRDDNEE [SEQ ID 30]
   PEPTIDE : FLAGDEDNVISQIQRPVKE [SEQ ID 31]
   PEPTIDE : LAIPVNRPGQLQS [SEQ ID 32]
   PEPTIDE : VLDLAIPVNRPGQLQ [SEQ ID 33]
   PEPTIDE : DLAIPVNRPGQLQ [SEQ ID 34]
   PEPTIDE : VFVIPAGHPVAVK [SEQ ID 35]
   PEPTIDE : TIFLPQHTDADYILVVLSGK [SEQ ID 36]
   PEPTIDE : NQRNFLAGDEDNVISQIQRPVKE [SEQ ID 37]
   PEPTIDE : LAIPVNRPGQLQ [SEQ ID 38]
   PEPTIDE : HPVAVKASSNLDLLGFG [SEQ ID 39]
   PEPTIDE : LAIPVNRPGQL [SEQ ID 40]
   PEPTIDE : DLAIPVNRPGQL [SEQ ID 41]
   PEPTIDE : SKPHTIFLPQHTDADYILVVLSGK [SEQ ID 42]
   PEPTIDE : FVIPAGHPVAVK [SEQ ID 43]
   PEPTIDE : DLAIPVNRPGQLQS [SEQ ID 44]
   PEPTIDE : SGDVFVIPAGHPVAVKASSNLD [SEQ ID 45]
   PEPTIDE : AIPVNRPGQLQSF [SEQ ID 46]
   PEPTIDE : ELAFPGSAQEVDR [SEQ ID 47]
   PEPTIDE : LAIPVNRPGQLQSFLLSG [SEQ ID 48]
   PEPTIDE : VFVIPAGHPVAVKASSNLDLLGFG [SEQ ID 49]
   PEPTIDE : AGHPVAVK [SEQ ID 50]
   PEPTIDE : HPVAVKASSNLDLLGFGINAE [SEQ ID 51]
   PEPTIDE : LAIPVNRPGQLQSFLLSGNQNQ [SEQ ID 52]
   PEPTIDE : SGDVFVIPAG [SEQ ID 53]
   PEPTIDE : GSLLLPHYNSRAIVIVTVNE [SEQ ID 54]
   PEPTIDE : NFLAGDEDNVISQIQRPVK [SEQ ID 55]
   PEPTIDE : SGDVFVIPAGHPVA [SEQ ID 56]
   PEPTIDE : GSLLLPHYNSRAIVIV [SEQ ID 57]
   PEPTIDE : RGDTIKLPAGTIAYLVNRDD [SEQ ID 58]
   PEPTIDE : SGDVFVIPAGHPVAVK [SEQ ID 59]
   PEPTIDE : LSSGDVFVIPAGHPVAVK [SEQ ID 60]
   PEPTIDE : LDLAIPVNRPGQL [SEQ ID 61]
   PEPTIDE : AIPVNRPGQL [SEQ ID 62]
   PEPTIDE : LAIPVNRPGQLQSFLL [SEQ ID 63]
   PEPTIDE : PHTIFLPQHTDADYILVVLSGK [SEQ ID 64]
   PEPTIDE : VFVIPAGHPVAVKASSNLD [SEQ ID 65]
   PEPTIDE : LAIPVNRPGQLQSFLLS [SEQ ID 66]
   PEPTIDE : VLDLAIPVNRPGQLQSF [SEQ ID 67]
   PEPTIDE : AIPVNRPGQLQS [SEQ ID 68]
   PEPTIDE : DTIKLPAGTIAYLVNRDDNE [SEQ ID 69]
   PEPTIDE : NYKAKLSSGDVFVIPAG [SEQ ID 70]
   PEPTIDE : GKAILTVLKPDDRNSFNLE [SEQ ID 71]
   PEPTIDE : YKSKPHTIFLPQHTDAD[SEQ ID 72]
   PEPTIDE : ASSNLDLLGFG[SEQ ID 73]
   PEPTIDE : DEEEEQGEEEINK[SEQ ID 74]
   PEPTIDE : YKSKPHTIFLPQHTD[SEQ ID 75]
   PEPTIDE : VLDLAIPVNR[SEQ ID 76]
   PEPTIDE : FFEITPEKNPQLQDLDIFVNSVEIK[SEQ ID 77]
   PEPTIDE : TIFLPQHTDADYIL[SEQ ID 78]
   PEPTIDE : SFLLSGNQNQQNYLSG[SEQ ID 79]
   PEPTIDE : SFLLSGNQNQQNYLSGFS[SEQ ID 80]
   PEPTIDE : NQQEQRKEDDEEEEQGEEE[SEQ ID 81]
   PEPTIDE : EEQGEEEINK[SEQ ID 82]
   PEPTIDE : SRGPIYSNE[SEQ ID 83]
   PEPTIDE : EDDEEEEQGEEEINK[SEQ ID 84]
   PEPTIDE : DDEEEEQGEEEINK[SEQ ID 85]
   PEPTIDE : KEDDEEEEQGEEEIN[SEQ ID 86]
   PEPTIDE : KEDDEEEEQGEE[SEQ ID 87]
   PEPTIDE : QRKEDDEEEEQGEEE[SEQ ID 88]
   PEPTIDE : KEDDEEEEQGEEEINK[SEQ ID 89]
   PEPTIDE : KEDDEEEEQGEEE[SEQ ID 90]
PROTEIN : Q9M3X6 - 3 - PEA
   PEPTIDE : HPVAITASSNLNLLG[SEQ ID 91]
   PEPTIDE : ASSNLNLLGFG[SEQ ID 92]
   PEPTIDE : ITASSNLNLLGFG[SEQ ID 93]
   PEPTIDE : ITASSNLNLLGFGINAE[SEQ ID 94]
   PEPTIDE : SSNLNLLGFG[SEQ ID 95]
   PEPTIDE : VDLVIPVNGPGKF[SEQ ID 96]
   PEPTIDE : LVIPVNGPGKFE[SEQ ID 97]
   PEPTIDE : LVIPVNGPGKFEA[SEQ ID 98]
   PEPTIDE : LRLVDLVIPVNGPGKFE[SEQ ID 99]
   PEPTIDE : YRAKPHTIFLPQHIDAD[SEQ ID 100]
   PEPTIDE : HPVAITASSNLNLLGFGINAE[SEQ ID 101]
   PEPTIDE : SNLNLLGFG[SEQ ID 102]
   PEPTIDE : HPVAITASSNLNLLGFGINAENNE[SEQ ID 103]
   PEPTIDE : LVDLVIPVNGPGKFE[SEQ ID 104]
   PEPTIDE : LVIPVNGPGKF[SEQ ID 105]
   PEPTIDE : TIKLPAGTTSYLVNQDDE[SEQ ID 106]
   PEPTIDE : DLRLVDLVIPVNGPGKFE[SEQ ID 107]
   PEPTIDE : EDLRLVDLVIPVNGPGKFE[SEQ ID 108]
   PEPTIDE : HPVAITASSNLNLLGFG[SEQ ID 109]
   PEPTIDE : LVDLVIPVNGPGKFEAFDLAK[SEQ ID 110]
   PEPTIDE : DNVISQIENPVKE[SEQ ID 111]
   PEPTIDE : VVIIPAGHPVAITASSNLNLLGFG[SEQ ID 112]
   PEPTIDE : LVDLVIPVNGPGKFEAF[SEQ ID 113]
   PEPTIDE : YPQLQDLDL[SEQ ID 114]
   PEPTIDE : VIPVNGPGKF[SEQ ID 115]
   PEPTIDE : SKKSLPSE[SEQ ID 116]
   PEPTIDE : LPQHIDADLILVVLSGK[SEQ ID 117]
   PEPTIDE : RGDTIKLPAGTTSYLVNQD[SEQ ID 118]
   PEPTIDE : IPVNGPGKF[SEQ ID 119]
   PEPTIDE : LPQHIDADL[SEQ ID 120]
   PEPTIDE : LVIPVNGPGK[SEQ ID 121]
   PEPTIDE : IFLPQHIDAD[SEQ ID 122]
   PEPTIDE : LPQHIDAD[SEQ ID 123]
   PEPTIDE : VIPVNGPGK[SEQ ID 124]
   PEPTIDE : IFLPQHIDA[SEQ ID 125]
   PEPTIDE : TIKLPAGTTSYLVNQDDEE[SEQ ID 126]
   PEPTIDE : HGEWRPSYEKEEDEEEGQRER[SEQ ID 127]
   PEPTIDE : EKRHGEWRPSYEKEEDEEEGQRE[SEQ ID 128]
   PEPTIDE : LPAGTTSYLVNQDDEEDLR[SEQ ID 129]
   PEPTIDE : PSYEKEEDEEEGQRER[SEQ ID 130]
   PEPTIDE : EKRHGEWRPSYE[SEQ ID 131]
   PEPTIDE : TIKLPAGTTSYLVNQDDEED[SEQ ID 132]
   PEPTIDE : HGEWRPSYEKQEDEEEK[SEQ ID133]
   PEPTIDE : EWRPSYEKEEDEEE[SEQ ID 134]
   PEPTIDE : PSYEKEEDEEEGQR[SEQ ID 135]
   PEPTIDE : EKEEDEEEGQR[SEQ ID 136]
   PEPTIDE : EWRPSYEKEEDEEEGQRE[SEQ ID 137]
   PEPTIDE : KEEDEEEGQR[SEQ ID 138]
   PEPTIDE : VQPGRERWEREEDEEQVDE[SEQ ID 139]
   PEPTIDE : DVVIIPAGHPVA[SEQ ID 140]
   PEPTIDE : HGEWRPSYEKQEDE[SEQ ID 141]
   PEPTIDE : EEDEEEGQR[SEQ ID 142]
   PEPTIDE : HGEWRPSYEKEEDEEEGQR[SEQ ID 143]
   PEPTIDE : EEWRGSQRREDPEE[SEQ ID 144]
   PEPTIDE : REEDEEQVDEEWRGSQRREDPEE[SEQ ID 145]
   PEPTIDE : RHGEWRPSY[SEQ ID 146]
   PEPTIDE : HGEWRPSYEKQEDEE[SEQ ID 147]
   PEPTIDE : VVIIPAGHPVA[SEQ ID 148]
   PEPTIDE : HGEWRPSYE[SEQ ID 149]
   PEPTIDE : KEEDEEEGQRER[SEQ ID 150]
   PEPTIDE : VVIIPAGHPVAIT[SEQ ID 151]
   PEPTIDE : EKRHGEWRPSYEKEEDE[SEQ ID 152]
   PEPTIDE : QVDEEWRGSQRREDPEE[SEQ ID 153]
   PEPTIDE : GDTIKLPAGTTSYLVNQDDEEDLR[SEQ ID 154]
   PEPTIDE : GSEPRVPAQRE[SEQ ID 155]
   PEPTIDE : EEKRHGEWRPSYEKE[SEQ ID 156]
   PEPTIDE : EWRPSYEKEEDEE[SEQ ID 157]
   PEPTIDE : NYDEGSEPRVPAQRE[SEQ ID 158]
   PEPTIDE : VIIPAGHPVAIT[SEQ ID 159]
   PEPTIDE : RHGEWRPSYEK[SEQ ID 160]
   PEPTIDE : NYDEGSEPR[SEQ ID 161]
   PEPTIDE : WRPSYEKEEDEE[SEQ ID 162]
   PEPTIDE : WRPSYEKQEDEEE[SEQ ID 163]
   PEPTIDE : EKRHGEWRPSYEKQEDEEE[SEQ ID 164]
   PEPTIDE : VVIIPAGHPVAITA[SEQ ID 165]
   PEPTIDE : KRHGEWRPSYE[SEQ ID 166]
   PEPTIDE : GSDDNVISQIENPVKE[SEQ ID 167]
   PEPTIDE : VVIIPAGHPV[SEQ ID 168]
   PEPTIDE : HGEWRPSY[SEQ ID 169]
   PEPTIDE : RPSYEKEEDEEEGQR[SEQ ID 170]
   PEPTIDE : HGEWRPSYEK[SEQ ID 171]
   PEPTIDE : KRHGEWRPSYEKEE[SEQ ID 172]
   PEPTIDE : VVIIPAGHPVAITAS[SEQ ID 173]
   PEPTIDE : RGDTIKLPAGTTSYLVNQDDEED[SEQ ID 174]
   PEPTIDE : KRHGEWRPSYEKQEDEEE[SEQ ID 175]
   PEPTIDE : DEEQVDEEWRGSQRREDPEE[SEQ ID 176]
   PEPTIDE : RHGEWRPSYE[SEQ ID 177]
   PEPTIDE : HGEWRPSYEKE[SEQ ID 178]
   PEPTIDE : KRHGEWRPSYEKEEDEEE[SEQ ID 179]
   PEPTIDE : EKRHGEWRPSYEKEEDEEE[SEQ ID 180]
   PEPTIDE : TIKLPAGTTSYLVNQDDEEDLRLVD[SEQ ID 181]
   PEPTIDE : WRPSYEKEEDEEEGQRE[SEQ ID 182]
   PEPTIDE : KRHGEWRPSYEKEEDEE[SEQ ID 183]
   PEPTIDE : VVIIPAGHPVAI[SEQ ID 184]
   PEPTIDE : EWRGSQRREDPEE[SEQ ID 185]
   PEPTIDE : HGEWRPSYEKQEDEEEKQK[SEQ ID 186]
   PEPTIDE : SGSDDNVISQIENPVKE[SEQ ID 187]
   PEPTIDE : RPSYEKEEDEEEGQRER[SEQ ID 188]
   PEPTIDE : EKEEDEEEGQRER[SEQ ID 189]
   PEPTIDE : HGEWRPSYEKQ[SEQ ID 190]
   PEPTIDE : WRPSYEKEEDEEE[SEQ ID 191]
   PEPTIDE : LAKNKNQYLRGFS[SEQ ID 192]
   PEPTIDE : NKNQYLRGFS[SEQ ID 193]
   PEPTIDE : LRGFSKNILE[SEQ ID 194]
   PEPTIDE : LAKNKNQYLRGFSKN[SEQ ID 195]
   PEPTIDE : TVLSPNDRNSY[SEQ ID 196]
   PEPTIDE : QYLRGFSKNILE[SEQ ID 197]
   PEPTIDE : GKAILTVLSPNDRNSYNLE[SEQ ID 198]
   PEPTIDE : RGFSKNILE[SEQ ID 199]
   PEPTIDE : NKNQYLRGFSKNILE[SEQ ID 200]
   PEPTIDE : ASSNLNLLGFGINAE[SEQ ID 201]
   PEPTIDE : ASSNLNLLGF[SEQ ID 202]
   PEPTIDE : LAKNKNQYLRGFSK[SEQ ID 203]
   PEPTIDE : RGDTIKLPAGTTSYLVNQDDEE[SEQ ID 204]
   PEPTIDE : ARLSPGDVVIIPAGHPVAITASSN[SEQ ID 205]
   PEPTIDE : VQRYEARLSPGD[SEQ ID 206]
   PEPTIDE : ARLSPGDVVIIPAGHPVAIT[SEQ ID 207]
   PEPTIDE : RGDTIKLPAGTTSYLVNQDDE[SEQ ID 208]
   PEPTIDE : ARLSPGDVVIIPAGHPVA[SEQ ID 209]
   PEPTIDE : GALMLPHYNSRAIVVLLVNE[SEQ ID 210]
   PEPTIDE : ARLSPGDVVIIPAGHPVAITASS[SEQ ID 211]
   PEPTIDE : LSPGDVVIIPAGHPVAITASSNLNLLGFGINAENNER[SEQ ID 212]
   PEPTIDE : ARLSPGDVVIIPAGHPVAITAS[SEQ ID 213]
   PEPTIDE : LSPGDVVIIPAGHPVAITASSNL[SEQ ID 214]
   PEPTIDE : ARLSPGDVVIIPAGHPVAITA[SEQ ID 215]
PROTEIN : Q0DEV5 - 5- RICE
   PEPTIDE : NWENVLLGLGVAGSAPGIEGDEIAPLAK[SEQ ID 216]
   PEPTIDE : YDQYKDAWDTSVVAEIK[SEQ ID 217]
   PEPTIDE : SSFDFIDGYDTPVEGR[SEQ ID 218]
   PEPTIDE : GPDTGVDYKDNQM[SEQ ID 219]
   PEPTIDE : ILNLNNNPYFK[SEQ ID 220]
   PEPTIDE : VVGTPAYEE[SEQ ID 221]
   PEPTIDE : IDGYDTPVEGR[SEQ ID 222]
   PEPTIDE : VVGTPAYE[SEQ ID 223]
   PEPTIDE : IYGPDTGVDYK[SEQ ID 224]
   PEPTIDE : VAGSAPGIEGDE[SEQ ID 225]
   PEPTIDE : IYGPDTGVDYKDNQMR[SEQ ID 226]
   PEPTIDE : VVGTPAYEEMVR[SEQ ID 227]
   PEPTIDE : DFIDGYDTPVEGR[SEQ ID 228]
   PEPTIDE : LGLGVAGSAPGIEGDEIAPLAK[SEQ ID 229]
   PEPTIDE : FNAPLAHLIMAGADVLAVPSR[SEQ ID 230]
   PEPTIDE : LGLGVAGSAPGIEGDE[SEQ ID 231]
   PEPTIDE : LGLGVAGSAPGIEGDEIAPL[SEQ ID 232]
   PEPTIDE : VLTVSPYYAEELISGIAR[SEQ ID 233]
   PEPTIDE : EALQAEAGLPVDR[SEQ ID 234]
   PEPTIDE : LGLGVAGSAPGIEGD[SEQ ID 235]
   PEPTIDE : IMAGADVLAVPSR[SEQ ID 236]
   PEPTIDE : GLGVAGSAPGIEGDE[SEQ ID 237]
   PEPTIDE : EALQAEAGLPVDRK[SEQ ID 238]
   PEPTIDE : TGGLGDVLGGLPPAMAANGHR[SEQ ID 239]
   PEPTIDE : LEEQKGPDVMA[SEQ ID 240]
   PEPTIDE : LGVAGSAPGIEGDEIAPLAK[SEQ ID 241]
   PEPTIDE : GLGVAGSAPGIEGDEIAPLAK[SEQ ID 242]
   PEPTIDE : TGGLGDVLGGLPPAM[SEQ ID 243]
   PEPTIDE : NVLLGLGVAGSAPGIEGDE[SEQ ID 244]
PROTEIN : P14323 - 6-RICE
   PEPTIDE : TVFDGVLRPGQL[SEQ ID 245]
   PEPTIDE : RLQSQNDQRGEIIHVK[SEQ ID 246]
PROTEIN : P29835 - 5 - RICE
   PEPTIDE : EGYYGEQQQQPGMTR[SEQ ID 247]
   PEPTIDE : GYYGEQQQQPGMTR[SEQ ID 248]
   PEPTIDE : EEGYYGEQQQQPGMTR[SEQ ID 249]
   PEPTIDE : YYGGEGSSSEQGYYGEGSSE[SEQ ID 250]
   PEPTIDE : YGGEGSSSEQGYYGEGSSE[SEQ ID 251]
   PEPTIDE : SSEEGYYGEQQQQPGMTR[SEQ ID 252]
   PEPTIDE : SEEGYYGEQQQQPGMTR[SEQ ID 253]
   PEPTIDE : QGYYGEGSSEE[SEQ ID 254]
   PEPTIDE : YGGEGSSSEQGYYGEGSSEEGY[SEQ ID 255]
   PEPTIDE : YGEQQQQPGMTR[SEQ ID 256]
   PEPTIDE : YYGEQQQQPGMTR[SEQ ID 257]
   PEPTIDE : SYEESMPMPLEQGWSSSSSE[SEQ ID 258]
   PEPTIDE : YYGEGSSEEGYYGEQQQQPGMTR[SEQ ID 259]
   PEPTIDE : QQQQPGMTRV[SEQ ID 260]
   PEPTIDE : GEQQQQPGMTR[SEQ ID 261]
   PEPTIDE : SYEESMPMPLEQGWSSSSSEY[SEQ ID 262]
   PEPTIDE : YYGGEGSSSEQGYYGEGSSEEGY[SEQ ID 263]
   PEPTIDE : YGEQQQQPGMTRVR[SEQ ID 264]
   PEPTIDE : GEGSSEEGYYGEQQQQPGMTR[SEQ ID 265]
   PEPTIDE : YGEGSSEEGYYGEQQQQPGMTR[SEQ ID 266]
   PEPTIDE : QQQQPGMTRVR[SEQ ID 267]
   PEPTIDE : QYAAQLPSMCRVEPQQCSIFAAGQY[SEQ ID 268]
PROTEIN : P0C1U8-5-*Staphylococcus aureus*
   PEPTIDE : APTGTFIASGVVVGKD[SEQ ID 413]
   PEPTIDE : LAIVKFSPNEQNKHIGE[SEQ ID 414]
   PEPTIDE : RHQITDTTNGHYAPVTYIQVE[SEQ ID 415]
   PEPTIDE : GDLAIVKFSPNEQNKHIGE[SEQ ID 416]
   PEPTIDE : NPDNPDNPNNPDNPNNPD[SEQ ID 417]
PROTEIN : P14614-4-RICE
   PEPTIDE : TVFNGVLRPGQL[SEQ ID 269]
   PEPTIDE : TVFNGVLRPGQLL[SEQ ID 270]
   PEPTIDE : SGFNNELLSEALGVNALVAK[SEQ ID 271]
   PEPTIDE : NGVLRPGQL[SEQ ID 272]
   PEPTIDE : ALVAKRLQGQNDQRGEI[SEQ ID 273]
   PEPTIDE : VPRYSNTPGM[SEQ ID 274]
   PEPTIDE : PRYSNTPGMV[SEQ ID 275]
   PEPTIDE : YSNTPGMVY[SEQ ID 276]
   PEPTIDE : LVPRYSNTPGM[SEQ ID 277]
   PEPTIDE : FYNEGDAPVV[SEQ ID 278]
   PEPTIDE : FYNEGDAPVVAL[SEQ ID 279]
   PEPTIDE : FEPLRRVRSEAGVTE[SEQ ID 280]
   PEPTIDE : FYNEGDAPVVALY[SEQ ID 281]
   PEPTIDE : FYNEGDAPVVA[SEQ ID 282]
PROTEIN : P09918 - 14 - *Pisum sativum* PEPTIDE : HGPVEMPYTLLYPSSK[SEQ ID 283]
PROTEIN : P02857-1-PEA
   PEPTIDE : LDALEPDNR[SEQ ID 284]
   PEPTIDE : DALEPDNR[SEQ ID 285]
   PEPTIDE : HGSLHKNAMFVPHYNLNANSIIYA[SEQ ID 286]
   PEPTIDE : LAGTSSVINNLPLDVVAATF[SEQ ID 287]
   PEPTIDE : FREGDIIAVPTGIVFW[SEQ ID 288]
   PEPTIDE : GTSSVINNLPLDVVAATFNLQRNE[SEQ ID 289]
   PEPTIDE : KGAIVKVKGGLSIISPPE[SEQ ID 290]
   PEPTIDE : RLAGTSSVINNLPLD[SEQ ID 291]
   PEPTIDE : AGTSSVINNLPLDVVAATFNLQRNE[SEQ ID 292]
   PEPTIDE : AGTSSVINNLPL[SEQ ID 293]
   PEPTIDE : LAGTSSVINNLPLDVVA[SEQ ID 294]
   PEPTIDE : AGTSSVINNLPLDV[SEQ ID 295]
   PEPTIDE : AGRIKTVTSLDLPVLRW[SEQ ID 296]
   PEPTIDE : AGRIKTVTSLDLPVLR[SEQ ID 297]
   PEPTIDE : FREGDIIAVPTGIVF[SEQ ID 298]
   PEPTIDE : AGTSSVINNLPLD[SEQ ID 299]
   PEPTIDE : LAGTSSVINNLPL[SEQ ID 300]
   PEPTIDE : LAGTSSVINNLPLDVV[SEQ ID 301]
   PEPTIDE : EGDIIAVPTGIVF[SEQ ID 302]
   PEPTIDE : LAGTSSVINNLPLDV[SEQ ID 303]
   PEPTIDE : AGRALTVPQNYAVAAKSLSD[SEQ ID 304]
   PEPTIDE : AGRALTVPQNYA[SEQ ID 305]
   PEPTIDE : LAGTSSVINNLPLD[SEQ ID 306]
   PEPTIDE : RAGIARLAGTSSVINNLPLDVVA[SEQ ID 307]
PROTEIN : P02855-4-PEA
   PEPTIDE : RASSNLNLLGFGINAE[SEQ ID 308]
   PEPTIDE : VTVNEGKGDFEL[SEQ ID 309]
   PEPTIDE : VRASSNLNLLGFGINAE[SEQ ID 310]
   PEPTIDE : VRASSNLNLLGFG[SEQ ID 311]
   PEPTIDE : HPVAVRASSNLNLLGFG[SEQ ID 312]
   PEPTIDE : TKNQVQSYKAKLTPGD[SEQ ID 313]
   PEPTIDE : HPVAVRASSNLNLLG[SEQ ID 314]
   PEPTIDE : KAKLTPGDVFVIPAG[SEQ ID 315]
   PEPTIDE : DLTFPGSAQEVDRLLENQK[SEQ ID 316]
   PEPTIDE : PAGHPVAVR[SEQ ID 317]
   PEPTIDE : AKLTPGDVFVIPAGHPVA[SEQ ID 318]
   PEPTIDE : SYKAKLTPGDVFVIPAGHPVA[SEQ ID 319]
   PEPTIDE : LTPGDVFVIPAGHPVAVR[SEQ ID 320]
   PEPTIDE : VQSYKAKLTPGDVFVIPAG[SEQ ID 321]
   PEPTIDE : YKAKLTPGDVFVIPAGHPVA[SEQ ID 322]
   PEPTIDE : FVIPAGHPVAVR[SEQ ID 323]
   PEPTIDE : YKAKLTPGDVFVIPAG[SEQ ID 324]
   PEPTIDE : DLTFPGSAQEVDR[SEQ ID 325]
   PEPTIDE : AKLTPGDVFVIPAGHPVAVR[SEQ ID 326]
   PEPTIDE : LTPGDVFVIPAG[SEQ ID 327]
   PEPTIDE : SYKAKLTPGDVFVIPAG[SEQ ID 328]
   PEPTIDE : SYKAKLTPGDVFVIPAGHPVAVR[SEQ ID 329]
   PEPTIDE : VIPAGHPVAVR[SEQ ID 330]
   PEPTIDE : QVQSYKAKLTPGDVFVIPAG[SEQ ID 331]
   PEPTIDE : AKLTPGDVFVIPAG[SEQ ID 332]
   PEPTIDE : HPVAVRASSNLNLLGFGINAE[SEQ ID 333]
   PEPTIDE : YKAKLTPGDVFVIPAGHPVAVR[SEQ ID 334]
   PEPTIDE : TKNQVQSYKAKLTPGDVFVIPAG[SEQ ID 335]
   PEPTIDE : PFNLKSSDPIYS[SEQ ID 336]
   PEPTIDE : IEKILLEE[SEQ ID 337]
   PEPTIDE : SRSEPFNLKSSDPIYS[SEQ ID 338]
   PEPTIDE : HPVAVRASSNLNL[SEQ ID 339]
PROTEIN : D3VNE1-5-PEA PEPTIDE : TLFLPQYTDADFILVVLSGK[SEQ ID 340]
PROTEIN : P07728 - 1-RICE
   PEPTIDE : EVEERSQNIF[SEQ ID 341] PEPTIDE : VEERSQNIFSGF[SEQ ID 342] PEPTIDE : ASLQEQEQGQVQ[SEQ ID 343] PEPTIDE : QEQEQGQVQSR[SEQ ID 344] PEPTIDE : ASLQEQEQGQVQSR[SEQ ID 345] PEPTIDE : EVEERSQNIFSGF[SEQ ID 346] PEPTIDE : VTDLNNGANQLDPRQRD[SEQ ID 347] PEPTIDE : VEHGLSLLQPYASL[SEQ ID 348] PEPTIDE : IYVTDLNNGANQLDPRQRDFL[SEQ ID 349] PEPTIDE : VTDLNNGANQLDPRQRDFL[SEQ ID 350] PEPTIDE : VEHGLSLLQPYASLQEQEQGQVQSR[SEQ ID 351] PEPTIDE : VTDLNNGANQLDPR[SEQ ID 352] PEPTIDE : IYVTDLNNGANQLDPRQRD[SEQ ID 353] PEPTIDE : YVTDLNNGANQLDPRQRDFL[SEQ ID 354] PEPTIDE : YVTDLNNGANQLDPR[SEQ ID 355] PEPTIDE : STELLSEALGVSSQVAR[SEQ ID 356] PEPTIDE : HGLSLLQPYASLQEQE[SEQ ID 357] PEPTIDE : SGFSTELLSEALGVSSQVAR[SEQ ID 358]
PROTEIN : P07730 - 2 - RICE
   PEPTIDE : GAFTPLQYKSYQD[SEQ ID 359]
   PEPTIDE : GLLLPHYTNGASLVY[SEQ ID 360]
   PEPTIDE : FLLAGNKRNPQAYRRE[SEQ ID 361]
   PEPTIDE : ALPTDVLANAYR[SEQ ID 362]
   PEPTIDE : DFLLAGNK[SEQ ID 363]
   PEPTIDE : DVLANAYR[SEQ ID 364]
   PEPTIDE : GAFTPLQYK[SEQ ID 365]
   PEPTIDE : QGDVIALPAGVAHW[SEQ ID 366]
   PEPTIDE : FGAFTPLQYKSY[SEQ ID 367]
   PEPTIDE : FLLAGNKRNPQAYR[SEQ ID 368]
   PEPTIDE : FGAFTPLQYKSYQ[SEQ ID 369]
   PEPTIDE : GLSLLQPYASLQEQE[SEQ ID 370]
   PEPTIDE : AFTPLQYK[SEQ ID 371]
   PEPTIDE : FGAFTPLQYKSYQD[SEQ ID 372]
   PEPTIDE : GDEFGAFTPLQYK[SEQ ID 373]
   PEPTIDE : FGAFTPLQYKSYQDV[SEQ ID 374]
   PEPTIDE : FGAFTPLQYK[SEQ ID 375]
   PEPTIDE : FGAFTPLQYKS[SEQ ID 376]
   PEPTIDE : VYIIQGRGITGPTF[SEQ ID 377]
   PEPTIDE : YIIQGRGITGPTF[SEQ ID 378]
   PEPTIDE : KTNPNSMVSHIAGK[SEQ ID 379]
   PEPTIDE : TNPNSMVSHIAGK[SEQ ID 380]
   PEPTIDE : PNSMVSHIAGKS[SEQ ID 381]
   PEPTIDE : NIDNPNRADTYNPRAGRVTN[SEQ ID 382]
   PEPTIDE : QRDFLLAGNKR[SEQ ID 383]
   PEPTIDE : LLQPYASLQEQE[SEQ ID 384]
   PEPTIDE : QRDFLLAGNK[SEQ ID 385]
   PEPTIDE : QEQEQGQMQSR[SEQ ID 386]
   PEPTIDE : SLLQPYASLQEQE[SEQ ID 387]
   PEPTIDE : ASLQEQEQGQM[SEQ ID 388]
   PEPTIDE : ASLQEQEQGQMQSR[SEQ ID 389]
   PEPTIDE : DFLLAGNKR[SEQ ID 390]
   PEPTIDE : QAFEPIRSVRSQAGTTEF[SEQ ID 391]
   PEPTIDE : KTNPNSMVSHIAGKSSIF[SEQ ID 392]
   PEPTIDE : VRRVIEPRGLLLPHYTNGASL[SEQ ID 393]
   PEPTIDE : FGAFTPLQYKSYQDVYN[SEQ ID 394]
   PEPTIDE : IALPAGVAHW[SEQ ID 395]
   PEPTIDE : RVRQNIDNPNRADTYNPRAGRVTNL[SEQ ID 396]
   PEPTIDE : NIDNPNRADTYNPRAGRVTNL[SEQ ID 397]
   PEPTIDE : GAFTPLQYKSYQDVYN[SEQ ID 398]
   PEPTIDE : PNSMVSHIAGKSSIFR[SEQ ID 399]
   PEPTIDE : RLQAFEPIRSVRSQAGTTE[SEQ ID 400]
   PEPTIDE : TNPNSMVSHIAGKSSIFR[SEQ ID 401]
PROTEIN : Q0D7S0 - 3 - RICE
   PEPTIDE : ELGAPDVGHPM[SEQ ID 402]
   PEPTIDE : LGAPDVGHPM[SEQ ID 403]
   PEPTIDE : ELGAPDVGHPMSEVF[SEQ ID 404]
   PEPTIDE : ELGAPDVGHPMS[SEQ ID 405]
   PEPTIDE : ELGAPDVGHPMSEVFR[SEQ ID 406]
   PEPTIDE : ELGAPDVGHPMSEV[SEQ ID 407]
   PEPTIDE : ELGAPDVGHPMSE[SEQ ID 408]
   PEPTIDE : LGAPDVGHPMSE[SEQ ID 409]
   PEPTIDE : YRELGAPDVGHPMSE[SEQ ID 410]
   PEPTIDE : LGAPDVGHPMSEV[SEQ ID 411]
   PEPTIDE : RELGAPDVGHPMSE[SEQ ID 412]

### ADDITIONAL PEPTIDES (AND SOURCE PROTEIN)

HPRPPKPDAPR [SEQ ID 452] - QOD9DO
LQQAPPPPQR [SEQ ID 453] - Q6AVS5
VGWGEQPWSPY [SEQ ID 454] - Q8H920
FHMPP [SEQ ID 455] - O49927
FRRP [SEQ ID 456] - P29835
FWM [SEQ ID 457] - P15838
HMPPS [SEQ ID 458] - 049927
PVEMPTLLYPS [SEQ ID 459]
HMPSS [SEQ ID 460] - O49927
HRFR [SEQ ID 461] - Q712V4
HRRS [SEQ ID 462] - P13918
HSPR [SEQ ID 463] - P14323
HWF [SEQ ID 464 - P14323
MFRR [SEQ ID 465] - Q41000
MFRRP [SEQ ID 466 - P29835
MPPS [SEQ ID 467] - 049927
MPRR [SEQ ID 468] - P15838
NMPS [SEQ ID 469] - P20698
PHMP [SEQ ID 470] - O49927
PHMPS [SEQ ID 471 - O49927
PRRF [SEQ ID 472] - P15838
WMK [SEQ ID 473] - O04434
DSINALEPDHR [SEQ ID 474] - P05692
ELTFPGSVQ [SEQ ID 475] - Q9M3X6
ELTFPGSVQE [SEQ ID 476] - Q9M3X6
IFEDAITIPGR [SEQ ID 477] - P09886
LDALEPDNRIESE [SEQ ID 478] - P02857
KTLDYWPSLR [SEQ ID 479] - P08688
RHGEWGPSY [SEQ ID 480]
ILVDGSHDIER [SEQ ID 481] - Q5N725
LVSHPIAAHEGR [SEQ ID 482] - P14614
NLAQAPAQALL [SEQ ID 483] - Q0DJ38
FLPQHTD [SEQ ID 484] - P13918
LEPDNR [SEQ ID 485] - P15838
LQSQND [SEQ ID 486] - P14323
LQSQNDQRGEI [SEQ ID 487] - P14323
QSQNDQRGEIIHVK [SEQ ID 488] - P14323
RGEIIHVK [SEQ ID 489] - P14323
RLQSQNDQ [SEQ ID 490] - P14323
RLQSQNDQRG [SEQ ID 491] - P14323
RLQSQNDQRGEIIH [SEQ ID 492] - P14323
VFDGVLRPG [SEQ ID 493] - P14323
HNPR [SEQ ID 494] - P14614
HPMS [SEQ ID 495] - Q0D7S0
HPSF [SEQ ID 496] - Q0DEV5
MPMP [SEQ ID 497] - P29835
PMPL [SEQ ID 498] - P29835
PNSM [SEQ ID 499] - P07728
WDP [SEQ ID 500] - B5A8N6
LRGFSK [SEQ ID 501] - Q9M3X6
RSQNIF [SEQ ID 502] - P07728
YLRGFS [SEQ ID 503] - Q9M3X6
GALMLPHYN [SEQ ID 504] - Q9M3X6
GALMLPHYNSR [SEQ ID 505] - Q9M3X6
KNPQLQDLDIFVNYVEIK [SEQ ID 700] P02855
PGQLQSFLLSGNQNQQNYLSGFSK [SEQ ID 701] P13918
RGPQQYAEWQINEK [SEQ ID 702] Q6K7K6
VLDLAIPVNRPGQL [SEQ ID 703] Q6K508
GYVGLTFPGCPATHQQQFQLFEQR [SEQ ID 704] P09918
LDVTPLSLGL [SEQ ID 717]
EEGIQLVAEAIR [SEQ ID 718]
YSLKPLVPR [SEQ ID 719]
WHT [SEQ ID 720]
WHN [SEQ ID 721]
NNPF [SEQ ID 722]
MRFR [SEQ ID 723]
MPPSS [SEQ ID 724]
HMPS [SEQ ID 725]
HMPPS [SEQ ID 726]
GHPM [SEQ ID 727]
FWN [SEQ ID 728]
FHMP [SEQ ID 729
WTIVQGLPIDE [SEQ ID 730]
GYPMYPLPR [SEQ ID 731]
HGGEGGRPY [SEQ ID 732]
LRGFSK
GALMLPHYN
GALMLPHYNSR
VFDGVLRPG
LQSQND
LQSQNDQRGEI
QSQNDQRGEIIHVK
RGEIIHVK [SEQ ID 740]
RLQSQNDQ
RLQSQNDQRG
RLQSQNDQRGEIIH
MPMP
PMPL
LEPDNR
GIARLAGTSSVIN
RSQNIF
PNSM
GHPM [SEQ ID 750]
HPMS
FLPQHTD
EWQINEK
GPQQYAEWQINEK
PQQYAEWQ
RGPQQYA
HNPR
WHN
WDP
HPSF [SEQ ID 760]
PGQLQSFLLSGNQNQQNYLSGF
QLQSFLLSGNQNQQNYLSGFSK
QSFLLSGNQNQQ
PGQLQSFLLSGN
QSFLLSGNQ
QNQQNYLSGFSK
YLRGFS
PVEMPTLLYPS
RGPQQYAEWQINE
GYVGLTFPGCPATHQQQFQLFEQR[SEQ ID 770]
KNPQLQDLDIFVNYVEIK
PGQLQSFLLSGNQNQQNYLSGFSK
RGPQQYAEWQINEK
VLDLAIPVNRPGQL
RGPQQYAEWQINEK [SEQ ID 775]
PROTEIN P02855 [SEQ ID 546-552]
   PEPTIDE : SRAIVIVTVNE
   PEPTIDE : AKLTPGDV
   PEPTIDE : IVIVTVNEGK
   PEPTIDE : LDALEPDNRIESEGGL (also in P02857)
   PEPTIDE : RPYYSNAPQE (also in P02857)
   PEPTIDE : LDALEPDNRIESEGGLIETWNPNNK (also in P02857)
   PEPTIDE : AIVIVTVNEGK (also in P13918)
PROTEIN P02857 [SEQ ID 553-565]
   PEPTIDE: LQVVNCNGNTVFDGEL
   PEPTIDE: QVVNCNGNTVFDGEL
   PEPTIDE : IIAVPTGIVF
   PEPTIDE: GRRYRDRHQKVNRFRE
   PEPTIDE: RPYYSNAPQEI
   PEPTIDE : RLDALEPDNRIE
   PEPTIDE : RLDALEPDNRIESE
   PEPTIDE : LDALEPDNRIESEGGLIETW
   PEPTIDE : LDALEPDNRIE
   PEPTIDE : LDALEPDNRIESEGGLIE
   PEPTIDE : LDALEPDNRIESEGGL (also in P02855)
   PEPTIDE : RPYYSNAPQE (also in P02857)
   PEPTIDE : LDALEPDNRIESEGGLIETWNPNNK (also in P02855)
PROTEIN P07728 [SEQ ID 566-609]
   PEPTIDE : VEHGLSLLQPYASLQEQEQGQVQSRER
   PEPTIDE : RSQNIFSGF
   PEPTIDE : GITGPTFPGCPESY
   PEPTIDE : CNGS
   PEPTIDE : SPREC
   PEPTIDE : PREC
   PEPTIDE : PRECR
   PEPTIDE : CPES
   PEPTIDE : SGCS
   PEPTIDE : CSNG
   PEPTIDE : RSQNIFSGFSTE
   PEPTIDE : VEEWSQNIFSGFST
   PEPTIDE : WSQNIFSGFSTEL
   PEPTIDE : WSQNIFSGFSTE
   PEPTIDE : STSQWQSSRR
   PEPTIDE : NRPI
   PEPTIDE : CDGS
   PEPTIDE : PRGC
   PEPTIDE : PRGCR
   PEPTIDE : RGCR
   PEPTIDE : GCRF
   PEPTIDE : PTFP
   PEPTIDE : PGCPE
   PEPTIDE : GCPE
   PEPTIDE : CPET
   PEPTIDE : AHWC
   PEPTIDE : HWCY
   PEPTIDE :SGCP
   PEPTIDE: SGCPN
   PEPTIDE : GCPN
   PEPTIDE : CPNG
   PEPTIDE : TFCTM
   PEPTIDE : FCTM
   PEPTIDE : FCTMR
   PEPTIDE : CTMR
   PEPTIDE : EGCA
   PEPTIDE : SQNIFSGFSTELL
   PEPTIDE : SQNIFSGFSTE
   PEPTIDE : QNDQRGEIVR
   PEPTIDE : SQNIFSGFSTEL (also in P07730)
   PEPTIDE : QLQCQNDQRGEI (also in P07730)
   PEPTIDE : LGQSTSQWQSSR (also in P07730)
   PEPTIDE : QQLLGQSTSQWQSSR (also in P07730)
   PEPTIDE : LLGQSTSQWQSSR (also in P07730)
PROTEIN P07730 [SEQ ID 610-619]
   PEPTIDE : NDQRGEIVR
   PEPTIDE : GQSTSQWQSSR
   PEPTIDE : STSQWQSSR
   PEPTIDE : GITGPTFPGCPET
   PEPTIDE : GITGPTFPGCPETY
   PEPTIDE : SQNIFSGFSTEL (also in P07728)
   PEPTIDE : QLQCQNDQRGEI (also in P07728)
   PEPTIDE : LGQSTSQWQSSR (also in P07728)
   PEPTIDE : QQLLGQSTSQWQSSR (also in P07728)
   PEPTIDE : LLGQSTSQWQSSR (also in P07728)
PROTEIN P09918 [620-630]
   PEPTIDE : IFFANQTYL
   PEPTIDE : EHLEPNLEGLTVEE
   PEPTIDE : IFFANQTYLPSETPAPLVHYREEELNNLRGDGTGER
   PEPTIDE : IHFEWDDDMGIPGAFYIK
   PEPTIDE : IFFANQTYLPSETPAPLVHYREEELNNLR
   PEPTIDE : TEQALPADLIK
   PEPTIDE : EHLEPNLEGLTVEEAIQNKK
   PEPTIDE : ISKEHLEPNLEGLTVEEAIQNKK
   PEPTIDE : LSLPHPQGDEHGAVSY
   PEPTIDE : ISKEHLEPNLEGLTVEEAIQNK
   PEPTIDE : EHLEPNLEGLTVEEAIQNK
PROTEIN POC1U8 [SEQ ID 631]
   PEPTIDE : LSTTGGNSGSPVFNEKNE
PROTEIN P13918 [SEQ ID 632-639]
   PEPTIDE : QSFLLSGNQNQQNYLSG
   PEPTIDE : VLDLAIPVNRPGQLQS
   PEPTIDE : VLDLAIPVNRPGQLQSFL
   PEPTIDE : FLLSGNQNQQNYLSG
   PEPTIDE : FLLSGNQNQQNYLSGFSK
   PEPTIDE : DPQNPFIFKSNKFQTLFE
   PEPTIDE : ELAFPGSAQEVDRILENQK
   PEPTIDE : AVVTVNEGK (also in P02855)
PROTEIN P14323 [SEQ ID 640-667]
   PEPTIDE : INAVAAKRLQSQNDQRGE
   PEPTIDE : NRAQQQQVYGSSIE
   PEPTIDE : PSTNPWHSPR
   PEPTIDE : CHGS
   PEPTIDE : CHGSM
   PEPTIDE : PWHS
   PEPTIDE : FREC
   PEPTIDE : RECR
   PEPTIDE : ECRF
   PEPTIDE : CRFD
   PEPTIDE: CRFDR
   PEPTIDE: CTGT
   PEPTIDE: FPGC
   PEPTIDE : FPGCP
   PEPTIDE : PGCP
   PEPTIDE: PGCPA
   PEPTIDE: PGCPAT
   PEPTIDE : GCPA
   PEPTIDE : CPAT
   PEPTIDE : ENFC
   PEPTIDE : NFCT
   PEPTIDE : NFCTI
   PEPTIDE : FCTI
   PEPTIDE : AQQQQVYGSSIEQH
   PEPTIDE : AQQQQVYGSSIEQHSGQNIFSGF
   PEPTIDE : AAKRLQSQNDQRGE
   PEPTIDE : QARSLKNNRGEE (also in P14614)
   PEPTIDE : FNPSTNPWHSPRQGS (also in Q0DEV5)
PROTEIN P14614 [SEQ ID 668]
   PEPTIDE : QARSLKNNRGEE (also in P14323)
PROTEIN Q0D7S0 [SEQ ID 669]
   PEPTIDE : AAASLPAFCNVDIPNGGGGVCYWLAR
PROTEIN Q0DEV5 [SEQ ID 670-680]
   PEPTIDE : VAGSAPGIEGDEIAPLAK
   PEPTIDE : LGVAGSAPGIEGDEIAPLAKEN
   PEPTIDE : GSAPGIEGDEIAPLAKE
   PEPTIDE : VAGSAPGIEGDEIAP
   PEPTIDE : GVAGSAPGIEGDEIAPLAK
   PEPTIDE : GVAGSAPGIEGDEIAPLAKEN
   PEPTIDE : VAGSAPGIEGDEIAPLAKEN
   PEPTIDE : SAPGIEGDEIAPLAK
   PEPTIDE : GSAPGIEGDEIAPLAK
   PEPTIDE : SAPGIEGDEIAPLAKEN
   PEPTIDE : FNPSTNPWHSPRQGS (also in P14323)
PROTEIN Q9M3X6 [SEQ ID 681-699]
   PEPTIDE : VDLVIPVNGPGK
   PEPTIDE : LVDLVIPVNGPGK
   PEPTIDE : IKLPAGTTSY
   PEPTIDE : IKLPAGTTSYL
   PEPTIDE : RRNPFLFKSNKF
   PEPTIDE : IENPVKELTFPGSVQEINR
   PEPTIDE : RRNPFLFKSNKFLT
   PEPTIDE : AKPHTIFLPQHIDA
   PEPTIDE : AKPHTIFLPQHIDAD
   PEPTIDE: KQKYRYQRE
   PEPTIDE: KQKYQYQRE
   PEPTIDE : MLPH
   PEPTIDE : RRNPFLFKSNKFLTLFENE
   PEPTIDE : PFLFKSNKFLTLFE
   PEPTIDE : SQERRNPFLFKSNKFLTLFE
   PEPTIDE : RRNPFLFKSNKFLTLFE
   PEPTIDE : SQERRNPFLFKSNKFLTLFENE
   PEPTIDE : LTFPGSVQE
   PEPTIDE : ELTFPGSVQEINR
Rice protein :Q6AVS5
Rice Protein: Q8H920
Pea protein: O49927
Pea protein :Q41000
Rice Protein : P20698
Pea Protein : O04434
Pea Protein: B5A8N6
Rice Protein: Q6K508
PEA PROTEIN : Q712V4
Pea Protein: P09886

### Examples of homologs for each protein

P13918 (Pea)
   >gi|137584|sp|P08438.1|VCL_VICFA RecName: Full=Vicilin; Flags: Precursor [Vicia faba] >gi |22057| emb|CAA68559.1| vicilin [Vicia faba var. minor] >gi|383931031|gb|AFH56916.1| vicilin **[Vicia faba]**
   >gi |502105533|ref|XP_004492829.1|PREDICTED: vicilin-like isoform X1 **[Cicer arietinum] ChickPea**
   >gi |29539109|emb|CAD87730.1| allergen Len c 1.0101 **[Lens culinaris] Lentil**
Q9M3X6 (Pea)
   >gi|164512526|emb|CAP06312.1| cvc [Pisum abyssinicum]
   >gi |164512538|emb|CAP06318.1| cvc [Lathyrus annuus]
   >gi |164512558| emb| CAP06328.1| cvc [Vicia villosa]
P09918 (Pea)
   >gi|357454557| ref |XP_003597559.1| Seed lipoxygenase-3 [Medicago truncatula] >gi|355486607|gb|AES67810.1| seed linoleate 9S-lipoxygenase [Medicago truncatula]
   >gi|734403888|gb|KHN32710.1| Seed linoleate 9S-lipoxygenase-3 [Glycine soja]
   >gi |593700103| ref|XP_007150490.1| hypothetical protein PHAVU_005G157000g [Phaseolus vulgaris]
   >gi |561023754|gb|ESW22484.1| hypothetical protein PHAVU_005G157000g [Phaseolus vulgaris]
P02857 (Pea)
   >gi 1483449 |emb| CAA83677.1| legumin A [Vicia sativa]
   >gi|600108|emb|CAA86824.1| legumin A precursor [Vicia narbonensis]
   >gi |502110016| ref|XP_004493779.1| PREDICTED: legumin-like [Cicer arietinum]
P02855 (Pea)
   >gi|164512536| emb|CAP06317.1| cvc [Lathyrus hirsutus]
   >gi |164512542|emb |CAP06320.1| cvc [Lathyrus cicera]
   >gi |164512544|emb|CAP06321.1| convicilin [Lathyrus sativus]
D3VNE1 (Pea)
   >gi |357507721|ref |XP_003624149.1| Provicilin [Medicago truncatula] >gi |87162569|gb|ABD28364.1| Cupin, RmIC-type [Medicago truncatula] >gi|355499164|gb|AES80367.1| vicilin 47 kDa protein [Medicago truncatula]
   >gi |164512560|emb |CAP06329.1| convicilin [Vicia peregrina]
   >gi |164512562| emb |CAP06330.1| convicilin [Vicia lutea]
P07728 (Rice)
   >gi|531874314|gb|AGT59174.1| glutelin, partial [Oryza sativa Indica Group]
   >gi |109894635|gb|ABG47337.1| glutelin precursor [Zizania latifolia]
   >gi |472867| emb |CAA52764.1| 11S globulin [Avena sativa]
P14614 (Rice)
   >gi |115445309|ref|NP_001046434.1| Os02g0248800 [Oryza sativa Japonica Group] >gi|37993738 1gb |AAR06952.1| glutelin type-B [Oryza sativa Japonica Group] >gi 1474977291 dbj |BAD19794.1| glutelin type-B [Oryza sativa Japonica Group] >gi |113535965|dbj| BAF08348.1| Os02g0248800 [Oryza sativa Japonica Group] >gi |215768942|dbj| BAH01171.1| unnamed protein product [Oryza sativa Japonica Group] >gi |284431772 |gb |ADB84627.1| glutelin [Oryza sativa Japonica Group]
   >gi |428674402| gb |AFZ41188.1| glutelin, partial [Oryza sativa Japonica Group]
   >gi |226510| prf||1515394A seed storage globulin
P07730 (Rice)
   >gi |225959| prf ||1404367A glutelin
   >gi |573943558| ref |XP_006654150.1| PREDICTED: glutelin type-A 3-like [Oryza brachyantha]
   >gi |721641733| ref |XP_010231907.1| PREDICTED: 12S seed storage globulin 1-like [Brachypodium distachyon]
Q0D7SO (Rice)
   >gi |169244463 |gb| ACA50505.1| seed allergenic protein RAG2 [Oryza sativa Japonica Group]
   >gi |5777592| emb |CAA44001.1| low molecular weight globulin [Oryza sativa]
   >gi |115471175| ref| NP_001059186.1| Os07g0214600 [Oryza sativa Japonica Group] >gi |23616954| dbj| BAC20657.1| allergen RA16 [Oryza sativa Japonica Group] >gi |113610722| dbj | BAF21100.1| Os07g0214600 [Oryza sativa Japonica Group] >gi |125557687|gb| EAZ03223.1| hypothetical protein Osl_25372 [Oryza sativa Indica Group]
Q0DEV5 (Rice)
   >gi| 83375868|gb|ABC17777.1| waxy [Oryza rufipogon]
   >gi|297614332|gb|ADI48504.1| glycogen synthetase [Oryza officinalis]
   >gi|389620054|gb|AFK93486.1| granule-bound starch synthase [Hordeum vulgare subsp. vulgare]
P14323 (Rice)
   >gi| 573918992|ref |XP_006647120.1| PREDICTED: glutelin type-B 2-like [Oryza brachyantha]
   >gi |573919041|ref |XP_006647142.1| PREDICTED: glutelin type-B 4-like [Oryza brachyantha]
   >gi| 109894635 |gb| ABG47337.1| glutelin precursor [Zizania latifolia]
P29835 (Rice)
   >gi|226508602|ref|NP_001152635.1| globulin precursor [Zea mays] >gi|195658363|gb|ACG48649.1| globulin precursor [Zea mays]
   >gi |242090997| ref |XP_002441331.1| hypothetical protein SORBIDRAFT_09g024570 [Sorghum bicolor]
   >gi|241946616|gb|EES19761.1| hypothetical protein SORBIDRAFT_09g024570 [Sorghum bicolor]
   >gi |514748428| ref|XP_004961615.1| PREDICTED: 19 kDa globulin-like [Setaria italica]
P0C1U8 (Bacterial) GLUC (Staphylococcus aureus)
   >gi |446599182| ref| WP_000676528.1| glutamyl endopeptidase [Staphylococcus aureus] >gi |253729369|gb| EES98098.1| trypsin [Staphylococcus aureus subsp. aureus TCH130] >gi| 341844549 |gb| EGS85761.1| glutamyl endopeptidase [Staphylococcus aureus subsp. aureus 21259] >gi| 537390486|gb|AGU61109.1| Glutamyl endopeptidase precursor [Staphylococcus aureus subsp. aureus CN1] >gi| 564714561|gb|ETD14665.1| glutamyl endopeptidase [Staphylococcus aureus subsp. aureus KPL1845] >gi| 577466329 |gb|EUG79766.1| glutamyl endopeptidase [Staphylococcus aureus M0139]
   >gi |580560623|gb|EVF84961.1| glutamyl endopeptidase [Staphylococcus aureus COAS6020]
   >gi |580687002|gb|EVH10169.1| glutamyl endopeptidase [Staphylococcus aureus UCIM6080]
   >gi |751815683|gb|KIN 24957.11 glutamyl endopeptidase [Staphylococcus aureus MRSA_CVM43477]
   >gi |781884797|dbj |BAR08486.1| glutamyl endopeptidase precursor [Staphylococcus aureus subsp. aureus]
   >gi |781887762|dbj |BAR11210.1| glutamyl endopeptidase precursor [Staphylococcus aureus subsp. aureus]

## Claims

1. A peptide of SEQUENCE ID NO: 283.

2. A growth promoting variant of a peptide of Claim 1, having 1 to 3 amino acid alterations compared with the peptide of Claim 1, in which the or each amino acid alteration is selected from insertion, addition, deletion and substitution of an amino acid.

3. A peptide having up to 50 amino acids and comprising a peptide of Claim 1 or 2, in which the peptide is growth promoting.

4. A peptide according to any preceding Claim, which is chemically modified, in which the peptide is growth promoting.

5. A peptide according to Claim 4, in which the peptide is chemically modified by modifications to side chains, incorporation of a protecting group, incorporation of unnatural amino acids and/or their derivatives during peptide synthesis, and the use of cross-linkers and other methods that impose conformational constraint on the peptide.

6. A conjugate comprising a peptide according to any preceding Claim, conjugated, linked or fused to a binding partner, in which the binding partner is optionally selected from a polyethylene glycol polymer, a molecular weight increasing compound, a lipophilic group, or an antibody molecule.

7. A composition comprising a peptide of any of Claims 1 to 5 or a conjugate of Claim 6.

8. A composition comprising a peptide of any of Claims 1 to 3.

9. A composition according to any of Claims 7 or 8, in which the composition is a powder.

10. A composition according to any of Claims 7 or 8, in which the composition is formulated for topical application to the skin of a human.

11. A composition according to Claim 10, in the form of a gel, cream, lotion or ointment.

12. A food or beverage, nutritional supplement, personal care composition, or pharmaceutical 5 composition, comprising a peptide of any of Claims 1 to 5.

13. A non-therapeutic method of preventing or inhibiting ageing of the human skin comprising a step of topically administering to the skin a growth promoting peptide of Claim 1 or 2, a variant of any of Claims 3-5, or a composition of any of Claims 7-9.

14. A topical composition according to Claim 10 or 11, for use in treating a wound in a mammal, wherein the composition is topically applied to the wound in the mammal.

## Patentansprüche

1. Peptid der SEQUENZ-ID NR.: 283.

2. Wachstumsfördernde Variante eines Peptids nach Anspruch 1, die 1 bis 3 Aminosäureveränderungen im Vergleich zu dem Peptid nach Anspruch 1 aufweist, in der die oder jede Aminosäureveränderung aus Insertion, Addition, Deletion und Substitution einer Aminosäure ausgewählt ist.

3. Peptid, das bis zu 50 Aminosäuren aufweist und ein Peptid nach Anspruch 1 oder 2 umfasst, in dem das Peptid wachstumsfördernd ist.

4. Peptid nach einem vorstehenden Anspruch, das chemisch modifiziert ist, in dem das Peptid wachstumsfördernd ist.

5. Peptid nach Anspruch 4, in dem das Peptid durch Folgendes chemisch modifiziert ist: Modifikationen an Seitenketten, Einbau einer Schutzgruppe, Einbau von nichtnatürlichen Aminosäuren und/oder ihren Derivaten während der Peptidsynthese und die Verwendung von Vernetzern und anderen Verfahren, die dem Peptid eine konformative Einschränkung auferlegen.

6. Konjugat, das ein Peptid nach einem vorstehenden Anspruch umfasst, das mit einem Bindungspartner konjugiert, verknüpft oder fusioniert ist, in dem der Bindungspartner optional aus einem Polyethylenglycolpolymer, einer das Molekulargewicht erhöhenden Verbindung, einer lipophilen Gruppe oder einem Antikörpermolekül ausgewählt ist.

7. Zusammensetzung, die ein Peptid nach einem der Ansprüche 1 bis 5 oder ein Konjugat nach Anspruch 6 umfasst.

8. Zusammensetzung, die ein Peptid nach einem der Ansprüche 1 bis 3 umfasst.

9. Zusammensetzung nach einem der Ansprüche 7 oder 8, in der die Zusammensetzung ein Pulver ist.

10. Zusammensetzung nach einem der Ansprüche 7 oder 8, in der die Zusammensetzung zur topischen Applikation auf die Haut eines Menschen formuliert ist.

11. Zusammensetzung nach Anspruch 10 in der Form eines Gel, einer Creme, Lotion oder Salbe.

12. Nahrungsmittel oder Getränk, Nahrungsergänzungsmittel, Körperpflegezusammensetzung oder pharmazeutische Zusammensetzung, das/die ein Peptid nach einem der Ansprüche 1 bis 5 umfasst.

13. Nicht-therapeutisches Verfahren zur Vorbeugung oder Hemmung des Alterns der menschlichen Haut, das einen Schritt der topischen Verabreichung auf die Haut eines wachstumsfördernden Peptids nach Anspruch 1 oder 2, einer Variante nach einem der Ansprüche 3-5 oder einer Zusammensetzung nach einem der Ansprüche 7-9 umfasst.

14. Topische Zusammensetzung nach Anspruch 10 oder 11 für die Verwendung bei der Behandlung einer Wunde bei einem Säuger, wobei die Zusammensetzung topisch auf die Wunde des Säugers appliziert wird.

## Revendications

1. Peptide de SEQUENCE ID NO : 283.

2. Variant, favorisant la croissance, d'un peptide selon la revendication 1, présentant de 1 à 3 altérations d'acides aminés par rapport au peptide selon la revendication 1, dans lequel la ou chaque altération d'acide aminé est choisie parmi une insertion, une addition, une délétion et une substitution d'un acide aminé.

3. Peptide ayant jusqu'à 50 acides aminés et comprenant un peptide selon la revendication 1 ou 2, le peptide étant un peptide favorisant la croissance.

4. Peptide selon l'une quelconque des revendications précédentes, qui est modifié chimiquement, le peptide étant un peptide favorisant la croissance.

5. Peptide selon la revendication 4, le peptide étant modifié chimiquement par des modifications apportées aux chaînes latérales, l'incorporation d'un groupement protecteur, l'incorporation d'acides aminés non naturels et/ou de leurs dérivés lors de la synthèse peptidique, et l'utilisation d'agents de réticulation et d'autres méthodes qui imposent une contrainte conformationnelle sur le peptide.

6. Conjugué comprenant un peptide selon l'une quelconque des revendications précédentes, qui est conjugué, lié ou fusionné avec un partenaire de liaison, le partenaire de liaison étant éventuellement choisi parmi un polymère de polyéthylène glycol, un composé d'augmentation du poids moléculaire, un groupement lipophile, ou une molécule d'anticorps.

7. Composition comprenant un peptide selon l'une quelconque des revendications 1 à 5, ou un conjugué selon la revendication 6.

8. Composition comprenant un peptide selon l'une quelconque des revendications 1 à 3.

9. Composition selon l'une quelconque des revendications 7 ou 8, la composition étant une poudre.

10. Composition selon l'une quelconque des revendications 7 ou 8, la composition étant formulée pour une application topique sur la peau d'un être humain.

11. Composition selon la revendication 10, sous la forme d'un gel, d'une crème, d'une lotion ou d'un onguent.

12. Aliment ou boisson, complément nutritionnel, composition de soins personnels, ou composition pharmaceutique, comprenant un peptide selon l'une quelconque des revendications 1 à 5.

13. Méthode non thérapeutique de prévention ou d'inhibition du vieillissement de la peau humaine, comprenant une étape d'administration par voie topique à la peau, d'un peptide favorisant la croissance selon la revendication 1 ou 2, d'un variant selon l'une quelconque des revendications 3-5, ou d'une composition selon l'une quelconque des revendications 7-9.

14. Composition topique selon la revendication 10 ou 11, pour une utilisation dans le traitement d'une plaie chez un mammifère, la composition étant appliquée par voie topique à la plaie chez le mammifère.


### SEQUENCE LISTING

<110> NURITAS LIMITED
<120> Cellular growth and proliferation promoting peptides, and uses thereof
<130> P11684PC00
<140> PCT/EP2016/067097
   <141> 2016-07-18
<150> EP15177017.9
   <151> 2015-07-16
<160> 775
<170> PatentIn version 3.5
<210> 1
   <211> 459
   <212> PRT
   <213> Pisum sativum
<400> 1
   Met Ala Ala Thr Thr Met Lys Ala Ser Phe Pro Leu Leu Met Leu Met
   1               5                   10                  15
   Gly Ile Ser Phe Leu Ala Ser Val Cys Val Ser Ser Arg Ser Asp Pro
               20                  25                  30
   Gln Asn Pro Phe Ile Phe Lys Ser Asn Lys Phe Gln Thr Leu Phe Glu
           35                  40                  45
   Asn Glu Asn Gly His Ile Arg Leu Leu Gln Lys Phe Asp Gln Arg Ser
       50                  55                  60
   Lys Ile Phe Glu Asn Leu Gln Asn Tyr Arg Leu Leu Glu Tyr Lys Ser
   65                  70                  75                  80
   Lys Pro His Thr Ile Phe Leu Pro Gln His Thr Asp Ala Asp Tyr Ile
                   85                  90                  95
   Leu Val Val Leu Ser Gly Lys Ala Ile Leu Thr Val Leu Lys Pro Asp
               100                 105                 110
   Asp Arg Asn Ser Phe Asn Leu Glu Arg Gly Asp Thr Ile Lys Leu Pro
           115                 120                 125
   Ala Gly Thr Ile Ala Tyr Leu Val Asn Arg Asp Asp Asn Glu Glu Leu
       130                 135                 140
   Arg Val Leu Asp Leu Ala Ile Pro Val Asn Arg Pro Gly Gln Leu Gln
   145 150 155 160
   Ser Phe Leu Leu Ser Gly Asn Gln Asn Gln Gln Asn Tyr Leu Ser Gly
   165 170 175
   Phe Ser Lys Asn Ile Leu Glu Ala Ser Phe Asn Thr Asp Tyr Glu Glu
   180 185 190
   Ile Glu Lys Val Leu Leu Glu Glu His Glu Lys Glu Thr Gln His Arg
   195 200 205
   Arg Ser Leu Lys Asp Lys Arg Gln Gln Ser Gln Glu Glu Asn Val Ile
   210 215 220
   Val Lys Leu Ser Arg Gly Gln Ile Glu Glu Leu Ser Lys Asn Ala Lys
   225 230 235 240
   Ser Thr Ser Lys Lys Ser Val Ser Ser Glu Ser Glu Pro Phe Asn Leu
   245 250 255
   Arg Ser Arg Gly Pro Ile Tyr Ser Asn Glu Phe Gly Lys Phe Phe Glu
   260 265 270
   Ile Thr Pro Glu Lys Asn Pro Gln Leu Gln Asp Leu Asp Ile Phe Val
   275 280 285
   Asn Ser Val Glu Ile Lys Glu Gly Ser Leu Leu Leu Pro His Tyr Asn
   290 295 300
   Ser Arg Ala Ile Val Ile Val Thr Val Asn Glu Gly Lys Gly Asp Phe
   305 310 315 320
   Glu Leu Val Gly Gln Arg Asn Glu Asn Gln Gln Glu Gln Arg Lys Glu
   325 330 335
   Asp Asp Glu Glu Glu Glu Gln Gly Glu Glu Glu Ile Asn Lys Gln Val
   340 345 350
   Gln Asn Tyr Lys Ala Lys Leu Ser Ser Gly Asp Val Phe Val Ile Pro
   355 360 365
   Ala Gly His Pro Val Ala Val Lys Ala Ser Ser Asn Leu Asp Leu Leu
   370 375 380
   Gly Phe Gly Ile Asn Ala Glu Asn Asn Gln Arg Asn Phe Leu Ala Gly
   385 390 395 400
   Asp Glu Asp Asn Val Ile Ser Gln Ile Gln Arg Pro Val Lys Glu Leu
   405 410 415
   Ala Phe Pro Gly Ser Ala Gln Glu Val Asp Arg Ile Leu Glu Asn Gln
   420 425 430
   Lys Gln Ser His Phe Ala Asp Ala Gln Pro Gln Gln Arg Glu Arg Gly
   435 440 445
   Ser Arg Glu Thr Arg Asp Arg Leu Ser Ser Val
   450 455
<210> 2
   <211> 613
   <212> PRT
   <213> Pisum sativum
<400> 2
   Met Ala Thr Thr Ile Lys Ser Arg Phe Pro Leu Leu Leu Leu Leu Gly
   1 5 10 15
   Ile Ile Phe Leu Ala Ser Val Val Cys Val Thr Tyr Ala Asn Tyr Asp
   20 25 30
   Glu Gly Ser Glu Pro Arg Val Pro Ala Gln Arg Glu Arg Gly Arg Gln
   35 40 45
   Glu Gly Glu Lys Glu Glu Lys Arg His Gly Glu Trp Arg Pro Ser Tyr
   50 55 60
   Glu Lys Glu Glu Asp Glu Glu Glu Gly Gln Arg Glu Arg Gly Arg Gln
   65 70 75 80
   Glu Gly Glu Lys Glu Glu Lys Arg His Gly Glu Trp Arg Pro Ser Tyr
   85 90 95
   Glu Lys Gln Glu Asp Glu Glu Glu Lys Gln Lys Tyr Arg Tyr Gln Arg
   100 105 110
   Glu Lys Glu Asp Glu Glu Glu Lys Gln Lys Tyr Gln Tyr Gln Arg Glu
   115 120 125
   Lys Lys Glu Gln Lys Glu Val Gln Pro Gly Arg Glu Arg Trp Glu Arg
   130 135 140
   Glu Glu Asp Glu Glu Gln Val Asp Glu Glu Trp Arg Gly Ser Gln Arg
   145 150 155 160
   Arg Glu Asp Pro Glu Glu Arg Ala Arg Leu Arg His Arg Glu Glu Arg
                   165                 170                 175
   Thr Lys Arg Asp Arg Arg His Gln Arg Glu Gly Glu Glu Glu Glu Arg
               180                 185                 190
   Ser Ser Glu Ser Gln Glu Arg Arg Asn Pro Phe Leu Phe Lys Ser Asn
           195                 200                 205
   Lys Phe Leu Thr Leu Phe Glu Asn Glu Asn Gly His Ile Arg Leu Leu
       210                 215                 220
   Gln Arg Phe Asp Lys Arg Ser Asp Leu Phe Glu Asn Leu Gln Asn Tyr
   225                 230                 235                 240
   Arg Leu Val Glu Tyr Arg Ala Lys Pro His Thr Ile Phe Leu Pro Gln
                   245                 250                 255
   His Ile Asp Ala Asp Leu Ile Leu Val Val Leu Ser Gly Lys Ala Ile
               260                 265                 270
   Leu Thr Val Leu Ser Pro Asn Asp Arg Asn Ser Tyr Asn Leu Glu Arg
           275                 280                 285
   Gly Asp Thr Ile Lys Leu Pro Ala Gly Thr Thr Ser Tyr Leu Val Asn
       290                 295                 300
   Gln Asp Asp Glu Glu Asp Leu Arg Leu Val Asp Leu Val Ile Pro Val
   305                 310                 315                 320
   Asn Gly Pro Gly Lys Phe Glu Ala Phe Asp Leu Ala Lys Asn Lys Asn
                   325                 330                 335
   Gln Tyr Leu Arg Gly Phe Ser Lys Asn Ile Leu Glu Ala Ser Tyr Asn
               340                 345                 350
   Thr Arg Tyr Glu Thr Ile Glu Lys Val Leu Leu Glu Glu Gln Glu Lys
           355                 360                 365
   Asp Arg Lys Arg Arg Gln Gln Gly Glu Glu Thr Asp Ala Ile Val Lys
       370                 375                 380
   Val Ser Arg Glu Gln Ile Glu Glu Leu Lys Lys Leu Ala Lys Ser Ser
   385                 390                 395                 400
   Ser Lys Lys Ser Leu Pro Ser Glu Phe Glu Pro Ile Asn Leu Arg Ser
                   405                 410                 415
   His Lys Pro Glu Tyr Ser Asn Lys Phe Gly Lys Leu Phe Glu Ile Thr
   420 425 430
   Pro Glu Lys Lys Tyr Pro Gln Leu Gln Asp Leu Asp Leu Phe Val Ser
   435 440 445
   Cys Val Glu Ile Asn Glu Gly Ala Leu Met Leu Pro His Tyr Asn Ser
   450 455 460
   Arg Ala Ile Val Val Leu Leu Val Asn Glu Gly Lys Gly Asn Leu Glu
   465 470 475 480
   Leu Leu Gly Leu Lys Asn Glu Gln Gln Glu Arg Glu Asp Arg Lys Glu
   485 490 495
   Arg Asn Asn Glu Val Gln Arg Tyr Glu Ala Arg Leu Ser Pro Gly Asp
   500 505 510
   Val Val Ile Ile Pro Ala Gly His Pro Val Ala Ile Thr Ala Ser Ser
   515 520 525
   Asn Leu Asn Leu Leu Gly Phe Gly Ile Asn Ala Glu Asn Asn Glu Arg
   530 535 540
   Asn Phe Leu Ser Gly Ser Asp Asp Asn Val Ile Ser Gln Ile Glu Asn
   545 550 555 560
   Pro Val Lys Glu Leu Thr Phe Pro Gly Ser Val Gln Glu Ile Asn Arg
   565 570 575
   Leu Ile Lys Asn Gln Lys Gln Ser His Phe Ala Asn Ala Glu Pro Glu
   580 585 590
   Gln Lys Glu Gln Gly Ser Gln Gly Lys Arg Ser Pro Leu Ser Ser Ile
   595 600 605
   Leu Gly Thr Phe Tyr
   610
<210> 3
   <211> 609
   <212> PRT
   <213> Oryza sativa
<400> 3 Pro
<210> 4
   <211> 499
   <212> PRT
   <213> Oryza sativa
<400> 4
   Met Ala Ser Ser Val Phe Ser Arg Phe Ser Ile Tyr Phe Cys Val Leu
   1 5 10 15
   Leu Leu Cys His Gly Ser Met Ala Gln Leu Phe Asn Pro Ser Thr Asn
   20 25 30
   Pro Trp His Ser Pro Arg Gln Gly Ser Phe Arg Glu Cys Arg Phe Asp
   35 40 45
   Arg Leu Gln Ala Phe Glu Pro Leu Arg Lys Val Arg Ser Glu Ala Gly
   50 55 60
   Val Thr Glu Tyr Phe Asp Glu Lys Asn Glu Leu Phe Gln Cys Thr Gly
   65 70 75 80
   Thr Phe Val Ile Arg Arg Val Ile Gln Pro Gln Gly Leu Leu Val Pro
   85 90 95
   Arg Tyr Thr Asn Ile Pro Gly Val Val Tyr Ile Ile Gln Gly Arg Gly
   100 105 110
   Ser Met Gly Leu Thr Phe Pro Gly Cys Pro Ala Thr Tyr Gln Gln Gln
   115 120 125
   Phe Gln Gln Phe Ser Ser Gln Gly Gln Ser Gln Ser Gln Lys Phe Arg
   130 135 140
   Asp Glu His Gln Lys Ile His Gln Phe Arg Gln Gly Asp Ile Val Ala
   145 150 155 160
   Leu Pro Ala Gly Val Ala His Trp Phe Tyr Asn Asp Gly Asp Ala Pro
   165 170 175
   Ile Val Ala Val Tyr Val Tyr Asp Val Asn Asn Asn Ala Asn Gln Leu
   180 185 190
   Glu Pro Arg Gln Lys Glu Phe Leu Leu Ala Gly Asn Asn Asn Arg Ala
   195 200 205
   Gln Gln Gln Gln Val Tyr Gly Ser Ser Ile Glu Gln His Ser Gly Gln
   210 215 220
   Asn Ile Phe Ser Gly Phe Gly Val Glu Met Leu Ser Glu Ala Leu Gly
   225 230 235 240
   Ile Asn Ala Val Ala Ala Lys Arg Leu Gln Ser Gln Asn Asp Gln Arg
   245 250 255
   Gly Glu Ile Ile His Val Lys Asn Gly Leu Gln Leu Leu Lys Pro Thr
   260 265 270
   Leu Thr Gln Gln Gln Glu Gln Ala Gln Ala Gln Asp Gln Tyr Gln Gln
   275 280 285
   Val Gln Tyr Ser Glu Arg Gln Gln Thr Ser Ser Arg Trp Asn Gly Leu
   290 295 300
   Glu Glu Asn Phe Cys Thr Ile Lys Val Arg Val Asn Ile Glu Asn Pro
   305 310 315 320
   Ser Arg Ala Asp Ser Tyr Asn Pro Arg Ala Gly Arg Ile Thr Ser Val
   325 330 335
   Asn Ser Gln Lys Phe Pro Ile Leu Asn Leu Ile Gln Met Ser Ala Thr
   340 345 350
   Arg Val Asn Leu Tyr Gln Asn Ala Ile Leu Ser Pro Phe Trp Asn Val
   355 360 365
   Asn Ala His Ser Leu Val Tyr Met Ile Gln Gly Arg Ser Arg Val Gln
       370                 375                 380
   Val Val Ser Asn Phe Gly Lys Thr Val Phe Asp Gly Val Leu Arg Pro
   385                 390                 395                 400
   Gly Gln Leu Leu Ile Ile Pro Gln His Tyr Ala Val Leu Lys Lys Ala
                   405                 410                 415
   Glu Arg Glu Gly Cys Gln Tyr Ile Ala Ile Lys Thr Asn Ala Asn Ala
               420                 425                 430
   Phe Val Ser His Leu Ala Gly Lys Asn Ser Val Phe Arg Ala Leu Pro
           435                 440                 445
   Val Asp Val Val Ala Asn Ala Tyr Arg Ile Ser Arg Glu Gln Ala Arg
       450                 455                 460
   Ser Leu Lys Asn Asn Arg Gly Glu Glu His Gly Ala Phe Thr Pro Arg
   465                 470                 475                 480
   Phe Gln Gln Gln Tyr Tyr Pro Gly Leu Ser Asn Glu Ser Glu Ser Glu
                   485                 490                 495
   Thr Ser Glu
<210> 5
   <211> 186
   <212> PRT
   <213> Oryza sativa
<400> 5
   Met Ala Ser Lys Val Val Phe Phe Ala Ala Ala Leu Met Ala Ala Met
   1               5                   10                  15
   Val Ala Ile Ser Gly Ala Gln Leu Ser Glu Ser Glu Met Arg Phe Arg
               20                  25                  30
   Asp Arg Gln Cys Gln Arg Glu Val Gln Asp Ser Pro Leu Asp Ala Cys
           35                  40                  45
   Arg Gln Val Leu Asp Arg Gln Leu Thr Gly Arg Glu Arg Phe Gln Pro
       50                  55                  60
   Met Phe Arg Arg Pro Gly Ala Leu Gly Leu Arg Met Gln Cys Cys Gln
   65                  70                  75                  80
   Gln Leu Gln Asp Val Ser Arg Glu Cys Arg Cys Ala Ala Ile Arg Arg
   85 90 95
   Met Val Arg Ser Tyr Glu Glu Ser Met Pro Met Pro Leu Glu Gln Gly
   100 105 110
   Trp Ser Ser Ser Ser Ser Glu Tyr Tyr Gly Gly Glu Gly Ser Ser Ser
   115 120 125
   Glu Gln Gly Tyr Tyr Gly Glu Gly Ser Ser Glu Glu Gly Tyr Tyr Gly
   130 135 140
   Glu Gln Gln Gln Gln Pro Gly Met Thr Arg Val Arg Leu Thr Arg Ala
   145 150 155 160
   Arg Gln Tyr Ala Ala Gln Leu Pro Ser Met Cys Arg Val Glu Pro Gln
   165 170 175
   Gln Cys Ser Ile Phe Ala Ala Gly Gln Tyr
   180 185
<210> 6
   <211> 500
   <212> PRT
   <213> Oryza sativa
<400> 6
   Met Ala Thr Ile Ala Phe Ser Arg Leu Ser Ile Tyr Phe Cys Val Leu
   1 5 10 15
   Leu Leu Cys His Gly Ser Met Ala Gln Leu Phe Gly Pro Asn Val Asn
   20 25 30
   Pro Trp His Asn Pro Arg Gln Gly Gly Phe Arg Glu Cys Arg Phe Asp
   35 40 45
   Arg Leu Gln Ala Phe Glu Pro Leu Arg Arg Val Arg Ser Glu Ala Gly
   50 55 60
   Val Thr Glu Tyr Phe Asp Glu Lys Asn Glu Gln Phe Gln Cys Thr Gly
   65 70 75 80
   Thr Phe Val Ile Arg Arg Val Ile Glu Pro Gln Gly Leu Leu Val Pro
   85 90 95
   Arg Tyr Ser Asn Thr Pro Gly Met Val Tyr Ile Ile Gln Gly Arg Gly
   100 105 110
   Ser Met Gly Leu Thr Phe Pro Gly Cys Pro Ala Thr Tyr Gln Gln Gln
           115                 120                 125
   Phe Gln Gln Phe Leu Pro Glu Gly Gln Ser Gln Ser Gln Lys Phe Arg
       130                 135                 140
   Asp Glu His Gln Lys Ile His Gln Phe Arg Gln Gly Asp Ile Val Ala
   145                 150                 155                 160
   Leu Pro Ala Gly Val Ala His Trp Phe Tyr Asn Glu Gly Asp Ala Pro
                   165                 170                 175
   Val Val Ala Leu Tyr Val Phe Asp Leu Asn Asn Asn Ala Asn Gln Leu
               180                 185                 190
   Glu Pro Arg Gln Lys Glu Phe Leu Leu Ala Gly Asn Asn Asn Arg Glu
           195                 200                 205
   Gln Gln Met Tyr Gly Arg Ser Ile Glu Gln His Ser Gly Gln Asn Ile
       210                 215                 220
   Phe Ser Gly Phe Asn Asn Glu Leu Leu Ser Glu Ala Leu Gly Val Asn
   225                 230                 235                 240
   Ala Leu Val Ala Lys Arg Leu Gln Gly Gln Asn Asp Gln Arg Gly Glu
                   245                 250                 255
   Ile Ile Arg Val Lys Asn Gly Leu Lys Leu Leu Arg Pro Ala Phe Ala
               260                 265                 270
   Gln Gln Gln Glu Gln Ala Gln Gln Gln Glu Gln Ala Gln Ala Gln Tyr
           275                 280                 285
   Gln Val Gln Tyr Ser Glu Glu Gln Gln Pro Ser Thr Arg Cys Asn Gly
       290                 295                 300
   Leu Asp Glu Asn Phe Cys Thr Ile Lys Ala Arg Leu Asn Ile Glu Asn
   305                 310                 315                 320
   Pro Ser His Ala Asp Thr Tyr Asn Pro Arg Ala Gly Arg Ile Thr Arg
                   325                 330                 335
   Leu Asn Ser Gln Lys Phe Pro Ile Leu Asn Leu Val Gln Leu Ser Ala
               340                 345                 350
   Thr Arg Val Asn Leu Tyr Gln Asn Ala Ile Leu Ser Pro Phe Trp Asn
           355                 360                 365
   Val Asn Ala His Ser Leu Val Tyr Ile Val Gln Gly His Ala Arg Val
   370 375 380
   Gln Val Val Ser Asn Leu Gly Lys Thr Val Phe Asn Gly Val Leu Arg
   385 390 395 400
   Pro Gly Gln Leu Leu Ile Ile Pro Gln His Tyr Val Val Leu Lys Lys
   405 410 415
   Ala Glu His Glu Gly Cys Gln Tyr Ile Ser Phe Lys Thr Asn Ala Asn
   420 425 430
   Ser Met Val Ser His Leu Ala Gly Lys Asn Ser Ile Phe Arg Ala Met
   435 440 445
   Pro Val Asp Val Ile Ala Asn Ala Tyr Arg Ile Ser Arg Glu Gln Ala
   450 455 460
   Arg Ser Leu Lys Asn Asn Arg Gly Glu Glu Leu Gly Ala Phe Thr Pro
   465 470 475 480
   Arg Tyr Gln Gln Gln Thr Tyr Pro Gly Phe Ser Asn Glu Ser Glu Asn
   485 490 495
   Glu Ala Leu Glu
   500
<210> 7
   <211> 861
   <212> PRT
   <213> Pisum sativum
<400> 7
   Met Phe Ser Gly Val Thr Gly Ile Leu Asn Arg Gly His Lys Ile Lys
   1 5 10 15
   Gly Thr Val Val Leu Met Arg Lys Asn Val Leu Asp Ile Asn Ser Leu
   20 25 30
   Thr Thr Val Gly Gly Val Ile Gly Gln Gly Phe Asp Ile Leu Gly Ser
   35 40 45
   Thr Val Asp Asn Leu Thr Ala Phe Leu Gly Arg Ser Val Ser Leu Gln
   50 55 60
   Leu Ile Ser Ala Thr Lys Pro Asp Ala Thr Gly Lys Gly Lys Leu Gly
   65 70 75 80
   Lys Ala Thr Phe Leu Glu Gly Ile Ile Ser Ser Leu Pro Thr Leu Gly
                   85                  90                  95
   Ala Gly Gln Ser Ala Phe Lys Ile His Phe Glu Trp Asp Asp Asp Met
               100                 105                 110
   Gly Ile Pro Gly Ala Phe Tyr Ile Lys Asn Phe Met Gln Thr Glu Phe
           115                 120                 125
   Phe Leu Val Ser Leu Thr Leu Asp Asp Ile Pro Asn His Gly Ser Ile
       130                 135                 140
   Tyr Phe Val Cys Asn Ser Trp Ile Tyr Asn Ala Lys His His Lys Ile
   145                 150                 155                 160
   Asp Arg Ile Phe Phe Ala Asn Gln Thr Tyr Leu Pro Ser Glu Thr Pro
                   165                 170                 175
   Ala Pro Leu Val His Tyr Arg Glu Glu Glu Leu Asn Asn Leu Arg Gly
               180                 185                 190
   Asp Gly Thr Gly Glu Arg Lys Glu Trp Glu Arg Ile Tyr Asp Tyr Asp
           195                 200                 205
   Val Tyr Asn Asp Leu Gly Asn Pro Asp Ser Gly Glu Asn His Ala Arg
       210                 215                 220
   Pro Val Leu Gly Gly Ser Glu Thr Tyr Pro Tyr Pro Arg Arg Gly Arg
   225                 230                 235                 240
   Thr Gly Arg Lys Pro Thr Arg Lys Asp Pro Asn Ser Glu Ser Arg Ser
                   245                 250                 255
   Asp Tyr Val Tyr Leu Pro Arg Asp Glu Ala Phe Gly His Leu Lys Ser
               260                 265                 270
   Ser Asp Phe Leu Thr Tyr Gly Leu Lys Ala Val Ser Gln Asn Val Val
           275                 280                 285
   Pro Ala Leu Glu Ser Val Phe Phe Asp Leu Asn Phe Thr Pro Asn Glu
       290                 295                 300
   Phe Asp Ser Phe Asp Glu Val His Gly Leu Tyr Glu Gly Gly Ile Lys
   305                 310                 315                 320
   Leu Pro Thr Asn Ile Leu Ser Gln Ile Ser Pro Leu Pro Val Leu Lys
                   325                 330                 335
   Glu Ile Phe Arg Thr Asp Gly Glu Asn Thr Leu Lys Tyr Pro Pro Pro
   340 345 350
   Lys Val Ile Gln Val Ser Arg Ser Gly Trp Met Thr Asp Glu Glu Phe
   355 360 365
   Ala Arg Glu Met Leu Ala Gly Val Asn Pro Asn Val Ile Cys Cys Leu
   370 375 380
   Gln Glu Phe Pro Pro Arg Ser Lys Leu Asp Ser Gln Ile Tyr Gly Asp
   385 390 395 400
   His Thr Ser Lys Ile Ser Lys Glu His Leu Glu Pro Asn Leu Glu Gly
   405 410 415
   Leu Thr Val Glu Glu Ala Ile Gln Asn Lys Lys Leu Phe Leu Leu Asp
   420 425 430
   His His Asp Ser Ile Met Pro Tyr Leu Arg Arg Ile Asn Ser Thr Ser
   435 440 445
   Thr Lys Ala Tyr Ala Thr Arg Thr Ile Leu Phe Leu Asn Asn Asn Gln
   450 455 460
   Asn Leu Lys Pro Leu Ala Ile Glu Leu Ser Leu Pro His Pro Gln Gly
   465 470 475 480
   Asp Glu His Gly Ala Val Ser Tyr Val Tyr Gln Pro Ala Leu Glu Gly
   485 490 495
   Val Glu Ser Ser Ile Trp Leu Leu Ala Lys Ala Tyr Val Ile Val Asn
   500 505 510
   Asp Ser Cys Tyr His Gln Leu Val Ser His Trp Leu Asn Thr His Ala
   515 520 525
   Val Val Glu Pro Phe Val Ile Ala Thr Asn Arg His Leu Ser Cys Leu
   530 535 540
   His Pro Ile Tyr Lys Leu Leu Tyr Pro His Tyr Arg Asp Thr Met Asn
   545 550 555 560
   Ile Asn Ser Leu Ala Arg Leu Ser Leu Val Asn Asp Gly Gly Ile Ile
   565 570 575
   Glu Lys Thr Phe Leu Trp Gly Arg Tyr Ser Met Glu Met Ser Ser Lys
   580 585 590
   Val Tyr Lys Asn Trp Val Phe Thr Glu Gln Ala Leu Pro Ala Asp Leu
   595 600 605
   Ile Lys Arg Gly Met Ala Ile Glu Asp Pro Ser Ser Pro Cys Gly Val
   610 615 620
   Lys Leu Val Val Glu Asp Tyr Pro Tyr Ala Val Asp Gly Leu Glu Ile
   625 630 635 640
   Trp Ala Ile Ile Lys Thr Trp Val Gln Asp Tyr Val Ser Leu Tyr Tyr
   645 650 655
   Thr Ser Asp Glu Lys Leu Arg Gln Asp Ser Glu Leu Gln Ala Trp Trp
   660 665 670
   Lys Glu Leu Val Glu Val Gly His Gly Asp Lys Lys Asn Glu Pro Trp
   675 680 685
   Trp Pro Lys Met Gln Thr Arg Glu Asp Leu Ile Glu Val Cys Ser Ile
   690 695 700
   Val Ile Trp Thr Ala Ser Ala Leu His Ala Ala Val Asn Phe Gly Gln
   705 710 715 720
   Tyr Ser Tyr Gly Gly Leu Ile Leu Asn Arg Pro Thr Leu Ser Arg Arg
   725 730 735
   Phe Met Pro Glu Lys Gly Ser Ala Glu Phe Glu Glu Leu Val Lys Ser
   740 745 750
   Pro Gln Lys Ala Tyr Leu Lys Thr Ile Thr Pro Lys Phe Gln Thr Leu
   755 760 765
   Ile Asp Leu Ser Val Ile Glu Ile Leu Ser Arg His Ala Ser Asp Glu
   770 775 780
   Leu Tyr Leu Gly Glu Arg Asp Asn Pro Asn Trp Thr Ser Asp Lys Arg
   785 790 795 800
   Ala Leu Glu Ala Phe Lys Lys Phe Gly Asn Lys Leu Ala Glu Ile Glu
   805 810 815
   Lys Lys Leu Thr Gln Arg Asn Asn Asp Glu Lys Leu Arg Asn Arg His
   820 825 830
   Gly Pro Val Glu Met Pro Tyr Thr Leu Leu Tyr Pro Ser Ser Lys Glu
   835 840 845
   Gly Leu Thr Phe Arg Gly Ile Pro Asn Ser Ile Ser Ile
   850 855 860
<210> 8
   <211> 517
   <212> PRT
   <213> Pisum sativum
<400> 8
   Met Ala Lys Leu Leu Ala Leu Ser Leu Ser Phe Cys Phe Leu Leu Leu
   1 5 10 15
   Gly Gly Cys Phe Ala Leu Arg Glu Gln Pro Gln Gln Asn Glu Cys Gln
   20 25 30
   Leu Glu Arg Leu Asp Ala Leu Glu Pro Asp Asn Arg Ile Glu Ser Glu
   35 40 45
   Gly Gly Leu Ile Glu Thr Trp Asn Pro Asn Asn Lys Gln Phe Arg Cys
   50 55 60
   Ala Gly Val Ala Leu Ser Arg Ala Thr Leu Gln Arg Asn Ala Leu Arg
   65 70 75 80
   Arg Pro Tyr Tyr Ser Asn Ala Pro Gln Glu Ile Phe Ile Gln Gln Gly
   85 90 95
   Asn Gly Tyr Phe Gly Met Val Phe Pro Gly Cys Pro Glu Thr Phe Glu
   100 105 110
   Glu Pro Gln Glu Ser Glu Gln Gly Glu Gly Arg Arg Tyr Arg Asp Arg
   115 120 125
   His Gln Lys Val Asn Arg Phe Arg Glu Gly Asp Ile Ile Ala Val Pro
   130 135 140
   Thr Gly Ile Val Phe Trp Met Tyr Asn Asp Gln Asp Thr Pro Val Ile
   145 150 155 160
   Ala Val Ser Leu Thr Asp Ile Arg Ser Ser Asn Asn Gln Leu Asp Gln
   165 170 175
   Met Pro Arg Arg Phe Tyr Leu Ala Gly Asn His Glu Gln Glu Phe Leu
   180 185 190
   Gln Tyr Gln His Gln Gln Gly Gly Lys Gln Glu Gln Glu Asn Glu Gly
           195                 200                 205
   Asn Asn Ile Phe Ser Gly Phe Lys Arg Asp Tyr Leu Glu Asp Ala Phe
       210                 215                 220
   Asn Val Asn Arg His Ile Val Asp Arg Leu Gln Gly Arg Asn Glu Asp
   225                 230                 235                 240
   Glu Glu Lys Gly Ala Ile Val Lys Val Lys Gly Gly Leu Ser Ile Ile
                   245                 250                 255
   Ser Pro Pro Glu Lys Gln Ala Arg His Gln Arg Gly Ser Arg Gln Glu
               260                 265                 270
   Glu Asp Glu Asp Glu Glu Lys Gln Pro Arg His Gln Arg Gly Ser Arg
           275                 280                 285
   Gln Glu Glu Glu Glu Asp Glu Asp Glu Glu Arg Gln Pro Arg His Gln
       290                 295                 300
   Arg Arg Arg Gly Glu Glu Glu Glu Glu Asp Lys Lys Glu Arg Gly Gly
   305                 310                 315                 320
   Ser Gln Lys Gly Lys Ser Arg Arg Gln Gly Asp Asn Gly Leu Glu Glu
                   325                 330                 335
   Thr Val Cys Thr Ala Lys Leu Arg Leu Asn Ile Gly Pro Ser Ser Ser
               340                 345                 350
   Pro Asp Ile Tyr Asn Pro Glu Ala Gly Arg Ile Lys Thr Val Thr Ser
           355                 360                 365
   Leu Asp Leu Pro Val Leu Arg Trp Leu Lys Leu Ser Ala Glu His Gly
       370                 375                 380
   Ser Leu His Lys Asn Ala Met Phe Val Pro His Tyr Asn Leu Asn Ala
   385                 390                 395                 400
   Asn Ser Ile Ile Tyr Ala Leu Lys Gly Arg Ala Arg Leu Gln Val Val
                   405                 410                 415
   Asn Cys Asn Gly Asn Thr Val Phe Asp Gly Glu Leu Glu Ala Gly Arg
               420                 425                 430
   Ala Leu Thr Val Pro Gln Asn Tyr Ala Val Ala Ala Lys Ser Leu Ser
           435                 440                 445
   Asp Arg Phe Ser Tyr Val Ala Phe Lys Thr Asn Asp Arg Ala Gly Ile
   450 455 460
   Ala Arg Leu Ala Gly Thr Ser Ser Val Ile Asn Asn Leu Pro Leu Asp
   465 470 475 480
   Val Val Ala Ala Thr Phe Asn Leu Gln Arg Asn Glu Ala Arg Gln Leu
   485 490 495
   Lys Ser Asn Asn Pro Phe Lys Phe Leu Val Pro Ala Arg Glu Ser Glu
   500 505 510
   Asn Arg Ala Ser Ala
   515
<210> 9
   <211> 275
   <212> PRT
   <213> Pisum sativum
<400> 9
   Asp Asn Ala Glu Ile Glu Lys Ile Leu Leu Glu Glu His Glu Lys Glu
   1 5 10 15
   Thr His His Arg Arg Gly Leu Arg Asp Lys Arg Gln Gln Ser Gln Glu
   20 25 30
   Lys Asn Val Ile Val Lys Val Ser Lys Lys Gln Ile Glu Glu Leu Ser
   35 40 45
   Lys Asn Ala Lys Ser Ser Ser Lys Lys Ser Val Ser Ser Arg Ser Glu
   50 55 60
   Pro Phe Asn Leu Lys Ser Ser Asp Pro Ile Tyr Ser Asn Gln Tyr Gly
   65 70 75 80
   Lys Phe Phe Glu Ile Thr Pro Lys Lys Asn Pro Gln Leu Gln Asp Leu
   85 90 95
   Asp Ile Phe Val Asn Tyr Val Glu Ile Lys Glu Gly Ser Leu Trp Leu
   100 105 110
   Pro His Tyr Asn Ser Arg Ala Ile Val Ile Val Thr Val Asn Glu Gly
   115 120 125
   Lys Gly Asp Phe Glu Leu Val Gly Gln Arg Asn Glu Asn Gln Gln Gly
   130 135 140
   Leu Arg Glu Glu Asp Asp Glu Glu Glu Glu Gln Arg Glu Glu Glu Thr
   145                 150                 155                 160
   Lys Asn Gln Val Gln Ser Tyr Lys Ala Lys Leu Thr Pro Gly Asp Val
                   165                 170                 175
   Phe Val Ile Pro Ala Gly His Pro Val Ala Val Arg Ala Ser Ser Asn
               180                 185                 190
   Leu Asn Leu Leu Gly Phe Gly Ile Asn Ala Glu Asn Asn Gln Arg Asn
           195                 200                 205
   Phe Leu Ala Gly Glu Glu Asp Asn Val Ile Ser Gln Ile Gln Lys Gln
       210                 215                 220
   Val Lys Asp Leu Thr Phe Pro Gly Ser Ala Gln Glu Val Asp Arg Leu
   225                 230                 235                 240
   Leu Glu Asn Gln Lys Gln Ser Tyr Phe Ala Asn Ala Gln Pro Gln Gln
                   245                 250                 255
   Arg Glu Thr Arg Ser Gln Glu Ile Lys Glu His Leu Tyr Ser Ile Leu
               260                 265                 270
   Gly Ala Phe
           275
<210> 10
   <211> 438
   <212> PRT
   <213> Pisum sativum
<400> 10
   Met Ala Ala Thr Pro Ile Lys Pro Leu Met Leu Leu Ala Ile Ala Phe
   1               5                   10                  15
   Leu Ala Ser Val Cys Val Ser Ser Arg Ser Asp Gln Glu Asn Pro Phe
               20                  25                  30
   Ile Phe Lys Ser Asn Arg Phe Gln Thr Leu Tyr Glu Asn Glu Asn Gly
           35                  40                  45
   His Ile Arg Leu Leu Gln Lys Phe Asp Lys Arg Ser Lys Ile Phe Glu
       50                  55                  60
   Asn Leu Gln Asn Tyr Arg Leu Leu Glu Tyr Lys Ser Lys Pro Arg Thr
   65                  70                  75                  80
   Leu Phe Leu Pro Gln Tyr Thr Asp Ala Asp Phe Ile Leu Val Val Leu
                   85                  90                  95
   Ser Gly Lys Ala Thr Leu Thr Val Leu Lys Ser Asn Asp Arg Asn Ser
               100                 105                 110
   Phe Asn Leu Glu Arg Gly Asp Thr Ile Lys Leu Pro Ala Gly Thr Ile
           115                 120                 125
   Ala Tyr Leu Ala Asn Arg Asp Asp Asn Glu Asp Leu Arg Val Leu Asp
       130                 135                 140
   Leu Thr Ile Pro Val Asn Lys Pro Gly Gln Leu Gln Ser Phe Leu Leu
   145                 150                 155                 160
   Ser Gly Thr Gln Asn Gln Pro Ser Leu Leu Ser Gly Phe Ser Lys Asn
                   165                 170                 175
   Ile Leu Glu Ala Ala Phe Asn Thr Asn Tyr Glu Glu Ile Glu Lys Val
               180                 185                 190
   Leu Leu Glu Gln Gln Glu Gln Glu Pro Gln His Arg Arg Ser Leu Lys
           195                 200                 205
   Asp Arg Arg Gln Glu Ile Asn Glu Glu Asn Val Ile Val Lys Val Ser
       210                 215                 220
   Arg Glu Gln Ile Glu Glu Leu Ser Lys Asn Ala Lys Ser Ser Ser Lys
   225                 230                 235                 240
   Lys Ser Val Ser Ser Glu Ser Gly Pro Phe Asn Leu Arg Ser Arg Asn
                   245                 250                 255
   Pro Ile Tyr Ser Asn Lys Phe Gly Lys Phe Phe Glu Ile Thr Pro Glu
               260                 265                 270
   Lys Asn Gln Gln Leu Gln Asp Leu Asp Ile Phe Val Asn Ser Val Asp
           275                 280                 285
   Ile Lys Glu Gly Ser Leu Leu Leu Pro Asn Tyr Asn Ser Arg Ala Ile
       290                 295                 300
   Val Ile Val Thr Val Thr Glu Gly Lys Gly Asp Phe Glu Leu Val Gly
   305                 310                 315                 320
   Gln Arg Asn Glu Asn Gln Gly Lys Glu Asn Asp Lys Glu Glu Glu Gln
                   325                 330                 335
   Glu Glu Glu Thr Ser Lys Gln Val Gln Leu Tyr Arg Ala Lys Leu Ser
   340 345 350
   Pro Gly Asp Val Phe Val Ile Pro Ala Gly His Pro Val Ala Ile Asn
   355 360 365
   Ala Ser Ser Asp Leu Asn Leu Ile Gly Phe Gly Ile Asn Ala Glu Asn
   370 375 380
   Asn Glu Arg Asn Phe Leu Ala Gly Glu Glu Asp Asn Val Ile Ser Gln
   385 390 395 400
   Val Glu Arg Pro Val Lys Glu Leu Ala Phe Pro Gly Ser Ser His Glu
   405 410 415
   Val Asp Arg Leu Leu Lys Asn Gln Lys Gln Ser Tyr Phe Ala Asn Ala
   420 425 430
   Gln Pro Leu Gln Arg Glu
   435
<210> 11
   <211> 499
   <212> PRT
   <213> Oryza sativa
<400> 11
   Met Ala Ser Ile Asn Arg Pro Ile Val Phe Phe Thr Val Cys Leu Phe
   1 5 10 15
   Leu Leu Cys Asn Gly Ser Leu Ala Gln Gln Leu Leu Gly Gln Ser Thr
   20 25 30
   Ser Gln Trp Gln Ser Ser Arg Arg Gly Ser Pro Arg Glu Cys Arg Phe
   35 40 45
   Asp Arg Leu Gln Ala Phe Glu Pro Ile Arg Ser Val Arg Ser Gln Ala
   50 55 60
   Gly Thr Thr Glu Phe Phe Asp Val Ser Asn Glu Gln Phe Gln Cys Thr
   65 70 75 80
   Gly Val Ser Val Val Arg Arg Val Ile Glu Pro Arg Gly Leu Leu Leu
   85 90 95
   Pro His Tyr Thr Asn Gly Ala Ser Leu Val Tyr Ile Ile Gln Gly Arg
   100 105 110
   Gly Ile Thr Gly Pro Thr Phe Pro Gly Cys Pro Glu Ser Tyr Gln Gln
           115                 120                 125
   Gln Phe Gln Gln Ser Gly Gln Ala Gln Leu Thr Glu Ser Gln Ser Gln
       130                 135                 140
   Ser Gln Lys Phe Lys Asp Glu His Gln Lys Ile His Arg Phe Arg Gln
   145                 150                 155                 160
   Gly Asp Val Ile Ala Leu Pro Ala Gly Val Ala His Trp Cys Tyr Asn
                   165                 170                 175
   Asp Gly Glu Val Pro Val Val Ala Ile Tyr Val Thr Asp Leu Asn Asn
               180                 185                 190
   Gly Ala Asn Gln Leu Asp Pro Arg Gln Arg Asp Phe Leu Leu Ala Gly
           195                 200                 205
   Asn Lys Arg Asn Pro Gln Ala Tyr Arg Arg Glu Val Glu Glu Arg Ser
       210                 215                 220
   Gln Asn Ile Phe Ser Gly Phe Ser Thr Glu Leu Leu Ser Glu Ala Leu
   225                 230                 235                 240
   Gly Val Ser Ser Gln Val Ala Arg Gln Leu Gln Cys Gln Asn Asp Gln
                   245                 250                 255
   Arg Gly Glu Ile Val Arg Val Glu His Gly Leu Ser Leu Leu Gln Pro
               260                 265                 270
   Tyr Ala Ser Leu Gln Glu Gln Glu Gln Gly Gln Val Gln Ser Arg Glu
           275                 280                 285
   Arg Tyr Gln Glu Gly Gln Tyr Gln Gln Ser Gln Tyr Gly Ser Gly Cys
       290                 295                 300
   Ser Asn Gly Leu Asp Glu Thr Phe Cys Thr Leu Arg Val Arg Gln Asn
   305                 310                 315                 320
   Ile Asp Asn Pro Asn Arg Ala Asp Thr Tyr Asn Pro Arg Ala Gly Arg
                   325                 330                 335
   Val Thr Asn Leu Asn Thr Gln Asn Phe Pro Ile Leu Ser Leu Val Gln
               340                 345                 350
   Met Ser Ala Val Lys Val Asn Leu Tyr Gln Asn Ala Leu Leu Ser Pro
   355 360 365
   Phe Trp Asn Ile Asn Ala His Ser Val Val Tyr Ile Thr Gln Gly Arg
   370 375 380
   Ala Arg Val Gln Val Val Asn Asn Asn Gly Lys Thr Val Phe Asn Gly
   385 390 395 400
   Glu Leu Arg Arg Gly Gln Leu Leu Ile Ile Pro Gln His Tyr Ala Val
   405 410 415
   Val Lys Lys Ala Gln Arg Glu Gly Cys Ala Tyr Ile Ala Phe Lys Thr
   420 425 430
   Asn Pro Asn Ser Met Val Ser His Ile Ala Gly Lys Ser Ser Ile Phe
   435 440 445
   Arg Ala Leu Pro Asn Asp Val Leu Ala Asn Ala Tyr Arg Ile Ser Arg
   450 455 460
   Glu Glu Ala Gln Arg Leu Lys His Asn Arg Gly Asp Glu Phe Gly Ala
   465 470 475 480
   Phe Thr Pro Ile Gln Tyr Lys Ser Tyr Gln Asp Val Tyr Asn Ala Ala
   485 490 495
   Glu Ser Ser
<210> 12
   <211> 499
   <212> PRT
   <213> Oryza sativa
<400> 12
   Met Ala Ser Ile Asn Arg Pro Ile Val Phe Phe Thr Val Cys Leu Phe
   1               5                   10                  15
   Leu Leu Cys Asp Gly Ser Leu Ala Gln Gln Leu Leu Gly Gln Ser Thr
               20                  25                  30
   Ser Gln Trp Gln Ser Ser Arg Arg Gly Ser Pro Arg Gly Cys Arg Phe
           35                  40                  45
   Asp Arg Leu Gln Ala Phe Glu Pro Ile Arg Ser Val Arg Ser Gln Ala
       50                  55                  60
   Gly Thr Thr Glu Phe Phe Asp Val Ser Asn Glu Leu Phe Gln Cys Thr
   65                  70                  75                  80
   Gly Val Ser Val Val Arg Arg Val Ile Glu Pro Arg Gly Leu Leu Leu
                   85                  90                  95
   Pro His Tyr Thr Asn Gly Ala Ser Leu Val Tyr Ile Ile Gln Gly Arg
               100                 105                 110
   Gly Ile Thr Gly Pro Thr Phe Pro Gly Cys Pro Glu Thr Tyr Gln Gln
           115                 120                 125
   Gln Phe Gln Gln Ser Gly Gln Ala Gln Leu Thr Glu Ser Gln Ser Gln
       130                 135                 140
   Ser His Lys Phe Lys Asp Glu His Gln Lys Ile His Arg Phe Arg Gln
   145                 150                 155                 160
   Gly Asp Val Ile Ala Leu Pro Ala Gly Val Ala His Trp Cys Tyr Asn
                   165                 170                 175
   Asp Gly Glu Val Pro Val Val Ala Ile Tyr Val Thr Asp Ile Asn Asn
               180                 185                 190
   Gly Ala Asn Gln Leu Asp Pro Arg Gln Arg Asp Phe Leu Leu Ala Gly
           195                 200                 205
   Asn Lys Arg Asn Pro Gln Ala Tyr Arg Arg Glu Val Glu Glu Trp Ser
       210                 215                 220
   Gln Asn Ile Phe Ser Gly Phe Ser Thr Glu Leu Leu Ser Glu Ala Phe
   225                 230                 235                 240
   Gly Ile Ser Asn Gln Val Ala Arg Gln Leu Gln Cys Gln Asn Asp Gln
                   245                 250                 255
   Arg Gly Glu Ile Val Arg Val Glu Arg Gly Leu Ser Leu Leu Gln Pro
               260                 265                 270
   Tyr Ala Ser Leu Gln Glu Gln Glu Gln Gly Gln Met Gln Ser Arg Glu
           275                 280                 285
   His Tyr Gln Glu Gly Gly Tyr Gln Gln Ser Gln Tyr Gly Ser Gly Cys
       290                 295                 300
   Pro Asn Gly Leu Asp Glu Thr Phe Cys Thr Met Arg Val Arg Gln Asn
   305                 310                 315                 320
   Ile Asp Asn Pro Asn Arg Ala Asp Thr Tyr Asn Pro Arg Ala Gly Arg
   325 330 335
   Val Thr Asn Leu Asn Ser Gln Asn Phe Pro Ile Leu Asn Leu Val Gln
   340 345 350
   Met Ser Ala Val Lys Val Asn Leu Tyr Gln Asn Ala Leu Leu Ser Pro
   355 360 365
   Phe Trp Asn Ile Asn Ala His Ser Ile Val Tyr Ile Thr Gln Gly Arg
   370 375 380
   Ala Gln Val Gln Val Val Asn Asn Asn Gly Lys Thr Val Phe Asn Gly
   385 390 395 400
   Glu Leu Arg Arg Gly Gln Leu Leu Ile Val Pro Gln His Tyr Val Val
   405 410 415
   Val Lys Lys Ala Gln Arg Glu Gly Cys Ala Tyr Ile Ala Phe Lys Thr
   420 425 430
   Asn Pro Asn Ser Met Val Ser His Ile Ala Gly Lys Ser Ser Ile Phe
   435 440 445
   Arg Ala Leu Pro Thr Asp Val Leu Ala Asn Ala Tyr Arg Ile Ser Arg
   450 455 460
   Glu Glu Ala Gln Arg Leu Lys His Asn Arg Gly Asp Glu Phe Gly Ala
   465 470 475 480
   Phe Thr Pro Leu Gln Tyr Lys Ser Tyr Gln Asp Val Tyr Asn Val Ala
   485 490 495
   Glu Ser Ser
<210> 13
   <211> 160
   <212> PRT
   <213> Oryza sativa
<400> 13
   Met Ala Ser Asn Lys Val Val Phe Ser Val Leu Leu Leu Ala Val Val
   1 5 10 15
   Ser Val Leu Ala Ala Thr Ala Thr Met Ala Glu Tyr His His Gln Asp
   20 25 30
   Gln Val Val Tyr Thr Pro Gly Pro Leu Cys Gln Pro Gly Met Gly Tyr
   35 40 45
   Pro Met Tyr Pro Leu Pro Arg Cys Arg Ala Leu Val Lys Arg Gln Cys
   50 55 60
   Val Gly Arg Gly Thr Ala Ala Ala Ala Glu Gln Val Arg Arg Asp Cys
   65 70 75 80
   Cys Arg Gln Leu Ala Ala Val Asp Asp Ser Trp Cys Arg Cys Glu Ala
   85 90 95
   Ile Ser His Met Leu Gly Gly Ile Tyr Arg Glu Leu Gly Ala Pro Asp
   100 105 110
   Val Gly His Pro Met Ser Glu Val Phe Arg Gly Cys Arg Arg Gly Asp
   115 120 125
   Leu Glu Arg Ala Ala Ala Ser Leu Pro Ala Phe Cys Asn Val Asp Ile
   130 135 140
   Pro Asn Gly Gly Gly Gly Val Cys Tyr Trp Leu Ala Arg Ser Gly Tyr
   145 150 155 160
<210> 14
   <211> 336
   <212> PRT
   <213> Staphylococcus aureus
<400> 14
   Met Lys Gly Lys Phe Leu Lys Val Ser Ser Leu Phe Val Ala Thr Leu
   1 5 10 15
   Thr Thr Ala Thr Leu Val Ser Ser Pro Ala Ala Asn Ala Leu Ser Ser
   20 25 30
   Lys Ala Met Asp Asn His Pro Gln Gln Thr Gln Ser Ser Lys Gln Gln
   35 40 45
   Thr Pro Lys Ile Gln Lys Gly Gly Asn Leu Lys Pro Leu Glu Gln Arg
   50 55 60
   Glu His Ala Asn Val Ile Leu Pro Asn Asn Asp Arg His Gln Ile Thr
   65 70 75 80
   Asp Thr Thr Asn Gly His Tyr Ala Pro Val Thr Tyr Ile Gln Val Glu
   85 90 95
   Ala Pro Thr Gly Thr Phe Ile Ala Ser Gly Val Val Val Gly Lys Asp
   100 105 110
   Thr Leu Leu Thr Asn Lys His Val Val Asp Ala Thr His Gly Asp Pro
   115 120 125
   His Ala Leu Lys Ala Phe Pro Ser Ala Ile Asn Gln Asp Asn Tyr Pro
   130 135 140
   Asn Gly Gly Phe Thr Ala Glu Gln Ile Thr Lys Tyr Ser Gly Glu Gly
   145 150 155 160
   Asp Leu Ala Ile Val Lys Phe Ser Pro Asn Glu Gln Asn Lys His Ile
   165 170 175
   Gly Glu Val Val Lys Pro Ala Thr Met Ser Asn Asn Ala Glu Thr Gln
   180 185 190
   Val Asn Gln Asn Ile Thr Val Thr Gly Tyr Pro Gly Asp Lys Pro Val
   195 200 205
   Ala Thr Met Trp Glu Ser Lys Gly Lys Ile Thr Tyr Leu Lys Gly Glu
   210 215 220
   Ala Met Gln Tyr Asp Leu Ser Thr Thr Gly Gly Asn Ser Gly Ser Pro
   225 230 235 240
   Val Phe Asn Glu Lys Asn Glu Val Ile Gly Ile His Trp Gly Gly Val
   245 250 255
   Pro Asn Glu Phe Asn Gly Ala Val Phe Ile Asn Glu Asn Val Arg Asn
   260 265 270
   Phe Leu Lys Gln Asn Ile Glu Asp Ile His Phe Ala Asn Asp Asp Gln
   275 280 285
   Pro Asn Asn Pro Asp Asn Pro Asp Asn Pro Asn Asn Pro Asp Asn Pro
   290 295 300
   Asn Asn Pro Asp Glu Pro Asn Asn Pro Asp Asn Pro Asn Asn Pro Asp
   305 310 315 320
   Asn Pro Asp Asn Gly Asp Asn Asn Asn Ser Asp Asn Pro Asp Ala Ala
   325 330 335
<210> 15
   <211> 13
   <212> PRT
   <213> Artificial Sequence
<220>
   <223> FRAGMENT OF SEQ ID 1
<400> 15
   Gln Asn Tyr Leu Ser Gly Phe Ser Lys Asn Ile Leu Glu
   1 5 10
<210> 16
   <211> 20
   <212> PRT
   <213> Artificial Sequence
<220>
   <223> FRAGMENT OF SEQ ID 1
<400> 16
   Thr Ile Lys Leu Pro Ala Gly Thr Ile Ala Tyr Leu Val Asn Arg Asp
   1 5 10 15
   Asp Asn Glu Glu
   20
<210> 17
   <211> 15
   <212> PRT
   <213> Artificial Sequence
<220>
   <223> FRAGMENT OF SEQ ID 1
<400> 17
   Leu Ala Ile Pro Val Asn Arg Pro Gly Gln Leu Gln Ser Phe Leu
   1 5 10 15
<210> 18
   <211> 11
   <212> PRT
   <213> Artificial Sequence
<220>
   <223> FRAGMENT OF SEQ ID 1
<400> 18
   Ala Ile Pro Val Asn Arg Pro Gly Gln Leu Gln
   1 5 10
<210> 19
   <211> 9
   <212> PRT
   <213> Artificial Sequence
<220>
   <223> FRAGMENT OF SEQ ID 1
<400> 19
   Pro Ala Gly His Pro Val Ala Val Lys
   1 5
<210> 20
   <211> 19
   <212> PRT
   <213> Artificial Sequence
<220>
   <223> FRAGMENT FOR SEQ ID 1
<400> 20
   Val Gln Asn Tyr Lys Ala Lys Leu Ser Ser Gly Asp Val Phe Val Ile
   1 5 10 15
   Pro Ala Gly
<210> 21
   <211> 24
   <212> PRT
   <213> Artificial Sequence
<220>
   <223> FRAGMENT OF SEQ ID 1
<400> 21
   Asn Asn Gln Arg Asn Phe Leu Ala Gly Asp Glu Asp Asn Val Ile Ser
   1 5 10 15
   Gln Ile Gln Arg Pro Val Lys Glu
   20
<210> 22
   <211> 23
   <212> PRT
   <213> Artificial Sequence
<220>
   <223> FRAGMENT OF SEQ ID 1
<400> 22
   Ile Asn Lys Gln Val Gln Asn Tyr Lys Ala Lys Leu Ser Ser Gly Asp
   1 5 10 15
   Val Phe Val Ile Pro Ala Gly
   20
<210> 23
   <211> 10
   <212> PRT
   <213> Artificial Sequence
<220>
   <223> FRAGMENT OF SEQ ID 1
<400> 23
   Leu Ala Ile Pro Val Asn Arg Pro Gly Gln
   1               5                   10
<210> 24
   <211> 20
   <212> PRT
   <213> Artificial Sequence
<220>
   <223> FRAGMENT OF SEQ ID 1
<400> 24
   Asn Phe Leu Ala Gly Asp Glu Asp Asn Val Ile Ser Gln Ile Gln Arg
   1               5                   10                  15
   Pro Val Lys Glu
               20
<210> 25
   <211> 15
   <212> PRT
   <213> Artificial Sequence
<220>
   <223> FRAGMENT OF SEQ ID 1
<400> 25
   Asp Leu Ala Ile Pro Val Asn Arg Pro Gly Gln Leu Gln Ser Phe
   1               5                   10                  15
<210> 26
   <211> 11
   <212> PRT
   <213> Artificial Sequence
<220>
   <223> FRAGMENT OF SEQ ID 1
<400> 26
   Val Ile Pro Ala Gly His Pro Val Ala Val Lys
   1               5                   10
<210> 27
   <211> 21
   <212> PRT
   <213> Artificial Sequence
<220>
   <223> FRAGMENT OF SEQ ID 1
<400> 27
   Asp Thr Ile Lys Leu Pro Ala Gly Thr Ile Ala Tyr Leu Val Asn Arg
   1               5                   10                  15
   Asp Asp Asn Glu Glu
               20
<210> 28
   <211> 14
   <212> PRT
   <213> Artificial Sequence
<220>
   <223> FRAGMENT OF SEQ ID 1
<400> 28
   Leu Ala Ile Pro Val Asn Arg Pro Gly Gln Leu Gln Ser Phe
   1               5                   10
<210> 29
   <211> 21
   <212> PRT
   <213> Artificial Sequence
<220>
   <223> FRAGMENT OF SEQ ID 1
<400> 29
   Lys Gln Val Gln Asn Tyr Lys Ala Lys Leu Ser Ser Gly Asp Val Phe
   1               5                   10                  15
   Val Ile Pro Ala Gly
               20
<210> 30
   <211> 23
   <212> PRT
   <213> Artificial Sequence
<220>
   <223> FRAGMENT SEQ ID 1
<400> 30
   Arg Gly Asp Thr Ile Lys Leu Pro Ala Gly Thr Ile Ala Tyr Leu Val
   1 5 10 15
   Asn Arg Asp Asp Asn Glu Glu
   20
<210> 31
   <211> 19
   <212> PRT
   <213> Artificial Sequence
<220>
   <223> FRAGMENT OF SEQ ID 1
<400> 31
   Phe Leu Ala Gly Asp Glu Asp Asn Val Ile Ser Gln Ile Gln Arg Pro
   1 5 10 15
   Val Lys Glu
<210> 32
   <211> 13
   <212> PRT
   <213> Artificial Sequence
<220>
   <223> FRAGMENT OF SEQ ID 1
<400> 32
   Leu Ala Ile Pro Val Asn Arg Pro Gly Gln Leu Gln Ser
   1 5 10
<210> 33
   <211> 15
   <212> PRT
   <213> Artificial Sequence
<220>
   <223> FRAGMENT OF SEQ ID 1
<400> 33
   Val Leu Asp Leu Ala Ile Pro Val Asn Arg Pro Gly Gln Leu Gln
   1 5 10 15
<210> 34
   <211> 13
   <212> PRT
   <213> Artificial Sequence
<220>
   <223> FRAGMENT OF SEQ ID 1
<400> 34
   Asp Leu Ala Ile Pro Val Asn Arg Pro Gly Gln Leu Gln
   1 5 10
<210> 35
   <211> 13
   <212> PRT
   <213> Artificial Sequence
<220>
   <223> FRAGMENT OF SEQ ID 1
<400> 35
   Val Phe Val Ile Pro Ala Gly His Pro Val Ala Val Lys
   1 5 10
<210> 36
   <211> 20
   <212> PRT
   <213> Artificial Sequence
<220>
   <223> FRAGMENT OF SEQ ID 1
<400> 36
   Thr Ile Phe Leu Pro Gln His Thr Asp Ala Asp Tyr Ile Leu Val Val
   1 5 10 15
   Leu Ser Gly Lys
   20
<210> 37
   <211> 23
   <212> PRT
   <213> Artificial Sequence
<220>
   <223> FRAGMENT OF SEQ ID 1
<400> 37
   Asn Gln Arg Asn Phe Leu Ala Gly Asp Glu Asp Asn Val Ile Ser Gln
   1 5 10 15
   Ile Gln Arg Pro Val Lys Glu
   20
<210> 38
   <211> 12
   <212> PRT
   <213> Artificial Sequence
<220>
   <223> FRAGMENT OF SEQ ID 1
<400> 38
   Leu Ala Ile Pro Val Asn Arg Pro Gly Gln Leu Gln
   1 5 10
<210> 39
   <211> 17
   <212> PRT
   <213> Artificial Sequence
<220>
   <223> FRAGMENT OF SEQ ID 1
<400> 39
   His Pro Val Ala Val Lys Ala Ser Ser Asn Leu Asp Leu Leu Gly Phe
   1 5 10 15
   Gly
<210> 40
   <211> 11
   <212> PRT
   <213> Artificial Sequence
<220>
   <223> FRAGMENT OF SEQ ID 1
<400> 40
   Leu Ala Ile Pro Val Asn Arg Pro Gly Gln Leu
   1 5 10
<210> 41
   <211> 12
   <212> PRT
   <213> Artificial Sequence
<220>
   <223> FRAGMENT SEQ ID 1
<400> 41
   Asp Leu Ala Ile Pro Val Asn Arg Pro Gly Gln Leu
   1 5 10
<210> 42
   <211> 24
   <212> PRT
   <213> Artificial Sequence
<220>
   <223> FRAGMENT OF SEQ ID 1
<400> 42
   Ser Lys Pro His Thr Ile Phe Leu Pro Gln His Thr Asp Ala Asp Tyr
   1               5                   10                  15
   Ile Leu Val Val Leu Ser Gly Lys
               20
<210> 43
   <211> 12
   <212> PRT
   <213> Artificial Sequence
<220>
   <223> FRAGMENT OF SEQ ID 1
<400> 43
   Phe Val Ile Pro Ala Gly His Pro Val Ala Val Lys
   1               5                   10
<210> 44
   <211> 14
   <212> PRT
   <213> Artificial Sequence
<220>
   <223> FRAGMENT OF SEQ ID 1
<400> 44
   Asp Leu Ala Ile Pro Val Asn Arg Pro Gly Gln Leu Gln Ser
   1               5                   10
<210> 45
   <211> 22
   <212> PRT
   <213> Artificial Sequence
<220>
   <223> FRAGMENT OF SEQ ID 1
<400> 45
   Ser Gly Asp Val Phe Val Ile Pro Ala Gly His Pro Val Ala Val Lys
   1               5                   10                  15
   Ala Ser Ser Asn Leu Asp
               20
<210> 46
   <211> 13
   <212> PRT
   <213> Artificial Sequence
<220>
   <223> FRAGMENT OF SEQ ID 1
<400> 46
   Ala Ile Pro Val Asn Arg Pro Gly Gln Leu Gln Ser Phe
   1 5 10
<210> 47
   <211> 13
   <212> PRT
   <213> Artificial Sequence
<220>
   <223> FRAGMENT OF SEQ ID 1
<400> 47
   Glu Leu Ala Phe Pro Gly Ser Ala Gln Glu Val Asp Arg
   1 5 10
<210> 48
   <211> 18
   <212> PRT
   <213> Artificial Sequence
<220>
   <223> FRAGMENT OF SEQ ID 1
<400> 48
   Leu Ala Ile Pro Val Asn Arg Pro Gly Gln Leu Gln Ser Phe Leu Leu
   1 5 10 15
   Ser Gly
<210> 49
   <211> 24
   <212> PRT
   <213> Artificial Sequence
<220>
   <223> FRAGMENT OF SEQ ID 1
<400> 49
   Val Phe Val Ile Pro Ala Gly His Pro Val Ala Val Lys Ala Ser Ser
   1 5 10 15
   Asn Leu Asp Leu Leu Gly Phe Gly
   20
<210> 50
   <211> 8
   <212> PRT
   <213> Artificial Sequence
<220>
   <223> FRAGMENT OF SEQ ID 1
<400> 50
   Ala Gly His Pro Val Ala Val Lys
   1 5
<210> 51
   <211> 21
   <212> PRT
   <213> Artificial Sequence
<220>
   <223> FRAGMENT OF SEQ ID 1
<400> 51
   His Pro Val Ala Val Lys Ala Ser Ser Asn Leu Asp Leu Leu Gly Phe
   1 5 10 15
   Gly Ile Asn Ala Glu
   20
<210> 52
   <211> 22
   <212> PRT
   <213> Artificial Sequence
<220>
   <223> FRAGMENT SEQ ID 1
<400> 52
   Leu Ala Ile Pro Val Asn Arg Pro Gly Gln Leu Gln Ser Phe Leu Leu
   1 5 10 15
   Ser Gly Asn Gln Asn Gln
   20
<210> 53
   <211> 10
   <212> PRT
   <213> Artificial Sequence
<220>
   <223> FRAGMENT SEQ ID 1
<400> 53
   Ser Gly Asp Val Phe Val Ile Pro Ala Gly
   1 5 10
<210> 54
   <211> 20
   <212> PRT
   <213> Artificial Sequence
<220>
   <223> FRAGMENT OF SEQ ID 1
<400> 54
   Gly Ser Leu Leu Leu Pro His Tyr Asn Ser Arg Ala Ile Val Ile Val
   1 5 10 15
   Thr Val Asn Glu
   20
<210> 55
   <211> 19
   <212> PRT
   <213> Artificial Sequence
<220>
   <223> FRAGMENT OF SEQ ID 1
<400> 55
   Asn Phe Leu Ala Gly Asp Glu Asp Asn Val Ile Ser Gln Ile Gln Arg
   1 5 10 15
   Pro Val Lys
<210> 56
   <211> 14
   <212> PRT
   <213> Artificial Sequence
<220>
   <223> FRAGMENT SEQ ID 1
<400> 56
   Ser Gly Asp Val Phe Val Ile Pro Ala Gly His Pro Val Ala
   1 5 10
<210> 57
   <211> 16
   <212> PRT
   <213> Artificial Sequence
<220>
   <223> FRAGMENT SEQ ID 1
<400> 57
   Gly Ser Leu Leu Leu Pro His Tyr Asn Ser Arg Ala Ile Val Ile Val
   1 5 10 15
<210> 58
   <211> 20
   <212> PRT
   <213> Artificial Sequence
<220>
   <223> FRAGMENT SEQ ID 1
<400> 58
   Arg Gly Asp Thr Ile Lys Leu Pro Ala Gly Thr Ile Ala Tyr Leu Val
   1               5                   10                  15
   Asn Arg Asp Asp
               20
<210> 59
   <211> 16
   <212> PRT
   <213> Artificial Sequence
<220>
   <223> FRAGMENT OF SEQ ID 1
<400> 59
   Ser Gly Asp Val Phe Val Ile Pro Ala Gly His Pro Val Ala Val Lys
   1               5                   10                  15
<210> 60
   <211> 18
   <212> PRT
   <213> Artificial Sequence
<220>
   <223> FRAGMENT SEQ ID 1
<400> 60
   Leu Ser Ser Gly Asp Val Phe Val Ile Pro Ala Gly His Pro Val Ala
   1               5                   10                  15
   Val Lys
<210> 61
   <211> 13
   <212> PRT
   <213> Artificial Sequence
<220>
   <223> FRAGMENT OF SEQ ID 1
<400> 61
   Leu Asp Leu Ala Ile Pro Val Asn Arg Pro Gly Gln Leu
   1               5                   10
<210> 62
   <211> 10
   <212> PRT
   <213> Artificial Sequence
<220>
   <223> FRAGMENT OF SEQ ID 1
<400> 62
   Ala Ile Pro Val Asn Arg Pro Gly Gln Leu
   1 5 10
<210> 63
   <211> 16
   <212> PRT
   <213> Artificial Sequence
<220>
   <223> FRAGMENT OF SEQ ID 1
<400> 63
   Leu Ala Ile Pro Val Asn Arg Pro Gly Gln Leu Gln Ser Phe Leu Leu
   1 5 10 15
<210> 64
   <211> 22
   <212> PRT
   <213> Artificial Sequence
<220>
   <223> FRAGMENT OF SEQ ID 1
<400> 64
   Pro His Thr Ile Phe Leu Pro Gln His Thr Asp Ala Asp Tyr Ile Leu
   1 5 10 15
   Val Val Leu Ser Gly Lys
   20
<210> 65
   <211> 19
   <212> PRT
   <213> Artificial Sequence
<220>
   <223> FRAGMENT OF SEQ ID 1
<400> 65
   Val Phe Val Ile Pro Ala Gly His Pro Val Ala Val Lys Ala Ser Ser
   1 5 10 15
   Asn Leu Asp
<210> 66
   <211> 17
   <212> PRT
   <213> Artificial Sequence
<220>
   <223> FRAGMENT OF SEQ ID 1
<400> 66 Ser
<210> 67
   <211> 17
   <212> PRT
   <213> Artificial Sequence
<220>
   <223> FRAGMENT OF SEQ ID 1
<400> 67
   Val Leu Asp Leu Ala Ile Pro Val Asn Arg Pro Gly Gln Leu Gln Ser
   1               5                   10                  15
   Phe
<210> 68
   <211> 12
   <212> PRT
   <213> Artificial Sequence
<220>
   <223> FRAGMENT OF SEQ ID 1
<400> 68
   Ala Ile Pro Val Asn Arg Pro Gly Gln Leu Gln Ser
   1               5                   10
<210> 69
   <211> 20
   <212> PRT
   <213> Artificial Sequence
<220>
   <223> FRAGMENT OF SEQ ID 1
<400> 69
   Asp Thr Ile Lys Leu Pro Ala Gly Thr Ile Ala Tyr Leu Val Asn Arg
   1               5                   10                  15
   Asp Asp Asn Glu
               20
<210> 70
   <211> 17
   <212> PRT
   <213> Artificial Sequence
<220>
   <223> FRAGMENT OF SEQ ID 1
<400> 70
   Asn Tyr Lys Ala Lys Leu Ser Ser Gly Asp Val Phe Val Ile Pro Ala
   1               5                   10                  15
   Gly
<210> 71
   <211> 19
   <212> PRT
   <213> Artificial Sequence
<220>
   <223> FRAGMENT OF SEQ ID 1
<400> 71
   Gly Lys Ala Ile Leu Thr Val Leu Lys Pro Asp Asp Arg Asn Ser Phe
   1               5                   10                  15
   Asn Leu Glu
<210> 72
   <211> 17
   <212> PRT
   <213> Artificial Sequence
<220>
   <223> FRAGMENT OF SEQ ID 1
<400> 72
   Tyr Lys Ser Lys Pro His Thr Ile Phe Leu Pro Gln His Thr Asp Ala
   1               5                   10                  15
   Asp
<210> 73
   <211> 11
   <212> PRT
   <213> Artificial Sequence
<220>
   <223> FRAGMENT OF SEQ ID 1
<400> 73
   Ala Ser Ser Asn Leu Asp Leu Leu Gly Phe Gly
   1               5                   10
<210> 74
   <211> 13
   <212> PRT
   <213> Artificial Sequence
<220>
   <223> FRAGMENT OF SEQ ID 1
<400> 74
   Asp Glu Glu Glu Glu Gln Gly Glu Glu Glu Ile Asn Lys
   1               5                   10
<210> 75
   <211> 15
   <212> PRT
   <213> Artificial Sequence
<220>
   <223> FRAGMENT OF SEQ ID 1
<400> 75
   Tyr Lys Ser Lys Pro His Thr Ile Phe Leu Pro Gln His Thr Asp
   1               5                   10                  15
<210> 76
   <211> 10
   <212> PRT
   <213> Artificial Sequence
<220>
   <223> FRAGMENT OF SEQ ID 1
<400> 76
   Val Leu Asp Leu Ala Ile Pro Val Asn Arg
   1               5                   10
<210> 77
   <211> 25
   <212> PRT
   <213> Artificial Sequence
<220>
   <223> FRAGMENT OF SEQ ID 1
<400> 77
   Phe Phe Glu Ile Thr Pro Glu Lys Asn Pro Gln Leu Gln Asp Leu Asp
   1               5                   10                  15
   Ile Phe Val Asn Ser Val Glu Ile Lys
               20                  25
<210> 78
   <211> 14
   <212> PRT
   <213> Artificial Sequence
<220>
   <223> FRAGMENT OF SEQ ID 1
<400> 78
   Thr Ile Phe Leu Pro Gln His Thr Asp Ala Asp Tyr Ile Leu
   1               5                   10
<210> 79
   <211> 16
   <212> PRT
   <213> Artificial Sequence
<220>
   <223> FRAGMENT OF SEQ ID 1
<400> 79
   Ser Phe Leu Leu Ser Gly Asn Gln Asn Gln Gln Asn Tyr Leu Ser Gly
   1               5                   10                  15
<210> 80
   <211> 18
   <212> PRT
   <213> Artificial Sequence
<220>
   <223> FRAGMENT OF SEQ ID 1
<400> 80
   Ser Phe Leu Leu Ser Gly Asn Gln Asn Gln Gln Asn Tyr Leu Ser Gly
   1               5                   10                  15
   Phe Ser
<210> 81
   <211> 19
   <212> PRT
   <213> Artificial Sequence
<220>
   <223> FRAGMENT OF SEQ ID 1
<400> 81
   Asn Gln Gln Glu Gln Arg Lys Glu Asp Asp Glu Glu Glu Glu Gln Gly
   1 5 10 15
   Glu Glu Glu
<210> 82
   <211> 10
   <212> PRT
   <213> Artificial Sequence
<220>
   <223> FRAGMENT OF SEQ ID 1
<400> 82
   Glu Glu Gln Gly Glu Glu Glu Ile Asn Lys
   1 5 10
<210> 83
   <211> 9
   <212> PRT
   <213> Artificial Sequence
<220>
   <223> FRAGMENT OF SEQ ID 1
<400> 83
   Ser Arg Gly Pro Ile Tyr Ser Asn Glu
   1 5
<210> 84
   <211> 15
   <212> PRT
   <213> Artificial Sequence
<220>
   <223> FRAGMENT OF SEQ ID 1
<400> 84
   Glu Asp Asp Glu Glu Glu Glu Gln Gly Glu Glu Glu Ile Asn Lys
   1 5 10 15
<210> 85
   <211> 14
   <212> PRT
   <213> Artificial Sequence
<220>
   <223> FRAGMENT OF SEQ ID 1
<400> 85
   Asp Asp Glu Glu Glu Glu Gln Gly Glu Glu Glu Ile Asn Lys
   1 5 10
<210> 86
   <211> 15
   <212> PRT
   <213> Artificial Sequence
<220>
   <223> FRAGMENT OF SEQ ID 1
<400> 86
   Lys Glu Asp Asp Glu Glu Glu Glu Gln Gly Glu Glu Glu Ile Asn
   1 5 10 15
<210> 87
   <211> 12
   <212> PRT
   <213> Artificial Sequence
<220>
   <223> FRAGMENT OF SEQ ID 1
<400> 87
   Lys Glu Asp Asp Glu Glu Glu Glu Gln Gly Glu Glu
   1 5 10
<210> 88
   <211> 15
   <212> PRT
   <213> Artificial Sequence
<220>
   <223> FRAGMENT OF SEQ ID 1
<400> 88
   Gln Arg Lys Glu Asp Asp Glu Glu Glu Glu Gln Gly Glu Glu Glu
   1 5 10 15
<210> 89
   <211> 16
   <212> PRT
   <213> Artificial Sequence
<220>
   <223> FRAGMENT OF SEQ ID 1
<400> 89
   Lys Glu Asp Asp Glu Glu Glu Glu Gln Gly Glu Glu Glu Ile Asn Lys
   1 5 10 15
<210> 90
   <211> 13
   <212> PRT
   <213> Artificial Sequence
<220>
   <223> FRAGMENT OF SEQ ID 1
<400> 90
   Lys Glu Asp Asp Glu Glu Glu Glu Gln Gly Glu Glu Glu
   1 5 10
<210> 91
   <211> 15
   <212> PRT
   <213> Artificial Sequence
<220>
   <223> FRAGMENT OF SEQ ID 2
<400> 91
   His Pro Val Ala Ile Thr Ala Ser Ser Asn Leu Asn Leu Leu Gly
   1 5 10 15
<210> 92
   <211> 11
   <212> PRT
   <213> Artificial Sequence
<220>
   <223> FRAGMENT OF SEQ ID 2
<400> 92
   Ala Ser Ser Asn Leu Asn Leu Leu Gly Phe Gly
   1 5 10
<210> 93
   <211> 13
   <212> PRT
   <213> Artificial Sequence
<220>
   <223> FRAGMENT OF SEQ ID 2
<400> 93
   Ile Thr Ala Ser Ser Asn Leu Asn Leu Leu Gly Phe Gly
   1 5 10
<210> 94
   <211> 17
   <212> PRT
   <213> Artificial Sequence
<220>
   <223> FRAGMENT OF SEQ ID 2
<400> 94
   Ile Thr Ala Ser Ser Asn Leu Asn Leu Leu Gly Phe Gly Ile Asn Ala
   1               5                   10                  15
   Glu
<210> 95
   <211> 10
   <212> PRT
   <213> Artificial Sequence
<220>
   <223> FRAGMENT OF SEQ ID 2
<400> 95
   Ser Ser Asn Leu Asn Leu Leu Gly Phe Gly
   1               5                   10
<210> 96
   <211> 13
   <212> PRT
   <213> Artificial Sequence
<220>
   <223> FRAGMENT OF SEQ ID 2
<400> 96
   Val Asp Leu Val Ile Pro Val Asn Gly Pro Gly Lys Phe
   1               5                   10
<210> 97
   <211> 12
   <212> PRT
   <213> Artificial Sequence
<220>
   <223> FRAGMENT OF SEQ ID 2
<400> 97
   Leu Val Ile Pro Val Asn Gly Pro Gly Lys Phe Glu
   1               5                   10
<210> 98
   <211> 13
   <212> PRT
   <213> Artificial Sequence
<220>
   <223> FRAGMENT OF SEQ ID 2
<400> 98
   Leu Val Ile Pro Val Asn Gly Pro Gly Lys Phe Glu Ala
   1               5                   10
<210> 99
   <211> 17
   <212> PRT
   <213> Artificial Sequence
<220>
   <223> FRAGMENT OF SEQ ID 2
<400> 99
   Leu Arg Leu Val Asp Leu Val Ile Pro Val Asn Gly Pro Gly Lys Phe
   1               5                   10                  15
   Glu
<210> 100
   <211> 17
   <212> PRT
   <213> Artificial Sequence
<220>
   <223> FRAGMENT OF SEQ ID 2
<400> 100
   Tyr Arg Ala Lys Pro His Thr Ile Phe Leu Pro Gln His Ile Asp Ala
   1               5                   10                  15
   Asp
<210> 101
   <211> 21
   <212> PRT
   <213> Artificial Sequence
<220>
   <223> FRAGMENT OF SEQ ID 2
<400> 101
   His Pro Val Ala Ile Thr Ala Ser Ser Asn Leu Asn Leu Leu Gly Phe
   1               5                   10                  15
   Gly Ile Asn Ala Glu
               20
<210> 102
   <211> 9
   <212> PRT
   <213> Artificial Sequence
<220>
   <223> FRAGMENT OF SEQ ID 2
<400> 102
   Ser Asn Leu Asn Leu Leu Gly Phe Gly
   1 5
<210> 103
   <211> 24
   <212> PRT
   <213> Artificial Sequence
<220>
   <223> FRAGMENT OF SEQ ID 2
<400> 103
   His Pro Val Ala Ile Thr Ala Ser Ser Asn Leu Asn Leu Leu Gly Phe
   1 5 10 15
   Gly Ile Asn Ala Glu Asn Asn Glu
   20
<210> 104
   <211> 15
   <212> PRT
   <213> Artificial Sequence
<220>
   <223> FRAGMENT OF SEQ ID 2
<400> 104
   Leu Val Asp Leu Val Ile Pro Val Asn Gly Pro Gly Lys Phe Glu
   1 5 10 15
<210> 105
   <211> 11
   <212> PRT
   <213> Artificial Sequence
<220>
   <223> FRAGMENT OF SEQ ID 2
<400> 105
   Leu Val Ile Pro Val Asn Gly Pro Gly Lys Phe
   1 5 10
<210> 106
   <211> 18
   <212> PRT
   <213> Artificial Sequence
<220>
   <223> FRAGMENT OF SEQ ID 2
<400> 106
   Thr Ile Lys Leu Pro Ala Gly Thr Thr Ser Tyr Leu Val Asn Gln Asp
   1 5 10 15
   Asp Glu
<210> 107
   <211> 18
   <212> PRT
   <213> Artificial Sequence
<220>
   <223> FRAGMENT OF SEQ ID 2
<400> 107
   Asp Leu Arg Leu Val Asp Leu Val Ile Pro Val Asn Gly Pro Gly Lys
   1 5 10 15
   Phe Glu
<210> 108
   <211> 19
   <212> PRT
   <213> Artificial Sequence
<220>
   <223> FRAGMENT OF SEQ ID 2
<400> 108
   Glu Asp Leu Arg Leu Val Asp Leu Val Ile Pro Val Asn Gly Pro Gly
   1 5 10 15
   Lys Phe Glu
<210> 109
   <211> 17
   <212> PRT
   <213> Artificial Sequence
<220>
   <223> FRAGMENT OF SEQ ID 2
<400> 109
   His Pro Val Ala Ile Thr Ala Ser Ser Asn Leu Asn Leu Leu Gly Phe
   1 5 10 15
   Gly
<210> 110
   <211> 21
   <212> PRT
   <213> Artificial Sequence
<220>
   <223> FRAGMENT OF SEQ ID 2
<400> 110
   Leu Val Asp Leu Val Ile Pro Val Asn Gly Pro Gly Lys Phe Glu Ala
   1 5 10 15
   Phe Asp Leu Ala Lys
   20
<210> 111
   <211> 13
   <212> PRT
   <213> Artificial Sequence
<220>
   <223> FRAGMENT OF SEQ ID 2
<400> 111
   Asp Asn Val Ile Ser Gln Ile Glu Asn Pro Val Lys Glu
   1 5 10
<210> 112
   <211> 24
   <212> PRT
   <213> Artificial Sequence
<220>
   <223> FRAGMENT OF SEQ ID 2
<400> 112
   Val Val Ile Ile Pro Ala Gly His Pro Val Ala Ile Thr Ala Ser Ser
   1 5 10 15
   Asn Leu Asn Leu Leu Gly Phe Gly
   20
<210> 113
   <211> 17
   <212> PRT
   <213> Artificial Sequence
<220>
   <223> FRAGMENT OF SEQ ID 2
<400> 113
   Leu Val Asp Leu Val Ile Pro Val Asn Gly Pro Gly Lys Phe Glu Ala
   1 5 10 15
   Phe
<210> 114
   <211> 9
   <212> PRT
   <213> Artificial Sequence
<220>
   <223> FRAGMENT OF SEQ ID 2
<400> 114
   Tyr Pro Gln Leu Gln Asp Leu Asp Leu
   1 5
<210> 115
   <211> 10
   <212> PRT
   <213> Artificial Sequence
<220>
   <223> FRAGMENT OF SEQ ID 2
<400> 115
   Val Ile Pro Val Asn Gly Pro Gly Lys Phe
   1 5 10
<210> 116
   <211> 8
   <212> PRT
   <213> Artificial Sequence
<220>
   <223> FRAGMENT OF SEQ ID 2
<400> 116
   Ser Lys Lys Ser Leu Pro Ser Glu
   1 5
<210> 117
   <211> 17
   <212> PRT
   <213> Artificial Sequence
<220>
   <223> FRAGMENT OF SEQ ID 2
<400> 117
   Leu Pro Gln His Ile Asp Ala Asp Leu Ile Leu Val Val Leu Ser Gly
   1 5 10 15
   Lys
<210> 118
   <211> 19
   <212> PRT
   <213> Artificial Sequence
<220>
   <223> FRAGMENT OF SEQ ID 2
<400> 118
   Arg Gly Asp Thr Ile Lys Leu Pro Ala Gly Thr Thr Ser Tyr Leu Val
   1 5 10 15
   Asn Gln Asp
<210> 119
   <211> 9
   <212> PRT
   <213> Artificial Sequence
<220>
   <223> FRAGMENT OF SEQ ID 2
<400> 119
   Ile Pro Val Asn Gly Pro Gly Lys Phe
   1 5
<210> 120
   <211> 9
   <212> PRT
   <213> Artificial Sequence
<220>
   <223> FRAGMENT OF SEQ ID 2
<400> 120
   Leu Pro Gln His Ile Asp Ala Asp Leu
   1 5
<210> 121
   <211> 10
   <212> PRT
   <213> Artificial Sequence
<220>
   <223> FRAGMENT OF SEQ ID 2
<400> 121
   Leu Val Ile Pro Val Asn Gly Pro Gly Lys
   1 5 10
<210> 122
   <211> 10
   <212> PRT
   <213> Artificial Sequence
<220>
   <223> FRAGMENT OF SEQ ID 2
<400> 122
   Ile Phe Leu Pro Gln His Ile Asp Ala Asp
   1 5 10
<210> 123
   <211> 8
   <212> PRT
   <213> Artificial Sequence
<220>
   <223> FRAGMENT OF SEQ ID 2
<400> 123
   Leu Pro Gln His Ile Asp Ala Asp
   1 5
<210> 124
   <211> 9
   <212> PRT
   <213> Artificial Sequence
<220>
   <223> FRAGMENT OF SEQ ID 2
<400> 124
   Val Ile Pro Val Asn Gly Pro Gly Lys
   1 5
<210> 125
   <211> 9
   <212> PRT
   <213> Artificial Sequence
<220>
   <223> FRAGMENT OF SEQ ID 2
<400> 125
   Ile Phe Leu Pro Gln His Ile Asp Ala
   1 5
<210> 126
   <211> 19
   <212> PRT
   <213> Artificial Sequence
<220>
   <223> FRAGMENT OF SEQ ID 2
<400> 126
   Thr Ile Lys Leu Pro Ala Gly Thr Thr Ser Tyr Leu Val Asn Gln Asp
   1               5                   10                  15
   Asp Glu Glu
<210> 127
   <211> 21
   <212> PRT
   <213> Artificial Sequence
<220>
   <223> FRAGMENT OF SEQ ID 2
<400> 127
   His Gly Glu Trp Arg Pro Ser Tyr Glu Lys Glu Glu Asp Glu Glu Glu
   1               5                   10                  15
   Gly Gln Arg Glu Arg
               20
<210> 128
   <211> 23
   <212> PRT
   <213> Artificial Sequence
<220>
   <223> FRAGMENT OF SEQ ID 2
<400> 128
   Glu Lys Arg His Gly Glu Trp Arg Pro Ser Tyr Glu Lys Glu Glu Asp
   1               5                   10                  15
   Glu Glu Glu Gly Gln Arg Glu
               20
<210> 129
   <211> 19
   <212> PRT
   <213> Artificial Sequence
<220>
   <223> EKRHGEWRPSYEKEEDEEEGQRE
<400> 129
   Leu Pro Ala Gly Thr Thr Ser Tyr Leu Val Asn Gln Asp Asp Glu Glu
   1               5                   10                  15
   Asp Leu Arg
<210> 130
   <211> 16
   <212> PRT
   <213> Artificial Sequence
<220>
   <223> EKRHGEWRPSYEKEEDEEEGQRE
<400> 130
   Pro Ser Tyr Glu Lys Glu Glu Asp Glu Glu Glu Gly Gln Arg Glu Arg
   1 5 10 15
<210> 131
   <211> 12
   <212> PRT
   <213> Artificial Sequence
<220>
   <223> EKRHGEWRPSYEKEEDEEEGQRE
<400> 131
   Glu Lys Arg His Gly Glu Trp Arg Pro Ser Tyr Glu
   1 5 10
<210> 132
   <211> 20
   <212> PRT
   <213> Artificial Sequence
<220>
   <223> EKRHGEWRPSYEKEEDEEEGQRE
<400> 132
   Thr Ile Lys Leu Pro Ala Gly Thr Thr Ser Tyr Leu Val Asn Gln Asp
   1 5 10 15
   Asp Glu Glu Asp
   20
<210> 133
   <211> 17
   <212> PRT
   <213> Artificial Sequence
<220>
   <223> EKRHGEWRPSYEKEEDEEEGQRE
<400> 133
   His Gly Glu Trp Arg Pro Ser Tyr Glu Lys Gln Glu Asp Glu Glu Glu
   1 5 10 15
   Lys
<210> 134
   <211> 14
   <212> PRT
   <213> Artificial Sequence
<220>
   <223> FRAGMENT OF SEQ ID 2
<400> 134
   Glu Trp Arg Pro Ser Tyr Glu Lys Glu Glu Asp Glu Glu Glu
   1               5                   10
<210> 135
   <211> 14
   <212> PRT
   <213> Artificial Sequence
<220>
   <223> FRAGMENT OF SEQ ID 2
<400> 135
   Pro Ser Tyr Glu Lys Glu Glu Asp Glu Glu Glu Gly Gln Arg
   1               5                   10
<210> 136
   <211> 11
   <212> PRT
   <213> Artificial Sequence
<220>
   <223> FRAGMENT OF SEQ ID 2
<400> 136
   Glu Lys Glu Glu Asp Glu Glu Glu Gly Gln Arg
   1               5                   10
<210> 137
   <211> 18
   <212> PRT
   <213> Artificial Sequence
<220>
   <223> FRAGMENT OF SEQ ID 2
<400> 137
   Glu Trp Arg Pro Ser Tyr Glu Lys Glu Glu Asp Glu Glu Glu Gly Gln
   1               5                   10                  15
   Arg Glu
<210> 138
   <211> 10
   <212> PRT
   <213> Artificial Sequence
<220>
   <223> FRAGMENT OF SEQ ID 2
<400> 138
   Lys Glu Glu Asp Glu Glu Glu Gly Gln Arg
   1 5 10
<210> 139
   <211> 19
   <212> PRT
   <213> Artificial Sequence
<220>
   <223> FRAGMENT OF SEQ ID 2
<400> 139
   Val Gln Pro Gly Arg Glu Arg Trp Glu Arg Glu Glu Asp Glu Glu Gln
   1 5 10 15
   Val Asp Glu
<210> 140
   <211> 12
   <212> PRT
   <213> Artificial Sequence
<220>
   <223> FRAGMENT OF SEQ ID 2
<400> 140
   Asp Val Val Ile Ile Pro Ala Gly His Pro Val Ala
   1 5 10
<210> 141
   <211> 14
   <212> PRT
   <213> Artificial Sequence
<220>
   <223> FRAGMENT OF SEQ ID 2
<400> 141
   His Gly Glu Trp Arg Pro Ser Tyr Glu Lys Gln Glu Asp Glu
   1 5 10
<210> 142
   <211> 9
   <212> PRT
   <213> Artificial Sequence
<220>
   <223> FRAGMENT OF SEQ ID 2
<400> 142
   Glu Glu Asp Glu Glu Glu Gly Gln Arg
   1               5
<210> 143
   <211> 19
   <212> PRT
   <213> Artificial Sequence
<220>
   <223> FRAGMENT OF SEQ ID 2
<400> 143
   His Gly Glu Trp Arg Pro Ser Tyr Glu Lys Glu Glu Asp Glu Glu Glu
   1               5                   10                  15
   Gly Gln Arg
<210> 144
   <211> 14
   <212> PRT
   <213> Artificial Sequence
<220>
   <223> FRAGMENT OF SEQ ID 2
<400> 144
   Glu Glu Trp Arg Gly Ser Gln Arg Arg Glu Asp Pro Glu Glu
   1               5                   10
<210> 145
   <211> 23
   <212> PRT
   <213> Artificial Sequence
<220>
   <223> FRAGMENT OF SEQ ID 2
<400> 145
   Arg Glu Glu Asp Glu Glu Gln Val Asp Glu Glu Trp Arg Gly Ser Gln
   1               5                   10                  15
   Arg Arg Glu Asp Pro Glu Glu
               20
<210> 146
   <211> 9
   <212> PRT
   <213> Artificial Sequence
<220>
   <223> FRAGMENT OF SEQ ID 2
<400> 146
   Arg His Gly Glu Trp Arg Pro Ser Tyr
   1 5
<210> 147
   <211> 15
   <212> PRT
   <213> Artificial Sequence
<220>
   <223> FRAGMENT OF SEQ ID 2
<400> 147
   His Gly Glu Trp Arg Pro Ser Tyr Glu Lys Gln Glu Asp Glu Glu
   1 5 10 15
<210> 148
   <211> 11
   <212> PRT
   <213> Artificial Sequence
<220>
   <223> FRAGMENT OF SEQ ID 2
<400> 148
   Val Val Ile Ile Pro Ala Gly His Pro Val Ala
   1 5 10
<210> 149
   <211> 9
   <212> PRT
   <213> Artificial Sequence
<220>
   <223> FRAGMENT OF SEQ ID 2
<400> 149
   His Gly Glu Trp Arg Pro Ser Tyr Glu
   1 5
<210> 150
   <211> 12
   <212> PRT
   <213> Artificial Sequence
<220>
   <223> FRAGMENT OF SEQ ID 2
<400> 150
   Lys Glu Glu Asp Glu Glu Glu Gly Gln Arg Glu Arg
   1 5 10
<210> 151
   <211> 13
   <212> PRT
   <213> Artificial Sequence
<220>
   <223> FRAGMENT OF SEQ ID 2
<400> 151
   Val Val Ile Ile Pro Ala Gly His Pro Val Ala Ile Thr
   1               5                   10
<210> 152
   <211> 17
   <212> PRT
   <213> Artificial Sequence
<220>
   <223> FRAGMENT OF SEQ ID 2
<400> 152
   Glu Lys Arg His Gly Glu Trp Arg Pro Ser Tyr Glu Lys Glu Glu Asp
   1               5                   10                  15
   Glu
<210> 153
   <211> 17
   <212> PRT
   <213> Artificial Sequence
<220>
   <223> FRAGMENT OF SEQ ID 2
<400> 153
   Gln Val Asp Glu Glu Trp Arg Gly Ser Gln Arg Arg Glu Asp Pro Glu
   1               5                   10                  15
   Glu
<210> 154
   <211> 24
   <212> PRT
   <213> Artificial Sequence
<220>
   <223> FRAGMENT OF SEQ ID 2
<400> 154
   Gly Asp Thr Ile Lys Leu Pro Ala Gly Thr Thr Ser Tyr Leu Val Asn
   1 5 10 15
   Gln Asp Asp Glu Glu Asp Leu Arg
   20
<210> 155
   <211> 11
   <212> PRT
   <213> Artificial Sequence
<220>
   <223> FRAGMENT OF SEQ ID 2
<400> 155
   Gly Ser Glu Pro Arg Val Pro Ala Gln Arg Glu
   1 5 10
<210> 156
   <211> 15
   <212> PRT
   <213> Artificial Sequence
<220>
   <223> FRAGMENT OF SEQ ID 2
<400> 156
   Glu Glu Lys Arg His Gly Glu Trp Arg Pro Ser Tyr Glu Lys Glu
   1 5 10 15
<210> 157
   <211> 13
   <212> PRT
   <213> Artificial Sequence
<220>
   <223> FRAGMENT OF SEQ ID 2
<400> 157
   Glu Trp Arg Pro Ser Tyr Glu Lys Glu Glu Asp Glu Glu
   1 5 10
<210> 158
   <211> 15
   <212> PRT
   <213> Artificial Sequence
<220>
   <223> FRAGMENT OF SEQ ID 2
<400> 158
   Asn Tyr Asp Glu Gly Ser Glu Pro Arg Val Pro Ala Gln Arg Glu
   1 5 10 15
<210> 159
   <211> 12
   <212> PRT
   <213> Artificial Sequence
<220>
   <223> FRAGMENT OF SEQ ID 2
<400> 159
   Val Ile Ile Pro Ala Gly His Pro Val Ala Ile Thr
   1 5 10
<210> 160
   <211> 11
   <212> PRT
   <213> Artificial Sequence
<220>
   <223> FRAGMENT OF SEQ ID 2
<400> 160
   Arg His Gly Glu Trp Arg Pro Ser Tyr Glu Lys
   1 5 10
<210> 161
   <211> 9
   <212> PRT
   <213> Artificial Sequence
<220>
   <223> FRAGMENT OF SEQ ID 2
<400> 161
   Asn Tyr Asp Glu Gly Ser Glu Pro Arg
   1 5
<210> 162
   <211> 12
   <212> PRT
   <213> Artificial Sequence
<220>
   <223> FRAGMENT OF SEQ ID 2
<400> 162
   Trp Arg Pro Ser Tyr Glu Lys Glu Glu Asp Glu Glu
   1 5 10
<210> 163
   <211> 13
   <212> PRT
   <213> Artificial Sequence
<220>
   <223> FRAGMENT OF SEQ ID 2
<400> 163
   Trp Arg Pro Ser Tyr Glu Lys Gln Glu Asp Glu Glu Glu
   1               5                   10
<210> 164
   <211> 19
   <212> PRT
   <213> Artificial Sequence
<220>
   <223> FRAGMENT OF SEQ ID 2
<400> 164
   Glu Lys Arg His Gly Glu Trp Arg Pro Ser Tyr Glu Lys Gln Glu Asp
   1               5                   10                  15
   Glu Glu Glu
<210> 165
   <211> 14
   <212> PRT
   <213> Artificial Sequence
<220>
   <223> FRAGMENT OF SEQ ID 2
<400> 165
   Val Val Ile Ile Pro Ala Gly His Pro Val Ala Ile Thr Ala
   1               5                   10
<210> 166
   <211> 11
   <212> PRT
   <213> Artificial Sequence
<220>
   <223> FRAGMENT OF SEQ ID 2
<400> 166
   Lys Arg His Gly Glu Trp Arg Pro Ser Tyr Glu
   1               5                   10
<210> 167
   <211> 16
   <212> PRT
   <213> Artificial Sequence
<220>
   <223> FRAGMENT OF SEQ ID 2
<400> 167
   Gly Ser Asp Asp Asn Val Ile Ser Gln Ile Glu Asn Pro Val Lys Glu
   1 5 10 15
<210> 168
   <211> 10
   <212> PRT
   <213> Artificial Sequence
<220>
   <223> FRAGMENT OF SEQ ID 2
<400> 168
   Val Val Ile Ile Pro Ala Gly His Pro Val
   1 5 10
<210> 169
   <211> 8
   <212> PRT
   <213> Artificial Sequence
<220>
   <223> FRAGMENT OF SEQ ID 2
<400> 169
   His Gly Glu Trp Arg Pro Ser Tyr
   1 5
<210> 170
   <211> 15
   <212> PRT
   <213> Artificial Sequence
<220>
   <223> FRAGMENT OF SEQ ID 2
<400> 170
   Arg Pro Ser Tyr Glu Lys Glu Glu Asp Glu Glu Glu Gly Gln Arg
   1 5 10 15
<210> 171
   <211> 10
   <212> PRT
   <213> Artificial Sequence
<220>
   <223> FRAGMENT OF SEQ ID 2
<400> 171
   His Gly Glu Trp Arg Pro Ser Tyr Glu Lys
   1 5 10
<210> 172
   <211> 14
   <212> PRT
   <213> Artificial Sequence
<220>
   <223> FRAGMENT OF SEQ ID 2
<400> 172
   Lys Arg His Gly Glu Trp Arg Pro Ser Tyr Glu Lys Glu Glu
   1               5                   10
<210> 173
   <211> 15
   <212> PRT
   <213> Artificial Sequence
<220>
   <223> FRAGMENT OF SEQ ID 2
<400> 173
   Val Val Ile Ile Pro Ala Gly His Pro Val Ala Ile Thr Ala Ser
   1               5                   10                  15
<210> 174
   <211> 23
   <212> PRT
   <213> Artificial Sequence
<220>
   <223> FRAGMENT OF SEQ ID 2
<400> 174
   Arg Gly Asp Thr Ile Lys Leu Pro Ala Gly Thr Thr Ser Tyr Leu Val
   1               5                   10                  15
   Asn Gln Asp Asp Glu Glu Asp
               20
<210> 175
   <211> 18
   <212> PRT
   <213> Artificial Sequence
<220>
   <223> FRAGMENT OF SEQ ID 2
<400> 175
   Lys Arg His Gly Glu Trp Arg Pro Ser Tyr Glu Lys Gln Glu Asp Glu
   1               5                   10                  15
   Glu Glu
<210> 176
   <211> 20
   <212> PRT
   <213> Artificial Sequence
<220>
   <223> FRAGMENT OF SEQ ID 2
<400> 176
   Asp Glu Glu Gln Val Asp Glu Glu Trp Arg Gly Ser Gln Arg Arg Glu
   1               5                   10                  15
   Asp Pro Glu Glu
               20
<210> 177
   <211> 10
   <212> PRT
   <213> Artificial Sequence
<220>
   <223> FRAGMENT OF SEQ ID 2
<400> 177
   Arg His Gly Glu Trp Arg Pro Ser Tyr Glu
   1               5                   10
<210> 178
   <211> 11
   <212> PRT
   <213> Artificial Sequence
<220>
   <223> FRAGMENT OF SEQ ID 2
<400> 178
   His Gly Glu Trp Arg Pro Ser Tyr Glu Lys Glu
   1               5                   10
<210> 179
   <211> 18
   <212> PRT
   <213> Artificial Sequence
<220>
   <223> FRAGMENT OF SEQ ID 2
<400> 179
   Lys Arg His Gly Glu Trp Arg Pro Ser Tyr Glu Lys Glu Glu Asp Glu
   1               5                   10                  15
   Glu Glu
<210> 180
   <211> 19
   <212> PRT
   <213> Artificial Sequence
<220>
   <223> FRAGMENT OF SEQ ID 2
<400> 180
   Glu Lys Arg His Gly Glu Trp Arg Pro Ser Tyr Glu Lys Glu Glu Asp
   1               5                   10                  15
   Glu Glu Glu
<210> 181
   <211> 25
   <212> PRT
   <213> Artificial Sequence
<220>
   <223> FRAGMENT OF SEQ ID 2
<400> 181
   Thr Ile Lys Leu Pro Ala Gly Thr Thr Ser Tyr Leu Val Asn Gln Asp
   1               5                   10                  15
   Asp Glu Glu Asp Leu Arg Leu Val Asp
               20                  25
<210> 182
   <211> 17
   <212> PRT
   <213> Artificial Sequence
<220>
   <223> FRAGMENT OF SEQ ID 2
<400> 182
   Trp Arg Pro Ser Tyr Glu Lys Glu Glu Asp Glu Glu Glu Gly Gln Arg
   1               5                   10                  15
   Glu
<210> 183
   <211> 17
   <212> PRT
   <213> Artificial Sequence
<220>
   <223> FRAGMENT OF SEQ ID 2
<400> 183
   Lys Arg His Gly Glu Trp Arg Pro Ser Tyr Glu Lys Glu Glu Asp Glu
   1 5 10 15
   Glu
<210> 184
   <211> 12
   <212> PRT
   <213> Artificial Sequence
<220>
   <223> FRAGMENT OF SEQ ID 2
<400> 184
   Val Val Ile Ile Pro Ala Gly His Pro Val Ala Ile
   1 5 10
<210> 185
   <211> 13
   <212> PRT
   <213> Artificial Sequence
<220>
   <223> FRAGMENT OF SEQ ID 2
<400> 185
   Glu Trp Arg Gly Ser Gln Arg Arg Glu Asp Pro Glu Glu
   1 5 10
<210> 186
   <211> 19
   <212> PRT
   <213> Artificial Sequence
<220>
   <223> FRAGMENT OF SEQ ID 2
<400> 186
   His Gly Glu Trp Arg Pro Ser Tyr Glu Lys Gln Glu Asp Glu Glu Glu
   1 5 10 15
   Lys Gln Lys
<210> 187
   <211> 17
   <212> PRT
   <213> Artificial Sequence
<220>
   <223> FRAGMENT OF SEQ ID 2
<400> 187
   Ser Gly Ser Asp Asp Asn Val Ile Ser Gln Ile Glu Asn Pro Val Lys
   1 5 10 15
   Glu
<210> 188
   <211> 17
   <212> PRT
   <213> Artificial Sequence
<220>
   <223> FRAGMENT OF SEQ ID 2
<400> 188
   Arg Pro Ser Tyr Glu Lys Glu Glu Asp Glu Glu Glu Gly Gln Arg Glu
   1 5 10 15
   Arg
<210> 189
   <211> 13
   <212> PRT
   <213> Artificial Sequence
<220>
   <223> FRAGMENT OF SEQ ID 2
<400> 189
   Glu Lys Glu Glu Asp Glu Glu Glu Gly Gln Arg Glu Arg
   1 5 10
<210> 190
   <211> 11
   <212> PRT
   <213> Artificial Sequence
<220>
   <223> FRAGMENT OF SEQ ID 2
<400> 190
   His Gly Glu Trp Arg Pro Ser Tyr Glu Lys Gln
   1 5 10
<210> 191
   <211> 13
   <212> PRT
   <213> Artificial Sequence
<220>
   <223> FRAGMENT OF SEQ ID 2
<400> 191
   Trp Arg Pro Ser Tyr Glu Lys Glu Glu Asp Glu Glu Glu
   1 5 10
<210> 192
   <211> 13
   <212> PRT
   <213> Artificial Sequence
<220>
   <223> FRAGMENT OF SEQ ID 2
<400> 192
   Leu Ala Lys Asn Lys Asn Gln Tyr Leu Arg Gly Phe Ser
   1 5 10
<210> 193
   <211> 10
   <212> PRT
   <213> Artificial Sequence
<220>
   <223> FRAGMENT OF SEQ ID 2
<400> 193
   Asn Lys Asn Gln Tyr Leu Arg Gly Phe Ser
   1 5 10
<210> 194
   <211> 10
   <212> PRT
   <213> Artificial Sequence
<220>
   <223> FRAGMENT OF SEQ ID 2
<400> 194
   Leu Arg Gly Phe Ser Lys Asn Ile Leu Glu
   1 5 10
<210> 195
   <211> 15
   <212> PRT
   <213> Artificial Sequence
<220>
   <223> FRAGMENT OF SEQ ID 2
<400> 195
   Leu Ala Lys Asn Lys Asn Gln Tyr Leu Arg Gly Phe Ser Lys Asn
   1 5 10 15
<210> 196
   <211> 11
   <212> PRT
   <213> Artificial Sequence
<220>
   <223> FRAGMENT OF SEQ ID 2
<400> 196
   Thr Val Leu Ser Pro Asn Asp Arg Asn Ser Tyr
   1 5 10
<210> 197
   <211> 12
   <212> PRT
   <213> Artificial Sequence
<220>
   <223> FRAGMENT OF SEQ ID 2
<400> 197
   Gln Tyr Leu Arg Gly Phe Ser Lys Asn Ile Leu Glu
   1 5 10
<210> 198
   <211> 19
   <212> PRT
   <213> Artificial Sequence
<220>
   <223> FRAGMENT OF SEQ ID 2
<400> 198
   Gly Lys Ala Ile Leu Thr Val Leu Ser Pro Asn Asp Arg Asn Ser Tyr
   1 5 10 15
   Asn Leu Glu
<210> 199
   <211> 9
   <212> PRT
   <213> Artificial Sequence
<220>
   <223> FRAGMENT OF SEQ ID 2
<400> 199
   Arg Gly Phe Ser Lys Asn Ile Leu Glu
   1 5
<210> 200
   <211> 15
   <212> PRT
   <213> Artificial Sequence
<220>
   <223> FRAGMENT OF SEQ ID 2
<400> 200
   Asn Lys Asn Gln Tyr Leu Arg Gly Phe Ser Lys Asn Ile Leu Glu
   1 5 10 15
<210> 201
   <211> 15
   <212> PRT
   <213> Artificial Sequence
<220>
   <223> FRAGMENT OF SEQ ID 2
<400> 201
   Ala Ser Ser Asn Leu Asn Leu Leu Gly Phe Gly Ile Asn Ala Glu
   1 5 10 15
<210> 202
   <211> 10
   <212> PRT
   <213> Artificial Sequence
<220>
   <223> FRAGMENT OF SEQ ID 2
<400> 202
   Ala Ser Ser Asn Leu Asn Leu Leu Gly Phe
   1 5 10
<210> 203
   <211> 14
   <212> PRT
   <213> Artificial Sequence
<220>
   <223> FRAGMENT OF SEQ ID 2
<400> 203
   Leu Ala Lys Asn Lys Asn Gln Tyr Leu Arg Gly Phe Ser Lys
   1 5 10
<210> 204
   <211> 22
   <212> PRT
   <213> Artificial Sequence
<220>
   <223> FRAGMENT OF SEQ ID 2
<400> 204
   Arg Gly Asp Thr Ile Lys Leu Pro Ala Gly Thr Thr Ser Tyr Leu Val
   1               5                   10                  15
   Asn Gln Asp Asp Glu Glu
               20
<210> 205
   <211> 24
   <212> PRT
   <213> Artificial Sequence
<220>
   <223> FRAGMENT OF SEQ ID 2
<400> 205
   Ala Arg Leu Ser Pro Gly Asp Val Val Ile Ile Pro Ala Gly His Pro
   1               5                   10                  15
   Val Ala Ile Thr Ala Ser Ser Asn
               20
<210> 206
   <211> 12
   <212> PRT
   <213> Artificial Sequence
<220>
   <223> FRAGMENT OF SEQ ID 2
<400> 206
   Val Gln Arg Tyr Glu Ala Arg Leu Ser Pro Gly Asp
   1               5                   10
<210> 207
   <211> 20
   <212> PRT
   <213> Artificial Sequence
<220>
   <223> FRAGMENT OF SEQ ID 2
<400> 207
   Ala Arg Leu Ser Pro Gly Asp Val Val Ile Ile Pro Ala Gly His Pro
   1               5                   10                  15
   Val Ala Ile Thr
               20
<210> 208
   <211> 21
   <212> PRT
   <213> Artificial Sequence
<220>
   <223> FRAGMENT OF SEQ ID 2
<400> 208
   Arg Gly Asp Thr Ile Lys Leu Pro Ala Gly Thr Thr Ser Tyr Leu Val
   1 5 10 15
   Asn Gln Asp Asp Glu
   20
<210> 209
   <211> 18
   <212> PRT
   <213> Artificial Sequence
<220>
   <223> FRAGMENT OF SEQ ID 2
<400> 209
   Ala Arg Leu Ser Pro Gly Asp Val Val Ile Ile Pro Ala Gly His Pro
   1 5 10 15
   Val Ala
<210> 210
   <211> 20
   <212> PRT
   <213> Artificial Sequence
<220>
   <223> FRAGMENT OF SEQ ID 2
<400> 210
   Gly Ala Leu Met Leu Pro His Tyr Asn Ser Arg Ala Ile Val Val Leu
   1 5 10 15
   Leu Val Asn Glu
   20
<210> 211
   <211> 23
   <212> PRT
   <213> Artificial Sequence
<220>
   <223> FRAGMENT OF SEQ ID 2
<400> 211
   Ala Arg Leu Ser Pro Gly Asp Val Val Ile Ile Pro Ala Gly His Pro
   1 5 10 15
   Val Ala Ile Thr Ala Ser Ser
   20
<210> 212
   <211> 37
   <212> PRT
   <213> Artificial Sequence
<220>
   <223> FRAGMENT OF SEQ ID 2
<400> 212
   Leu Ser Pro Gly Asp Val Val Ile Ile Pro Ala Gly His Pro Val Ala
   1 5 10 15
   Ile Thr Ala Ser Ser Asn Leu Asn Leu Leu Gly Phe Gly Ile Asn Ala
   20 25 30
   Glu Asn Asn Glu Arg
   35
<210> 213
   <211> 22
   <212> PRT
   <213> Artificial Sequence
<220>
   <223> FRAGMENT OF SEQ ID 2
<400> 213
   Ala Arg Leu Ser Pro Gly Asp Val Val Ile Ile Pro Ala Gly His Pro
   1 5 10 15
   Val Ala Ile Thr Ala Ser
   20
<210> 214
   <211> 23
   <212> PRT
   <213> Artificial Sequence
<220>
   <223> FRAGMENT OF SEQ ID 2
<400> 214
   Leu Ser Pro Gly Asp Val Val Ile Ile Pro Ala Gly His Pro Val Ala
   1 5 10 15
   Ile Thr Ala Ser Ser Asn Leu
   20
<210> 215
   <211> 21
   <212> PRT
   <213> Artificial Sequence
<220>
   <223> FRAGMENT OF SEQ ID 2
<400> 215
   Ala Arg Leu Ser Pro Gly Asp Val Val Ile Ile Pro Ala Gly His Pro
   1 5 10 15
   Val Ala Ile Thr Ala
   20
<210> 216
   <211> 28
   <212> PRT
   <213> Artificial Sequence
<220>
   <223> FRAGMENT OF SEQ ID 3
<400> 216
   Asn Trp Glu Asn Val Leu Leu Gly Leu Gly Val Ala Gly Ser Ala Pro
   1 5 10 15
   Gly Ile Glu Gly Asp Glu Ile Ala Pro Leu Ala Lys
   20 25
<210> 217
   <211> 17
   <212> PRT
   <213> Artificial Sequence
<220>
   <223> FRAGMENT OF SEQ ID 3
<400> 217
   Tyr Asp Gln Tyr Lys Asp Ala Trp Asp Thr Ser Val Val Ala Glu Ile
   1 5 10 15
   Lys
<210> 218
   <211> 16
   <212> PRT
   <213> Artificial Sequence
<220>
   <223> FRAGMENT OF SEQ ID 3
<400> 218
   Ser Ser Phe Asp Phe Ile Asp Gly Tyr Asp Thr Pro Val Glu Gly Arg
   1               5                   10                  15
<210> 219
   <211> 13
   <212> PRT
   <213> Artificial Sequence
<220>
   <223> FRAGMENT OF SEQ ID 3
<400> 219
   Gly Pro Asp Thr Gly Val Asp Tyr Lys Asp Asn Gln Met
   1               5                   10
<210> 220
   <211> 11
   <212> PRT
   <213> Artificial Sequence
<220>
   <223> FRAGMENT OF SEQ ID 3
<400> 220
   Ile Leu Asn Leu Asn Asn Asn Pro Tyr Phe Lys
   1               5                   10
<210> 221
   <211> 9
   <212> PRT
   <213> Artificial Sequence
<220>
   <223> FRAGMENT OF SEQ ID 3
<400> 221
   Val Val Gly Thr Pro Ala Tyr Glu Glu
   1               5
<210> 222
   <211> 11
   <212> PRT
   <213> Artificial Sequence
<220>
   <223> FRAGMENT OF SEQ ID 3
<400> 222
   Ile Asp Gly Tyr Asp Thr Pro Val Glu Gly Arg
   1 5 10
<210> 223
   <211> 8
   <212> PRT
   <213> Artificial Sequence
<220>
   <223> FRAGMENT OF SEQ ID 3
<400> 223
   Val Val Gly Thr Pro Ala Tyr Glu
   1 5
<210> 224
   <211> 11
   <212> PRT
   <213> Artificial Sequence
<220>
   <223> FRAGMENT OF SEQ ID 3
<400> 224
   Ile Tyr Gly Pro Asp Thr Gly Val Asp Tyr Lys
   1 5 10
<210> 225
   <211> 12
   <212> PRT
   <213> Artificial Sequence
<220>
   <223> FRAGMENT OF SEQ ID 3
<400> 225
   Val Ala Gly Ser Ala Pro Gly Ile Glu Gly Asp Glu
   1 5 10
<210> 226
   <211> 16
   <212> PRT
   <213> Artificial Sequence
<220>
   <223> FRAGMENT OF SEQ ID 3
<400> 226
   Ile Tyr Gly Pro Asp Thr Gly Val Asp Tyr Lys Asp Asn Gln Met Arg
   1 5 10 15
<210> 227
   <211> 12
   <212> PRT
   <213> Artificial Sequence
<220>
   <223> FRAGMENT OF SEQ ID 3
<400> 227
   Val Val Gly Thr Pro Ala Tyr Glu Glu Met Val Arg
   1               5                   10
<210> 228
   <211> 13
   <212> PRT
   <213> Artificial Sequence
<220>
   <223> FRAGMENT OF SEQ ID 3
<400> 228
   Asp Phe Ile Asp Gly Tyr Asp Thr Pro Val Glu Gly Arg
   1               5                   10
<210> 229
   <211> 22
   <212> PRT
   <213> Artificial Sequence
<220>
   <223> FRAGMENT OF SEQ ID 3
<400> 229
   Leu Gly Leu Gly Val Ala Gly Ser Ala Pro Gly Ile Glu Gly Asp Glu
   1               5                   10                  15
   Ile Ala Pro Leu Ala Lys
               20
<210> 230
   <211> 21
   <212> PRT
   <213> Artificial Sequence
<220>
   <223> FRAGMENT OF SEQ ID 3
<400> 230
   Phe Asn Ala Pro Leu Ala His Leu Ile Met Ala Gly Ala Asp Val Leu
   1               5                   10                  15
   Ala Val Pro Ser Arg
               20
<210> 231
   <211> 16
   <212> PRT
   <213> Artificial Sequence
<220>
   <223> FRAGMENT OF SEQ ID 3
<400> 231
   Leu Gly Leu Gly Val Ala Gly Ser Ala Pro Gly Ile Glu Gly Asp Glu
   1               5                   10                  15
<210> 232
   <211> 20
   <212> PRT
   <213> Artificial Sequence
<220>
   <223> FRAGMENT OF SEQ ID 3
<400> 232
   Leu Gly Leu Gly Val Ala Gly Ser Ala Pro Gly Ile Glu Gly Asp Glu
   1               5                   10                  15
   Ile Ala Pro Leu
               20
<210> 233
   <211> 18
   <212> PRT
   <213> Artificial Sequence
<220>
   <223> FRAGMENT OF SEQ ID 3
<400> 233
   Val Leu Thr Val Ser Pro Tyr Tyr Ala Glu Glu Leu Ile Ser Gly Ile
   1               5                   10                  15
   Ala Arg
<210> 234
   <211> 13
   <212> PRT
   <213> Artificial Sequence
<220>
   <223> FRAGMENT OF SEQ ID 3
<400> 234
   Glu Ala Leu Gln Ala Glu Ala Gly Leu Pro Val Asp Arg
   1 5 10
<210> 235
   <211> 15
   <212> PRT
   <213> Artificial Sequence
<220>
   <223> FRAGMENT OF SEQ ID 3
<400> 235
   Leu Gly Leu Gly Val Ala Gly Ser Ala Pro Gly Ile Glu Gly Asp
   1 5 10 15
<210> 236
   <211> 13
   <212> PRT
   <213> Artificial Sequence
<220>
   <223> FRAGMENT OF SEQ ID 3
<400> 236
   Ile Met Ala Gly Ala Asp Val Leu Ala Val Pro Ser Arg
   1 5 10
<210> 237
   <211> 15
   <212> PRT
   <213> Artificial Sequence
<220>
   <223> FRAGMENT OF SEQ ID 3
<400> 237
   Gly Leu Gly Val Ala Gly Ser Ala Pro Gly Ile Glu Gly Asp Glu
   1 5 10 15
<210> 238
   <211> 14
   <212> PRT
   <213> Artificial Sequence
<220>
   <223> FRAGMENT OF SEQ ID 3
<400> 238
   Glu Ala Leu Gln Ala Glu Ala Gly Leu Pro Val Asp Arg Lys
   1 5 10
<210> 239
   <211> 21
   <212> PRT
   <213> Artificial Sequence
<220>
   <223> FRAGMENT OF SEQ ID 3
<400> 239
   Thr Gly Gly Leu Gly Asp Val Leu Gly Gly Leu Pro Pro Ala Met Ala
   1               5                   10                  15
   Ala Asn Gly His Arg
               20
<210> 240
   <211> 11
   <212> PRT
   <213> Artificial Sequence
<220>
   <223> FRAGMENT OF SEQ ID 3
<400> 240
   Leu Glu Glu Gln Lys Gly Pro Asp Val Met Ala
   1               5                   10
<210> 241
   <211> 20
   <212> PRT
   <213> Artificial Sequence
<220>
   <223> FRAGMENT OF SEQ ID 3
<400> 241
   Leu Gly Val Ala Gly Ser Ala Pro Gly Ile Glu Gly Asp Glu Ile Ala
   1               5                   10                  15
   Pro Leu Ala Lys
               20
<210> 242
   <211> 21
   <212> PRT
   <213> Artificial Sequence
<220>
   <223> FRAGMENT OF SEQ ID 3
<400> 242
   Gly Leu Gly Val Ala Gly Ser Ala Pro Gly Ile Glu Gly Asp Glu Ile
   1 5 10 15
   Ala Pro Leu Ala Lys
   20
<210> 243
   <211> 15
   <212> PRT
   <213> Artificial Sequence
<220>
   <223> FRAGMENT OF SEQ ID 3
<400> 243
   Thr Gly Gly Leu Gly Asp Val Leu Gly Gly Leu Pro Pro Ala Met
   1 5 10 15
<210> 244
   <211> 19
   <212> PRT
   <213> Artificial Sequence
<220>
   <223> FRAGMENT OF SEQ ID 3
<400> 244
   Asn Val Leu Leu Gly Leu Gly Val Ala Gly Ser Ala Pro Gly Ile Glu
   1 5 10 15
   Gly Asp Glu
<210> 245
   <211> 12
   <212> PRT
   <213> Artificial Sequence
<220>
   <223> FRAGMENT OF SEQ ID 4
<400> 245
   Thr Val Phe Asp Gly Val Leu Arg Pro Gly Gln Leu
   1 5 10
<210> 246
   <211> 16
   <212> PRT
   <213> Artificial Sequence
<220>
   <223> FRAGMENT OF SEQ ID 4
<400> 246
   Arg Leu Gln Ser Gln Asn Asp Gln Arg Gly Glu Ile Ile His Val Lys
   1 5 10 15
<210> 247
   <211> 15
   <212> PRT
   <213> Artificial Sequence
<220>
   <223> FRAGMENT OF SEQ ID 5
<400> 247
   Glu Gly Tyr Tyr Gly Glu Gln Gln Gln Gln Pro Gly Met Thr Arg
   1 5 10 15
<210> 248
   <211> 14
   <212> PRT
   <213> Artificial Sequence
<220>
   <223> FRAGMENT OF SEQ ID 5
<400> 248
   Gly Tyr Tyr Gly Glu Gln Gln Gln Gln Pro Gly Met Thr Arg
   1 5 10
<210> 249
   <211> 16
   <212> PRT
   <213> Artificial Sequence
<220>
   <223> FRAGMENT OF SEQ ID 5
<400> 249
   Glu Glu Gly Tyr Tyr Gly Glu Gln Gln Gln Gln Pro Gly Met Thr Arg
   1 5 10 15
<210> 250
   <211> 20
   <212> PRT
   <213> Artificial Sequence
<220>
   <223> FRAGMENT OF SEQ ID 5
<400> 250
   Tyr Tyr Gly Gly Glu Gly Ser Ser Ser Glu Gln Gly Tyr Tyr Gly Glu
   1 5 10 15
   Gly Ser Ser Glu
   20
<210> 251
   <211> 19
   <212> PRT
   <213> Artificial Sequence
<220>
   <223> FRAGMENT OF SEQ ID 5
<400> 251
   Tyr Gly Gly Glu Gly Ser Ser Ser Glu Gln Gly Tyr Tyr Gly Glu Gly
   1               5                   10                  15
   Ser Ser Glu
<210> 252
   <211> 18
   <212> PRT
   <213> Artificial Sequence
<220>
   <223> FRAGMENT OF SEQ ID 5
<400> 252
   Ser Ser Glu Glu Gly Tyr Tyr Gly Glu Gln Gln Gln Gln Pro Gly Met
   1               5                   10                  15
   Thr Arg
<210> 253
   <211> 17
   <212> PRT
   <213> Artificial Sequence
<220>
   <223> FRAGMENT OF SEQ ID 5
<400> 253
   Ser Glu Glu Gly Tyr Tyr Gly Glu Gln Gln Gln Gln Pro Gly Met Thr
   1               5                   10                  15
   Arg
<210> 254
   <211> 11
   <212> PRT
   <213> Artificial Sequence
<220>
   <223> FRAGMENT OF SEQ ID 5
<400> 254
   Gln Gly Tyr Tyr Gly Glu Gly Ser Ser Glu Glu
   1               5                   10
<210> 255
   <211> 22
   <212> PRT
   <213> Artificial Sequence
<220>
   <223> FRAGMENT OF SEQ ID 5
<400> 255
   Tyr Gly Gly Glu Gly Ser Ser Ser Glu Gln Gly Tyr Tyr Gly Glu Gly
   1               5                   10                  15
   Ser Ser Glu Glu Gly Tyr
               20
<210> 256
   <211> 12
   <212> PRT
   <213> Artificial Sequence
<220>
   <223> FRAGMENT OF SEQ ID 5
<400> 256
   Tyr Gly Glu Gln Gln Gln Gln Pro Gly Met Thr Arg
   1               5                   10
<210> 257
   <211> 13
   <212> PRT
   <213> Artificial Sequence
<220>
   <223> FRAGMENT OF SEQ ID 5
<400> 257
   Tyr Tyr Gly Glu Gln Gln Gln Gln Pro Gly Met Thr Arg
   1               5                   10
<210> 258
   <211> 20
   <212> PRT
   <213> Artificial Sequence
<220>
   <223> FRAGMENT OF SEQ ID 5
<400> 258
   Ser Tyr Glu Glu Ser Met Pro Met Pro Leu Glu Gln Gly Trp Ser Ser
   1 5 10 15
   Ser Ser Ser Glu
   20
<210> 259
   <211> 23
   <212> PRT
   <213> Artificial Sequence
<220>
   <223> FRAGMENT OF SEQ ID 5
<400> 259
   Tyr Tyr Gly Glu Gly Ser Ser Glu Glu Gly Tyr Tyr Gly Glu Gln Gln
   1 5 10 15
   Gln Gln Pro Gly Met Thr Arg
   20
<210> 260
   <211> 10
   <212> PRT
   <213> Artificial Sequence
<220>
   <223> FRAGMENT OF SEQ ID 5
<400> 260
   Gln Gln Gln Gln Pro Gly Met Thr Arg Val
   1 5 10
<210> 261
   <211> 11
   <212> PRT
   <213> Artificial Sequence
<220>
   <223> FRAGMENT OF SEQ ID 5
<400> 261
   Gly Glu Gln Gln Gln Gln Pro Gly Met Thr Arg
   1 5 10
<210> 262
   <211> 21
   <212> PRT
   <213> Artificial Sequence
<220>
   <223> FRAGMENT OF SEQ ID 5
<400> 262
   Ser Tyr Glu Glu Ser Met Pro Met Pro Leu Glu Gln Gly Trp Ser Ser
   1               5                   10                  15
   Ser Ser Ser Glu Tyr
               20
<210> 263
   <211> 23
   <212> PRT
   <213> Artificial Sequence
<220>
   <223> FRAGMENT OF SEQ ID 5
<400> 263
   Tyr Tyr Gly Gly Glu Gly Ser Ser Ser Glu Gln Gly Tyr Tyr Gly Glu
   1               5                   10                  15
   Gly Ser Ser Glu Glu Gly Tyr
               20
<210> 264
   <211> 14
   <212> PRT
   <213> Artificial Sequence
<220>
   <223> FRAGMENT OF SEQ ID 5
<400> 264
   Tyr Gly Glu Gln Gln Gln Gln Pro Gly Met Thr Arg Val Arg
   1               5                   10
<210> 265
   <211> 21
   <212> PRT
   <213> Artificial Sequence
<220>
   <223> FRAGMENT OF SEQ ID 5
<400> 265
   Gly Glu Gly Ser Ser Glu Glu Gly Tyr Tyr Gly Glu Gln Gln Gln Gln
   1               5                   10                  15
   Pro Gly Met Thr Arg
               20
<210> 266
   <211> 22
   <212> PRT
   <213> Artificial Sequence
<220>
   <223> FRAGMENT OF SEQ ID 5
<400> 266
   Tyr Gly Glu Gly Ser Ser Glu Glu Gly Tyr Tyr Gly Glu Gln Gln Gln
   1               5                   10                  15
   Gln Pro Gly Met Thr Arg
               20
<210> 267
   <211> 11
   <212> PRT
   <213> Artificial Sequence
<220>
   <223> FRAGMENT OF SEQ ID 5
<400> 267
   Gln Gln Gln Gln Pro Gly Met Thr Arg Val Arg
   1               5                   10
<210> 268
   <211> 25
   <212> PRT
   <213> Artificial Sequence
<220>
   <223> FRAGMENT OF SEQ ID 5
<400> 268
   Gln Tyr Ala Ala Gln Leu Pro Ser Met Cys Arg Val Glu Pro Gln Gln
   1               5                   10                  15
   Cys Ser Ile Phe Ala Ala Gly Gln Tyr
               20                  25
<210> 269
   <211> 12
   <212> PRT
   <213> Artificial Sequence
<220>
   <223> FRAGMENT OF SEQ ID 6
<400> 269
   Thr Val Phe Asn Gly Val Leu Arg Pro Gly Gln Leu
   1               5                   10
<210> 270
   <211> 13
   <212> PRT
   <213> Artificial Sequence
<220>
   <223> FRAGMENT OF SEQ ID 6
<400> 270
   Thr Val Phe Asn Gly Val Leu Arg Pro Gly Gln Leu Leu
   1               5                   10
<210> 271
   <211> 20
   <212> PRT
   <213> Artificial Sequence
<220>
   <223> FRAGMENT OF SEQ ID 6
<400> 271
   Ser Gly Phe Asn Asn Glu Leu Leu Ser Glu Ala Leu Gly Val Asn Ala
   1               5                   10                  15
   Leu Val Ala Lys
               20
<210> 272
   <211> 9
   <212> PRT
   <213> Artificial Sequence
<220>
   <223> FRAGMENT OF SEQ ID 6
<400> 272
   Asn Gly Val Leu Arg Pro Gly Gln Leu
   1               5
<210> 273
   <211> 17
   <212> PRT
   <213> Artificial Sequence
<220>
   <223> FRAGMENT OF SEQ ID 6
<400> 273
   Ala Leu Val Ala Lys Arg Leu Gln Gly Gln Asn Asp Gln Arg Gly Glu
   1               5                   10                  15
   Ile
<210> 274
   <211> 10
   <212> PRT
   <213> Artificial Sequence
<220>
   <223> FRAGMENT OF SEQ ID 6
<400> 274
   Val Pro Arg Tyr Ser Asn Thr Pro Gly Met
   1 5 10
<210> 275
   <211> 10
   <212> PRT
   <213> Artificial Sequence
<220>
   <223> FRAGMENT OF SEQ ID 6
<400> 275
   Pro Arg Tyr Ser Asn Thr Pro Gly Met Val
   1 5 10
<210> 276
   <211> 9
   <212> PRT
   <213> Artificial Sequence
<220>
   <223> FRAGMENT OF SEQ ID 6
<400> 276
   Tyr Ser Asn Thr Pro Gly Met Val Tyr
   1 5
<210> 277
   <211> 11
   <212> PRT
   <213> Artificial Sequence
<220>
   <223> FRAGMENT OF SEQ ID 6
<400> 277
   Leu Val Pro Arg Tyr Ser Asn Thr Pro Gly Met
   1 5 10
<210> 278
   <211> 10
   <212> PRT
   <213> Artificial Sequence
<220>
   <223> FRAGMENT OF SEQ ID 6
<400> 278
   Phe Tyr Asn Glu Gly Asp Ala Pro Val Val
   1               5                   10
<210> 279
   <211> 12
   <212> PRT
   <213> Artificial Sequence
<220>
   <223> FRAGMENT OF SEQ ID 6
<400> 279
   Phe Tyr Asn Glu Gly Asp Ala Pro Val Val Ala Leu
   1               5                   10
<210> 280
   <211> 15
   <212> PRT
   <213> Artificial Sequence
<220>
   <223> FRAGMENT OF SEQ ID 6
<400> 280
   Phe Glu Pro Leu Arg Arg Val Arg Ser Glu Ala Gly Val Thr Glu
   1               5                   10                  15
<210> 281
   <211> 13
   <212> PRT
   <213> Artificial Sequence
<220>
   <223> FRAGMENT OF SEQ ID 6
<400> 281
   Phe Tyr Asn Glu Gly Asp Ala Pro Val Val Ala Leu Tyr
   1               5                   10
<210> 282
   <211> 11
   <212> PRT
   <213> Artificial Sequence
<220>
   <223> FRAGMENT OF SEQ ID 6
<400> 282
   Phe Tyr Asn Glu Gly Asp Ala Pro Val Val Ala
   1               5                   10
<210> 283
   <211> 16
   <212> PRT
   <213> Artificial Sequence
<220>
   <223> FRAGMENT OF SEQ ID 7
<400> 283
   His Gly Pro Val Glu Met Pro Tyr Thr Leu Leu Tyr Pro Ser Ser Lys
   1 5 10 15
<210> 284
   <211> 9
   <212> PRT
   <213> Artificial Sequence
<220>
   <223> FRAGMENT OF SEQ ID 8
<400> 284
   Leu Asp Ala Leu Glu Pro Asp Asn Arg
   1 5
<210> 285
   <211> 8
   <212> PRT
   <213> Artificial Sequence
<220>
   <223> FRAGMENT OF SEQ ID 8
<400> 285
   Asp Ala Leu Glu Pro Asp Asn Arg
   1 5
<210> 286
   <211> 24
   <212> PRT
   <213> Artificial Sequence
<220>
   <223> FRAGMENT OF SEQ ID 8
<400> 286
   His Gly Ser Leu His Lys Asn Ala Met Phe Val Pro His Tyr Asn Leu
   1 5 10 15
   Asn Ala Asn Ser Ile Ile Tyr Ala
   20
<210> 287
   <211> 20
   <212> PRT
   <213> Artificial Sequence
<220>
   <223> FRAGMENT OF SEQ ID 8
<400> 287
   Leu Ala Gly Thr Ser Ser Val Ile Asn Asn Leu Pro Leu Asp Val Val
   1               5                   10                  15
   Ala Ala Thr Phe
               20
<210> 288
   <211> 16
   <212> PRT
   <213> Artificial Sequence
<220>
   <223> FRAGMENT OF SEQ ID 8
<400> 288
   Phe Arg Glu Gly Asp Ile Ile Ala Val Pro Thr Gly Ile Val Phe Trp
   1               5                   10                  15
<210> 289
   <211> 24
   <212> PRT
   <213> Artificial Sequence
<220>
   <223> FRAGMENT OF SEQ ID 8
<400> 289
   Gly Thr Ser Ser Val Ile Asn Asn Leu Pro Leu Asp Val Val Ala Ala
   1               5                   10                  15
   Thr Phe Asn Leu Gln Arg Asn Glu
               20
<210> 290
   <211> 18
   <212> PRT
   <213> Artificial Sequence
<220>
   <223> FRAGMENT OF SEQ ID 8
<400> 290
   Lys Gly Ala Ile Val Lys Val Lys Gly Gly Leu Ser Ile Ile Ser Pro
   1 5 10 15
   Pro Glu
<210> 291
   <211> 15
   <212> PRT
   <213> Artificial Sequence
<220>
   <223> FRAGMENT OF SEQ ID 8
<400> 291
   Arg Leu Ala Gly Thr Ser Ser Val Ile Asn Asn Leu Pro Leu Asp
   1 5 10 15
<210> 292
   <211> 25
   <212> PRT
   <213> Artificial Sequence
<220>
   <223> FRAGMENT OF SEQ ID 8
<400> 292
   Ala Gly Thr Ser Ser Val Ile Asn Asn Leu Pro Leu Asp Val Val Ala
   1 5 10 15
   Ala Thr Phe Asn Leu Gln Arg Asn Glu
   20 25
<210> 293
   <211> 12
   <212> PRT
   <213> Artificial Sequence
<220>
   <223> FRAGMENT OF SEQ ID 8
<400> 293
   Ala Gly Thr Ser Ser Val Ile Asn Asn Leu Pro Leu
   1 5 10
<210> 294
   <211> 17
   <212> PRT
   <213> Artificial Sequence
<220>
   <223> FRAGMENT OF SEQ ID 8
<400> 294
   Leu Ala Gly Thr Ser Ser Val Ile Asn Asn Leu Pro Leu Asp Val Val
   1 5 10 15
   Ala
<210> 295
   <211> 14
   <212> PRT
   <213> Artificial Sequence
<220>
   <223> FRAGMENT OF SEQ ID 8
<400> 295
   Ala Gly Thr Ser Ser Val Ile Asn Asn Leu Pro Leu Asp Val
   1 5 10
<210> 296
   <211> 17
   <212> PRT
   <213> Artificial Sequence
<220>
   <223> FRAGMENT OF SEQ ID 8
<400> 296 Trp
<210> 297
   <211> 16
   <212> PRT
   <213> Artificial Sequence
<220>
   <223> FRAGMENT OF SEQ ID 8
<400> 297
   Ala Gly Arg Ile Lys Thr Val Thr Ser Leu Asp Leu Pro Val Leu Arg
   1 5 10 15
<210> 298
   <211> 15
   <212> PRT
   <213> Artificial Sequence
<220>
   <223> FRAGMENT OF SEQ ID 8
<400> 298
   Phe Arg Glu Gly Asp Ile Ile Ala Val Pro Thr Gly Ile Val Phe
   1 5 10 15
<210> 299
   <211> 13
   <212> PRT
   <213> Artificial Sequence
<220>
   <223> FRAGMENT OF SEQ ID 8
<400> 299
   Ala Gly Thr Ser Ser Val Ile Asn Asn Leu Pro Leu Asp
   1 5 10
<210> 300
   <211> 13
   <212> PRT
   <213> Artificial Sequence
<220>
   <223> FRAGMENT OF SEQ ID 8
<400> 300
   Leu Ala Gly Thr Ser Ser Val Ile Asn Asn Leu Pro Leu
   1 5 10
<210> 301
   <211> 16
   <212> PRT
   <213> Artificial Sequence
<220>
   <223> FRAGMENT OF SEQ ID 8
<400> 301
   Leu Ala Gly Thr Ser Ser Val Ile Asn Asn Leu Pro Leu Asp Val Val
   1 5 10 15
<210> 302
   <211> 13
   <212> PRT
   <213> Artificial Sequence
<220>
   <223> FRAGMENT OF SEQ ID 8
<400> 302
   Glu Gly Asp Ile Ile Ala Val Pro Thr Gly Ile Val Phe
   1 5 10
<210> 303
   <211> 15
   <212> PRT
   <213> Artificial Sequence
<220>
   <223> FRAGMENT OF SEQ ID 8
<400> 303
   Leu Ala Gly Thr Ser Ser Val Ile Asn Asn Leu Pro Leu Asp Val
   1 5 10 15
<210> 304
   <211> 20
   <212> PRT
   <213> Artificial Sequence
<220>
   <223> FRAGMENT OF SEQ ID 8
<400> 304
   Ala Gly Arg Ala Leu Thr Val Pro Gln Asn Tyr Ala Val Ala Ala Lys
   1 5 10 15
   Ser Leu Ser Asp
   20
<210> 305
   <211> 12
   <212> PRT
   <213> Artificial Sequence
<220>
   <223> FRAGMENT OF SEQ ID 8
<400> 305
   Ala Gly Arg Ala Leu Thr Val Pro Gln Asn Tyr Ala
   1 5 10
<210> 306
   <211> 14
   <212> PRT
   <213> Artificial Sequence
<220>
   <223> FRAGMENT OF SEQ ID 8
<400> 306
   Leu Ala Gly Thr Ser Ser Val Ile Asn Asn Leu Pro Leu Asp
   1 5 10
<210> 307
   <211> 23
   <212> PRT
   <213> Artificial Sequence
<220>
   <223> FRAGMENT OF SEQ ID 8
<400> 307
   Arg Ala Gly Ile Ala Arg Leu Ala Gly Thr Ser Ser Val Ile Asn Asn
   1               5                   10                  15
   Leu Pro Leu Asp Val Val Ala
               20
<210> 308
   <211> 16
   <212> PRT
   <213> Artificial Sequence
<220>
   <223> FRAGMENT OF SEQ ID 9
<400> 308
   Arg Ala Ser Ser Asn Leu Asn Leu Leu Gly Phe Gly Ile Asn Ala Glu
   1               5                   10                  15
<210> 309
   <211> 12
   <212> PRT
   <213> Artificial Sequence
<220>
   <223> FRAGMENT OF SEQ ID 9
<400> 309
   Val Thr Val Asn Glu Gly Lys Gly Asp Phe Glu Leu
   1               5                   10
<210> 310
   <211> 17
   <212> PRT
   <213> Artificial Sequence
<220>
   <223> FRAGMENT OF SEQ ID 9
<400> 310
   Val Arg Ala Ser Ser Asn Leu Asn Leu Leu Gly Phe Gly Ile Asn Ala
   1               5                   10                  15
   Glu
<210> 311
   <211> 13
   <212> PRT
   <213> Artificial Sequence
<220>
   <223> FRAGMENT OF SEQ ID 9
<400> 311
   Val Arg Ala Ser Ser Asn Leu Asn Leu Leu Gly Phe Gly
   1               5                   10
<210> 312
   <211> 17
   <212> PRT
   <213> Artificial Sequence
<220>
   <223> FRAGMENT OF SEQ ID 9
<400> 312
   His Pro Val Ala Val Arg Ala Ser Ser Asn Leu Asn Leu Leu Gly Phe
   1               5                   10                  15
   Gly
<210> 313
   <211> 16
   <212> PRT
   <213> Artificial Sequence
<220>
   <223> FRAGMENT OF SEQ ID 9
<400> 313
   Thr Lys Asn Gln Val Gln Ser Tyr Lys Ala Lys Leu Thr Pro Gly Asp
   1               5                   10                  15
<210> 314
   <211> 15
   <212> PRT
   <213> Artificial Sequence
<220>
   <223> FRAGMENT OF SEQ ID 9
<400> 314
   His Pro Val Ala Val Arg Ala Ser Ser Asn Leu Asn Leu Leu Gly
   1               5                   10                  15
<210> 315
   <211> 15
   <212> PRT
   <213> Artificial Sequence
<220>
   <223> FRAGMENT OF SEQ ID 9
<400> 315
   Lys Ala Lys Leu Thr Pro Gly Asp Val Phe Val Ile Pro Ala Gly
   1 5 10 15
<210> 316
   <211> 19
   <212> PRT
   <213> Artificial Sequence
<220>
   <223> FRAGMENT OF SEQ ID 9
<400> 316
   Asp Leu Thr Phe Pro Gly Ser Ala Gln Glu Val Asp Arg Leu Leu Glu
   1 5 10 15
   Asn Gln Lys
<210> 317
   <211> 9
   <212> PRT
   <213> Artificial Sequence
<220>
   <223> FRAGMENT OF SEQ ID 9
<400> 317
   Pro Ala Gly His Pro Val Ala Val Arg
   1 5
<210> 318
   <211> 18
   <212> PRT
   <213> Artificial Sequence
<220>
   <223> FRAGMENT OF SEQ ID 9
<400> 318
   Ala Lys Leu Thr Pro Gly Asp Val Phe Val Ile Pro Ala Gly His Pro
   1 5 10 15
   Val Ala
<210> 319
   <211> 21
   <212> PRT
   <213> Artificial Sequence
<220>
   <223> FRAGMENT OF SEQ ID 9
<400> 319
   Ser Tyr Lys Ala Lys Leu Thr Pro Gly Asp Val Phe Val Ile Pro Ala
   1               5                   10                  15
   Gly His Pro Val Ala
               20
<210> 320
   <211> 18
   <212> PRT
   <213> Artificial Sequence
<220>
   <223> FRAGMENT OF SEQ ID 9
<400> 320
   Leu Thr Pro Gly Asp Val Phe Val Ile Pro Ala Gly His Pro Val Ala
   1               5                   10                  15
   Val Arg
<210> 321
   <211> 19
   <212> PRT
   <213> Artificial Sequence
<220>
   <223> FRAGMENT OF SEQ ID 9
<400> 321
   Val Gln Ser Tyr Lys Ala Lys Leu Thr Pro Gly Asp Val Phe Val Ile
     1               5                   10                  15
   Pro Ala Gly
<210> 322
   <211> 20
   <212> PRT
   <213> Artificial Sequence
<220>
   <223> FRAGMENT OF SEQ ID 9
<400> 322
   Tyr Lys Ala Lys Leu Thr Pro Gly Asp Val Phe Val Ile Pro Ala Gly
   1 5 10 15
   His Pro Val Ala
   20
<210> 323
   <211> 12
   <212> PRT
   <213> Artificial Sequence
<220>
   <223> FRAGMENT OF SEQ ID 9
<400> 323
   Phe Val Ile Pro Ala Gly His Pro Val Ala Val Arg
   1 5 10
<210> 324
   <211> 16
   <212> PRT
   <213> Artificial Sequence
<220>
   <223> FRAGMENT OF SEQ ID 9
<400> 324
   Tyr Lys Ala Lys Leu Thr Pro Gly Asp Val Phe Val Ile Pro Ala Gly
   1 5 10 15
<210> 325
   <211> 13
   <212> PRT
   <213> Artificial Sequence
<220>
   <223> FRAGMENT OF SEQ ID 9
<400> 325
   Asp Leu Thr Phe Pro Gly Ser Ala Gln Glu Val Asp Arg
   1 5 10
<210> 326
   <211> 20
   <212> PRT
   <213> Artificial Sequence
<220>
   <223> FRAGMENT OF SEQ ID 9
<400> 326
   Ala Lys Leu Thr Pro Gly Asp Val Phe Val Ile Pro Ala Gly His Pro
   1               5                   10                  15
   Val Ala Val Arg
               20
<210> 327
   <211> 12
   <212> PRT
   <213> Artificial Sequence
<220>
   <223> FRAGMENT OF SEQ ID 9
<400> 327
   Leu Thr Pro Gly Asp Val Phe Val Ile Pro Ala Gly
   1               5                   10
<210> 328
   <211> 17
   <212> PRT
   <213> Artificial Sequence
<220>
   <223> FRAGMENT OF SEQ ID 9
<400> 328
   Ser Tyr Lys Ala Lys Leu Thr Pro Gly Asp Val Phe Val Ile Pro Ala
   1               5                   10                  15
   Gly
<210> 329
   <211> 23
   <212> PRT
   <213> Artificial Sequence
<220>
   <223> FRAGMENT OF SEQ ID 9
<400> 329
   Ser Tyr Lys Ala Lys Leu Thr Pro Gly Asp Val Phe Val Ile Pro Ala
   1               5                   10                  15
   Gly His Pro Val Ala Val Arg
               20
<210> 330
   <211> 11
   <212> PRT
   <213> Artificial Sequence
<220>
   <223> FRAGMENT OF SEQ ID 9
<400> 330
   Val Ile Pro Ala Gly His Pro Val Ala Val Arg
   1 5 10
<210> 331
   <211> 20
   <212> PRT
   <213> Artificial Sequence
<220>
   <223> FRAGMENT OF SEQ ID 9
<400> 331
   Gln Val Gln Ser Tyr Lys Ala Lys Leu Thr Pro Gly Asp Val Phe Val
   1 5 10 15
   Ile Pro Ala Gly
   20
<210> 332
   <211> 14
   <212> PRT
   <213> Artificial Sequence
<220>
   <223> FRAGMENT OF SEQ ID 9
<400> 332
   Ala Lys Leu Thr Pro Gly Asp Val Phe Val Ile Pro Ala Gly
   1 5 10
<210> 333
   <211> 21
   <212> PRT
   <213> Artificial Sequence
<220>
   <223> FRAGMENT OF SEQ ID 9
<400> 333
   His Pro Val Ala Val Arg Ala Ser Ser Asn Leu Asn Leu Leu Gly Phe
   1 5 10 15
   Gly Ile Asn Ala Glu
   20
<210> 334
   <211> 22
   <212> PRT
   <213> Artificial Sequence
<220>
   <223> FRAGMENT OF SEQ ID 9
<400> 334
   Tyr Lys Ala Lys Leu Thr Pro Gly Asp Val Phe Val Ile Pro Ala Gly
   1 5 10 15
   His Pro Val Ala Val Arg
   20
<210> 335
   <211> 23
   <212> PRT
   <213> Artificial Sequence
<220>
   <223> FRAGMENT OF SEQ ID 9
<400> 335
   Thr Lys Asn Gln Val Gln Ser Tyr Lys Ala Lys Leu Thr Pro Gly Asp
   1 5 10 15
   Val Phe Val Ile Pro Ala Gly
   20
<210> 336
   <211> 12
   <212> PRT
   <213> Artificial Sequence
<220>
   <223> FRAGMENT OF SEQ ID 9
<400> 336
   Pro Phe Asn Leu Lys Ser Ser Asp Pro Ile Tyr Ser
   1 5 10
<210> 337
   <211> 8
   <212> PRT
   <213> Artificial Sequence
<220>
   <223> FRAGMENT OF SEQ ID 9
<400> 337
   Ile Glu Lys Ile Leu Leu Glu Glu
   1 5
<210> 338
   <211> 16
   <212> PRT
   <213> Artificial Sequence
<220>
   <223> FRAGMENT OF SEQ ID 9
<400> 338
   Ser Arg Ser Glu Pro Phe Asn Leu Lys Ser Ser Asp Pro Ile Tyr Ser
   1               5                   10                  15
<210> 339
   <211> 13
   <212> PRT
   <213> Artificial Sequence
<220>
   <223> FRAGMENT OF SEQ ID 9
<400> 339
   His Pro Val Ala Val Arg Ala Ser Ser Asn Leu Asn Leu
   1               5                   10
<210> 340
   <211> 20
   <212> PRT
   <213> Artificial Sequence
<220>
   <223> FRAGMENT OF SEQ ID 10
<400> 340
   Thr Leu Phe Leu Pro Gln Tyr Thr Asp Ala Asp Phe Ile Leu Val Val
   1               5                   10                  15
   Leu Ser Gly Lys
               20
<210> 341
   <211> 10
   <212> PRT
   <213> Artificial Sequence
<220>
   <223> FRAGMENT OF SEQ ID 11
<400> 341
   Glu Val Glu Glu Arg Ser Gln Asn Ile Phe
   1               5                   10
<210> 342
   <211> 12
   <212> PRT
   <213> Artificial Sequence
<220>
   <223> FRAGMENT OF SEQ ID 11
<400> 342
   Val Glu Glu Arg Ser Gln Asn Ile Phe Ser Gly Phe
   1               5                   10
<210> 343
   <211> 12
   <212> PRT
   <213> Artificial Sequence
<220>
   <223> FRAGMENT OF SEQ ID 11
<400> 343
   Ala Ser Leu Gln Glu Gln Glu Gln Gly Gln Val Gln
   1               5                   10
<210> 344
   <211> 11
   <212> PRT
   <213> Artificial Sequence
<220>
   <223> FRAGMENT OF SEQ ID 11
<400> 344
   Gln Glu Gln Glu Gln Gly Gln Val Gln Ser Arg
   1               5                   10
<210> 345
   <211> 14
   <212> PRT
   <213> Artificial Sequence
<220>
   <223> FRAGMENT OF SEQ ID 11
<400> 345
   Ala Ser Leu Gln Glu Gln Glu Gln Gly Gln Val Gln Ser Arg
   1               5                   10
<210> 346
   <211> 13
   <212> PRT
   <213> Artificial Sequence
<220>
   <223> FRAGMENT OF SEQ ID 11
<400> 346
   Glu Val Glu Glu Arg Ser Gln Asn Ile Phe Ser Gly Phe
   1 5 10
<210> 347
   <211> 17
   <212> PRT
   <213> Artificial Sequence
<220>
   <223> FRAGMENT OF SEQ ID 11
<400> 347
   Val Thr Asp Leu Asn Asn Gly Ala Asn Gln Leu Asp Pro Arg Gln Arg
   1 5 10 15
   Asp
<210> 348
   <211> 14
   <212> PRT
   <213> Artificial Sequence
<220>
   <223> FRAGMENT OF SEQ ID 11
<400> 348
   Val Glu His Gly Leu Ser Leu Leu Gln Pro Tyr Ala Ser Leu
   1 5 10
<210> 349
   <211> 21
   <212> PRT
   <213> Artificial Sequence
<220>
   <223> FRAGMENT OF SEQ ID 11
<400> 349
   Ile Tyr Val Thr Asp Leu Asn Asn Gly Ala Asn Gln Leu Asp Pro Arg
   1 5 10 15
   Gln Arg Asp Phe Leu
   20
<210> 350
   <211> 19
   <212> PRT
   <213> Artificial Sequence
<220>
   <223> FRAGMENT OF SEQ ID 11
<400> 350
   Val Thr Asp Leu Asn Asn Gly Ala Asn Gln Leu Asp Pro Arg Gln Arg
   1 5 10 15
   Asp Phe Leu
<210> 351
   <211> 25
   <212> PRT
   <213> Artificial Sequence
<220>
   <223> FRAGMENT OF SEQ ID 11
<400> 351
   Val Glu His Gly Leu Ser Leu Leu Gln Pro Tyr Ala Ser Leu Gln Glu
   1 5 10 15
   Gln Glu Gln Gly Gln Val Gln Ser Arg
   20 25
<210> 352
   <211> 14
   <212> PRT
   <213> Artificial Sequence
<220>
   <223> FRAGMENT OF SEQ ID 11
<400> 352
   Val Thr Asp Leu Asn Asn Gly Ala Asn Gln Leu Asp Pro Arg
   1 5 10
<210> 353
   <211> 19
   <212> PRT
   <213> Artificial Sequence
<220>
   <223> FRAGMENT OF SEQ ID 11
<400> 353
   Ile Tyr Val Thr Asp Leu Asn Asn Gly Ala Asn Gln Leu Asp Pro Arg
   1 5 10 15
   Gln Arg Asp
<210> 354
   <211> 20
   <212> PRT
   <213> Artificial Sequence
<220>
   <223> FRAGMENT OF SEQ ID 11
<400> 354
   Tyr Val Thr Asp Leu Asn Asn Gly Ala Asn Gln Leu Asp Pro Arg Gln
   1 5 10 15
   Arg Asp Phe Leu
   20
<210> 355
   <211> 15
   <212> PRT
   <213> Artificial Sequence
<220>
   <223> FRAGMENT OF SEQ ID 11
<400> 355
   Tyr Val Thr Asp Leu Asn Asn Gly Ala Asn Gln Leu Asp Pro Arg
   1 5 10 15
<210> 356
   <211> 17
   <212> PRT
   <213> Artificial Sequence
<220>
   <223> FRAGMENT OF SEQ ID 11
<400> 356
   Ser Thr Glu Leu Leu Ser Glu Ala Leu Gly Val Ser Ser Gln Val Ala
   1 5 10 15
   Arg
<210> 357
   <211> 16
   <212> PRT
   <213> Artificial Sequence
<220>
   <223> FRAGMENT OF SEQ ID 11
<400> 357
   His Gly Leu Ser Leu Leu Gln Pro Tyr Ala Ser Leu Gln Glu Gln Glu
   1 5 10 15
<210> 358
   <211> 20
   <212> PRT
   <213> Artificial Sequence
<220>
   <223> FRAGMENT OF SEQ ID 11
<400> 358
   Ser Gly Phe Ser Thr Glu Leu Leu Ser Glu Ala Leu Gly Val Ser Ser
   1 5 10 15
   Gln Val Ala Arg
   20
<210> 359
   <211> 13
   <212> PRT
   <213> Artificial Sequence
<220>
   <223> FRAGMENT OF SEQ ID 12
<400> 359
   Gly Ala Phe Thr Pro Leu Gln Tyr Lys Ser Tyr Gln Asp
   1 5 10
<210> 360
   <211> 15
   <212> PRT
   <213> Artificial Sequence
<220>
   <223> FRAGMENT OF SEQ ID 12
<400> 360
   Gly Leu Leu Leu Pro His Tyr Thr Asn Gly Ala Ser Leu Val Tyr
   1 5 10 15
<210> 361
   <211> 16
   <212> PRT
   <213> Artificial Sequence
<220>
   <223> FRAGMENT OF SEQ ID 12
<400> 361
   Phe Leu Leu Ala Gly Asn Lys Arg Asn Pro Gln Ala Tyr Arg Arg Glu
   1 5 10 15
<210> 362
   <211> 12
   <212> PRT
   <213> Artificial Sequence
<220>
   <223> FRAGMENT OF SEQ ID 12
<400> 362
   Ala Leu Pro Thr Asp Val Leu Ala Asn Ala Tyr Arg
   1               5                   10
<210> 363
   <211> 8
   <212> PRT
   <213> Artificial Sequence
<220>
   <223> FRAGMENT OF SEQ ID 12
<400> 363
   Asp Phe Leu Leu Ala Gly Asn Lys
   1               5
<210> 364
   <211> 8
   <212> PRT
   <213> Artificial Sequence
<220>
   <223> FRAGMENT OF SEQ ID 12
<400> 364
   Asp Val Leu Ala Asn Ala Tyr Arg
   1               5
<210> 365
   <211> 9
   <212> PRT
   <213> Artificial Sequence
<220>
   <223> FRAGMENT OF SEQ ID 12
<400> 365
   Gly Ala Phe Thr Pro Leu Gln Tyr Lys
   1               5
<210> 366
   <211> 14
   <212> PRT
   <213> Artificial Sequence
<220>
   <223> FRAGMENT OF SEQ ID 12
<400> 366
   Gln Gly Asp Val Ile Ala Leu Pro Ala Gly Val Ala His Trp
   1               5                   10
<210> 367
   <211> 12
   <212> PRT
   <213> Artificial Sequence
<220>
   <223> FRAGMENT OF SEQ ID 12
<400> 367
   Phe Gly Ala Phe Thr Pro Leu Gln Tyr Lys Ser Tyr
   1               5                   10
<210> 368
   <211> 14
   <212> PRT
   <213> Artificial Sequence
<220>
   <223> FRAGMENT OF SEQ ID 12
<400> 368
   Phe Leu Leu Ala Gly Asn Lys Arg Asn Pro Gln Ala Tyr Arg
   1               5                   10
<210> 369
   <211> 13
   <212> PRT
   <213> Artificial Sequence
<220>
   <223> FRAGMENT OF SEQ ID 12
<400> 369
   Phe Gly Ala Phe Thr Pro Leu Gln Tyr Lys Ser Tyr Gln
   1               5                   10
<210> 370
   <211> 15
   <212> PRT
   <213> Artificial Sequence
<220>
   <223> FRAGMENT OF SEQ ID 12
<400> 370
   Gly Leu Ser Leu Leu Gln Pro Tyr Ala Ser Leu Gln Glu Gln Glu
   1               5                   10                  15
<210> 371
   <211> 8
   <212> PRT
   <213> Artificial Sequence
<220>
   <223> FRAGMENT OF SEQ ID 12
<400> 371
   Ala Phe Thr Pro Leu Gln Tyr Lys
   1               5
<210> 372
   <211> 14
   <212> PRT
   <213> Artificial Sequence
<220>
   <223> FRAGMENT OF SEQ ID 12
<400> 372
   Phe Gly Ala Phe Thr Pro Leu Gln Tyr Lys Ser Tyr Gln Asp
   1               5                   10
<210> 373
   <211> 13
   <212> PRT
   <213> Artificial Sequence
<220>
   <223> FRAGMENT OF SEQ ID 12
<400> 373
   Gly Asp Glu Phe Gly Ala Phe Thr Pro Leu Gln Tyr Lys
   1               5                   10
<210> 374
   <211> 15
   <212> PRT
   <213> Artificial Sequence
<220>
   <223> FRAGMENT OF SEQ ID 12
<400> 374
   Phe Gly Ala Phe Thr Pro Leu Gln Tyr Lys Ser Tyr Gln Asp Val
   1               5                   10                  15
<210> 375
   <211> 10
   <212> PRT
   <213> Artificial Sequence
<220>
   <223> FRAGMENT OF SEQ ID 12
<400> 375
   Phe Gly Ala Phe Thr Pro Leu Gln Tyr Lys
   1               5                   10
<210> 376
   <211> 11
   <212> PRT
   <213> Artificial Sequence
<220>
   <223> FRAGMENT OF SEQ ID 12
<400> 376
   Phe Gly Ala Phe Thr Pro Leu Gln Tyr Lys Ser
   1               5                   10
<210> 377
   <211> 14
   <212> PRT
   <213> Artificial Sequence
<220>
   <223> FRAGMENT OF SEQ ID 12
<400> 377
   Val Tyr Ile Ile Gln Gly Arg Gly Ile Thr Gly Pro Thr Phe
   1               5                   10
<210> 378
   <211> 13
   <212> PRT
   <213> Artificial Sequence
<220>
   <223> FRAGMENT OF SEQ ID 12
<400> 378
   Tyr Ile Ile Gln Gly Arg Gly Ile Thr Gly Pro Thr Phe
   1               5                   10
<210> 379
   <211> 14
   <212> PRT
   <213> Artificial Sequence
<220>
   <223> FRAGMENT OF SEQ ID 12
<400> 379
   Lys Thr Asn Pro Asn Ser Met Val Ser His Ile Ala Gly Lys
   1               5                   10
<210> 380
   <211> 13
   <212> PRT
   <213> Artificial Sequence
<220>
   <223> FRAGMENT OF SEQ ID 12
<400> 380
   Thr Asn Pro Asn Ser Met Val Ser His Ile Ala Gly Lys
   1               5                   10
<210> 381
   <211> 12
   <212> PRT
   <213> Artificial Sequence
<220>
   <223> FRAGMENT OF SEQ ID 12
<400> 381
   Pro Asn Ser Met Val Ser His Ile Ala Gly Lys Ser
   1               5                   10
<210> 382
   <211> 20
   <212> PRT
   <213> Artificial Sequence
<220>
   <223> FRAGMENT OF SEQ ID 12
<400> 382
   Asn Ile Asp Asn Pro Asn Arg Ala Asp Thr Tyr Asn Pro Arg Ala Gly
   1               5                   10                  15
   Arg Val Thr Asn
               20
<210> 383
   <211> 11
   <212> PRT
   <213> Artificial Sequence
<220>
   <223> FRAGMENT OF SEQ ID 12
<400> 383
   Gln Arg Asp Phe Leu Leu Ala Gly Asn Lys Arg
   1               5                   10
<210> 384
   <211> 12
   <212> PRT
   <213> Artificial Sequence
<220>
   <223> FRAGMENT OF SEQ ID 12
<400> 384
   Leu Leu Gln Pro Tyr Ala Ser Leu Gln Glu Gln Glu
   1 5 10
<210> 385
   <211> 10
   <212> PRT
   <213> Artificial Sequence
<220>
   <223> FRAGMENT OF SEQ ID 12
<400> 385
   Gln Arg Asp Phe Leu Leu Ala Gly Asn Lys
   1 5 10
<210> 386
   <211> 11
   <212> PRT
   <213> Artificial Sequence
<220>
   <223> FRAGMENT OF SEQ ID 12
<400> 386
   Gln Glu Gln Glu Gln Gly Gln Met Gln Ser Arg
   1 5 10
<210> 387
   <211> 13
   <212> PRT
   <213> Artificial Sequence
<220>
   <223> FRAGMENT OF SEQ ID 12
<400> 387
   Ser Leu Leu Gln Pro Tyr Ala Ser Leu Gln Glu Gln Glu
   1 5 10
<210> 388
   <211> 11
   <212> PRT
   <213> Artificial Sequence
<220>
   <223> FRAGMENT OF SEQ ID 12
<400> 388
   Ala Ser Leu Gln Glu Gln Glu Gln Gly Gln Met
   1 5 10
<210> 389
   <211> 14
   <212> PRT
   <213> Artificial Sequence
<220>
   <223> FRAGMENT OF SEQ ID 12
<400> 389
   Ala Ser Leu Gln Glu Gln Glu Gln Gly Gln Met Gln Ser Arg
   1               5                   10
<210> 390
   <211> 9
   <212> PRT
   <213> Artificial Sequence
<220>
   <223> FRAGMENT OF SEQ ID 12
<400> 390
   Asp Phe Leu Leu Ala Gly Asn Lys Arg
   1               5
<210> 391
   <211> 18
   <212> PRT
   <213> Artificial Sequence
<220>
   <223> FRAGMENT OF SEQ ID 12
<400> 391
   Gln Ala Phe Glu Pro Ile Arg Ser Val Arg Ser Gln Ala Gly Thr Thr
   1               5                   10                  15
   Glu Phe
<210> 392
   <211> 18
   <212> PRT
   <213> Artificial Sequence
<220>
   <223> FRAGMENT OF SEQ ID 12
<400> 392
   Lys Thr Asn Pro Asn Ser Met Val Ser His Ile Ala Gly Lys Ser Ser
   1               5                   10                  15
   Ile Phe
<210> 393
   <211> 21
   <212> PRT
   <213> Artificial Sequence
<220>
   <223> FRAGMENT OF SEQ ID 12
<400> 393
   Val Arg Arg Val Ile Glu Pro Arg Gly Leu Leu Leu Pro His Tyr Thr
   1               5                   10                  15
   Asn Gly Ala Ser Leu
               20
<210> 394
   <211> 17
   <212> PRT
   <213> Artificial Sequence
<220>
   <223> FRAGMENT OF SEQ ID 12
<400> 394
   Phe Gly Ala Phe Thr Pro Leu Gln Tyr Lys Ser Tyr Gln Asp Val Tyr
   1               5                   10                  15
   Asn
<210> 395
   <211> 10
   <212> PRT
   <213> Artificial Sequence
<220>
   <223> FRAGMENT OF SEQ ID 12
<400> 395
   Ile Ala Leu Pro Ala Gly Val Ala His Trp
   1               5                   10
<210> 396
   <211> 25
   <212> PRT
   <213> Artificial Sequence
<220>
   <223> FRAGMENT OF SEQ ID 12
<400> 396
   Arg Val Arg Gln Asn Ile Asp Asn Pro Asn Arg Ala Asp Thr Tyr Asn
   1               5                   10                  15
   Pro Arg Ala Gly Arg Val Thr Asn Leu
               20                  25
<210> 397
   <211> 21
   <212> PRT
   <213> Artificial Sequence
<220>
   <223> FRAGMENT OF SEQ ID 12
<400> 397
   Asn Ile Asp Asn Pro Asn Arg Ala Asp Thr Tyr Asn Pro Arg Ala Gly
   1               5                   10                  15
   Arg Val Thr Asn Leu
               20
<210> 398
   <211> 16
   <212> PRT
   <213> Artificial Sequence
<220>
   <223> FRAGMENT OF SEQ ID 12
<400> 398
   Gly Ala Phe Thr Pro Leu Gln Tyr Lys Ser Tyr Gln Asp Val Tyr Asn
   1               5                   10                  15
<210> 399
   <211> 16
   <212> PRT
   <213> Artificial Sequence
<220>
   <223> FRAGMENT OF SEQ ID 12
<400> 399
   Pro Asn Ser Met Val Ser His Ile Ala Gly Lys Ser Ser Ile Phe Arg
   1               5                   10                  15
<210> 400
   <211> 19
   <212> PRT
   <213> Artificial Sequence
<220>
   <223> FRAGMENT OF SEQ ID 12
<400> 400
   Arg Leu Gln Ala Phe Glu Pro Ile Arg Ser Val Arg Ser Gln Ala Gly
   1               5                   10                  15
   Thr Thr Glu
<210> 401
   <211> 18
   <212> PRT
   <213> Artificial Sequence
<220>
   <223> FRAGMENT OF SEQ ID 12
<400> 401
   Thr Asn Pro Asn Ser Met Val Ser His Ile Ala Gly Lys Ser Ser Ile
   1               5                   10                  15
   Phe Arg
<210> 402
   <211> 11
   <212> PRT
   <213> Artificial Sequence
<220>
   <223> FRAGMENT OF SEQ ID 13
<400> 402
   Glu Leu Gly Ala Pro Asp Val Gly His Pro Met
   1               5                   10
<210> 403
   <211> 10
   <212> PRT
   <213> Artificial Sequence
<220>
   <223> FRAGMENT OF SEQ ID 13
<400> 403
   Leu Gly Ala Pro Asp Val Gly His Pro Met
   1               5                   10
<210> 404
   <211> 15
   <212> PRT
   <213> Artificial Sequence
<220>
   <223> FRAGMENT OF SEQ ID 13
<400> 404
   Glu Leu Gly Ala Pro Asp Val Gly His Pro Met Ser Glu Val Phe
   1 5 10 15
<210> 405
   <211> 12
   <212> PRT
   <213> Artificial Sequence
<220>
   <223> FRAGMENT OF SEQ ID 13
<400> 405
   Glu Leu Gly Ala Pro Asp Val Gly His Pro Met Ser
   1 5 10
<210> 406
   <211> 16
   <212> PRT
   <213> Artificial Sequence
<220>
   <223> FRAGMENT OF SEQ ID 13
<400> 406
   Glu Leu Gly Ala Pro Asp Val Gly His Pro Met Ser Glu Val Phe Arg
   1 5 10 15
<210> 407
   <211> 14
   <212> PRT
   <213> Artificial Sequence
<220>
   <223> FRAGMENT OF SEQ ID 13
<400> 407
   Glu Leu Gly Ala Pro Asp Val Gly His Pro Met Ser Glu Val
   1 5 10
<210> 408
   <211> 13
   <212> PRT
   <213> Artificial Sequence
<220>
   <223> FRAGMENT OF SEQ ID 13
<400> 408
   Glu Leu Gly Ala Pro Asp Val Gly His Pro Met Ser Glu
   1 5 10
<210> 409
   <211> 12
   <212> PRT
   <213> Artificial Sequence
<220>
   <223> FRAGMENT OF SEQ ID 13
<400> 409
   Leu Gly Ala Pro Asp Val Gly His Pro Met Ser Glu
   1               5                   10
<210> 410
   <211> 15
   <212> PRT
   <213> Artificial Sequence
<220>
   <223> FRAGMENT OF SEQ ID 13
<400> 410
   Tyr Arg Glu Leu Gly Ala Pro Asp Val Gly His Pro Met Ser Glu
   1               5                   10                  15
<210> 411
   <211> 13
   <212> PRT
   <213> Artificial Sequence
<220>
   <223> FRAGMENT OF SEQ ID 13
<400> 411
   Leu Gly Ala Pro Asp Val Gly His Pro Met Ser Glu Val
   1               5                   10
<210> 412
   <211> 14
   <212> PRT
   <213> Artificial Sequence
<220>
   <223> FRAGMENT OF SEQ ID 13
<400> 412
   Arg Glu Leu Gly Ala Pro Asp Val Gly His Pro Met Ser Glu
   1               5                   10
<210> 413
   <211> 16
   <212> PRT
   <213> Artificial Sequence
<220>
   <223> FRAGMENT OF SEQ ID 14
<400> 413
   Ala Pro Thr Gly Thr Phe Ile Ala Ser Gly Val Val Val Gly Lys Asp
   1 5 10 15
<210> 414
   <211> 17
   <212> PRT
   <213> Artificial Sequence
<220>
   <223> FRAGMENT OF SEQ ID 14
<400> 414
   Leu Ala Ile Val Lys Phe Ser Pro Asn Glu Gln Asn Lys His Ile Gly
   1 5 10 15
   Glu
<210> 415
   <211> 21
   <212> PRT
   <213> Artificial Sequence
<220>
   <223> FRAGMENT OF SEQ ID 14
<400> 415
   Arg His Gln Ile Thr Asp Thr Thr Asn Gly His Tyr Ala Pro Val Thr
   1 5 10 15
   Tyr Ile Gln Val Glu
   20
<210> 416
   <211> 19
   <212> PRT
   <213> Artificial Sequence
<220>
   <223> FRAGMENT OF SEQ ID 14
<400> 416
   Gly Asp Leu Ala Ile Val Lys Phe Ser Pro Asn Glu Gln Asn Lys His
   1 5 10 15
   Ile Gly Glu
<210> 417
   <211> 18
   <212> PRT
   <213> Artificial Sequence
<220>
   <223> FRAGMENT OF SEQ ID 14
<400> 417
   Asn Pro Asp Asn Pro Asp Asn Pro Asn Asn Pro Asp Asn Pro Asn Asn
   1 5 10 15
   Pro Asp
<210> 418
   <211> 9
   <212> PRT
   <213> Artificial Sequence
<220>
   <223> PEPTIDE
<400> 418
   Gln Ser Phe Ile Leu Ser Gly Asn Glu
   1 5
<210> 419
   <211> 9
   <212> PRT
   <213> Artificial Sequence
<220>
   <223> PEPTIDE
<400> 419
   Glu Ser Phe Leu Leu Ser Gly Asn Gln
   1 5
<210> 420
   <211> 9
   <212> PRT
   <213> Artificial Sequence
<220>
   <223> PEPTIDE
<400> 420
   Gln Ser Tyr Leu Leu Ser Gly Asn Gln
   1 5
<210> 421
   <211> 9
   <212> PRT
   <213> Artificial Sequence
<220>
   <223> PEPTIDE
<400> 421
   Gln Ser Phe Leu Leu Ser Gly Asp Gln
   1 5
<210> 422
   <211> 9
   <212> PRT
   <213> Artificial Sequence
<220>
   <223> PEPTIDE
<400> 422
   Gln Ser Tyr Leu Leu Ser Gly Asn Glu
   1 5
<210> 423
   <211> 9
   <212> PRT
   <213> Artificial Sequence
<220>
   <223> PEPTIDE
<400> 423
   Glu Ser Phe Leu Leu Ser Gly Asn Glu
   1 5
<210> 424
   <211> 9
   <212> PRT
   <213> Artificial Sequence
<220>
   <223> PEPTIDE
<400> 424
   Glu Ser Tyr Leu Leu Ser Gly Asn Gln
   1 5
<210> 425
   <211> 9
   <212> PRT
   <213> Artificial Sequence
<220>
   <223> PEPTIDE
<400> 425
   Gln Ser Phe Leu Leu Ser Gly Asp Glu
   1 5
<210> 426
   <211> 9
   <212> PRT
   <213> Artificial Sequence
<220>
   <223> PEPTIDE
<400> 426
   Gln Ser Tyr Leu Leu Ser Gly Asp Gln
   1 5
<210> 427
   <211> 9
   <212> PRT
   <213> Artificial Sequence
<220>
   <223> PEPTIDE
<400> 427
   Gln Ser Phe Arg Leu Ser Gly Asn Gln
   1 5
<210> 428
   <211> 9
   <212> PRT
   <213> Artificial Sequence
<220>
   <223> PEPTIDE
<400> 428
   Gln Ser Phe Leu Leu Ser Tyr Asn Gln
   1 5
<210> 429
   <211> 9
   <212> PRT
   <213> Artificial Sequence
<220>
   <223> PEPTIDE
<400> 429
   Gln Phe Phe Leu Leu Ser Gly Asn Gln
   1 5
<210> 430
   <211> 9
   <212> PRT
   <213> Artificial Sequence
<220>
   <223> PEPTIDE
<400> 430
   Gln Ser Phe Leu Leu Ser Gly Ala Gln
   1 5
<210> 431
   <211> 9
   <212> PRT
   <213> Artificial Sequence
<220>
   <223> PEPTIDE
<400> 431
   Gln Ser Phe Leu Leu Ser Gly Asn Pro
   1 5
<210> 432
   <211> 9
   <212> PRT
   <213> Artificial Sequence
<220>
   <223> PEPTIDE
<400> 432
   Gln Ser Phe Arg Arg Ser Gly Asn Gln
   1 5
<210> 433
   <211> 9
   <212> PRT
   <213> Artificial Sequence
<220>
   <223> PEPTIDE
<400> 433
   Gln Ser Phe Leu Leu Ser Tyr Ile Gln
   1 5
<210> 434
   <211> 9
   <212> PRT
   <213> Artificial Sequence
<220>
   <223> PEPTIDE
<400> 434
   Gln Phe Phe Leu Leu Ser Gly Asn Leu
   1 5
<210> 435
   <211> 10
   <212> PRT
   <213> Artificial Sequence
<220>
   <223> PEPTIDE
<400> 435
   Gln Ser Phe Leu Leu Ser Gly Ala Gln Ala
   1               5                   10
<210> 436
   <211> 9
   <212> PRT
   <213> Artificial Sequence
<220>
   <223> PEPTIDE
<400> 436
   Gln Ser Phe Leu Leu Ser Gly Asn Pro
   1               5
<210> 437
   <211> 10
   <212> PRT
   <213> Artificial Sequence
<220>
   <223> PEPTIDE
<400> 437
   Gln Ser Phe Leu Leu Ser Gly Asn Gln Gln
   1               5                   10
<210> 438
   <211> 10
   <212> PRT
   <213> Artificial Sequence
<220>
   <223> PEPTIDE
<400> 438
   Gln Ser Phe Leu Leu Leu Ser Gly Asn Gln
   1               5                   10
<210> 439
   <211> 11
   <212> PRT
   <213> Artificial Sequence
<220>
   <223> PEPTIDE
<400> 439
   Ala Gln Ser Phe Gly Leu Leu Ser Gly Asn Gln
   1 5 10
<210> 440
   <211> 11
   <212> PRT
   <213> Artificial Sequence
<220>
   <223> PEPTIDE
<400> 440
   Arg Gln Ser Phe Leu Leu Ile Ser Gly Asn Gln
   1 5 10
<210> 441
   <211> 10
   <212> PRT
   <213> Artificial Sequence
<220>
   <223> PEPTIDE
<400> 441
   Gln Ser Phe Leu Leu Ser Gly Asn Gln Lys
   1 5 10
<210> 442
   <211> 8
   <212> PRT
   <213> Artificial Sequence
<220>
   <223> PEPTIDE
<400> 442
   Gln Phe Leu Leu Ser Gly Asn Gln
   1 5
<210> 443
   <211> 8
   <212> PRT
   <213> Artificial Sequence
<220>
   <223> PEPTIDE
<400> 443
   Ser Phe Leu Leu Ser Gly Asn Gln
   1 5
<210> 444
   <211> 8
   <212> PRT
   <213> Artificial Sequence
<220>
   <223> PEPTIDE
<400> 444
   Gln Ser Phe Leu Leu Ser Gly Asn
   1               5
<210> 445
   <211> 9
   <212> PRT
   <213> Artificial Sequence
<220>
   <223> PEPTIDE
<400> 445
   Gln Ser Phe Leu Leu Ser Gly Asn Gln
   1               5
<210> 446
   <211> 8
   <212> PRT
   <213> Artificial Sequence
<220>
   <223> PEPTIDE
<400> 446
   Gln Ser Phe Leu Ser Gly Asn Gln
   1               5
<210> 447
   <211> 8
   <212> PRT
   <213> Artificial Sequence
<220>
   <223> PEPTIDE
<400> 447
   Gln Ser Leu Leu Ser Gly Asn Gln
   1               5
<210> 448
   <211> 8
   <212> PRT
   <213> Artificial Sequence
<220>
   <223> PEPTIDE
<400> 448
   Ser Phe Leu Leu Ser Gly Asn Gln
   1               5
<210> 449
   <211> 8
   <212> PRT
   <213> Artificial Sequence
<220>
   <223> PEPTIDE
<400> 449
   Gln Ser Phe Leu Leu Ser Gly Asn
   1               5
<210> 450
   <211> 7
   <212> PRT
   <213> Artificial Sequence
<220>
   <223> PEPTIDE
<400> 450
   Ser Phe Leu Leu Ser Gly Asn
   1               5
<210> 451
   <211> 7
   <212> PRT
   <213> Artificial Sequence
<220>
   <223> PEPTIDE
<400> 451
   Gln Ser Phe Ser Gly Asn Gln
   1               5
<210> 452
   <211> 11
   <212> PRT
   <213> Artificial Sequence
<220>
   <223> PEPTIDE
<400> 452
   His Pro Arg Pro Pro Lys Pro Asp Ala Pro Arg
   1               5                   10
<210> 453
   <211> 10
   <212> PRT
   <213> Artificial Sequence
<220>
   <223> PEPTIDE
<400> 453
   Leu Gln Gln Ala Pro Pro Pro Pro Gln Arg
   1               5                   10
<210> 454
   <211> 11
   <212> PRT
   <213> Artificial Sequence
<220>
   <223> PEPTIDE
<400> 454
   Val Gly Trp Gly Glu Gln Pro Trp Ser Pro Tyr
   1               5                   10
<210> 455
   <211> 5
   <212> PRT
   <213> Artificial Sequence
<220>
   <223> PEPTIDE
<400> 455
   Phe His Met Pro Pro
   1               5
<210> 456
   <211> 4
   <212> PRT
   <213> Artificial Sequence
<220>
   <223> PEPTIE
<400> 456
   Phe Arg Arg Pro
   1
<210> 457
   <211> 4
   <212> PRT
   <213> Artificial Sequence
<220>
   <223> PEPTIDE
<400> 457
   Phe Trp Met Ala
   1
<210> 458
   <211> 5
   <212> PRT
   <213> Artificial Sequence
<220>
   <223> PEPTIDE
<400> 458
   His Met Pro Pro Ser
   1 5
<210> 459
   <211> 11
   <212> PRT
   <213> Artificial Sequence
<220>
   <223> PEPTIDE
<400> 459
   Pro Val Glu Met Pro Thr Leu Leu Tyr Pro Ser
   1 5 10
<210> 460
   <211> 5
   <212> PRT
   <213> Artificial Sequence
<220>
   <223> PEPTIDE
<400> 460
   His Met Pro Ser Ser
   1 5
<210> 461
   <211> 4
   <212> PRT
   <213> Artificial Sequence
<220>
   <223> PEPTIDE
<400> 461
   His Arg Phe Arg
   1
<210> 462
   <211> 4
   <212> PRT
   <213> Artificial Sequence
<220>
   <223> PEPTIDE
<400> 462
   His Arg Arg Ser
   1
<210> 463
   <211> 4
   <212> PRT
   <213> Artificial Sequence
<220>
   <223> PEPTIDE
<400> 463
   His Ser Pro Arg
   1
<210> 464
   <211> 4
   <212> PRT
   <213> Artificial Sequence
<220>
   <223> PEPTIDE
<400> 464
   His Trp Phe Ala
   1
<210> 465
   <211> 4
   <212> PRT
   <213> Artificial Sequence
<220>
   <223> PEPTIDE
<400> 465
   Met Phe Arg Arg
   1
<210> 466
   <211> 5
   <212> PRT
   <213> Artificial Sequence
<220>
   <223> PEPTIDE
<400> 466
   Met Phe Arg Arg Pro
   1 5
<210> 467
   <211> 4
   <212> PRT
   <213> Artificial Sequence
<220>
   <223> PEPTIDE
<400> 467
   Met Pro Pro Ser
   1
<210> 468
   <211> 4
   <212> PRT
   <213> Artificial Sequence
<220>
   <223> PEPTIDE
<400> 468
   Met Pro Arg Arg
   1
<210> 469
   <211> 4
   <212> PRT
   <213> Artificial Sequence
<220>
   <223> PEPTIDE
<400> 469
   Asn Met Pro Ser
   1
<210> 470
   <211> 4
   <212> PRT
   <213> Artificial Sequence
<220>
   <223> PEPTIDE
<400> 470
   Pro His Met Pro
   1
<210> 471
   <211> 5
   <212> PRT
   <213> Artificial Sequence
<220>
   <223> PEPTIDE
<400> 471
   Pro His Met Pro Ser
   1               5
<210> 472
   <211> 4
   <212> PRT
   <213> Artificial Sequence
<220>
   <223> PEPTIDE
<400> 472
   Pro Arg Arg Phe
   1
<210> 473
   <211> 4
   <212> PRT
   <213> Artificial Sequence
<220>
   <223> PEPTIDE
<400> 473
   Trp Met Lys Ala
   1
<210> 474
   <211> 11
   <212> PRT
   <213> Artificial Sequence
<220>
   <223> PEPTIDE
<400> 474
   Asp Ser Ile Asn Ala Leu Glu Pro Asp His Arg
   1               5                   10
<210> 475
   <211> 9
   <212> PRT
   <213> Artificial Sequence
<220>
   <223> PEPTIDE
<400> 475
   Glu Leu Thr Phe Pro Gly Ser Val Gln
   1 5
<210> 476
   <211> 10
   <212> PRT
   <213> Artificial Sequence
<220>
   <223> PEPTIDE
<400> 476
   Glu Leu Thr Phe Pro Gly Ser Val Gln Glu
   1 5 10
<210> 477
   <211> 11
   <212> PRT
   <213> Artificial Sequence
<220>
   <223> PEPTIDE
<400> 477
   Ile Phe Glu Asp Ala Ile Thr Ile Pro Gly Arg
   1 5 10
<210> 478
   <211> 13
   <212> PRT
   <213> Artificial Sequence
<220>
   <223> PEPTIDE
<400> 478
   Leu Asp Ala Leu Glu Pro Asp Asn Arg Ile Glu Ser Glu
   1 5 10
<210> 479
   <211> 10
   <212> PRT
   <213> Artificial Sequence
<220>
   <223> PEPTIDE
<400> 479
   Lys Thr Leu Asp Tyr Trp Pro Ser Leu Arg
   1 5 10
<210> 480
   <211> 9
   <212> PRT
   <213> Artificial Sequence
<220>
   <223> PEPTIDE
<400> 480
   Arg His Gly Glu Trp Gly Pro Ser Tyr
   1               5
<210> 481
   <211> 11
   <212> PRT
   <213> Artificial Sequence
<220>
   <223> PEPTIDE
<400> 481
   Ile Leu Val Asp Gly Ser His Asp Ile Glu Arg
   1               5                   10
<210> 482
   <211> 11
   <212> PRT
   <213> Artificial Sequence
<220>
   <223> PEPTIDE
<400> 482
   Ile Leu Val Asp Gly Ser His Asp Ile Glu Arg
   1               5                   10
<210> 483
   <211> 11
   <212> PRT
   <213> Artificial Sequence
<220>
   <223> PEPTIDE
<400> 483
   Asn Leu Ala Gln Ala Pro Ala Gln Ala Leu Leu
   1               5                   10
<210> 484
   <211> 7
   <212> PRT
   <213> Artificial Sequence
<220>
   <223> PEPTIDE
<400> 484
   Phe Leu Pro Gln His Thr Asp
   1 5
<210> 485
   <211> 6
   <212> PRT
   <213> Artificial Sequence
<220>
   <223> PEPTIDE
<400> 485
   Leu Glu Pro Asp Asn Arg
   1 5
<210> 486
   <211> 6
   <212> PRT
   <213> Artificial Sequence
<220>
   <223> PEPTIDE
<400> 486
   Leu Gln Ser Gln Asn Asp
   1 5
<210> 487
   <211> 11
   <212> PRT
   <213> Artificial Sequence
<220>
   <223> PEPTIDE
<400> 487
   Leu Gln Ser Gln Asn Asp Gln Arg Gly Glu Ile
   1 5 10
<210> 488
   <211> 14
   <212> PRT
   <213> Artificial Sequence
<220>
   <223> PEPTIDE
<400> 488
   Gln Ser Gln Asn Asp Gln Arg Gly Glu Ile Ile His Val Lys
   1 5 10
<210> 489
   <211> 8
   <212> PRT
   <213> Artificial Sequence
<220>
   <223> PEPTIDE
<400> 489
   Arg Gly Glu Ile Ile His Val Lys
   1 5
<210> 490
   <211> 8
   <212> PRT
   <213> Artificial Sequence
<220>
   <223> PEPTIDE
<400> 490
   Arg Leu Gln Ser Gln Asn Asp Gln
   1 5
<210> 491
   <211> 10
   <212> PRT
   <213> Artificial Sequence
<220>
   <223> PEPTIDE
<400> 491
   Arg Leu Gln Ser Gln Asn Asp Gln Arg Gly
   1 5 10
<210> 492
   <211> 14
   <212> PRT
   <213> Artificial Sequence
<220>
   <223> PEPTIDE
<400> 492
   Arg Leu Gln Ser Gln Asn Asp Gln Arg Gly Glu Ile Ile His
   1 5 10
<210> 493
   <211> 9
   <212> PRT
   <213> Artificial Sequence
<220>
   <223> PEPTIDE
<400> 493
   Val Phe Asp Gly Val Leu Arg Pro Gly
   1               5
<210> 494
   <211> 4
   <212> PRT
   <213> Artificial Sequence
<220>
   <223> PEPTIDE
<400> 494
   His Asn Pro Arg
   1
<210> 495
   <211> 4
   <212> PRT
   <213> Artificial Sequence
<220>
   <223> PEPTIDE
<400> 495
   His Pro Met Ser
   1
<210> 496
   <211> 4
   <212> PRT
   <213> Artificial Sequence
<220>
   <223> PEPTIDE
<400> 496
   His Pro Ser Phe
   1
<210> 497
   <211> 4
   <212> PRT
   <213> Artificial Sequence
<220>
   <223> PEPTIDE
<400> 497
   Met Pro Met Pro
   1
<210> 498
   <211> 4
   <212> PRT
   <213> Artificial Sequence
<220>
   <223> PEPTIDE
<400> 498
   Pro Met Pro Leu
   1
<210> 499
   <211> 4
   <212> PRT
   <213> Artificial Sequence
<220>
   <223> PEPTIDE
<400> 499
   Pro Asn Ser Met
   1
<210> 500
   <211> 4
   <212> PRT
   <213> Artificial Sequence
<220>
   <223> PEPTIDE
<400> 500
   Trp Asp Pro Ala
   1
<210> 501
   <211> 6
   <212> PRT
   <213> Artificial Sequence
<220>
   <223> PEPTIDE
<400> 501
   Leu Arg Gly Phe Ser Lys
   1               5
<210> 502
   <211> 6
   <212> PRT
   <213> Artificial Sequence
<220>
   <223> PEPTIDE
<400> 502
   Arg Ser Gln Asn Ile Phe
   1               5
<210> 503
   <211> 6
   <212> PRT
   <213> Artificial Sequence
<220>
   <223> PEPTIDE
<400> 503
   Tyr Leu Arg Gly Phe Ser
   1               5
<210> 504
   <211> 9
   <212> PRT
   <213> Artificial Sequence
<220>
   <223> PEPTIDE
<400> 504
   Gly Ala Leu Met Leu Pro His Tyr Asn
   1               5
<210> 505
   <211> 11
   <212> PRT
   <213> Artificial Sequence
<220>
   <223> PEPTIDE
<400> 505
   Gly Ala Leu Met Leu Pro His Tyr Asn Ser Arg
   1               5                   10
<210> 506
   <211> 463
   <212> PRT
   <213> Vicia faba
<400> 506
   Met Ala Ala Thr Thr Leu Lys Asp Ser Phe Pro Leu Leu Thr Leu Leu
   1               5                   10                  15
   Gly Ile Ala Phe Leu Ala Ser Val Cys Leu Ser Ser Arg Ser Asp Gln
               20                  25                  30
   Asp Asn Pro Phe Val Phe Glu Ser Asn Arg Phe Gln Thr Leu Phe Glu
           35                  40                  45
   Asn Glu Asn Gly His Ile Arg Leu Leu Gln Lys Phe Asp Gln His Ser
       50                  55                  60
   Lys Leu Leu Glu Asn Leu Gln Asn Tyr Arg Leu Leu Glu Tyr Lys Ser
   65                  70                  75                  80
   Lys Pro His Thr Ile Phe Leu Pro Gln Gln Thr Asp Ala Asp Phe Ile
                   85                  90                  95
   Leu Val Val Leu Ser Gly Lys Ala Ile Leu Thr Val Leu Leu Pro Asn
               100                 105                 110
   Asp Arg Asn Ser Phe Ser Leu Glu Arg Gly Asp Thr Ile Lys Leu Pro
           115                 120                 125
   Ala Gly Thr Ile Gly Tyr Leu Val Asn Arg Asp Asp Glu Glu Asp Leu
       130                 135                 140
   Arg Val Leu Asp Leu Val Ile Pro Val Asn Arg Pro Gly Glu Pro Gln
   145                 150                 155                 160
   Ser Phe Leu Leu Ser Gly Asn Gln Asn Gln Pro Ser Ile Leu Ser Gly
                   165                 170                 175
   Phe Ser Lys Asn Ile Leu Glu Ala Ser Phe Asn Thr Asp Tyr Lys Glu
               180                 185                 190
   Ile Glu Lys Val Leu Leu Glu Glu His Gly Lys Glu Lys Tyr His Arg
           195                 200                 205
   Arg Gly Leu Lys Asp Arg Arg Gln Arg Gly Gln Glu Glu Asn Val Ile
       210                 215                 220
   Val Lys Ile Ser Arg Lys Gln Ile Glu Glu Leu Asn Lys Asn Ala Lys
   225                 230                 235                 240
   Ser Ser Ser Lys Lys Ser Thr Ser Ser Glu Ser Glu Pro Phe Asn Leu
                   245                 250                 255
   Arg Ser Arg Glu Pro Ile Tyr Ser Asn Lys Phe Gly Lys Phe Phe Glu
   260 265 270
   Ile Thr Pro Lys Arg Asn Pro Gln Leu Gln Asp Leu Asn Ile Phe Val
   275 280 285
   Asn Tyr Val Glu Ile Asn Glu Gly Ser Leu Leu Leu Pro His Tyr Asn
   290 295 300
   Ser Arg Ala Ile Val Ile Val Thr Val Asn Glu Gly Lys Gly Asp Phe
   305 310 315 320
   Glu Leu Val Gly Gln Arg Asn Glu Asn Gln Gln Gly Leu Arg Glu Glu
   325 330 335
   Tyr Asp Glu Glu Lys Glu Gln Gly Glu Glu Glu Ile Arg Lys Gln Val
   340 345 350
   Gln Asn Tyr Lys Ala Lys Leu Ser Pro Gly Asp Val Leu Val Ile Pro
   355 360 365
   Ala Gly Tyr Pro Val Ala Ile Lys Ala Ser Ser Asn Leu Asn Leu Val
   370 375 380
   Gly Phe Gly Ile Asn Ala Glu Asn Asn Gln Arg Tyr Phe Leu Ala Gly
   385 390 395 400
   Glu Glu Asp Asn Val Ile Ser Gln Ile His Lys Pro Val Lys Glu Leu
   405 410 415
   Ala Phe Pro Gly Ser Ala Gln Glu Val Asp Thr Leu Leu Glu Asn Gln
   420 425 430
   Lys Gln Ser His Phe Ala Asn Ala Gln Pro Arg Glu Arg Glu Arg Gly
   435 440 445
   Ser Gln Glu Ile Lys Asp His Leu Tyr Ser Ile Leu Gly Ser Phe
   450 455 460
<210> 507
   <211> 446
   <212> PRT
   <213> Cicer arietinum
<400> 507
   Met Ala Ile Lys Ala Arg Phe Pro Leu Leu Val Leu Leu Gly Ile Val
   1 5 10 15
   Phe Leu Ala Ser Val Cys Ala Lys Ser Asp Lys Glu Asn Pro Phe Phe
               20                  25                  30
   Phe Lys Ser Asn Asn Cys Gln Thr Leu Phe Glu Asn Glu Asn Gly His
           35                  40                  45
   Val Arg Leu Leu Gln Arg Phe Asp Lys Arg Ser Gln Leu Phe Glu Asn
       50                  55                  60
   Leu Gln Asn Tyr Arg Leu Met Glu Tyr Asn Ser Lys Pro His Thr Leu
   65                  70                  75                  80
   Phe Leu Pro Gln His Asn Asp Ala Asp Phe Ile Leu Val Val Leu Arg
                   85                  90                  95
   Gly Arg Ala Ile Leu Thr Val Leu Asn Pro Asn Asp Arg Asn Thr Phe
               100                 105                 110
   Lys Leu Glu Arg Gly Asp Thr Ile Lys Leu Pro Ala Gly Thr Ile Ala
           115                 120                 125
   Tyr Leu Ala Asn Arg Asp Asp Asn Glu Asp Leu Arg Val Leu Asp Leu
       130                 135                 140
   Ala Ile Pro Val Asn Arg Pro Gly Gln Phe Gln Ser Phe Ser Leu Ser
   145                 150                 155                 160
   Gly Asn Glu Asn Gln Gln Ser Tyr Phe Gln Gly Phe Ser Lys Lys Ile
                   165                 170                 175
   Leu Glu Ala Ser Phe Asn Ser Asp Tyr Glu Glu Ile Glu Arg Val Leu
               180                 185                 190
   Leu Glu Glu Gln Glu Gln Lys Pro Glu Gln Arg Arg Gly His Lys Gly
           195                 200                 205
   Arg Gln Gln Ser Gln Glu Thr Asp Val Ile Val Lys Ile Ser Arg Glu
       210                 215                 220
   Gln Ile Glu Glu Leu Ser Lys Asn Ala Lys Ser Asn Cys Lys Lys Ser
   225                 230                 235                 240
   Val Ser Ser Glu Ser Glu Pro Phe Asn Leu Arg Ser Arg Ser Pro Ile
                   245                 250                 255
   Tyr Ser Asn Arg Phe Gly Asn Phe Phe Glu Ile Thr Pro Glu Lys Asn
                   260                 265                 270
   Pro Gln Leu Lys Asp Leu Asp Ile Phe Val Asn Ser Val Glu Ile Lys
           275                 280                 285
   Glu Gly Ser Leu Leu Leu Pro His Phe Asn Ser Arg Ala Thr Val Ile
       290                 295                 300
   Leu Val Val Asn Glu Gly Lys Gly Glu Val Glu Leu Val Gly Leu Arg
   305                 310                 315                 320
   Asn Glu Asn Glu Gln Glu Asn Lys Lys Glu Asp Glu Glu Glu Glu Glu
                   325                 330                 335
   Asp Arg Asn Val Gln Val Gln Arg Phe Gln Ser Lys Leu Ser Ser Gly
               340                 345                 350
   Asp Val Val Val Ile Pro Ala Ser His Pro Phe Ser Ile Asn Ala Ser
           355                 360                 365
   Ser Asp Leu Phe Leu Leu Gly Phe Gly Ile Asn Ala Gln Asn Asn Gln
       370                 375                 380
   Arg Asn Phe Leu Ala Gly Glu Glu Asp Asn Val Ile Ser Gln Ile Gln
   385                 390                 395                 400
   Arg Pro Val Lys Glu Val Ala Phe Pro Gly Ser Ala Glu Glu Val Asp
                   405                 410                 415
   Arg Leu Leu Lys Asn Gln Arg Gln Ser His Phe Ala Asn Ala Gln Pro
               420                 425                 430
   Gln Gln Lys Arg Lys Gly Ser Gln Arg Ile Arg Ser Pro Phe
           435                 440                 445
<210> 508
   <211> 418
   <212> PRT
   <213> Lens culinaris
<400> 508
   Ser Arg Ser Asp Gln Glu Asn Pro Phe Ile Phe Lys Ser Asn Arg Phe
   1 5 10 15
   Gln Thr Ile Tyr Glu Asn Glu Asn Gly His Ile Arg Leu Leu Gln Arg
   20 25 30
   Phe Asp Lys Arg Ser Lys Ile Phe Glu Asn Leu Gln Asn Tyr Arg Leu
           35                  40                  45
   Leu Glu Tyr Lys Ser Lys Pro His Thr Ile Phe Leu Pro Gln Phe Thr
       50                  55                  60
   Asp Ala Asp Phe Ile Leu Val Val Leu Ser Gly Lys Ala Ile Leu Thr
   65                  70                  75                  80
   Val Leu Asn Ser Asn Asp Arg Asn Ser Phe Asn Leu Glu Arg Gly Asp
                   85                  90                  95
   Thr Ile Lys Leu Pro Ala Gly Thr Ile Ala Tyr Leu Ala Asn Arg Asp
               100                 105                 110
   Asp Asn Glu Asp Leu Arg Val Leu Asp Leu Ala Ile Pro Val Asn Arg
           115                 120                 125
   Pro Gly Gln Leu Gln Ser Phe Leu Leu Ser Gly Thr Gln Asn Gln Pro
       130                 135                 140
   Ser Phe Leu Ser Gly Phe Ser Lys Asn Ile Leu Glu Ala Ala Phe Asn
   145                 150                 155                 160
   Thr Glu Tyr Glu Glu Ile Glu Lys Val Leu Leu Glu Glu Gln Glu Gln
                   165                 170                 175
   Lys Ser Gln His Arg Arg Ser Leu Arg Asp Lys Arg Gln Glu Ile Thr
               180                 185                 190
   Asn Glu Asp Val Ile Val Lys Val Ser Arg Glu Gln Ile Glu Glu Leu
           195                 200                 205
   Ser Lys Asn Ala Lys Ser Ser Ser Lys Lys Ser Val Ser Ser Glu Ser
       210                 215                 220
   Glu Pro Phe Asn Leu Arg Ser Arg Asn Pro Ile Tyr Ser Asn Lys Phe
   225                 230                 235                 240
   Gly Lys Phe Phe Glu Ile Thr Pro Glu Lys Asn Pro Gln Leu Gln Asp
                   245                 250                 255
   Leu Asp Ile Phe Val Asn Ser Val Glu Ile Lys Glu Gly Ser Leu Leu
               260                 265                 270
   Leu Pro Asn Tyr Asn Ser Arg Ala Ile Val Ile Val Thr Val Asn Glu
           275                 280                 285
   Gly Lys Gly Asp Phe Glu Leu Val Gly Gln Arg Asn Glu Asn Gln Gln
   290 295 300
   Glu Gln Arg Glu Glu Asn Asp Glu Glu Glu Gly Gln Glu Glu Glu Thr
   305 310 315 320
   Thr Lys Gln Val Gln Arg Tyr Arg Ala Arg Leu Ser Pro Gly Asp Val
   325 330 335
   Leu Val Ile Pro Ala Gly His Pro Val Ala Ile Asn Ala Ser Ser Asp
   340 345 350
   Leu Asn Leu Ile Gly Phe Gly Ile Asn Ala Lys Asn Asn Gln Arg Asn
   355 360 365
   Phe Leu Ala Gly Glu Glu Asp Asn Val Ile Ser Gln Ile Gln Arg Pro
   370 375 380
   Val Lys Glu Leu Ala Phe Pro Gly Ser Ser Arg Glu Val Asp Arg Leu
   385 390 395 400
   Leu Thr Asn Gln Lys Gln Ser His Phe Ala Asn Ala Gln Pro Leu Gln
   405 410 415
   Ile Glu
<210> 509
   <211> 526
   <212> PRT
   <213> Pisum elatius
<400> 509
   Met Ala Thr Thr Val Glu Ser Arg Phe Pro Leu Leu Leu Phe Pro Gly
   1 5 10 15
   Ile Ile Phe Leu Ala Ser Val Cys Val Thr Tyr Ala Asn Tyr Asp Glu
   20 25 30
   Gly Ser Glu Thr Arg Val Pro Gly Gln Arg Glu Arg Gly Arg Gln Glu
   35 40 45
   Gly Glu Lys Glu Glu Lys Arg His Gly Glu Trp Arg Pro Ser Tyr Glu
   50 55 60
   Lys Glu Glu Asp Glu Glu Glu Lys Gln Lys Tyr Arg Tyr Gln Arg Glu
   65 70 75 80
   Lys Glu Asp Glu Glu Glu Lys Gln Lys Tyr Arg Tyr Gln Arg Glu Lys
                   85                  90                  95
   Lys Glu Glu Lys Glu Val Gln Pro Gly Arg Glu Arg Trp Glu Arg Glu
               100                 105                 110
   Glu Asp Glu Glu Gln Val Asp Glu Glu Trp Arg Gly Ser Gln Arg Arg
           115                 120                 125
   Gln Asp Pro Glu Glu Arg Ala Arg Leu Arg His Arg Glu Glu Arg Thr
       130                 135                 140
   Lys Arg Asp Arg Arg His Lys Arg Glu Gly Glu Glu Glu Glu Arg Ser
   145                 150                 155                 160
   Ser Glu Ser Gln Glu Gln Arg Asn Pro Phe Leu Phe Lys Ser Asn Lys
                   165                 170                 175
   Phe Leu Thr Leu Phe Glu Asn Glu Asn Gly His Ile Arg Arg Leu Gln
               180                 185                 190
   Arg Phe Asp Lys Arg Ser Asp Leu Phe Glu Asn Leu Gln Asn Tyr Arg
           195                 200                 205
   Leu Val Glu Tyr Arg Ala Lys Pro His Thr Ile Phe Leu Pro Gln His
       210                 215                 220
   Ile Asp Ala Asp Leu Ile Leu Val Val Leu Asn Gly Lys Ala Ile Leu
   225                 230                 235                 240
   Thr Val Leu Ser Pro Asn Asp Arg Asn Ser Tyr Asn Leu Glu Arg Gly
                   245                 250                 255
   Asp Thr Ile Lys Ile Pro Ala Gly Thr Thr Ser Tyr Leu Val Asn Gln
               260                 265                 270
   Asp Asp Glu Glu Asp Leu Arg Val Val Asp Phe Val Ile Pro Val Asn
           275                 280                 285
   Arg Pro Gly Lys Phe Glu Ala Phe Gly Leu Ser Glu Asn Lys Asn Gln
       290                 295                 300
   Tyr Leu Arg Gly Phe Ser Lys Asn Ile Leu Glu Ala Ser Leu Asn Thr
   305                 310                 315                 320
   Lys Tyr Glu Thr Ile Glu Lys Val Leu Leu Glu Glu Gln Glu Lys Lys
                   325                 330                 335
   Pro Gln Gln Leu Arg Asp Arg Lys Arg Arg Gln Gln Gly Gly Glu Arg
   340 345 350
   Asp Ala Ile Ile Lys Val Ser Arg Glu Gln Ile Glu Glu Leu Arg Lys
   355 360 365
   Leu Ala Lys Ser Ser Ser Lys Lys Ser Leu Pro Ser Glu Phe Glu Pro
   370 375 380
   Phe Asn Leu Arg Ser His Lys Pro Glu Tyr Ser Asn Lys Phe Gly Lys
   385 390 395 400
   Leu Phe Glu Ile Thr Pro Glu Lys Lys Tyr Pro Gln Leu Gln Asp Leu
   405 410 415
   Asp Ile Leu Val Ser Cys Val Glu Ile Asn Lys Gly Ala Leu Met Leu
   420 425 430
   Pro His Tyr Asn Ser Arg Ala Ile Val Val Leu Leu Val Asn Glu Gly
   435 440 445
   Lys Gly Asn Leu Glu Leu Leu Gly Leu Lys Asn Glu Gln Gln Glu Arg
   450 455 460
   Glu Asp Arg Lys Glu Arg Asn Asn Glu Val Gln Arg Tyr Glu Ala Arg
   465 470 475 480
   Leu Ser Pro Gly Asp Val Val Ile Ile Pro Ala Gly His Pro Val Ala
   485 490 495
   Ile Ser Ala Ser Ser Asn Leu Asn Leu Leu Gly Phe Gly Thr Asn Ala
   500 505 510
   Glu Asn Asn Gln Arg Asn Phe Leu Ser Gly Ser Asp Asp Asn
   515 520 525
<210> 510
   <211> 541
   <212> PRT
   <213> Lathyrus aphaca
<400> 510
   Met Ala Thr Thr Ile Lys Ser Arg Phe Pro Leu Leu Leu Leu Leu Gly
   1 5 10 15
   Ile Ile Phe Leu Ala Ser Val Cys Val Thr Trp Ala Asn Tyr Asp Glu
   20 25 30
   Gly Ser Glu Pro Arg Val Pro Gly Gln Arg Glu Arg Gly Arg Gln Glu
           35                  40                  45
   Gly Glu Lys Glu Glu Lys Arg His Gly Glu Trp Arg Pro Ser Tyr Glu
       50                  55                  60
   Glu Glu Tyr Asp Glu Gly Leu Glu Pro Lys Val Pro Gly Lys Arg Glu
   65                  70                  75                  80
   Arg Gly Arg Gln Glu Gly Glu Lys Glu Glu Lys Arg His Glu Glu Trp
                   85                  90                  95
   Arg Pro Ser Tyr Glu Lys Glu Glu Asp Glu Glu Glu Lys Gln Lys Tyr
               100                 105                 110
   Asn Tyr Gln Arg Glu Lys Lys Glu His Lys Glu Val Gln Pro Gly Arg
           115                 120                 125
   Glu Arg Trp Glu Arg Lys Gln Asp Glu Lys Gln Val Glu Glu Asp Glu
       130                 135                 140
   Glu Pro Gly Glu Glu Gln Trp Arg Gly Ser Lys Arg His Glu Asp Pro
   145                 150                 155                 160
   Glu Glu Arg Ala Arg Leu Arg His Arg Glu Glu Lys Thr Lys Ser Tyr
                   165                 170                 175
   Val Glu Asp Asn Glu Glu Thr Ser Ser Lys Glu Gly Arg Asn Pro Phe
               180                 185                 190
   Leu Phe Lys Ser Asn Lys Phe Leu Thr Leu Phe Glu Asn Glu Asn Gly
           195                 200                 205
   His Ile Arg Arg Leu Gln Arg Phe Asp Glu Arg Ser Asp Ile Phe Glu
       210                 215                 220
   Asn Leu Gln Asn Tyr Arg Leu Val Glu Tyr Arg Ala Lys Pro His Thr
   225                 230                 235                 240
   Met Phe Leu Pro Gln His Ile Asp Ala Asp Leu Ile Leu Val Val Leu
                   245                 250                 255
   Asn Gly Lys Ala Ile Leu Thr Val Leu Ser Pro Asn Asp Arg Asn Ser
               260                 265                 270
   Tyr Asn Leu Glu Arg Gly Asp Thr Val Lys Leu Pro Ala Gly Thr Thr
           275                 280                 285
   Ser Tyr Leu Val Asn Gln Asp Asp Glu Glu Asp Leu Arg Val Val Asp
       290                 295                 300
   Leu Ala Ile Pro Val Asn Arg Pro Gly Lys Phe Glu Ala Phe Gly Leu
   305                 310                 315                 320
   Ser Ala Asn Lys Asn Gln Tyr Leu Arg Gly Phe Ser Lys Asn Ile Leu
                   325                 330                 335
   Glu Ala Ser Leu Asn Thr Lys Tyr Glu Thr Ile Glu Lys Val Leu Leu
               340                 345                 350
   Glu Glu Arg Arg Asp Gln Lys Gly Arg Gln Gln Gly Gln Glu Thr Asn
           355                 360                 365
   Ala Ile Val Lys Val Ser Arg Glu Gln Ile Glu Glu Leu Arg Lys Leu
       370                 375                 380
   Ala Lys Ser Ser Ser Lys Lys Ser Leu Leu Ser Glu Ser Glu Pro Leu
   385                 390                 395                 400
   Asn Leu Arg Ser Gln Asn Pro Lys Tyr Ser Asn Lys Phe Gly Lys Phe
                   405                 410                 415
   Phe Glu Ile Thr Pro Gln Lys Lys Tyr Pro Gln Leu Gln Asp Leu Asp
               420                 425                 430
   Val Ser Ile Ser Cys Val Glu Ile Asn Lys Gly Ala Leu Leu Leu Pro
           435                 440                 445
   His Tyr Asn Ser Arg Ser Ile Gly Ile Leu Leu Val Asn Glu Gly Lys
       450                 455                 460
   Gly Asn Leu Glu Leu Val Gly Phe Lys Asn Glu Gln Gln Arg Gln Arg
   465                 470                 475                 480
   Glu Asn Glu Glu Thr Asn Lys Lys Leu Gln Arg Tyr Glu Ala Arg Leu
                   485                 490                 495
   Ser Ser Gly Asp Val Val Val Ile Pro Glu Gly His Pro Val Ala Ile
               500                 505                 510
   Ser Ala Ser Ser Asn Leu Asn Leu Leu Gly Phe Gly Ile Asn Ala Ala
           515                 520                 525
   Asn Asn Gln Arg Asn Phe Leu Thr Gly Ser Asp Asp Asn
   530 535 540
<210> 511
   <211> 589
   <212> PRT
   <213> Vicia villosa
<400> 511
   Met Ala Thr Thr Ile Lys Ser Arg Phe Pro Val Leu Leu Leu Leu Gly
   1               5                   10                  15
   Ile Ile Phe Leu Thr Ser Val Cys Val Thr Tyr Ala Asn Tyr Asp Glu
               20                  25                  30
   Gly Arg Glu Pro Ser Val Pro Gly Gln Arg Glu Arg Gly Arg Gln Glu
           35                  40                  45
   Gly Glu Lys Glu Glu Lys Arg His Gly Glu Trp Arg Pro Ser Glu Glu
       50                  55                  60
   Asp Glu Glu Glu Lys Tyr Lys Tyr Glu Glu Gly Arg Val Pro Gly Gln
   65                  70                  75                  80
   Arg Glu Arg Gly Arg Gln Glu Gly Glu Lys Glu Glu Lys Arg His Gly
                   85                  90                  95
   Lys Trp Arg Pro Ser Glu Glu Glu Asp Glu Glu Glu Lys Tyr Arg Tyr
               100                 105                 110
   Glu Glu Gly Ser Glu Pro Arg Gly Pro Gly Gln Arg Glu Thr Gly Arg
           115                 120                 125
   Gln Glu Gly Glu Lys Glu Lys Gln Arg Pro Glu Arg Glu Pro Ser Tyr
       130                 135                 140
   Glu Lys Glu Glu Asp Glu Glu Glu Lys Gln Lys Tyr Gln Tyr His Arg
   145                 150                 155                 160
   Glu Lys Lys Glu Gln Arg Glu Val Arg Pro Gly Arg Glu Arg Phe Glu
                   165                 170                 175
   Arg His Glu Asp Glu Glu Gln Trp Arg Gly Ile Gln Arg His Glu Asp
               180                 185                 190
   Pro Glu Glu Arg Ala Arg Glu Arg Tyr Arg Ala Glu Ile Ala Lys Arg
           195                 200                 205
   Gln Val Glu Glu Glu Arg Glu Glu Arg Asp Ile Pro His Glu Arg Glu
   210 215 220
   Gln Arg Asn Pro Phe Leu Phe Lys Ser Asn Lys Phe Gln Thr Leu Phe
   225 230 235 240
   Gln Asn Glu Asn Gly Tyr Ile Arg Arg Leu Gln Arg Phe Asp Lys Arg
   245 250 255
   Ser Asp Leu Phe Glu Asn Leu Gln Asn Tyr Arg Leu Val Glu Tyr Arg
   260 265 270
   Ala Lys Pro His Thr Ile Phe Leu Pro Gln His Ile Asp Ala Asp Leu
   275 280 285
   Ile Ile Val Val Leu Ser Gly Arg Ala Ile Leu Thr Val Leu Ser Pro
   290 295 300
   Asp Asp Arg Asn Ser Tyr Asn Leu Glu Arg Gly Asp Thr Ile Lys Leu
   305 310 315 320
   Pro Ala Gly Thr Thr Ser Tyr Leu Val Asn Gln Asp Asp Glu Glu Asp
   325 330 335
   Leu Arg Val Val Asp Leu Ala Ile Pro Val Asn Arg Pro Gly Lys Val
   340 345 350
   Glu Ser Phe Leu Leu Ser Gly Asn Lys Asn Gln Tyr Leu Arg Gly Phe
   355 360 365
   Ser Lys Asn Ile Leu Glu Ala Ser Phe Asn Thr Asn Tyr Glu Thr Ile
   370 375 380
   Glu Arg Val Leu Leu Glu Glu Gln Asp Lys Glu Ser Gln Gln Ser Ile
   385 390 395 400
   Gly Gln Lys Arg Arg Ser Gln Arg Gln Glu Thr Asn Ala Leu Val Lys
   405 410 415
   Val Ser Arg Glu Gln Leu Glu Asp Leu Lys Arg Leu Ala Lys Ser Ser
   420 425 430
   Ser Gln Glu Gly Leu Ser Ser Gln Phe Glu Pro Ile Asn Leu Arg Ser
   435 440 445
   Gln Asn Pro Lys Tyr Ser Asn Lys Phe Gly Lys Val Phe Glu Ile Thr
   450 455 460
   Pro Glu Lys Lys Tyr Pro Gln Leu Gln Asp Leu Asp Leu Phe Val Ser
   465 470 475 480
   Ser Val Asp Ile Lys Glu Gly Ala Leu Met Leu Pro His Tyr Asn Ser
   485 490 495
   Arg Ala Ile Val Val Leu Leu Val Asn Glu Gly Arg Gly Asn Leu Glu
   500 505 510
   Leu Val Gly Leu Lys Asn Glu Gln Gln Glu Gln Arg Glu Lys Glu Asp
   515 520 525
   Glu Gln Gln Glu Arg Asn Asn Gln Val Gln Arg Tyr Glu Ala Arg Leu
   530 535 540
   Ser Pro Gly Asp Val Val Ile Ile Pro Ala Gly His Pro Val Ala Val
   545 550 555 560
   Arg Ala Ser Ser Asp Leu Asn Leu Leu Ala Phe Gly Ile Asn Ala Glu
   565 570 575
   Asn Asn Gln Arg Asn Phe Leu Ala Gly Ser Asp Asp Asn
   580 585
<210> 512
   <211> 861
   <212> PRT
   <213> Medicago truncatula
<400> 512
   Met Phe Ser Gly Val Thr Gly Ile Leu Asn Arg Gly His Lys Ile Lys
   1 5 10 15
   Gly Thr Val Val Leu Met Arg Lys Asn Val Leu Asp Ile Asn Ser Leu
   20 25 30
   Thr Ser Val Gly Gly Val Ile Gly Gln Gly Phe Asp Ile Leu Gly Ser
   35 40 45
   Thr Leu Asp Asn Leu Thr Ala Phe Leu Gly Arg Ser Val Ser Leu Gln
   50 55 60
   Leu Ile Ser Ala Thr Lys Pro Asp Ala Asn Gly Lys Gly Lys Leu Gly
   65 70 75 80
   Lys Ala Thr Phe Leu Glu Gly Ile Ile Thr Ser Leu Pro Thr Leu Gly
   85 90 95
   Ala Gly Gln Ser Ala Phe Lys Ile His Phe Glu Trp Asp Asp Asp Met
   100 105 110
   Gly Ile Pro Gly Ala Phe Tyr Ile Lys Asn Phe Met Gln Thr Glu Phe
   115 120 125
   Phe Leu Val Ser Leu Thr Leu Glu Asp Ile Pro Asn His Gly Ser Ile
   130 135 140
   Tyr Phe Val Cys Asn Ser Trp Ile Tyr Asn Ala Lys His His Lys Leu
   145 150 155 160
   Asp Arg Ile Phe Phe Ala Asn Lys Ala Tyr Leu Pro Ser Glu Thr Pro
   165 170 175
   Ala Pro Leu Val His Tyr Arg Glu Glu Glu Leu Asn Asn Leu Arg Gly
   180 185 190
   Asp Gly Thr Gly Glu Arg Lys Glu Trp Glu Arg Ile Tyr Asp Tyr Asp
   195 200 205
   Val Tyr Asn Asp Leu Gly Asn Pro Glu Lys Gly Asp Asn His Ala Arg
   210 215 220
   Pro Val Leu Gly Gly Ser Asp Thr Tyr Pro Tyr Pro Arg Arg Gly Arg
   225 230 235 240
   Thr Gly Arg Lys Pro Asn Pro Lys Asp Pro Lys Ser Glu Ser Arg Ser
   245 250 255
   Asp Phe Val Tyr Leu Pro Arg Asp Glu Ala Phe Gly His Leu Lys Ser
   260 265 270
   Ser Asp Phe Leu Thr Tyr Gly Leu Lys Ala Val Ser Gln Asn Val Val
   275 280 285
   Pro Ala Leu Glu Ser Val Ile Phe Asp Leu Asn Phe Thr Pro Asn Glu
   290 295 300
   Phe Asp Ser Phe Asp Glu Val His Gly Leu Tyr Glu Gly Gly Ile Lys
   305 310 315 320
   Leu Pro Thr Asp Val Leu Ser Lys Ile Ser Pro Leu Pro Val Leu Lys
   325 330 335
   Glu Ile Phe Arg Thr Asp Gly Glu Gln Phe Leu Lys Tyr Pro Pro Pro
   340 345 350
   Lys Val Leu Gln Val Ser Arg Ser Ala Trp Met Thr Asp Glu Glu Phe
           355                 360                 365
   Ala Arg Glu Met Leu Ala Gly Val Asn Pro Asn Val Ile Cys Cys Leu
       370                 375                 380
   Gln Glu Phe Pro Pro Arg Ser Lys Leu Asp Ser Gln Val Tyr Gly Asp
   385                 390                 395                 400
   His Thr Ser Lys Ile Thr Lys Glu His Leu Glu Pro Asn Leu Glu Gly
                   405                 410                 415
   Leu Thr Val Glu Glu Ala Ile Gln Asn Lys Lys Leu Phe Leu Leu Asp
               420                 425                 430
   His His Asp Ser Ile Met Pro Tyr Leu Arg Arg Ile Asn Ser Thr Pro
           435                 440                 445
   Thr Lys Ala Tyr Ala Thr Arg Thr Ile Leu Phe Leu Ser Ser Asp Lys
       450                 455                 460
   Thr Leu Lys Pro Leu Ala Ile Glu Leu Ser Leu Pro His Pro Asp Gly
   465                 470                 475                 480
   Asp Glu His Gly Ala Val Ser His Val Tyr Gln Pro Ala Leu Glu Gly
                   485                 490                 495
   Val Glu Ser Thr Ile Trp Leu Leu Ala Lys Ala Tyr Val Val Val Asn
               500                 505                 510
   Asp Ser Cys Tyr His Gln Leu Val Ser His Trp Leu Asn Thr His Ala
           515                 520                 525
   Val Val Glu Pro Phe Val Ile Ala Thr Asn Arg His Leu Ser Tyr Leu
       530                 535                 540
   His Pro Ile Tyr Lys Leu Leu Tyr Pro His Tyr Arg Asp Thr Met Asn
   545                 550                 555                 560
   Ile Asn Ser Leu Ala Arg Gln Ser Leu Val Asn Asp Gly Gly Ile Ile
                   565                 570                 575
   Glu Lys Thr Phe Leu Trp Gly Arg Tyr Ser Met Glu Met Ser Ser Lys
               580                 585                 590
   Val Tyr Lys Asn Trp Thr Leu Pro Gly Gln Ala Leu Pro Ala Asp Leu
           595                 600                 605
   Ile Lys Arg Gly Met Ala Ile Glu Glu Pro Ser Ser Pro Cys Gly Val
   610 615 620
   Lys Leu Val Val Glu Asp Tyr Pro Tyr Ala His Asp Gly Leu Glu Ile
   625 630 635 640
   Trp Ala Ala Ile Lys Thr Trp Val Gln Asp Tyr Val Ser Leu Tyr Tyr
   645 650 655
   Thr Thr Asp Asp Ile Leu Arg Gln Asp Ser Glu Leu Gln Ala Trp Trp
   660 665 670
   Lys Glu Leu Val Glu Val Gly His Gly Asp Lys Lys Asn Glu Pro Trp
   675 680 685
   Trp Pro Lys Met Gln Ala Arg Glu Glu Leu Val Glu Val Cys Thr Thr
   690 695 700
   Val Ile Trp Ile Ala Ser Ala Leu His Ala Ala Val Asn Phe Gly Gln
   705 710 715 720
   Tyr Ser Tyr Gly Gly Leu Ile Leu Asn Arg Pro Thr Leu Ser Arg Arg
   725 730 735
   Phe Met Pro Glu Lys Gly Ser Ala Glu Tyr Asn Glu Leu Val Lys Ser
   740 745 750
   Pro Gln Lys Ala Tyr Leu Lys Thr Ile Thr Pro Lys Phe Gln Thr Leu
   755 760 765
   Ile Asp Leu Ser Val Ile Glu Ile Leu Ser Arg His Ala Ser Asp Glu
   770 775 780
   Val Tyr Leu Gly Glu Arg Asp Asn Pro Asn Trp Thr Ser Asp Thr Arg
   785 790 795 800
   Ala Leu Glu Ala Phe Lys Lys Phe Gly Asn Lys Leu Ala Glu Ile Glu
   805 810 815
   Lys Asn Leu Ala Gln Arg Asn Asn Asp Glu Lys Leu Arg His Arg Leu
   820 825 830
   Gly Pro Val Gln Met Pro Tyr Thr Leu Leu His Pro Ser Ser Glu Glu
   835 840 845
   Gly Leu Thr Phe Arg Gly Ile Pro Asn Ser Ile Ser Ile
   850 855 860
<210> 513
   <211> 857
   <212> PRT
   <213> Glycine soja
<400> 513
   Met Leu Gly Gly Leu Leu His Arg Gly His Lys Ile Lys Gly Thr Val
   1               5                   10                  15
   Val Leu Met Arg Lys Asn Val Leu Asp Val Asn Ser Val Thr Ser Val
               20                  25                  30
   Gly Gly Ile Ile Gly Gln Gly Leu Asp Leu Val Gly Ser Thr Leu Asp
           35                  40                  45
   Thr Leu Thr Ala Phe Leu Gly Arg Ser Val Ser Leu Gln Leu Ile Ser
       50                  55                  60
   Ala Thr Lys Ala Asp Ala Asn Gly Lys Gly Lys Leu Gly Lys Ala Thr
   65                  70                  75                  80
   Phe Leu Glu Gly Ile Ile Thr Ser Leu Pro Thr Leu Gly Ala Gly Gln
                   85                  90                  95
   Ser Ala Phe Lys Ile Asn Phe Glu Trp Asp Asp Gly Ser Gly Ile Pro
               100                 105                 110
   Gly Ala Phe Tyr Ile Lys Asn Phe Met Gln Thr Glu Phe Phe Leu Val
           115                 120                 125
   Ser Leu Thr Leu Glu Asp Ile Pro Asn His Gly Ser Ile His Phe Val
       130                 135                 140
   Cys Asn Ser Trp Ile Tyr Asn Ala Lys Leu Phe Lys Ser Asp Arg Ile
   145                 150                 155                 160
   Phe Phe Ala Asn Gln Thr Tyr Leu Pro Ser Glu Thr Pro Ala Pro Leu
                   165                 170                 175
   Val Lys Tyr Arg Glu Glu Glu Leu His Asn Leu Arg Gly Asp Gly Thr
               180                 185                 190
   Gly Glu Arg Lys Glu Trp Glu Arg Ile Tyr Asp Tyr Asp Val Tyr Asn
           195                 200                 205
   Asp Leu Gly Asp Pro Asp Lys Gly Glu Asn His Ala Arg Pro Val Leu
       210                 215                 220
   Gly Gly Asn Asp Thr Phe Pro Tyr Pro Arg Arg Gly Arg Thr Gly Arg
   225                 230                 235                 240
   Lys Pro Thr Arg Lys Asp Pro Asn Ser Glu Ser Arg Ser Asn Asp Val
                   245                 250                 255
   Tyr Leu Pro Arg Asp Glu Ala Phe Gly His Leu Lys Ser Ser Asp Phe
               260                 265                 270
   Leu Thr Tyr Gly Leu Lys Ser Val Ser Gln Asn Val Leu Pro Leu Leu
           275                 280                 285
   Gln Ser Ala Phe Asp Leu Asn Phe Thr Pro Arg Glu Phe Asp Ser Phe
       290                 295                 300
   Asp Glu Val His Gly Leu Tyr Ser Gly Gly Ile Lys Leu Pro Thr Asp
   305                 310                 315                 320
   Ile Ile Ser Lys Ile Ser Pro Leu Pro Val Leu Lys Glu Ile Phe Arg
                   325                 330                 335
   Thr Asp Gly Glu Gln Ala Leu Lys Phe Pro Pro Pro Lys Val Ile Gln
               340                 345                 350
   Val Ser Lys Ser Ala Trp Met Thr Asp Glu Glu Phe Ala Arg Glu Met
           355                 360                 365
   Leu Ala Gly Val Asn Pro Asn Leu Ile Arg Cys Leu Lys Asp Phe Pro
       370                 375                 380
   Pro Arg Ser Lys Leu Asp Ser Gln Val Tyr Gly Asp His Thr Ser Gln
   385                 390                 395                 400
   Ile Thr Lys Glu His Leu Glu Pro Asn Leu Glu Gly Leu Thr Val Asp
                   405                 410                 415
   Glu Ala Ile Gln Asn Lys Arg Leu Phe Leu Leu Asp His His Asp Pro
               420                 425                 430
   Ile Met Pro Tyr Leu Arg Arg Ile Asn Ala Thr Ser Thr Lys Ala Tyr
           435                 440                 445
   Ala Thr Arg Thr Ile Leu Phe Leu Lys Asn Asp Gly Thr Leu Arg Pro
       450                 455                 460
   Leu Ala Ile Glu Leu Ser Leu Pro His Pro Gln Gly Asp Gln Ser Gly
   465                 470                 475                 480
   Ala Phe Ser Gln Val Phe Leu Pro Ala Asp Glu Gly Val Glu Ser Ser
                   485                 490                 495
   Ile Trp Leu Leu Ala Lys Ala Tyr Val Val Val Asn Asp Ser Cys Tyr
               500                 505                 510
   His Gln Leu Val Ser His Trp Leu Asn Thr His Ala Val Val Glu Pro
           515                 520                 525
   Phe Ile Ile Ala Thr Asn Arg His Leu Ser Val Val His Pro Ile Tyr
       530                 535                 540
   Lys Leu Leu His Pro His Tyr Arg Asp Thr Met Asn Ile Asn Gly Leu
   545                 550                 555                 560
   Ala Arg Leu Ser Leu Val Asn Asp Gly Gly Val Ile Glu Gln Thr Phe
                   565                 570                 575
   Leu Trp Gly Arg Tyr Ser Val Glu Met Ser Ala Val Val Tyr Lys Asp
               580                 585                 590
   Trp Val Phe Thr Asp Gln Ala Leu Pro Ala Asp Leu Ile Lys Arg Gly
           595                 600                 605
   Met Ala Ile Glu Asp Pro Ser Cys Pro His Gly Ile Arg Leu Val Ile
       610                 615                 620
   Glu Asp Tyr Pro Tyr Ala Val Asp Gly Leu Glu Ile Trp Asp Ala Ile
   625                 630                 635                 640
   Lys Thr Trp Val His Glu Tyr Val Phe Leu Tyr Tyr Lys Ser Asp Asp
                   645                 650                 655
   Thr Leu Arg Glu Asp Pro Glu Leu Gln Ala Cys Trp Lys Glu Leu Val
               660                 665                 670
   Glu Val Gly His Gly Asp Lys Lys Asn Glu Pro Trp Trp Pro Lys Met
           675                 680                 685
   Gln Thr Arg Glu Glu Leu Val Glu Ser Cys Ala Ile Ile Ile Trp Thr
       690                 695                 700
   Ala Ser Ala Leu His Ala Ala Val Asn Phe Gly Gln Tyr Pro Tyr Gly
   705                 710                 715                 720
   Gly Leu Ile Leu Asn Arg Pro Thr Leu Ser Arg Arg Phe Met Pro Glu
   725 730 735
   Lys Gly Ser Ala Glu Tyr Glu Glu Leu Arg Lys Asn Pro Gln Lys Ala
   740 745 750
   Tyr Leu Lys Thr Ile Thr Pro Lys Phe Gln Thr Leu Ile Asp Leu Ser
   755 760 765
   Val Ile Glu Ile Leu Ser Arg His Ala Ser Asp Glu Val Tyr Leu Gly
   770 775 780
   Glu Arg Asp Asn Pro Asn Trp Thr Ser Asp Thr Arg Ala Leu Glu Ala
   785 790 795 800
   Phe Lys Arg Phe Gly Asn Lys Leu Ala Gln Ile Glu Asn Lys Leu Ser
   805 810 815
   Glu Arg Asn Asn Asp Glu Lys Leu Arg Asn Arg Cys Gly Pro Val Gln
   820 825 830
   Met Pro Tyr Thr Leu Leu Leu Pro Ser Ser Lys Glu Gly Leu Thr Phe
   835 840 845
   Arg Gly Ile Pro Asn Ser Ile Ser Ile
   850 855
<210> 514
   <211> 860
   <212> PRT
   <213> Phaseolus vulgaris
<400> 514
   Met Phe Ser Gly Val Ser Gly Leu Ile Asn Arg Gly His Lys Leu Lys
   1 5 10 15
   Gly Thr Val Val Leu Met Arg Lys Asn Val Leu Asp Val Asn Ser Val
   20 25 30
   Thr Ser Val Gly Gly Ile Val Gly Gln Gly Leu Asp Ile Leu Gly Ser
   35 40 45
   Thr Val Asp Asn Leu Thr Ala Phe Leu Gly Arg Ser Val Ser Leu Gln
   50 55 60
   Leu Ile Ser Ala Thr Lys Pro Asp Ala Asn Gly Lys Gly Lys Leu Gly
   65 70 75 80
   Lys Ala Thr Phe Leu Glu Gly Ile Ile Thr Ser Leu Pro Thr Leu Gly
                   85                  90                  95
   Ala Gly Gln Ser Ala Phe Lys Ile His Phe Glu Trp Asp Asp Glu Met
               100                 105                 110
   Gly Ile Pro Gly Ala Phe Tyr Ile Lys Asn Phe Met Gln Thr Glu Phe
           115                 120                 125
   Tyr Leu Val Ser Leu Thr Leu Glu Asp Ile Pro Asn His Gly Ser Leu
       130                 135                 140
   His Phe Leu Cys Asn Ser Trp Ile Tyr Asn Ala Lys His Phe Lys Asn
   145                 150                 155                 160
   Asp Arg Ile Phe Phe Val Asn Gln Ile Tyr Leu Pro Ser Glu Thr Pro
                   165                 170                 175
   Ala Pro Leu Val Lys Tyr Arg Glu Glu Glu Leu Val Asn Met Arg Gly
               180                 185                 190
   Asp Gly Thr Gly Glu Arg Lys Glu Trp Asp Arg Ile Tyr Asp Tyr Asp
           195                 200                 205
   Val Tyr Asn Asp Leu Gly Asp Pro Asp Lys Gly Glu Asn Asn Ala Arg
       210                 215                 220
   Pro Ile Leu Gly Gly Ser Asp Thr Leu Pro Tyr Pro Arg Arg Gly Arg
   225                 230                 235                 240
   Thr Gly Arg Arg Pro Thr Arg Lys Asp Pro Lys Ser Glu Ser Arg Ser
                   245                 250                 255
   Ser Asp Ile Tyr Leu Pro Arg Asp Glu Ala Phe Gly His Leu Lys Ser
               260                 265                 270
   Ser Asp Phe Leu Thr Tyr Gly Leu Lys Ser Val Ser Gln Asn Phe Leu
           275                 280                 285
   Pro Ala Leu Gln Ser Ala Phe Asp Leu Asn Phe Thr Pro Asn Glu Phe
       290                 295                 300
   Asp Ser Phe Glu Glu Val His Gly Leu Tyr Ser Gly Gly Ile Lys Leu
   305                 310                 315                 320
   Pro Thr Asp Val Leu Ser Lys Ile Ser Pro Leu Pro Val Leu Lys Glu
                   325                 330                 335
   Ile Phe Arg Thr Asp Gly Glu Gln Thr Leu Lys Phe Pro Pro Pro Lys
               340                 345                 350
   Val Val Gln Asp Ser Lys Ser Ala Trp Met Thr Asp Glu Glu Phe Ala
           355                 360                 365
   Arg Glu Met Ile Cys Gly Val Asn Pro Asn Leu Ile Arg Leu Leu Gln
       370                 375                 380
   Asp Phe Pro Pro Gln Ser Lys Leu Asp Ser Gln Val Tyr Gly Asp His
   385                 390                 395                 400
   Thr Ser Gln Ile Thr Lys Glu Asn Leu Glu Pro Asn Leu Glu Gly Leu
                   405                 410                 415
   Thr Val Asp Glu Ala Ile Gln Ser Lys Arg Leu Phe Leu Leu Asp His
               420                 425                 430
   His Asp Ser Ile Met Pro Tyr Leu Arg Arg Ile Asn Ala Thr Ser Ser
           435                 440                 445
   Lys Ala Tyr Ala Thr Arg Thr Ile Leu Phe Leu Lys Lys Asp Arg Thr
       450                 455                 460
   Leu Lys Pro Leu Ala Ile Glu Leu Ser Leu Pro His Pro Gly Gly Asp
   465                 470                 475                 480
   Lys Ser Gly Val Val Ser Gln Val Phe Leu Pro Ala Asp Glu Gly Val
                   485                 490                 495
   Glu Ser Ser Val Trp Leu Leu Ala Lys Ser Tyr Val Ile Val Asn Asp
               500                 505                 510
   Ser Ser Tyr His Gln Leu Val Ser His Trp Leu Asn Thr His Ala Val
           515                 520                 525
   Val Glu Pro Phe Val Ile Ala Thr Asn Arg His Leu Ser Val Val His
       530                 535                 540
   Pro Ile Tyr Lys Leu Leu His Pro His Tyr Arg Asp Thr Met Asn Ile
   545                 550                 555                 560
   Asn Ala Leu Ala Arg Gly Asp Leu Val Asn His Gly Gly Ile Ile Glu
                   565                 570                 575
   Lys Thr Phe Val Trp Gly Arg Tyr Ser Met Glu Met Ser Ala Val Ile
   580 585 590
   Tyr Lys Asp Trp Val Phe Thr Asp Gln Ala Leu Pro Ala Asp Leu Ile
   595 600 605
   Lys Arg Gly Ile Ala Thr Glu Asp Pro Glu Cys Pro His Gly Leu Arg
   610 615 620
   Leu Phe Ile Glu Asp Tyr Pro Tyr Ala Val Asp Gly Leu Glu Ile Trp
   625 630 635 640
   Asp Ala Ile Lys Thr Trp Val His Glu Tyr Val Phe Leu Tyr Tyr Lys
   645 650 655
   Ser Asp Asp Thr Leu Lys Glu Asp Pro Glu Leu Gln Ala Trp Trp Lys
   660 665 670
   Glu Leu Val Glu Val Gly His Gly Asp Lys Lys Asn Glu Pro Trp Trp
   675 680 685
   Pro Lys Met Gln Thr Arg Glu Glu Leu Val Glu Ala Cys Ser Ile Val
   690 695 700
   Ile Trp Thr Ala Ser Ala Leu His Ala Ala Val Asn Phe Gly Gln Tyr
   705 710 715 720
   Pro Tyr Gly Gly Leu Ile Leu Asn Arg Pro Thr Leu Ser Arg Arg Phe
   725 730 735
   Met Pro Glu Glu Gly Ser Ala Glu Tyr Glu Glu Leu Lys Lys Ser Pro
   740 745 750
   Gln Lys Ala Leu Leu Lys Thr Ile Thr Pro Lys Phe Gln Thr Leu Val
   755 760 765
   Asp Leu Ser Val Ile Glu Ile Leu Ser Arg His Ala Ser Asp Glu Val
   770 775 780
   Tyr Leu Gly Glu Arg Asp Asn Pro Asn Trp Thr Ser Asp Thr Arg Ala
   785 790 795 800
   Leu Glu Ala Phe Lys Arg Phe Gly Lys Lys Leu Ser Glu Ile Glu Lys
   805 810 815
   Lys Leu Ser Gln Arg Asn Asn Asp Glu Lys Leu Arg Asn Arg Tyr Gly
   820 825 830
   Pro Val Met Met Pro Tyr Thr Leu Leu Phe Pro Ser Ser Asp Glu Gly
           835                 840                 845
   Leu Thr Phe Arg Gly Ile Pro Asn Ser Ile Ser Ile
       850                 855                 860
<210> 515
   <211> 498
   <212> PRT
   <213> Vicia sativa
<400> 515
   Met Ala Lys Leu Leu Ala Leu Ser Leu Ser Phe Cys Phe Leu Leu Phe
   1               5                   10                  15
   Ser Ser Cys Phe Ala Leu Arg Glu Gln Ser Gln Gln Asn Glu Cys Gln
               20                  25                  30
   Leu Glu Arg Ile Asn Ala Leu Glu Pro Asp Asn Arg Ile Glu Ser Glu
           35                  40                  45
   Gly Gly Leu Ile Glu Thr Trp Asn Pro Asn Asn Arg Gln Phe Arg Cys
       50                  55                  60
   Ala Arg Val Ala Leu Ser Arg Ala Thr Leu Gln Arg Asn Ala Leu Arg
   65                  70                  75                  80
   Arg Pro Tyr Tyr Ser Asn Ala Pro Gln Glu Ile Tyr Ile Gln Gln Gly
                   85                  90                  95
   Asn Gly Tyr Phe Gly Met Val Phe Pro Gly Cys Pro Glu Thr His Glu
               100                 105                 110
   Glu Pro Gln Gln Ser Glu Gln Gly Glu Gly Arg Arg Tyr Arg Asp Ser
           115                 120                 125
   His Gln Lys Val Asn Arg Phe Arg Glu Gly Asp Ile Ile Ala Val Pro
       130                 135                 140
   Thr Gly Ile Ala Phe Trp Met Tyr Asn Asp Gln Asp Thr Pro Val Ile
   145                 150                 155                 160
   Ala Ile Ser Leu Thr Asp Thr Gly Ser Ser Asn Asn Gln Leu Asp Gln
                   165                 170                 175
   Met Pro Arg Arg Phe Tyr Leu Ala Gly Asn Gln Glu Gln Glu Phe Leu
               180                 185                 190
   Arg Tyr Gln His Gln Gln Gly Gly Lys Gln Glu Gln Asp Asn Asp Gly
           195                 200                 205
   Asn Asn Ile Phe Ser Gly Phe Lys Arg Asp Phe Leu Glu Asp Ala Phe
       210                 215                 220
   Asn Val Asn Arg His Ile Val Asp Arg Leu Gln Gly Arg Asn Glu Asp
   225                 230                 235                 240
   Glu Glu Lys Gly Ala Ile Val Lys Val Lys Gly Gly Leu Ser Ile Ile
                   245                 250                 255
   Ala Pro Pro Glu Arg Gln Ala Arg His Glu Arg Gly Ser Arg Gln Glu
               260                 265                 270
   Glu Asp Glu Asp Glu Lys Glu Glu Arg Gln Pro Ser His His Lys Ser
           275                 280                 285
   Arg Arg Asp Glu Asp Glu Asp Asp Lys Glu Lys Arg His Ser Gln Lys
       290                 295                 300
   Gly Gln Ser Arg Arg Gln Gly Asp Asn Gly Leu Glu Glu Thr Val Cys
   305                 310                 315                 320
   Thr Ala Lys Leu Arg Ala Asn Ile Gly Ser Ser Pro Ser Pro Asp Ile
                   325                 330                 335
   Tyr Asn Pro Gln Ala Gly Arg Ile Lys Thr Val Thr Ser Leu Asp Leu
               340                 345                 350
   Pro Val Leu Arg Trp Leu Lys Leu Ser Ala Glu His Gly Ser Leu His
           355                 360                 365
   Lys Asn Ala Met Phe Val Pro His Tyr Asn Leu Asn Ala Asn Ser Val
       370                 375                 380
   Ile Tyr Ala Leu Lys Gly Arg Ala Arg Leu Gln Val Val Asn Cys Asn
   385                 390                 395                 400
   Gly Asn Thr Val Phe Asp Gly Glu Leu Glu Ala Gly Arg Ala Leu Thr
                   405                 410                 415
   Val Pro Gln Asn Tyr Ala Val Ala Ala Lys Ser Leu Ser Glu Arg Phe
               420                 425                 430
   Thr Tyr Val Ala Phe Lys Thr Asp Asp Arg Ala Ser Ile Ala Arg Leu
           435                 440                 445
   Ala Gly Thr Ser Ser Val Ile Asp Asp Leu Pro Leu Asp Val Val Ala
       450                 455                 460
   Ala Thr Phe Asn Met Gln Arg Asn Glu Ala Arg Gln Leu Lys Ser Asn
   465                 470                 475                 480
   Asn Pro Phe Lys Phe Leu Val Pro Pro Arg Gln Ser Glu Met Arg Ala
                   485                 490                 495
   Ser Ala
<210> 516
   <211> 482
   <212> PRT
   <213> Vicia narbonensis
<400> 516
   Met Ala Lys Leu Leu Ala Leu Ser Leu Ser Leu Cys Phe Leu Leu Phe
   1               5                   10                  15
   Ser Asn Ser Phe Ala Leu Arg Glu Gln Ser Gln Gln Asn Glu Cys Gln
               20                  25                  30
   Leu Glu Arg Leu Asp Ala Leu Glu Pro Asp Asn Arg Ile Glu Ser Glu
           35                  40                  45
   Gly Gly Leu Ile Glu Thr Trp Asn Pro Asn Asn Arg Gln Phe Arg Cys
       50                  55                  60
   Ala Gly Val Ala Leu Ser Arg Val Thr Leu Gln Arg Asn Ala Leu Arg
   65                  70                  75                  80
   Arg Pro Tyr Tyr Ser Asn Ala Pro Gln Glu Ile Tyr Ile Gln Gln Gly
                   85                  90                  95
   Asn Gly Tyr Phe Gly Val Val Phe Pro Gly Cys Pro Glu Thr Phe Glu
               100                 105                 110
   Glu Pro Gln Glu Ser Glu Gln Arg Glu Arg Arg Arg Tyr Arg Asp Ser
           115                 120                 125
   His Gln Lys Val Asn Arg Phe Arg Glu Gly Asp Ile Ile Ala Val Pro
       130                 135                 140
   Thr Gly Asn Val Leu Trp Met Tyr Asn Asp Gln Asp Thr Pro Val Ile
   145                 150                 155                 160
   Ala Ile Ser Leu Thr Asp Thr Gly Ser Ser Asn Asn Gln Leu Asp Gln
                   165                 170                 175
   Ile Pro Arg Arg Phe Tyr Leu Ala Gly Asn Gln Glu Gln Glu Phe Leu
               180                 185                 190
   Arg Tyr Gln Arg Glu Gln Gly Gly Lys Gln Glu Gln Glu Asn Asp Gly
           195                 200                 205
   Asn Asn Ile Phe Ser Gly Phe Lys Arg Asp Phe Leu Glu Asp Ala Leu
       210                 215                 220
   Asn Val Asn Arg His Ile Val Asp Arg Leu Gln Gly Arg Asn Glu Asp
   225                 230                 235                 240
   Glu Glu Lys Gly Ala Ile Val Lys Val Lys Gly Gly Leu Ser Ile Ile
                   245                 250                 255
   Thr Pro Pro Glu Arg Gln Arg Gly Ser Arg Gln Glu Glu Asp Glu Asp
               260                 265                 270
   Glu Lys Glu Glu Arg Gln Pro Ser Arg Arg Arg Asp Glu Ser Gln Lys
           275                 280                 285
   Gly Glu Ser Arg Arg His Gly Asp Asn Gly Leu Glu Glu Thr Val Cys
       290                 295                 300
   Thr Ala Lys Leu Arg Val Asn Ile Gly Ser Ser Pro Ser Pro Asp Ile
   305                 310                 315                 320
   Tyr Asn Pro Gln Ala Gly Arg Ile Asn Thr Val Thr Ser Leu Asp Leu
                   325                 330                 335
   Pro Val Leu Arg Trp Leu Lys Leu Ser Ala Glu His Gly Ser Leu Arg
               340                 345                 350
   Lys Asn Ala Leu Ile Val Pro His Tyr Asn Arg Asn Ala Asn Ser Val
           355                 360                 365
   Ile Tyr Ala Leu Lys Gly Arg Ala Arg Leu Gln Val Val Asn Cys Asn
       370                 375                 380
   Gly Asn Thr Val Phe Asp Gly Glu Leu Glu Ala Gly Arg Ala Leu Thr
   385                 390                 395                 400
   Val Pro Gln Asn Tyr Ala Val Ala Ala Lys Ser Leu Ser Glu Arg Phe
                   405                 410                 415
   Thr Tyr Val Ala Phe Lys Thr Asn Asp Arg Asp Gly Ile Ala Arg Leu
   420 425 430
   Ala Gly Thr Ser Ser Val Ile Asn Asp Leu Pro Leu Asp Val Val Ala
   435 440 445
   Ala Thr Phe Asn Leu Gln Arg Asn Glu Ala Arg Gln Leu Lys Ser Asn
   450 455 460
   Asn Pro Phe Lys Leu Leu Val Pro Pro Arg Glu Ser Glu Lys Arg Ala
   465 470 475 480
   Ser Ala
<210> 517
   <211> 499
   <212> PRT
   <213> Cicer arietinum
<220>
   <221> misc_feature
   <222> (189)..(196)
   <223> Xaa can be any naturally occurring amino acid
<400> 517
   Met Ala Lys Leu Leu Ala Leu Ser Leu Ser Phe Cys Phe Leu Leu Phe
   1 5 10 15
   Gly Ser Cys Phe Ala Leu Arg Asp Gln Pro Glu Gln Asn Glu Cys Gln
   20 25 30
   Leu Glu His Leu Asn Ala Leu Glu Pro Asp Asn Arg Ile Lys Ser Glu
   35 40 45
   Gly Gly Leu Ile Glu Thr Trp Asn Pro Ser Asn Lys Gln Phe Arg Cys
   50 55 60
   Ala Gly Val Ala Leu Ser Arg Ala Thr Leu Gln Pro Asn Ser Leu Arg
   65 70 75 80
   Arg Pro Phe Tyr Thr Asn Ala Pro Gln Glu Ile Phe Ile Gln Gln Gly
   85 90 95
   Asn Gly Tyr Phe Gly Met Val Phe Pro Gly Cys Val Glu Thr Phe Glu
   100 105 110
   Glu Pro Arg Glu Ser Glu Gln Gly Glu Gly Ser Lys Phe Arg Asp Ser
   115 120 125
   His Gln Lys Val Asn Arg Phe Arg Glu Gly Asp Ile Ile Ala Val Pro
   130 135 140
   Thr Gly Val Val Phe Trp Met Phe Asn Asp Gln Asp Thr Pro Val Ile
   145 150 155 160
   Ala Val Ser Leu Ile Asp Thr Ser Ser Phe Gln Asn Gln Leu Asp Gln
   165 170 175
   Met Pro Arg Arg Phe Tyr Leu Ala Gly Asn His Glu Xaa Xaa Xaa Xaa
   180 185 190
   Xaa Xaa Xaa Xaa Gln Gln Glu Gly Ser Glu Glu Glu Glu Asn Glu Gly
   195 200 205
   Gly Asn Ile Phe Ser Gly Phe Lys Arg Asp Phe Leu Glu Asp Ala Leu
   210 215 220
   Asn Val Asn Arg Arg Ile Val Asn Lys Leu Gln Gly Arg Asn Glu Asp
   225 230 235 240
   Glu Glu Lys Gly Ala Ile Val Lys Val Lys Gly Gly Leu Ser Ile Ile
   245 250 255
   Thr Pro Pro Glu Lys Glu Pro Arg Gln Lys Arg Gly Ser Arg Gln Glu
   260 265 270
   Glu Asp Glu Asp Glu Asp Glu Lys Arg Gln Pro His Arg His Ser Arg
   275 280 285
   Gln Asp Glu Asp Glu Asp Glu Lys Arg Gln Pro Arg Arg His Ser Arg
   290 295 300
   Gly Gly Ser Lys Ser Gln Arg Asp Asn Gly Phe Glu Glu Thr Ile Cys
   305 310 315 320
   Thr Ala Arg Leu His Gln Asn Ile Gly Ser Ser Ser Ser Pro Asp Ile
   325 330 335
   Tyr Asn Pro Gln Ala Gly Arg Ile Lys Thr Val Thr Ser Phe Asp Leu
   340 345 350
   Pro Ala Leu Arg Phe Leu Lys Leu Ser Ala Glu Phe Gly Ser Leu His
   355 360 365
   Lys Asn Ala Met Phe Val Pro His Tyr Asn Leu Asn Ala Asn Ser Ile
       370                 375                 380
   Leu Tyr Ala Leu Lys Gly Arg Ala Arg Leu Gln Ile Val Asn Cys Lys
   385                 390                 395                 400
   Gly Asn Ser Val Phe Asp Gly Glu Leu Glu Ala Gly Arg Ala Leu Ile
                   405                 410                 415
   Val Pro Gln Asn Phe Ala Ile Ala Ala Lys Ser Leu Ser Asp Arg Phe
               420                 425                 430
   Ser Tyr Val Ala Phe Lys Thr Asn Asp Arg Ala Ala Ile Gly Arg Leu
           435                 440                 445
   Leu Gly Ala Ser Ser Leu Ile Asn Gly Met Pro Glu Glu Val Val Ala
       450                 455                 460
   Ala Ala Phe Asn Met Glu Arg Asn Glu Ala Arg Gln Leu Lys Phe Asn
   465                 470                 475                 480
   Ser Pro Phe Ser Phe Leu Val Pro Pro Arg Ser Asp Ser Asp Asn Lys
                   485                 490                 495
   Ala Ala Ala
<210> 518
   <211> 576
   <212> PRT
   <213> Lathyrus hirsutus
<400> 518
   Met Ala Ile Ile Ile Lys Ser Arg Phe Pro Leu Leu Leu Leu Leu Gly
   1               5                   10                  15
   Ile Ile Phe Leu Ala Ser Val Cys Ala Thr Trp Ala Asn Tyr Asp Glu
               20                  25                  30
   Gly Ser Glu Pro Arg Val Pro Gly Gln Arg Glu Arg Gly Arg Gln Glu
           35                  40                  45
   Gly Glu Lys Ala Glu Lys Ser His Glu Lys Trp Arg Pro Ser Tyr Glu
       50                  55                  60
   Glu Glu Tyr Asp Glu Gly Ser Glu Pro Arg Val Pro Gly Lys Arg Glu
   65                  70                  75                  80
   Arg Gly Arg Gln Glu Gly Glu Lys Glu Glu Lys Arg His Gly Glu Trp
                   85                  90                  95
   Arg Pro Ser His Glu Glu Glu Tyr Asp Glu Gly Ser Glu Pro Arg Val
               100                 105                 110
   Pro Thr His Gly Glu Arg Gly Arg Gln Glu Gly Glu Lys Glu Glu Lys
           115                 120                 125
   Arg His Glu Glu Trp Arg Pro Ser Tyr Glu Lys Glu Glu Asp Glu Glu
       130                 135                 140
   Glu Lys Glu Lys Tyr Lys Tyr Gln Arg Glu Lys Lys Glu Gln Lys Glu
   145                 150                 155                 160
   Val Gln Pro Gly Arg Glu Lys Trp Glu Arg Lys Gln Asp Glu Lys His
                   165                 170                 175
   Val Glu Glu Asp Glu Asp Gln Glu Glu Glu Gln Trp Arg Gly Ser Lys
               180                 185                 190
   Arg Arg Glu Asp Pro Glu Glu Arg Ala Arg Leu Arg Tyr Arg Glu Glu
           195                 200                 205
   Arg Thr Lys Ser Asn Val Glu Glu Glu Thr Glu Glu Arg Arg Asn Pro
       210                 215                 220
   Phe Leu Phe Lys Ser Asn Lys Phe Leu Thr Leu Phe Glu Asn Glu Asn
   225                 230                 235                 240
   Gly His Ile Arg Arg Leu Gln Arg Phe Asp Glu Arg Ser Asp Ile Phe
                   245                 250                 255
   Glu Asn Leu Gln Asn Tyr Arg Leu Val Glu Tyr Lys Ala Lys Pro His
               260                 265                 270
   Thr Met Phe Leu Pro Gln His Ile Asp Ala Asp Leu Ile Ile Val Val
           275                 280                 285
   Leu Asn Gly Lys Ala Ile Leu Thr Val Leu Ser Pro Asn Asp Arg Asn
       290                 295                 300
   Ser Tyr Asn Leu Glu Arg Gly Asp Thr Ile Lys Leu Pro Ala Gly Thr
   305                 310                 315                 320
   Thr Ser Tyr Leu Val Asn Gln Asp Asp Glu Glu Asp Leu Arg Val Val
                   325                 330                 335
   Asp Leu Ala Ile Pro Val Asn Arg Pro Gly Lys Phe Glu Ala Phe Gly
               340                 345                 350
   Leu Ser Ala Asn Lys Asn Gln Tyr Leu Arg Gly Phe Ser Lys Asn Ile
           355                 360                 365
   Leu Glu Ala Phe Leu Asn Thr Lys Tyr Glu Thr Ile Glu Lys Val Leu
       370                 375                 380
   Leu Glu Glu Gln Glu Arg Arg Asp Arg Lys Gly Arg Gln Gln Gly Gln
   385                 390                 395                 400
   Glu Thr Asn Ala Ile Val Lys Val Ser Arg Glu Gln Ile Glu Glu Leu
                   405                 410                 415
   Arg Lys Leu Ala Lys Ser Ser Ser Lys Lys Ser Leu Leu Ser Glu Ser
               420                 425                 430
   Glu Pro Ile Asn Leu Arg Ser Gln Asn Pro Lys Tyr Ser Asn Lys Phe
           435                 440                 445
   Gly Lys Leu Phe Glu Ile Thr Pro Glu Lys Lys Tyr Pro Gln Leu Gln
       450                 455                 460
   Asp Leu Asp Val Ser Ile Ser Cys Val Glu Ile Asn Glu Gly Ala Pro
   465                 470                 475                 480
   Leu Leu Pro His Tyr Asn Ser Arg Ala Ile Val Leu Leu Leu Val Asn
                   485                 490                 495
   Glu Gly Lys Gly Asn Leu Glu Leu Val Gly Phe Lys Asn Glu Gln Gln
               500                 505                 510
   Arg Gln Arg Glu Asn Glu Glu Arg Asn Lys Lys Val Gln Arg Tyr Glu
           515                 520                 525
   Ala Arg Leu Ser Pro Gly Asp Val Val Val Ile Pro Ala Gly His Pro
       530                 535                 540
   Val Ala Ile Ser Ala Ser Leu Asn Leu Asn Leu Val Gly Phe Gly Val
   545                 550                 555                 560
   Asn Ala Glu Asn Asn Gln Arg Asn Phe Leu Thr Gly Ser Asp Asp Asn
                   565                 570                 575
<210> 519
   <211> 564
   <212> PRT
   <213> Lathyrus cicera
<400> 519
   Met Ala Thr Ile Ile Lys Ser Arg Phe Pro Leu Leu Leu Leu Leu Gly
   1               5                   10                  15
   Ile Ile Phe Leu Ala Ser Val Cys Val Thr Leu Ala Asn Tyr Asp Glu
               20                  25                  30
   Gly Ser Glu Pro Arg Val Pro Ala Gln Arg Glu Arg Gly Arg Gln Glu
           35                  40                  45
   Gly Glu Lys Glu Glu Lys Arg His Gly Glu Trp Arg Pro Ser His Glu
       50                  55                  60
   Lys Glu Tyr Asp Glu Gly Ser Glu Pro Arg Val Pro Gly Arg Arg Glu
   65                  70                  75                  80
   Arg Gly Arg Gln Glu Gly Glu Lys Glu Glu Lys Arg His Gly Glu Trp
                   85                  90                  95
   Arg Pro Ser Tyr Glu Lys Glu Tyr Asp Glu Gly Ser Glu Pro Arg Val
               100                 105                 110
   Pro Gly Arg Arg Glu Arg Gly Arg Gln Glu Gly Glu Lys Glu Glu Lys
           115                 120                 125
   Arg His Gly Glu Trp Arg Pro Ser Tyr Glu Lys Glu Tyr Asp Glu Glu
       130                 135                 140
   Glu Lys Gln Lys Tyr Gln Tyr Glu Arg Glu Lys Glu Glu Gln Lys Glu
   145                 150                 155                 160
   Val Gln Pro Gly Arg Glu Arg Trp Glu Arg Lys Glu Asp Glu Glu Lys
                   165                 170                 175
   Glu Glu Asp Gln Trp Arg Gly Ser Gln Arg His Glu Asp Pro Glu Glu
               180                 185                 190
   Arg Ala Arg Leu Arg Tyr Arg Lys Glu Arg Thr Lys Lys Tyr Val Glu
           195                 200                 205
   Glu Asp Thr Glu Glu Thr Ser Ser Glu Ser Gln Gly Arg Arg Asn Pro
       210                 215                 220
   Phe Leu Phe Lys Ser Asn Lys Phe Leu Thr Leu Phe Glu Asn Glu Asn
   225                 230                 235                 240
   Gly Tyr Ile Arg Arg Leu Gln Arg Phe Asp Glu Arg Ser Asp Ile Phe
                   245                 250                 255
   Glu Asn Leu Gln Asn Tyr Arg Leu Val Glu Tyr Arg Ala Lys Pro His
               260                 265                 270
   Thr Ile Phe Leu Pro Gln His Ile Asp Ala Asp Leu Ile Leu Val Ile
           275                 280                 285
   Leu Asn Gly Lys Ala Ile Leu Thr Val Leu Ser Pro Asn Asp Arg Asn
       290                 295                 300
   Ser Tyr Asn Leu Glu Arg Gly Asp Thr Ile Lys Leu Pro Ala Gly Thr
   305                 310                 315                 320
   Thr Ser Tyr Leu Val Asn Glu Asp Asp Glu Glu Asp Leu Arg Val Val
                   325                 330                 335
   Asp Leu Val Ile Pro Val Asn Arg Pro Gly Lys Phe Glu Ala Phe Asp
               340                 345                 350
   Leu Asn Gln Tyr Leu Gly Gly Phe Ser Lys Ser Val Leu Glu Ala Ser
           355                 360                 365
   Leu Asn Thr Lys Tyr Glu Thr Ile Glu Lys Val Leu Leu Glu Glu Gln
       370                 375                 380
   Gln Lys Gln Gly Gln Glu Thr Asn Ala Ile Val Lys Val Ser Arg Glu
   385                 390                 395                 400
   Gln Ile Glu Glu Leu Arg Lys Leu Ala Lys Ser Ser Ser Lys Lys Ser
                   405                 410                 415
   Leu Leu Ser Glu Leu Glu Pro Val Asn Leu Arg Ser His Ser Pro Lys
               420                 425                 430
   Tyr Ser Asn Lys Phe Gly Lys Phe Phe Glu Ile Thr Pro Glu Lys Lys
           435                 440                 445
   Tyr Pro Gln Leu Gln Asp Leu Asp Val Ser Ile Ser Cys Val Glu Ile
       450                 455                 460
   Asn Glu Gly Ala Leu Leu Leu Pro His Tyr Asn Ser Arg Ala Ile Val
   465                 470                 475                 480
   Val Val Leu Val Asn Glu Gly Lys Gly Asn Leu Glu Leu Leu Gly Val
   485 490 495
   Gln Asn Glu Asp Glu Gln Gln Glu Arg Lys Glu Arg Asn Lys Glu Val
   500 505 510
   Gln Arg Tyr Glu Ala Arg Leu Ser Pro Gly Asp Val Val Ile Ile Pro
   515 520 525
   Ser Gly His Pro Val Ala Val Ser Ala Ser Ser Asn Leu Asn Leu Leu
   530 535 540
   Gly Phe Gly Ile Asn Ala Glu Asn Asn Gln Arg Asn Phe Leu Ser Gly
   545 550 555 560
   Ser Asp Asp Asn
<210> 520
   <211> 527
   <212> PRT
   <213> Lathyrus sativus
<400> 520
   Met Ala Thr Ile Ile Lys Ser Arg Phe Pro Leu Leu Leu Leu Leu Gly
   1 5 10 15
   Ile Ile Phe Leu Ala Ser Val Cys Val Thr Tyr Ala Asn Tyr Asp Glu
   20 25 30
   Gly Ser Glu Pro Arg Val Pro Ala Gln Arg Glu Arg Gly Arg Gln Glu
   35 40 45
   Gly Glu Lys Glu Glu Lys Arg His Gly Glu Trp Arg Pro Ser Ser Glu
   50 55 60
   Lys Glu Tyr Asp Glu Gly Ser Glu Pro Arg Val Pro Gly Arg Arg Glu
   65 70 75 80
   Arg Gly Arg Gln Glu Gly Glu Lys Glu Glu Lys Arg His Gly Glu Trp
   85 90 95
   Arg Pro Ser Tyr Glu Lys Glu Tyr Asp Glu Glu Glu Lys Gln Lys Tyr
   100 105 110
   Gln Tyr Glu Arg Glu Lys Lys Glu Gln Lys Glu Val Glu Pro Gly Arg
   115 120 125
   Glu Arg Trp Glu Arg Lys Glu Asp Glu Glu Lys Glu Glu Asp Gln Trp
       130                 135                 140
   Arg Gly Ser Gln Arg His Glu Asp Pro Glu Glu Arg Ala Arg Leu Arg
   145                 150                 155                 160
   Tyr Arg Lys Glu Arg Thr Lys Lys Tyr Val Glu Glu Asp Thr Glu Glu
                   165                 170                 175
   Thr Ser Ser Glu Ser Gln Gly Arg Arg Asn Pro Phe Leu Phe Lys Ser
               180                 185                 190
   Asn Lys Phe Leu Thr Leu Phe Glu Asn Glu Asn Gly Tyr Ile Arg Arg
           195                 200                 205
   Leu Gln Arg Phe Asp Glu Arg Ser Asp Leu Phe Glu Asn Leu Gln Asn
       210                 215                 220
   Tyr Arg Leu Val Glu Tyr Arg Ala Lys Pro His Thr Ile Phe Leu Pro
   225                 230                 235                 240
   Gln His Ile Asp Ala Asp Leu Ile Leu Val Ile Leu Asn Gly Lys Ala
                   245                 250                 255
   Ile Leu Thr Val Leu Ser Pro Asn Asp Arg Asn Ser Tyr Asn Leu Glu
               260                 265                 270
   Arg Gly Asp Thr Ile Lys Leu Pro Ala Gly Thr Thr Ser Tyr Leu Val
           275                 280                 285
   Asn Glu Asp Asp Glu Glu Asp Leu Arg Val Val Asp Leu Val Ile Pro
       290                 295                 300
   Val Asn Arg Pro Gly Lys Phe Glu Ala Phe Asp Leu Asn Gln Tyr Leu
   305                 310                 315                 320
   Gly Gly Phe Ser Lys Ser Val Leu Lys Ala Ser Leu Asn Thr Lys Tyr
                   325                 330                 335
   Glu Thr Ile Glu Lys Val Leu Leu Glu Glu Gln Gln Lys Gln Gly Gln
               340                 345                 350
   Glu Thr Asn Ala Ile Val Lys Val Ser Arg Glu Gln Ile Glu Glu Leu
           355                 360                 365
   Arg Lys Leu Ala Lys Ser Ser Ser Lys Lys Ser Leu Leu Ser Glu Leu
       370                 375                 380
   Glu Pro Val Asn Leu Arg Ser His Ser Pro Lys Tyr Ser Asn Lys Phe
   385 390 395 400
   Gly Lys Phe Phe Glu Ile Thr Pro Glu Lys Lys Tyr Pro Gln Leu Gln
   405 410 415
   Asp Leu Asp Val Ser Ile Ser Cys Val Glu Ile Asn Glu Gly Ala Leu
   420 425 430
   Leu Leu Pro His Tyr Asn Ser Arg Ala Ile Val Val Leu Leu Val Asn
   435 440 445
   Glu Gly Lys Gly Asn Leu Glu Leu Leu Gly Val Gln Asp Glu Asp Glu
   450 455 460
   Gln Gln Glu Arg Lys Lys Arg Asn Lys Glu Val Gln Arg Tyr Glu Ala
   465 470 475 480
   Arg Leu Ser Pro Ser Asp Val Val Ile Ile Pro Ala Gly His Pro Val
   485 490 495
   Ala Val Ser Ala Ser Ser Asn Leu Asn Leu Leu Gly Phe Gly Ile Asn
   500 505 510
   Ala Glu Asn Asn Glu Arg Asn Phe Leu Ser Gly Ser Asp Asp Asn
   515 520 525
<210> 521
   <211> 463
   <212> PRT
   <213> Medicago truncatula
<400> 521
   Met Ala Ile Lys Ala Pro Phe Gln Leu Leu Met Leu Leu Gly Ile Phe
   1 5 10 15
   Phe Leu Ala Ser Val Cys Val Ser Ser Arg Asp Asp Arg His Asp Gln
   20 25 30
   Glu Asn Pro Phe Phe Phe Asn Ala Asn His Phe Gln Thr Leu Phe Glu
   35 40 45
   Asn Glu Asn Gly His Ile Arg Leu Leu Gln Arg Phe Asp Lys Arg Ser
   50 55 60
   Lys Ile Phe Glu Asn Leu Gln Asn Tyr Arg Leu Leu Glu Tyr His Ser
   65 70 75 80
   Lys Pro His Thr Leu Phe Leu Pro Gln His Asn Asp Ala Asp Phe Ile
                   85                  90                  95
   Leu Ala Val Leu Ser Gly Lys Ala Ile Leu Thr Val Leu Asn Pro Asp
               100                 105                 110
   Asn Arg Asn Ser Phe Asn Leu Glu Arg Gly Asp Thr Ile Lys Leu Pro
           115                 120                 125
   Ala Gly Ser Ile Ala Tyr Leu Ala Asn Arg Asp Asp Asn Glu Asp Leu
       130                 135                 140
   Arg Val Leu Asp Leu Ala Ile Pro Val Asn Arg Pro Gly Lys Phe Gln
   145                 150                 155                 160
   Ser Phe Ser Leu Ser Gly Ser Gln Asn Gln Gln Ser Phe Phe Ser Gly
                   165                 170                 175
   Phe Ser Lys Asn Ile Leu Glu Ala Ala Phe Asn Ala Asn Tyr Glu Glu
               180                 185                 190
   Ile Glu Arg Val Leu Ile Glu Glu His Glu Gln Glu Pro Gln His Arg
           195                 200                 205
   Arg Gly Leu Arg Lys Asp Arg Arg Gln Gln Ser Gln Asp Ser Asn Val
       210                 215                 220
   Ile Val Lys Val Ser Arg Glu Gln Ile Glu Glu Leu Ser Arg His Ala
   225                 230                 235                 240
   Lys Ser Ser Ser Arg Arg Ser Gly Ser Ser Glu Ser Ala Pro Phe Asn
                   245                 250                 255
   Leu Arg Ser Arg Glu Pro Ile Tyr Ser Asn Glu Phe Gly Asn Phe Phe
               260                 265                 270
   Glu Ile Thr Pro Glu Lys Asn Pro Gln Leu Lys Asp Leu Asp Ile Leu
           275                 280                 285
   Val Asn Tyr Ala Glu Ile Arg Glu Gly Ser Leu Leu Leu Pro His Phe
       290                 295                 300
   Asn Ser Arg Ala Thr Val Ile Val Val Val Asp Glu Gly Lys Gly Glu
   305                 310                 315                 320
   Phe Glu Leu Val Gly Gln Arg Asn Glu Asn Gln Gln Glu Gln Arg Glu
                   325                 330                 335
   Glu Asp Glu Gln Gln Glu Glu Glu Arg Ser Gln Gln Val Gln Arg Tyr
   340 345 350
   Arg Ala Arg Leu Ser Pro Gly Asp Val Tyr Val Ile Pro Ala Gly His
   355 360 365
   Pro Thr Val Val Ser Ala Ser Ser Asp Leu Ser Leu Leu Gly Phe Gly
   370 375 380
   Ile Asn Ala Glu Asn Asn Glu Arg Asn Phe Leu Ala Gly Glu Glu Asp
   385 390 395 400
   Asn Val Ile Ser Gln Ile Glu Arg Pro Val Lys Glu Val Ala Phe Pro
   405 410 415
   Gly Ser Ala Gln Asp Val Glu Ser Leu Leu Lys Asn Gln Arg Gln Ser
   420 425 430
   Tyr Phe Ala Asn Ala Gln Pro Gln Gln Arg Glu Arg Glu Glu Gly Arg
   435 440 445
   Ser Gln Arg Gln Arg Glu Leu Ile Ser Ser Ile Leu Gly Val Phe
   450 455 460
<210> 522
   <211> 499
   <212> PRT
   <213> Vicia pannonica
<400> 522
   Met Ala Thr Thr Phe Lys Ser Arg Phe Ser Leu Leu Leu Leu Leu Gly
   1 5 10 15
   Ile Ile Phe Leu Ala Phe Val Cys Val Thr Cys Ala Asn Tyr Asp Glu
   20 25 30
   Gly Ser Glu Pro Arg Val Pro Gly Gln Arg Glu Arg Gly Arg Gln Glu
   35 40 45
   Gly Glu Lys Glu Glu Gln Ser Arg Glu Arg His Pro Gln Arg Glu Pro
   50 55 60
   Ser Arg Glu Lys Glu Glu Asp Glu Glu Glu Lys Gln Lys Tyr Asp Glu
   65 70 75 80
   Gly Thr Glu Pro Arg Val Pro Gly Gln Arg Glu Arg Gly Arg Gln Glu
   85 90 95
   Gly Glu Lys Glu Glu Gln Arg Arg Glu Arg His Pro Gly Gln Arg Glu
               100                 105                 110
   Pro Ser Gln Glu Glu Asp Glu Glu Arg Glu Glu Ser Asp Arg Arg Gln
           115                 120                 125
   Glu Gly Ser Ser Lys Ser Glu Glu Gln Arg Asn Pro Phe Leu Phe Lys
       130                 135                 140
   Ser Asn Lys Phe Leu Thr Leu Phe Gln Asn Gly Asn Gly His Ile Arg
   145                 150                 155                 160
   Leu Leu Gln Arg Phe Asp Lys Arg Ser Asp Leu Phe Glu Asn Leu Gln
                   165                 170                 175
   Asn Tyr Arg Leu Leu Glu Tyr Arg Ala Lys Pro His Thr Ile Phe Leu
               180                 185                 190
   Pro Gln His Ile Asp Ala Asp Leu Ile Leu Val Val Leu Ser Gly Arg
           195                 200                 205
   Ala Ile Leu Thr Val Leu Ser Pro Asp Asp Arg Asn Ser Tyr Asn Leu
       210                 215                 220
   Glu Arg Gly Asp Thr Ile Lys Leu Pro Ala Gly Thr Thr Ser Tyr Pro
   225                 230                 235                 240
   Leu Asn Gln Asp Asp Glu Glu Asp Leu Arg Val Val Asp Leu Ala Ile
                   245                 250                 255
   Ser Val Asn Arg Pro Gly Lys Val Glu Ser Phe Asn Leu Ser Gly Asn
               260                 265                 270
   Lys Asn Gln Tyr Leu Arg Gly Phe Ser Glu Asn Ile Leu Glu Ala Ser
           275                 280                 285
   Phe Asn Thr Lys Tyr Glu Thr Ile Glu Lys Val Leu Leu Glu Glu Gln
       290                 295                 300
   Asp Lys Glu Ser Gln Gln Pro Arg Gly Gln Arg Leu Gln Arg Gln Glu
   305                 310                 315                 320
   Thr Asn Ala Leu Val Lys Val Ser Arg Glu Gln Val Glu Glu Leu Lys
                   325                 330                 335
   Arg Leu Ala Arg Thr Ser Ser Lys Lys Gly Val Ser Ser Glu Phe Glu
   340 345 350
   Pro Phe Asn Leu Arg Ser His Gly Pro Lys Tyr Ser Asn Lys Phe Gly
   355 360 365
   Lys Phe Phe Glu Ile Thr Pro Glu Lys Lys Tyr Pro Gln Leu Gln Asp
   370 375 380
   Leu Asp Ile Ser Val Ser Ser Val Glu Ile Asn Glu Gly Ala Leu Phe
   385 390 395 400
   Leu Pro His Tyr Asn Ser Arg Ala Ile Val Val Val Leu Val Asp Glu
   405 410 415
   Gly Lys Gly Asn Leu Glu Leu Val Gly Phe Lys Asn Glu Gln Gln Glu
   420 425 430
   Gln Arg Glu Lys Glu Asp Glu Gln Glu Glu Arg Asn Lys Gln Val Gln
   435 440 445
   Arg Tyr Glu Ala Lys Leu Ser Pro Gly Asp Val Val Ile Ile Pro Ala
   450 455 460
   Gly His Pro Val Ala Val Ser Ala Ser Ser Asn Leu Asn Leu Leu Gly
   465 470 475 480
   Phe Gly Ile Asn Ala Glu Asn Asn Gln Arg Asn Phe Leu Thr Gly Ser
   485 490 495
   Asp Asp Asn
<210> 523
   <211> 515
   <212> PRT
   <213> Vicia ludoviciana
<400> 523
   Met Ala Thr Thr Ile Lys Leu Arg Phe Pro Leu Leu Leu Leu Leu Gly
   1               5                   10                  15
   Val Ile Leu Leu Ala Ser Val Cys Val Thr Cys Ala Asn Tyr Asp Glu
               20                  25                  30
   Gly Ser Glu Pro Arg Val Pro Gly Arg Pro Glu Gly Glu Lys Glu Glu
           35                  40                  45
   Lys His Arg Gly Lys Leu Arg Pro Ser Tyr Glu Lys Glu Glu Asp Glu
   50 55 60
   Gly Glu Lys Gln Arg Tyr His Tyr Glu Lys Lys Glu Gln Lys Glu Ala
   65 70 75 80
   Gln Pro Arg Arg Glu Lys Lys Glu Gln Lys Glu Glu Glu Lys Gln Val
   85 90 95
   Glu Glu Glu Ser Arg Glu Ser Gln Arg Tyr Glu Asp Pro Gly Glu Arg
   100 105 110
   Ala Arg Glu Arg Tyr Arg Ala Glu Ile Ile Lys Arg Gln Val Glu Lys
   115 120 125
   Glu Arg Glu Glu Arg Asp Arg Arg His Gln Arg Glu Gly Glu Glu Glu
   130 135 140
   Glu Gly Ser Ser Lys Ser Arg Asn Pro Phe Leu Phe Lys Ser Asn Asn
   145 150 155 160
   Phe Leu Thr Leu Phe Glu Asn Glu Asn Gly His Ile Arg Leu Leu Gln
   165 170 175
   Arg Phe Asp Lys Arg Ser Asp Leu Phe Glu Asn Leu Gln Asn Tyr Arg
   180 185 190
   Leu Val Glu Tyr Arg Ala Lys Pro His Thr Ile Phe Leu Pro Gln His
   195 200 205
   Ile Asp Ala Asp Leu Ile Leu Val Val Leu Ser Gly Lys Ala Ile Leu
   210 215 220
   Thr Val Leu Ser Pro Asn Asn Arg Asn Ser Tyr Asn Leu Lys Arg Gly
   225 230 235 240
   Asp Thr Ile Lys Leu Pro Ala Gly Thr Thr Ser Tyr Leu Leu Asn Ser
   245 250 255
   Asp Asp Glu Glu Asp Leu Arg Met Val Asp Leu Ala Ile Ser Val Asn
   260 265 270
   Arg Pro Gly Lys Val Glu Ser Phe Asn Leu Ser Gly Asn Lys Asn Gln
   275 280 285
   Tyr Leu Arg Gly Phe Ser Lys Asn Ile Leu Glu Ala Ser Phe Asn Thr
   290 295 300
   Lys Tyr Glu Thr Ile Glu Lys Val Leu Leu Glu Glu Gln Asp Lys Glu
   305 310 315 320
   Ser Gln Gln Ser Ile Gly Gln Lys Arg Ile Ser Gln Arg Gln Glu Thr
   325 330 335
   Asn Ala Leu Val Lys Val Ser Arg Glu Gln Ile Glu Glu Pro Lys Arg
   340 345 350
   Leu Ala Arg Ser Ser Ser Arg Lys Gly Val Ser Ser Glu Phe Glu Pro
   355 360 365
   Ile Asn Leu Arg Ser Gln Arg Pro Lys Tyr Ser Asn Lys Phe Gly Lys
   370 375 380
   Phe Tyr Glu Ile Ser Pro Glu Lys Lys Tyr Pro Gln Leu Gln Asp Leu
   385 390 395 400
   Asp Val Ser Val Ser Ser Val Glu Ile Asn Glu Gly Ala Leu Leu Leu
   405 410 415
   Pro His Tyr Asn Ser Arg Ala Ile Val Thr Val Leu Val Asn Glu Gly
   420 425 430
   Lys Gly Asn Leu Glu Leu Ile Gly Phe Gln Asn Glu Gln Gln Gly Gln
   435 440 445
   Arg Glu Lys Glu Asp Glu Gln Gln His Glu Arg Asn Lys Gln Val Gln
   450 455 460
   Arg Tyr Asp Ala Arg Leu Ser Ser Gly Asp Val Val Ile Ile Pro Ala
   465 470 475 480
   Gly His Pro Val Ala Val Ser Ala Ser Ser Asn Leu Asp Leu Leu Gly
   485 490 495
   Phe Gly Ile Asn Ala Glu Asn Ser Gln Arg Asn Phe Leu Thr Gly Ser
   500 505 510
   Asp Asp Asn
   515
<210> 524
   <211> 454
   <212> PRT
   <213> Oryza sativa
<400> 524
   Cys Arg Phe Asp Arg Leu Gln Ala Phe Glu Pro Ile Arg Ser Val Arg
   1               5                   10                  15
   Ser Gln Ala Gly Thr Thr Glu Phe Phe Asp Val Ser Asn Glu Gln Phe
               20                  25                  30
   Gln Cys Thr Gly Val Ser Ala Val Arg Arg Val Ile Glu Pro Arg Gly
           35                  40                  45
   Leu Leu Leu Pro His Tyr Thr Asn Gly Ala Ser Leu Val Tyr Ile Ile
       50                  55                  60
   Gln Gly Arg Gly Ile Thr Gly Pro Thr Phe Pro Gly Cys Pro Glu Ser
   65                  70                  75                  80
   Tyr Gln Gln Gln Phe Gln Gln Ser Gly Gln Ala Gln Leu Thr Glu Ser
                   85                  90                  95
   Gln Ser Gln Ser His Lys Phe Lys Asp Glu His Gln Lys Ile His Arg
               100                 105                 110
   Phe Arg Gln Gly Asp Val Ile Ala Leu Pro Ala Gly Val Ala His Trp
           115                 120                 125
   Cys Tyr Asn Asp Gly Glu Val Pro Val Val Ala Ile Tyr Val Thr Asp
       130                 135                 140
   Leu Asn Asn Gly Ala Asn Gln Leu Asp Pro Arg Gln Arg Asp Phe Leu
   145                 150                 155                 160
   Leu Ala Gly Asn Lys Arg Asn Pro Gln Ala Tyr Arg Arg Glu Val Glu
                   165                 170                 175
   Glu Arg Ser Gln Asn Ile Phe Ser Gly Phe Ser Thr Glu Leu Leu Ser
               180                 185                 190
   Glu Ala Leu Gly Val Ser Ser Gln Val Ala Arg Gln Leu Gln Cys Gln
           195                 200                 205
   Asn Asp Gln Arg Gly Glu Ile Val Arg Val Glu His Gly Leu Ser Leu
       210                 215                 220
   Leu Gln Pro Tyr Ala Ser Leu Gln Glu Gln Glu Gln Gly Gln Val Gln
   225                 230                 235                 240
   Ser Arg Glu Arg Tyr Gln Glu Gly Gln Tyr Gln Gln Ser Gln Tyr Gly
                   245                 250                 255
   Ser Gly Cys Ser Asn Gly Leu Asp Glu Thr Phe Cys Thr Met Lys Val
   260 265 270
   Arg Gln Asn Ile Asp Asn Pro Asn Arg Ala Asp Thr Tyr Asn Pro Arg
   275 280 285
   Ala Gly Arg Val Thr Asn Leu Asn Thr Gln Asn Phe Pro Ile Leu Asn
   290 295 300
   Leu Val Gln Met Ser Ala Val Lys Val Asn Leu Tyr Gln Asn Ala Leu
   305 310 315 320
   Leu Ser Pro Phe Trp Asn Ile Asn Ala His Ser Val Val Tyr Ile Thr
   325 330 335
   Gln Gly Arg Ala Arg Val Gln Val Val Asn Asn Asn Gly Lys Thr Val
   340 345 350
   Phe Asn Gly Glu Leu Arg Arg Gly Gln Leu Leu Ile Ile Pro Gln His
   355 360 365
   Tyr Ala Val Val Lys Lys Ala Gln Arg Glu Gly Cys Ala Tyr Ile Ala
   370 375 380
   Phe Lys Thr Asn Pro Asn Ser Met Val Ser His Ile Ala Gly Lys Ser
   385 390 395 400
   Ser Ile Phe Arg Ala Leu Pro Asn Asp Val Leu Ala Asn Ala Tyr Arg
   405 410 415
   Ile Ser Arg Glu Glu Ala Gln Arg Leu Lys His Asn Arg Gly Asp Glu
   420 425 430
   Phe Gly Ala Phe Thr Pro Ile Gln Tyr Lys Ser Tyr Gln Asp Val Tyr
   435 440 445
   Asn Ala Ala Glu Ser Ser
   450
<210> 525
   <211> 500
   <212> PRT
   <213> Zizania latifolia
<400> 525
   Met Asn Met Ala Thr Ile Asn Gly Pro Thr Ile Phe Phe Thr Val Cys
   1 5 10 15
   Leu Phe Leu Leu Cys His Gly Ser Leu Ala Gln Leu Leu Gly Gln Ser
               20                  25                  30
   Thr Ser Gln Trp Gln Ser Ser His Arg Gly Ser Ser Arg Gln Cys Arg
           35                  40                  45
   Phe Asp Arg Leu Gln Ala Phe Glu Pro Val Arg Ser Val Arg Ser Gln
       50                  55                  60
   Ala Gly Thr Thr Glu Phe Phe Asp Ala Ser Asn Glu Leu Phe Gln Cys
   65                  70                  75                  80
   Ala Gly Val Ser Ile Val Arg Arg Ile Ile Glu Pro Arg Gly Leu Leu
                   85                  90                  95
   Leu Pro Gln Tyr Thr Asn Gly Ala Thr Ile Met Tyr Ile Ile Gln Gly
               100                 105                 110
   Arg Gly Ile Thr Gly Gln Thr Phe Pro Gly Cys Pro Glu Ser Tyr Gln
           115                 120                 125
   Gln Gln Phe Gln Gln Ser Met Gln Ala Gln Leu Thr Gly Ser Gln Ser
       130                 135                 140
   Gln Ser Gln Lys Phe Lys Asp Glu His Gln Lys Ile Asn Arg Phe Arg
   145                 150                 155                 160
   Gln Gly Asp Val Ile Ala Leu Pro Ala Gly Val Ala His Trp Cys Tyr
                   165                 170                 175
   Asn Asp Gly Glu Val Pro Val Val Ala Ile Tyr Val Ile Asp Ile Asn
               180                 185                 190
   Asn Ala Ala Asn Gln Leu Asp Pro Arg Gln Arg Asp Phe Leu Leu Ala
           195                 200                 205
   Gly Asn Met Arg Ser Pro Gln Ala Tyr Arg Arg Glu Val Glu Asn Gln
       210                 215                 220
   Ser Gln Asn Ile Phe Ser Gly Phe Ser Ala Glu Leu Leu Ser Glu Ala
   225                 230                 235                 240
   Leu Gly Ile Ser Thr Gly Val Ala Arg Gln Leu Gln Cys Gln Asn Asp
                   245                 250                 255
   Gln Arg Gly Glu Ile Val Arg Val Glu His Gly Leu Ser Leu Leu Gln
               260                 265                 270
   Pro Tyr Ala Ser Leu Gln Glu Gln Glu Gln Lys Gln Glu Gln Pro Arg
   275 280 285
   Glu Arg Tyr Gln Val Thr Gln His Gln Gln Ser Gln Tyr Gly Gly Gly
   290 295 300
   Cys Ser Asn Gly Leu Asp Glu Thr Phe Cys Ala Met Arg Ile Trp Gln
   305 310 315 320
   Asn Ile Asp Asn Pro Asn Leu Ala Asp Thr Tyr Asn Pro Arg Ala Gly
   325 330 335
   Arg Val Thr Asn Leu Asn Ser Gln Lys Phe Pro Ile Leu Asn Leu Ile
   340 345 350
   Gln Met Ser Ala Val Lys Val Asn Leu Tyr Gln Asn Ala Leu Leu Ser
   355 360 365
   Pro Phe Trp Asn Ile Asn Ser His Ser Val Val Tyr Val Thr Gln Gly
   370 375 380
   Cys Ala Arg Val Gln Val Val Asn Asn Asn Gly Lys Thr Val Phe Asn
   385 390 395 400
   Gly Glu Leu Arg Arg Gly Gln Leu Leu Ile Ile Pro Gln His Tyr Val
   405 410 415
   Val Val Lys Lys Ala Gln Arg Glu Gly Cys Ala Tyr Ile Ala Phe Lys
   420 425 430
   Thr Asn Pro Asn Ser Met Val Ser His Ile Val Gly Lys Ser Ser Ile
   435 440 445
   Phe Arg Ala Leu Pro Thr Asp Val Leu Ala Asn Ala Tyr Arg Ile Ser
   450 455 460
   Arg Glu Asp Ala Gln Arg Leu Lys His Asn Arg Gly Asp Glu Leu Gly
   465 470 475 480
   Ala Phe Thr Pro Leu Gln Tyr Lys Ser Tyr Gln Asp Val Ser Ser Val
   485 490 495
   Ala Ala Ser Ser
   500
<210> 526
   <211> 527
   <212> PRT
   <213> Avena sativa
<400> 526
   Met Ala Thr Thr Ser Phe Pro Ser Met Leu Phe Tyr Phe Cys Ile Phe
   1               5                   10                  15
   Leu Leu Phe His Gly Ser Met Ala Gln Leu Phe Gly Gln Ser Ser Thr
               20                  25                  30
   Pro Trp Gln Ser Ser Arg Gln Gly Gly Leu Arg Gly Cys Arg Phe Asp
           35                  40                  45
   Arg Leu Gln Ala Phe Glu Pro Leu Arg Gln Val Arg Ser Gln Ala Gly
       50                  55                  60
   Ile Thr Glu Tyr Phe Asp Glu Gln Asn Glu Gln Phe Arg Cys Thr Gly
   65                  70                  75                  80
   Val Ser Val Ile Arg Arg Val Ile Glu Pro Gln Gly Leu Val Leu Pro
                   85                  90                  95
   Gln Tyr His Asn Ala Pro Ala Leu Val Tyr Ile Leu Gln Gly Arg Gly
               100                 105                 110
   Phe Thr Gly Leu Thr Phe Pro Gly Cys Pro Ala Thr Phe Gln Gln Gln
           115                 120                 125
   Phe Gln Pro Phe Asp Gln Ser Gln Phe Ala Gln Gly Gln Arg Gln Ser
       130                 135                 140
   Gln Thr Ile Lys Asp Glu His Gln Arg Val Gln Arg Phe Lys Gln Gly
   145                 150                 155                 160
   Asp Val Val Ala Leu Pro Ala Gly Ile Val His Trp Cys Tyr Asn Asp
                   165                 170                 175
   Gly Asp Ala Pro Ile Val Ala Ile Tyr Val Phe Asp Val Asn Asn Asn
               180                 185                 190
   Ala Asn Gln Leu Glu Pro Arg Gln Lys Glu Phe Leu Leu Ala Gly Asn
           195                 200                 205
   Asn Lys Arg Glu Gln Gln Ser Gly Asn Asn Ile Phe Ser Gly Leu Ser
       210                 215                 220
   Val Gln Leu Leu Ser Glu Ala Leu Gly Ile Ser Gln Gln Ala Ala Gln
   225 230 235 240
   Arg Ile Gln Ser Gln Asn Asp Gln Arg Gly Glu Ile Ile Arg Val Ser
   245 250 255
   Gln Gly Leu Gln Phe Leu Lys Pro Ile Val Ser Gln Gln Val Pro Gly
   260 265 270
   Glu Gln Gln Val Tyr Gln Pro Ile Gln Thr Gln Glu Gly Gln Ala Thr
   275 280 285
   Gln Tyr Gln Val Gly Gln Ser Thr Gln Tyr Gln Val Gly Lys Ser Thr
   290 295 300
   Pro Tyr Gln Gly Gly Gln Ser Ser Gln Tyr Gln Ala Gly Gln Ser Trp
   305 310 315 320
   Asp Gln Ser Phe Asn Gly Leu Glu Glu Asn Phe Cys Ser Leu Glu Ala
   325 330 335
   Arg Lys Asn Ile Glu Asn Pro Gln His Ala Asp Thr Tyr Asn Pro Arg
   340 345 350
   Ala Gly Arg Ile Thr Arg Leu Asn Ser Lys Asn Phe Pro Ile Leu Asn
   355 360 365
   Ile Val Gln Met Ser Ala Thr Arg Val Asn Leu Tyr Gln Asn Ala Ile
   370 375 380
   Leu Ser Pro Phe Trp Asn Ile Asn Ala His Ser Val Ile Tyr Met Ile
   385 390 395 400
   Gln Gly His Ala Arg Val Gln Val Val Asn Asn Asn Gly Gln Thr Val
   405 410 415
   Phe Asn Asp Ile Leu Arg Arg Gly Gln Leu Leu Ile Val Pro Gln His
   420 425 430
   Phe Val Val Leu Lys Lys Ala Glu Arg Glu Gly Cys Gln Tyr Ile Ser
   435 440 445
   Phe Lys Thr Asn Pro Asn Ser Met Val Ser His Ile Ala Gly Lys Ser
   450 455 460
   Ser Ile Leu Arg Ala Leu Pro Ile Asp Val Leu Ala Asn Ala Tyr Arg
   465 470 475 480
   Ile Ser Arg Gln Glu Ala Arg Asn Leu Lys Asn Asn Arg Gly Glu Glu
   485 490 495
   Phe Gly Ala Phe Thr Pro Lys Leu Thr Gln Lys Gly Phe Gln Ser Tyr
   500 505 510
   Gln Asp Ile Glu Glu Gly Ser Ser Ser Pro Val Arg Ala Ser Glu
   515 520 525
<210> 527
   <211> 495
   <212> PRT
   <213> Oryza sativa
<400> 527
   Met Thr Ile Ser Val Phe Ser Arg Phe Ser Ile Tyr Phe Cys Val Leu
   1 5 10 15
   Leu Leu Cys Asn Gly Ser Met Ala Gln Leu Phe Asp Pro Ala Thr Asn
   20 25 30
   Gln Trp Gln Thr His Arg Gln Gly Ser Phe Arg Glu Cys Arg Phe Glu
   35 40 45
   Arg Leu Gln Ala Phe Glu Pro Leu Gln Asn Val Arg Ser Glu Ala Gly
   50 55 60
   Val Thr Glu Tyr Phe Asp Glu Thr Asn Glu Leu Phe Gln Cys Thr Gly
   65 70 75 80
   Thr Phe Val Ile Arg Arg Val Ile Gln Pro Gln Gly Leu Leu Ile Pro
   85 90 95
   Arg Tyr Ala Asn Thr Pro Gly Met Val Tyr Ile Ile Gln Gly Arg Gly
   100 105 110
   Ser Met Gly Leu Thr Phe Pro Gly Cys Pro Ala Thr Tyr Gln Gln Gln
   115 120 125
   Ser Gln Gln Phe Leu Phe Gln Gly Glu Ser Gln Ser Gln Lys Phe Ile
   130 135 140
   Asp Glu His Gln Lys Ile His Gln Phe Arg Gln Gly Asp Ile Val Val
   145 150 155 160
   Leu Pro Thr Gly Val Ala His Trp Phe Tyr Asn Asp Gly Asp Thr Pro
   165 170 175
   Val Val Ala Leu Tyr Val Tyr Asp Ile Asn Asn Ser Ala Asn Gln Leu
               180                 185                 190
   Glu Pro Arg His Arg Glu Phe Leu Leu Ala Gly Lys Asn Asn Arg Val
           195                 200                 205
   Gln Gln Val Tyr Gly Arg Ser Ile Gln Gln His Ser Gly Gln Asn Ile
       210                 215                 220
   Phe Asn Gly Phe Ser Val Glu Pro Leu Ser Glu Ala Leu Asn Ile Asn
   225                 230                 235                 240
   Thr Val Thr Thr Lys Arg Leu Gln Ser Gln Asn Asp Gln Arg Gly Glu
                   245                 250                 255
   Ile Ile His Val Lys Asn Gly Leu Gln Leu Leu Lys Pro Thr Leu Thr
               260                 265                 270
   Gln Arg Gln Glu Gln Glu Gln Ala Gln Tyr Gln Glu Val Gln Tyr Ser
           275                 280                 285
   Glu Lys Pro Gln Thr Ser Ser Arg Trp Asn Gly Leu Glu Glu Asn Leu
       290                 295                 300
   Cys Thr Ile Lys Thr Arg Leu Asn Ile Glu Asn Pro Ser Arg Ala Asp
   305                 310                 315                 320
   Ser Tyr Asp Pro Arg Ala Gly Arg Ile Thr Ser Leu Asp Ser Gln Lys
                   325                 330                 335
   Phe Pro Ile Leu Asn Ile Ile Gln Met Ser Ala Thr Arg Val Asn Leu
               340                 345                 350
   Tyr Gln Asn Ala Ile Leu Thr Pro Phe Trp Asn Val Asn Ala His Ser
           355                 360                 365
   Leu Met Tyr Val Ile Arg Gly Arg Ala Arg Val Gln Val Val Ser Asn
       370                 375                 380
   Phe Gly Lys Thr Val Phe Asp Gly Val Leu Arg Pro Glu Gln Leu Leu
   385                 390                 395                 400
   Ile Ile Pro Gln Asn Tyr Val Val Leu Lys Lys Ala Gln His Glu Gly
                   405                 410                 415
   Cys Gln Tyr Ile Ala Ile Asn Thr Asn Ala Asn Ala Phe Val Ser His
               420                 425                 430
   Leu Ala Gly Val Asp Ser Val Phe His Ala Leu Pro Val Asp Val Ile
   435 440 445
   Ala Asn Ala Tyr Cys Ile Ser Arg Glu Glu Ala Arg Arg Leu Lys Asn
   450 455 460
   Asn Arg Gly Asp Glu Tyr Gly Pro Phe Pro Pro Arg Leu Gln Gln Gln
   465 470 475 480
   Ile Tyr Pro Glu Phe Ser Asn Glu Ser Lys Gly Glu Thr Ser Glu
   485 490 495
<210> 528
   <211> 480
   <212> PRT
   <213> Oryza sativa
<400> 528
   Leu Leu Cys His Gly Ser Met Ala Gln Ile Phe Ser Leu Gly Ile Asn
   1 5 10 15
   Pro Trp Gln Asn Pro Arg Gln Gly Gly Ser Arg Glu Cys Arg Phe Asp
   20 25 30
   Arg Leu Gln Ala Phe Glu Pro Leu Arg Lys Val Arg His Glu Ala Gly
   35 40 45
   Val Thr Glu Tyr Phe Asp Glu Lys Asn Glu Gln Phe Gln Cys Thr Gly
   50 55 60
   Thr Leu Val Ile Arg Arg Ile Ile Glu Pro Gln Gly Leu Leu Leu Pro
   65 70 75 80
   Arg Tyr Ser Asn Thr Pro Gly Leu Val Tyr Ile Ile Gln Gly Thr Gly
   85 90 95
   Val Leu Gly Leu Thr Phe Pro Gly Cys Pro Ala Thr Tyr Gln Lys Gln
   100 105 110
   Phe Arg His Phe Gly Leu Glu Gly Gly Ser Gln Arg Gln Gly Lys Lys
   115 120 125
   Leu Arg Asp Glu Asn Gln Lys Ile His Gln Phe Arg Gln Gly Asp Val
   130 135 140
   Val Ala Leu Pro Ser Gly Ile Pro His Trp Phe Tyr Asn Glu Gly Asp
   145 150 155 160
   Thr Pro Val Val Ala Leu Phe Val Phe Asp Val Asn Asn Asn Ala Asn
                   165                 170                 175
   Gln Leu Glu Pro Arg Gln Lys Glu Phe Leu Leu Ala Gly Asn Asn Ile
               180                 185                 190
   Glu Gln Gln Val Ser Asn Pro Ser Ile Asn Lys His Ser Gly Gln Asn
           195                 200                 205
   Ile Phe Asn Gly Phe Asn Thr Lys Leu Leu Ser Glu Ala Leu Gly Val
       210                 215                 220
   Asn Ile Glu Val Thr Arg Arg Leu Gln Ser Gln Asn Asp Arg Arg Gly
   225                 230                 235                 240
   Asp Ile Ile Arg Val Lys Asn Gly Leu Arg Leu Ile Lys Pro Thr Ile
                   245                 250                 255
   Thr Gln Gln Gln Glu Gln Thr Gln Asp Gln Tyr Gln Gln Ile Gln Tyr
               260                 265                 270
   His Arg Glu Gln Arg Ser Thr Ser Lys Tyr Asn Gly Leu Asp Glu Asn
           275                 280                 285
   Phe Cys Ala Ile Arg Ala Arg Leu Asn Ile Glu Asn Pro Asn His Ala
       290                 295                 300
   Asp Thr Tyr Asn Pro Arg Ala Gly Arg Ile Thr Asn Leu Asn Ser Gln
   305                 310                 315                 320
   Lys Phe Ser Ile Leu Asn Leu Val Gln Met Ser Ala Thr Arg Val Asn
                   325                 330                 335
   Leu Tyr Gln Asn Ala Ile Leu Ser Pro Phe Trp Asn Ile Asn Ala His
               340                 345                 350
   Ser Leu Val Tyr Thr Ile Gln Gly Arg Ala Arg Val Gln Val Val Ser
           355                 360                 365
   Asn His Gly Lys Ala Val Phe Asn Gly Val Leu Arg Pro Gly Gln Leu
       370                 375                 380
   Leu Ile Ile Pro Gln Asn Tyr Val Val Met Lys Lys Ala Glu Leu Glu
   385                 390                 395                 400
   Gly Phe Gln Phe Ile Ala Phe Lys Thr Asn Pro Asn Ala Met Val Asn
                   405                 410                 415
   His Ile Ala Gly Lys Asn Ser Val Leu Arg Ala Met Pro Val Asp Val
   420 425 430
   Ile Ala Asn Ala Tyr Arg Ile Ser Arg Gln Glu Ala Arg Ser Leu Lys
   435 440 445
   Asn Asn Arg Gly Glu Glu Ile Gly Ala Phe Thr Pro Arg Tyr Gln Gln
   450 455 460
   Gln Lys Ile His Gln Glu Tyr Ser Asn Pro Asn Glu Ser Glu Thr Gln
   465 470 475 480
<210> 529
   <211> 518
   <212> PRT
   <213> Oryza sativa
<400> 529
   Met Ala Thr Thr Arg Phe Pro Ser Leu Leu Phe Tyr Ser Cys Ile Phe
   1 5 10 15
   Leu Leu Cys Asn Gly Ser Met Ala Gln Leu Phe Gly Gln Ser Phe Thr
   20 25 30
   Pro Trp Gln Ser Ser Arg Gln Gly Gly Leu Arg Gly Cys Arg Phe Asp
   35 40 45
   Arg Leu Gln Ala Phe Glu Pro Leu Arg Gln Val Arg Ser Gln Ala Gly
   50 55 60
   Ile Thr Glu Tyr Phe Asp Glu Gln Asn Glu Gln Phe Arg Cys Ala Gly
   65 70 75 80
   Val Ser Val Ile Arg Arg Val Ile Glu Pro Gln Gly Leu Leu Leu Pro
   85 90 95
   Gln Tyr His Asn Ala Pro Gly Leu Val Tyr Ile Leu Gln Gly Arg Gly
   100 105 110
   Phe Thr Gly Leu Thr Phe Pro Gly Cys Pro Ala Thr Phe Gln Gln Gln
   115 120 125
   Phe Gln Pro Phe Asp Gln Ala Arg Phe Ala Gln Gly Gln Ser Lys Ser
   130 135 140
   Gln Asn Leu Lys Asp Glu His Gln Arg Val His His Ile Lys Gln Gly
   145 150 155 160
   Asp Val Val Ala Leu Pro Ala Gly Ile Val His Trp Cys Tyr Asn Asp
   165 170 175
   Gly Asp Ala Pro Ile Val Ala Val Tyr Val Phe Asp Val Asn Asn Asn
   180 185 190
   Ala Asn Gln Leu Glu Pro Arg Gln Lys Glu Phe Leu Leu Ala Gly Asn
   195 200 205
   Asn Lys Arg Glu Gln Gln Phe Gly Gln Asn Ile Phe Ser Gly Phe Ser
   210 215 220
   Val Gln Leu Leu Ser Glu Ala Leu Gly Ile Ser Gln Gln Ala Ala Gln
   225 230 235 240
   Lys Ile Gln Ser Gln Asn Asp Gln Arg Gly Glu Ile Ile Arg Val Ser
   245 250 255
   Gln Gly Leu Gln Phe Leu Lys Pro Phe Val Ser Gln Gln Gly Pro Val
   260 265 270
   Glu His Gln Ala Tyr Gln Pro Ile Gln Ser Gln Gln Glu Gln Ser Thr
   275 280 285
   Gln Tyr Gln Val Gly Gln Ser Pro Gln Tyr Gln Glu Gly Gln Ser Thr
   290 295 300
   Gln Tyr Gln Ser Gly Gln Ser Trp Asp Gln Ser Phe Asn Gly Leu Glu
   305 310 315 320
   Glu Asn Phe Cys Ser Leu Glu Ala Arg Gln Asn Ile Glu Asn Pro Lys
   325 330 335
   Arg Ala Asp Thr Tyr Asn Pro Arg Ala Gly Arg Ile Thr His Leu Asn
   340 345 350
   Ser Lys Asn Phe Pro Thr Leu Asn Leu Val Gln Met Ser Ala Thr Arg
   355 360 365
   Val Asn Leu Tyr Gln Asn Ala Ile Leu Ser Pro Tyr Trp Asn Ile Asn
   370 375 380
   Ala His Ser Val Met His Met Ile Gln Gly Arg Ala Arg Val Gln Val
   385 390 395 400
   Val Asn Asn His Gly Gln Thr Val Phe Asn Asp Ile Leu Arg Arg Gly
   405 410 415
   Gln Leu Leu Ile Ile Pro Gln His Tyr Val Val Leu Lys Lys Ala Glu
   420 425 430
   Arg Glu Gly Cys Gln Tyr Ile Ser Phe Lys Thr Thr Pro Asn Ser Met
   435 440 445
   Val Ser Tyr Ile Ala Gly Lys Thr Ser Ile Leu Arg Ala Leu Pro Val
   450 455 460
   Asp Val Leu Ala Asn Ala Tyr Arg Ile Ser Arg Gln Glu Ser Gln Asn
   465 470 475 480
   Leu Lys Asn Asn Arg Gly Glu Glu Phe Gly Ala Phe Thr Pro Lys Phe
   485 490 495
   Ala Gln Thr Gly Ser Gln Ser Tyr Gln Asp Glu Gly Glu Ser Ser Ser
   500 505 510
   Thr Glu Lys Ala Ser Glu
   515
<210> 530
   <211> 498
   <212> PRT
   <213> Oryza sativa
<400> 530
   Met Ala Ser Thr Asn Arg Pro Ile Val Phe Phe Thr Val Cys Leu Phe
   1               5                   10                  15
   Leu Leu Cys Asp Gly Ser Leu Ala Gln Gln Leu Leu Gly Gln Ser Thr
               20                  25                  30
   Ser Gln Trp Gln Ser Ser Arg Arg Gly Ser Pro Arg Gly Cys Arg Phe
           35                  40                  45
   Asp Arg Leu Gln Ala Phe Glu Pro Ile Arg Ser Val Arg Ser Gln Ala
       50                  55                  60
   Gly Thr Thr Glu Phe Phe Asp Val Ser Asn Glu Leu Phe Gln Cys Thr
   65                  70                  75                  80
   Gly Val Ser Val Val Arg Arg Val Ile Glu Pro Arg Gly Leu Leu Leu
                   85                  90                  95
   Pro His Tyr Thr Asn Gly Ala Ser Leu Val Tyr Ile Ile Gln Gly Arg
               100                 105                 110
   Gly Ile Thr Gly Pro Thr Phe Pro Gly Cys Pro Glu Thr Tyr Gln Gln
           115                 120                 125
   Gln Phe Gln Gln Ser Gly Gln Ala Gly Leu Thr Glu Ser Gln Ser Gln
       130                 135                 140
   Ser His Lys Phe Lys Asp Glu His Gln Lys Ile His Arg Phe Arg Gln
   145                 150                 155                 160
   Gly Asp Val Ile Ala Leu Pro Ala Gly Val Ala His Trp Cys Tyr Asn
                   165                 170                 175
   Asp Cys Glu Val Pro Val Val Ala Ile Tyr Val Thr Asp Ile Asn Asn
               180                 185                 190
   Gly Ala Asn Gln Leu Asp Pro Arg Gln Arg Asp Phe Leu Leu Ala Gly
           195                 200                 205
   Asn Lys Arg Asn Pro Gln Ala Tyr Arg Arg Glu Val Glu Glu Trp Ser
       210                 215                 220
   Gln Asn Ile Phe Ser Gly Phe Ser Thr Glu Leu Leu Ser Glu Ala Phe
   225                 230                 235                 240
   Gly Ile Ser Asn Gln Val Ala Arg Gln Leu Gln Cys Gln Asn Asp Gln
                   245                 250                 255
   Lys Gly Glu Ile Val Arg Val Glu Arg Gly Leu Ser Leu Leu Gln Pro
               260                 265                 270
   Tyr Ala Ser Leu Gln Glu Gln Glu Gln Gly Gln Met Gln Ser Arg Glu
           275                 280                 285
   His Tyr Gln Glu Gly Gly Tyr Gln Gln Ser Gln Tyr Gly Ser Gly Cys
       290                 295                 300
   Pro Asn Gly Leu Asp Glu Thr Phe Cys Val Asn Lys Val Arg Gln Asn
   305                 310                 315                 320
   Ile Asp Asn Pro Asn Arg Ala Asp Thr Tyr Asn Pro Arg Ala Gly Arg
                   325                 330                 335
   Val Thr Asn Leu Ser Gln Asn Phe Pro Ile Leu Asn Leu Val Gln Met
               340                 345                 350
   Ser Ala Val Lys Val Asn Leu Tyr Gln Asn Thr Asp Thr Trp Ile Ser
   355 360 365
   Met Gly Gln Glu Glu Asn Ala Leu Leu Ser Pro Phe Trp Asn Ile Asn
   370 375 380
   Ala His Ser Ile Val Tyr Ile Thr Gln Gly Arg Ala Gln Val Gln Val
   385 390 395 400
   Leu Arg Arg Gly Gln Leu Leu Ile Val Pro Gln His Tyr Val Val Val
   405 410 415
   Lys Lys Ala Gln Arg Glu Gly Cys Ala Tyr Ile Ala Phe Lys Thr Asn
   420 425 430
   Pro Asn Ser Met Val Ser His Ile Ala Gly Lys Ser Ser Ile Phe Arg
   435 440 445
   Ala Leu Pro Thr Asp Val Leu Ala Asn Ala Tyr Arg Ile Ser Arg Glu
   450 455 460
   Glu Ala Gln Arg Leu Lys His Asn Arg Gly Asp Glu Phe Gly Ala Phe
   465 470 475 480
   Thr Pro Leu Gln Tyr Lys Ser Tyr Gln Asp Val Tyr Asn Val Ala Glu
   485 490 495
   Ser Ser
<210> 531
   <211> 491
   <212> PRT
   <213> Oryza brachyantha
<400> 531
   Met Lys Ser Ser Ile Val Phe Ser Thr Ile Cys Leu Val Leu Leu Cys
   1 5 10 15
   His Gly Ser Leu Ala Gln Leu Leu Ser Gln Ser Thr Ser Gln Trp Gln
   20 25 30
   Ser Ser Arg Arg Gly Ser Pro Arg Gln Cys Arg Phe Asp Gln Leu Gln
   35 40 45
   Ala Phe Glu Pro Ile Arg Thr Val Arg Ser Gln Ala Gly Val Thr Glu
   50 55 60
   Phe Tyr Asp Val Ser Asn Glu Leu Phe Gln Cys Thr Gly Val Ser Val
   65                  70                  75                  80
   Val Arg Arg Val Ile Glu Pro Arg Gly Leu Leu Leu Pro His Tyr Ser
                   85                  90                  95
   Asn Gly Ala Thr Leu Val Tyr Ile Ile Gln Gly Arg Gly Ile Thr Gly
               100                 105                 110
   Pro Thr Phe Pro Gly Cys Pro Glu Thr Tyr Gln Gln Gln Phe Gln Gln
           115                 120                 125
   Ser Gly Glu Ala Gln Pro Phe Glu Gly Gln Ser His Lys Phe Arg Asp
       130                 135                 140
   Glu His Gln Lys Ile His Arg Phe Arg Gln Gly Asp Val Val Ala Leu
   145                 150                 155                 160
   Pro Ala Gly Val Ala His Trp Cys Tyr Asn Asp Gly Glu Val Pro Ile
                   165                 170                 175
   Val Ala Ile Tyr Val Thr Asp Ile Tyr Asn Ser Ala Asn Gln Leu Asp
               180                 185                 190
   Pro Arg His Arg Asp Phe Phe Leu Ala Gly Asn Asn Lys Val Ala Gln
           195                 200                 205
   Gln Leu Tyr Arg Ser Glu Ala Arg Glu Asn Ser Lys Asn Ile Phe Gly
       210                 215                 220
   Gly Phe Ser Val Glu Leu Leu Ser Glu Ala Leu Gly Ile Ser Arg Gly
   225                 230                 235                 240
   Val Ala Arg Gln Leu Gln Cys Gln Asn Asp Gln Arg Gly Glu Ile Val
                   245                 250                 255
   Arg Val Glu His Gly Leu Ala Leu Leu Gln Pro Tyr Ala Ser Val Gln
               260                 265                 270
   Glu Gln Gln Gln Glu Gln Val Gln Ser Arg Asp Tyr Glu Gln Thr Gln
           275                 280                 285
   Tyr Gln Gln Lys Gln Pro Gln Gly Ser Cys Ser Asn Gly Leu Asp Glu
       290                 295                 300
   Thr Phe Cys Thr Met Arg Leu Arg Gln Asn Ile Asp Asn Pro Asn Leu
   305                 310                 315                 320
   Ala Asp Thr Tyr Asn Pro Lys Ala Gly Arg Ile Thr Tyr Leu Asn Gly
                   325                 330                 335
   Gln Lys Phe Pro Ile Leu Asn Leu Val Gln Met Ser Ala Val Lys Val
               340                 345                 350
   Asn Leu Tyr Gln Asn Ala Val Leu Ser Pro Phe Trp Asn Ile Asn Ala
           355                 360                 365
   His Ser Val Val Tyr Ile Thr Gln Gly Arg Ala Arg Val Gln Val Val
       370                 375                 380
   Asn Asn Asn Gly Lys Thr Val Phe Asp Gly Glu Leu Arg Gln Gly Gln
   385                 390                 395                 400
   Leu Leu Ile Ile Pro Gln His His Val Val Leu Lys Lys Ala Gln Arg
                   405                 410                 415
   Glu Gly Cys Ser Tyr Ile Ala Leu Lys Thr Asn Pro Asn Ser Ile Val
               420                 425                 430
   Ser His Ile Ala Gly Lys Asn Ser Ile Phe Arg Ala Leu Pro Gly Asp
           435                 440                 445
   Val Val Thr Asn Ala Tyr Arg Ile Ser Arg Glu Glu Ala Lys Arg Ile
       450                 455                 460
   Lys His Asn Arg Gly Asp Glu Ser Gly Val Phe Ala Pro Ser His Ala
   465                 470                 475                 480
   Tyr Arg Ser Tyr Gln Asp Met Ser Val Ala Ala
                   485                 490
<210> 532
   <211> 492
   <212> PRT
   <213> Brachypodium distachyon
<400> 532
   Met Ala His Thr Ser Phe Ser Ser Phe Leu Ser Tyr Phe Cys Leu Phe
   1               5                   10                  15
   Leu Leu Phe His Gly Ser Met Ala Gln Val Leu Gly Gln Val Ser Thr
               20                  25                  30
   Trp Gln Ser Ser Arg Gln Gly Gly Ser Arg Asp Cys Ser Phe Asp Arg
           35                  40                  45
   Leu Gln Ala Ile Glu Pro Val Thr Gln Val Arg Ser Gln Ala Gly Leu
       50                  55                  60
   Thr Glu Tyr Phe Asp Glu Gln Asn Glu Gln Phe Arg Cys Ala Gly Val
   65                  70                  75                  80
   Phe Val Ile Arg Arg Val Ile Glu Pro Arg Gly Leu Leu Leu Pro Arg
                   85                  90                  95
   Tyr His Asn Thr Pro Gly Leu Val Tyr Ile Leu Gln Gly Asn Gly Phe
               100                 105                 110
   Val Gly Leu Thr Phe Pro Gly Cys Pro Glu Thr Phe Arg Glu Gln Phe
           115                 120                 125
   Gln Gln Phe Arg Gln Thr Gln Ser Thr Leu Gly Gln Ser Gln Cys Gln
       130                 135                 140
   Ser Gln Lys Leu Gly Asp Val His Gln Arg Val His Gln Phe Thr Gln
   145                 150                 155                 160
   Gly Asp Val Val Ala Leu Pro Thr Gly Val Ala His Trp Ile Tyr Asn
                   165                 170                 175
   Gly Gly Asp Ala Pro Val Val Ile Val Tyr Val Phe Asp Val Asn Asn
               180                 185                 190
   Asn Ala Asn Gln Leu Glu Pro Arg Gln Lys Glu Phe Leu Leu Gly Gly
           195                 200                 205
   Asn Tyr Asn Gly Val Leu Gln Tyr Gly Gln Asn Ile Phe Ser Gly Phe
       210                 215                 220
   Asn Ala Gln Leu Leu Ser Gln Ala Phe Gly Ile Asn Glu Gln Thr Ser
   225                 230                 235                 240
   Gln Arg Ile Gln Asn Gln Asn Asp Gly Arg Gly Asp Ile Ile Arg Val
                   245                 250                 255
   Asp Asn Gly Leu Gln Phe Leu Lys Pro Val Val Thr Gln Gln Gln Pro
               260                 265                 270
   Glu Gln Pro Phe Met Pro Ile Gln His Gln Thr Gly Gln Ser Ser Arg
           275                 280                 285
   Asn Gly Leu Glu Glu Asn Phe Cys Ser Leu Glu Pro Arg Gln Asn Ile
       290                 295                 300
   Glu Asp Pro Asn Arg Ala Asp Thr Tyr Asn Pro Arg Ala Gly Ser Ile
   305 310 315 320
   Thr Arg Leu Asn Gly Gln Asn Phe Pro Ile Leu Asn Leu Val Gln Met
   325 330 335
   Ser Ala Thr Arg Val Asn Leu Gln Lys Asn Ala Ile Leu Ser Pro Phe
   340 345 350
   Trp Asn Ile Asn Ala His Ser Val Val Tyr Val Ile Gln Gly His Ala
   355 360 365
   Leu Val Gln Val Val Asn Asn Gln Gly His Asn Val Phe Asn Gly Leu
   370 375 380
   Leu His Arg Gly Gln Leu Leu Ile Ile Pro Gln Asn Tyr Val Val Leu
   385 390 395 400
   Lys Lys Ala Glu Ser Glu Gly Tyr Gln Tyr Ile Ala Phe Lys Thr Asn
   405 410 415
   Ala Asn Ser Met Val Ser His Ile Ala Gly Lys Asn Ser Ile Leu Arg
   420 425 430
   Ala Leu Pro Val Asp Val Ile Ala Asn Ala Tyr Arg Ile Ser Arg Gln
   435 440 445
   Glu Ala Gln Asn Leu Lys Asn Asn Arg Gly Glu Glu Thr Gly Val Leu
   450 455 460
   Thr Pro Asn Phe Ser Gln Ser Thr Cys Gln Ser Tyr Gln Thr Glu Asp
   465 470 475 480
   Val Gln Ser Leu Arg Pro Met Ser His Trp Ser Glu
   485 490
<210> 533
   <211> 166
   <212> PRT
   <213> Oryza sativa
<220>
   <221> misc_feature
   <222> (120)..(120)
   <223> Xaa can be any naturally occurring amino acid
<400> 533
   Met Ala Ser Asn Lys Val Val Phe Ser Ala Leu Leu Leu Ile Ile Val
   1 5 10 15
   Ser Val Leu Ala Ala Thr Ala Thr Met Ala Asp His His Lys Asp Gln
   20 25 30
   Val Val Tyr Ser Leu Gly Glu Arg Cys Gln Pro Gly Met Gly Tyr Pro
   35 40 45
   Met Tyr Ser Leu Pro Arg Cys Arg Ala Val Val Lys Arg Gln Cys Val
   50 55 60
   Gly His Gly Ala Pro Gly Gly Ala Val Asp Glu Gln Leu Arg Gln Asp
   65 70 75 80
   Cys Cys Arg Gln Leu Ala Ala Val Asp Asp Ser Trp Cys Arg Cys Ser
   85 90 95
   Ala Leu Asn His Met Val Gly Gly Ile Tyr Arg Glu Leu Gly Ala Thr
   100 105 110
   Asp Val Gly His Pro Met Ala Xaa Val Phe Pro Gly Cys Arg Arg Gly
   115 120 125
   Asp Leu Glu Arg Ala Ala Ala Ser Leu Pro Ala Phe Cys Asn Val Asp
   130 135 140
   Ile Pro Asn Gly Thr Gly Gly Val Cys Tyr Trp Leu Gly Tyr Pro Arg
   145 150 155 160
   Thr Pro Arg Thr Gly His
   165
<210> 534
   <211> 165
   <212> PRT
   <213> Oryza sativa
<400> 534
   Met Ala Ser Asn Lys Val Val Phe Ser Ala Leu Leu Leu Ile Ile Val
   1 5 10 15
   Ser Val Leu Arg Arg Asp Gly Thr Met Ala Asp His His Lys Asp Gln
   20 25 30
   Val Val Tyr Ser Leu Gly Glu Arg Cys Gln Pro Gly Met Gly Tyr Pro
   35 40 45
   Met Tyr Ser Leu Pro Arg Cys Arg Ala Val Val Lys Arg Gln Cys Val
   50 55 60
   Gly His Gly Ala Pro Gly Ala Val Asp Glu Gln Leu Arg Gln Asp Cys
   65 70 75 80
   Cys Arg Gln Leu Ala Ala Val Asp Asp Ser Trp Cys Arg Cys Ser Ala
   85 90 95
   Leu Asn His Met Val Gly Gly Ile Tyr Arg Glu Leu Gly Ala Thr Asp
   100 105 110
   Val Gly His Pro Met Ala Glu Val Phe Pro Gly Cys Arg Arg Gly Asp
   115 120 125
   Leu Glu Arg Ala Ala Ala Ser Leu Pro Ala Phe Cys Asn Val Asp Ile
   130 135 140
   Pro Asn Gly Thr Gly Gly Val Cys Tyr Trp Leu Gly Tyr Pro Arg Thr
   145 150 155 160
   Pro Arg Thr Gly His
   165
<210> 535
   <211> 157
   <212> PRT
   <213> Oryza sativa
<400> 535
   Met Ala Ser Asn Lys Val Val Ile Ser Ala Leu Leu Val Val Val Val
   1 5 10 15
   Ser Val Leu Ala Ala Thr Thr Thr Met Ala Asp His His Gln Glu Gln
   20 25 30
   Val Val Tyr Thr Pro Gly Gln Leu Cys Gln Pro Gly Ile Gly Tyr Pro
   35 40 45
   Thr Tyr Pro Leu Pro Arg Cys Arg Ala Phe Val Lys Arg Gln Cys Val
   50 55 60
   Ala Pro Gly Thr Val Asp Glu Gln Val Arg Arg Gly Cys Cys Arg Gln
   65 70 75 80
   Leu Ala Ala Ile Asp Ser Ser Trp Cys Arg Cys Asp Ala Leu Asn His
   85 90 95
   Met Leu Arg Ile Ile Tyr Arg Glu Ser Gly Ala Ala Asp Ala Gly His
   100 105 110
   Pro Met Ala Glu Val Phe Arg Gly Cys Arg Arg Gly Asp Ile Glu Arg
   115 120 125
   Ala Ala Ala Ser Leu Pro Ala Phe Cys Asn Val Asp Ile Pro Asn Gly
   130 135 140
   Val Gly Gly Val Cys Tyr Trp Leu Pro Gly Thr Gly Tyr
   145 150 155
<210> 536
   <211> 605
   <212> PRT
   <213> Oryza rufipogon
<400> 536
   Met Ser Ala Leu Thr Thr Ser Gln Leu Ala Thr Ser Ala Thr Gly Phe
   1 5 10 15
   Gly Ile Ala Asp Arg Ser Ala Pro Ser Ser Leu Leu Arg His Gly Phe
   20 25 30
   Gln Gly Leu Lys Pro Arg Ser Pro Ala Gly Gly Asp Ala Thr Ser Leu
   35 40 45
   Ser Val Thr Thr Ser Ala Arg Ala Thr Pro Lys Gln Gln Arg Ser Val
   50 55 60
   Gln Arg Gly Ser Arg Arg Phe Pro Ser Val Val Val Tyr Ala Thr Gly
   65 70 75 80
   Ala Gly Met Asn Val Val Phe Val Gly Ala Glu Met Ala Pro Trp Ser
   85 90 95
   Lys Thr Gly Gly Leu Gly Asp Val Leu Gly Gly Leu Pro Pro Ala Met
   100 105 110
   Ala Ala Asn Gly His Arg Val Met Val Ile Ser Pro Arg Tyr Asp Gln
   115 120 125
   Tyr Lys Asp Ala Trp Asp Thr Ser Val Val Ala Glu Ile Lys Val Ala
   130 135 140
   Asp Arg Tyr Glu Arg Val Arg Phe Phe His Cys Tyr Lys Arg Gly Val
   145 150 155 160
   Asp Arg Val Phe Val Asp His Pro Ser Phe Leu Glu Lys Val Trp Gly
                   165                 170                 175
   Lys Thr Gly Glu Lys Ile Tyr Gly Pro Asp Thr Gly Val Asp Tyr Lys
               180                 185                 190
   Asp Asn Gln Met Arg Phe Ser Leu Leu Cys Gln Ala Pro Arg Ile Leu
           195                 200                 205
   Asn Leu Asn Asn Asn Pro Tyr Phe Lys Gly Thr Tyr Gly Glu Asp Val
       210                 215                 220
   Val Phe Val Cys Asn Asp Trp His Thr Gly Pro Leu Ala Ser Tyr Leu
   225                 230                 235                 240
   Lys Asn Asn Tyr Gln Pro Asn Gly Ile Tyr Arg Asn Ala Lys Val Ala
                   245                 250                 255
   Phe Cys Ile His Asn Ile Ser Tyr Gln Gly Arg Phe Ala Phe Glu Asp
               260                 265                 270
   Tyr Pro Glu Leu Asn Leu Ser Glu Arg Phe Arg Ser Ser Phe Asp Phe
           275                 280                 285
   Ile Asp Gly Tyr Asp Thr Pro Val Glu Gly Arg Lys Ile Asn Trp Met
       290                 295                 300
   Lys Ala Gly Ile Leu Glu Ala Asp Arg Val Leu Thr Val Ser Pro Tyr
   305                 310                 315                 320
   Tyr Ala Glu Glu Leu Ile Ser Gly Ile Ala Arg Gly Cys Glu Leu Asp
                   325                 330                 335
   Asn Ile Met Arg Leu Thr Gly Ile Thr Gly Ile Val Asn Gly Met Asp
               340                 345                 350
   Val Ser Glu Trp Asp Pro Ser Lys Asp Lys Tyr Ile Thr Ala Lys Tyr
           355                 360                 365
   Asp Ala Thr Thr Ala Ile Glu Ala Lys Ala Leu Asn Lys Glu Ala Leu
       370                 375                 380
   Gln Ala Glu Ala Gly Leu Pro Val Asp Arg Lys Ile Pro Leu Ile Ala
   385                 390                 395                 400
   Phe Ile Gly Arg Leu Glu Glu Gln Lys Gly Pro Asp Val Met Ala Ala
                   405                 410                 415
   Ala Ile Pro Glu Leu Met Gln Glu Asp Val Gln Ile Val Leu Leu Gly
   420 425 430
   Thr Gly Lys Lys Lys Phe Glu Lys Leu Leu Lys Ser Met Glu Glu Lys
   435 440 445
   Tyr Pro Gly Lys Val Arg Ala Val Val Lys Phe Asn Ala Pro Leu Ala
   450 455 460
   His Leu Ile Met Ala Gly Ala Asp Val Leu Ala Val Pro Ser Arg Phe
   465 470 475 480
   Glu Pro Cys Gly Leu Ile Gln Leu Gln Gly Met Arg Tyr Gly Thr Pro
   485 490 495
   Cys Ala Cys Ala Ser Thr Gly Gly Leu Val Asp Thr Val Ile Glu Gly
   500 505 510
   Lys Thr Gly Phe His Met Gly Arg Leu Ser Val Asp Cys Lys Val Val
   515 520 525
   Glu Pro Ser Asp Val Lys Lys Val Ala Ala Thr Leu Lys Arg Ala Ile
   530 535 540
   Lys Val Val Gly Thr Pro Ala Tyr Glu Glu Met Val Arg Asn Cys Met
   545 550 555 560
   Asn Gln Asp Leu Ser Trp Lys Gly Pro Ala Lys Asn Trp Glu Asn Val
   565 570 575
   Leu Leu Gly Leu Gly Val Ala Gly Ser Ala Pro Gly Ile Glu Gly Asp
   580 585 590
   Glu Ile Ala Pro Leu Ala Lys Glu Asn Val Ala Ala Pro
   595 600 605
<210> 537
   <211> 609
   <212> PRT
   <213> Oryza nivarra
<400> 537
   Met Ser Ala Leu Thr Thr Ser Gln Leu Ala Thr Ser Ala Thr Gly Phe
   1 5 10 15
   Gly Ile Ala Asp Arg Ser Ala Pro Ser Ser Leu Leu Arg His Gly Phe
   20 25 30
   Gln Gly Leu Lys Pro Arg Ser Pro Ala Gly Gly Asp Ala Ser Ser Leu
           35                  40                  45
   Ser Val Thr Thr Ser Ala Arg Ala Thr Pro Lys Gln Gln Arg Ser Val
       50                  55                  60
   Gln Arg Gly Ser Arg Arg Phe Pro Ser Val Val Val Tyr Ala Thr Gly
   65                  70                  75                  80
   Ala Gly Met Asn Val Val Phe Val Gly Ala Glu Met Ala Pro Trp Ser
                   85                  90                  95
   Lys Thr Gly Gly Leu Gly Asp Val Leu Gly Gly Leu Pro Pro Ala Met
               100                 105                 110
   Ala Ala Asn Gly His Arg Val Met Val Ile Ser Pro Arg His Asp Gln
           115                 120                 125
   Tyr Lys Asp Ala Trp Asp Thr Ser Val Val Ala Glu Ile Lys Val Ala
       130                 135                 140
   Asp Arg Tyr Glu Arg Val Arg Phe Phe His Cys Tyr Lys Arg Gly Val
   145                 150                 155                 160
   Asp Arg Val Phe Ile Asp His Pro Ser Phe Leu Glu Lys Val Trp Gly
                   165                 170                 175
   Lys Thr Gly Glu Lys Ile Tyr Gly Pro Asp Thr Gly Val Asp Tyr Lys
               180                 185                 190
   Asp Asn Gln Met Arg Phe Ser Leu Leu Cys Gln Ala Ala Leu Glu Ala
           195                 200                 205
   Pro Arg Ile Leu Asn Leu Asn Asn Asn Pro Tyr Phe Lys Gly Thr Tyr
       210                 215                 220
   Gly Glu Asp Val Val Phe Val Cys Asn Asp Trp His Thr Gly Pro Leu
   225                 230                 235                 240
   Pro Ser Tyr Leu Lys Asn Asn Tyr Gln Pro Asn Gly Ile Tyr Arg Asn
                   245                 250                 255
   Ala Lys Val Ala Phe Cys Ile His Asn Ile Ser Tyr Gln Gly Arg Phe
               260                 265                 270
   Ala Phe Glu Asp Tyr Pro Glu Leu Asn Leu Ser Glu Arg Phe Arg Ser
           275                 280                 285
   Ser Phe Asp Phe Ile Asp Gly Tyr Asp Thr Pro Val Glu Gly Arg Lys
       290                 295                 300
   Ile Asn Trp Met Lys Ala Gly Ile Leu Glu Ser Asp Arg Val Leu Thr
   305                 310                 315                 320
   Val Ser Pro Tyr Tyr Ala Glu Glu Leu Ile Ser Gly Ile Ala Arg Gly
                   325                 330                 335
   Cys Glu Leu Asp Asn Ile Met Arg Leu Thr Gly Ile Thr Gly Ile Val
               340                 345                 350
   Asn Gly Met Asp Val Ser Glu Trp Asp Pro Ser Lys Asp Lys Tyr Ile
           355                 360                 365
   Ala Ala Lys Tyr Asp Ala Thr Thr Ala Ile Glu Ala Lys Ala Leu Asn
       370                 375                 380
   Lys Glu Ala Leu Gln Ala Glu Ala Gly Leu Pro Val Asp Arg Lys Ile
   385                 390                 395                 400
   Pro Leu Ile Ala Phe Ile Gly Arg Leu Glu Glu Gln Lys Gly Pro Asp
                   405                 410                 415
   Val Met Ala Ala Ala Ile Pro Glu Leu Met Gln Glu Asn Val Gln Ile
               420                 425                 430
   Val Leu Leu Gly Thr Gly Lys Lys Lys Phe Glu Lys Leu Leu Lys Ser
           435                 440                 445
   Met Glu Glu Lys Tyr Pro Gly Lys Val Arg Ala Val Val Lys Phe Asn
       450                 455                 460
   Ala Pro Leu Ala His Leu Ile Met Ala Gly Ala Asp Val Leu Ala Val
   465                 470                 475                 480
   Pro Ser Arg Phe Glu Pro Cys Gly Leu Ile Gln Leu Gln Gly Met Arg
                   485                 490                 495
   Tyr Gly Thr Pro Cys Ala Cys Ala Ser Thr Gly Gly Leu Val Asp Thr
               500                 505                 510
   Val Ile Glu Gly Lys Thr Gly Phe His Met Gly Arg Leu Ser Val Asp
           515                 520                 525
   Cys Lys Val Val Glu Pro Ser Asp Val Gln Lys Val Ala Thr Thr Leu
   530 535 540
   Lys Arg Ala Ile Lys Ile Val Gly Thr Pro Ala Tyr Asn Glu Met Val
   545 550 555 560
   Arg Asn Cys Met Asn Gln Asp Leu Ser Trp Lys Gly Pro Ala Lys Asn
   565 570 575
   Trp Glu Asn Val Leu Leu Gly Leu Gly Val Ala Gly Ser Ala Pro Gly
   580 585 590
   Val Glu Gly Glu Glu Ile Ala Pro Leu Ala Lys Glu Asn Val Ala Ala
   595 600 605
   Pro
<210> 538
   <211> 603
   <212> PRT
   <213> Hordeum vulgare
<400> 538
   Met Ala Ala Leu Ala Thr Ser Gln Leu Ala Thr Ser Gly Thr Val Leu
   1               5                   10                  15
   Gly Val Thr Asp Arg Phe Arg Arg Pro Gly Phe Gln Gly Leu Arg Pro
               20                  25                  30
   Arg Asn Pro Ala Asp Ala Ala Leu Gly Met Arg Thr Ile Gly Ala Ser
           35                  40                  45
   Ala Ala Pro Lys Gln Ser Arg Lys Ala His Arg Gly Ser Arg Arg Cys
       50                  55                  60
   Leu Ser Val Val Val Arg Ala Thr Gly Ser Gly Met Asn Leu Val Phe
   65                  70                  75                  80
   Val Gly Ala Glu Met Ala Pro Trp Ser Lys Thr Gly Gly Leu Gly Asp
                   85                  90                  95
   Val Leu Gly Gly Leu Pro Pro Ala Met Ala Ala Asn Gly His Arg Val
               100                 105                 110
   Met Val Val Ser Pro Arg Tyr Asp Gln Tyr Lys Asp Ala Trp Asp Thr
           115                 120                 125
   Ser Val Ile Ser Glu Ile Lys Val Ala Asp Glu Tyr Glu Arg Val Arg
   130 135 140
   Phe Phe His Cys Tyr Lys Arg Gly Val Asp Arg Val Phe Ile Asp His
   145 150 155 160
   Pro Trp Phe Leu Glu Lys Val Arg Gly Lys Thr Lys Glu Lys Ile Tyr
   165 170 175
   Gly Pro Asp Ala Gly Thr Asp Tyr Glu Asp Asn Gln Gln Arg Phe Ser
   180 185 190
   Leu Leu Cys Gln Ala Ala Leu Glu Ala Pro Arg Ile Leu Asn Leu Asn
   195 200 205
   Asn Asn Pro Tyr Phe Ser Gly Pro Tyr Gly Glu Asp Val Val Phe Val
   210 215 220
   Cys Asn Asp Trp His Thr Gly Leu Leu Ala Cys Tyr Leu Lys Ser Asn
   225 230 235 240
   Tyr Gln Ser Asn Gly Ile Tyr Arg Thr Ala Lys Val Ala Phe Cys Ile
   245 250 255
   His Asn Ile Ser Tyr Gln Gly Arg Phe Ser Phe Asp Asp Phe Ala Gln
   260 265 270
   Leu Asn Leu Pro Asp Arg Phe Lys Ser Ser Phe Asp Phe Ile Asp Gly
   275 280 285
   Tyr Asp Lys Pro Val Glu Gly Arg Lys Ile Asn Trp Met Lys Ala Gly
   290 295 300
   Ile Leu Gln Ala Asp Lys Val Leu Thr Val Ser Pro Tyr Tyr Ala Glu
   305 310 315 320
   Glu Leu Ile Ser Asp Glu Ala Arg Gly Cys Glu Leu Asp Asn Ile Met
   325 330 335
   Arg Leu Thr Gly Ile Thr Gly Ile Val Asn Gly Met Asp Val Ser Glu
   340 345 350
   Trp Asp Pro Thr Lys Asp Lys Phe Leu Ala Val Asn Tyr Asp Ile Thr
   355 360 365
   Thr Ala Leu Glu Ala Lys Ala Leu Asn Lys Glu Ala Leu Gln Ala Glu
   370 375 380
   Val Gly Leu Pro Val Asp Arg Lys Val Pro Leu Val Ala Phe Ile Gly
   385 390 395 400
   Arg Leu Glu Glu Gln Lys Gly Pro Asp Val Met Ile Ala Ala Ile Pro
   405 410 415
   Glu Ile Leu Lys Glu Glu Asp Val Gln Ile Ile Leu Leu Gly Thr Gly
   420 425 430
   Lys Lys Lys Phe Glu Lys Leu Leu Lys Ser Met Glu Glu Lys Phe Pro
   435 440 445
   Gly Lys Val Arg Ala Val Val Arg Phe Asn Ala Pro Leu Ala His Gln
   450 455 460
   Met Met Ala Gly Ala Asp Leu Leu Ala Val Thr Ser Arg Phe Glu Pro
   465 470 475 480
   Cys Gly Leu Ile Gln Leu Gln Gly Met Arg Tyr Gly Thr Pro Cys Val
   485 490 495
   Cys Ala Ser Thr Gly Gly Leu Val Asp Thr Ile Val Glu Gly Lys Thr
   500 505 510
   Gly Phe His Met Gly Arg Leu Ser Val Asp Cys Asn Val Val Glu Pro
   515 520 525
   Ala Asp Val Lys Lys Val Ala Thr Thr Leu Lys Arg Ala Val Lys Val
   530 535 540
   Val Gly Thr Pro Ala Tyr Gln Glu Met Val Lys Asn Cys Met Ile Gln
   545 550 555 560
   Asp Leu Ser Trp Lys Gly Pro Ala Lys Asn Trp Glu Asp Val Leu Leu
   565 570 575
   Glu Leu Gly Val Glu Gly Ser Glu Pro Gly Ile Val Gly Glu Glu Ile
   580 585 590
   Ala Pro Leu Ala Met Glu Asn Val Ala Ala Pro
   595 600
<210> 539
   <211> 495
   <212> PRT
   <213> Oryza brachyantha
<400> 539
   Met Ala Thr Thr Val Phe Ser Arg Phe Ser Thr Tyr Phe Cys Val Leu
   1               5                   10                  15
   Leu Leu Cys His Gly Ser Met Ala Gln Leu Phe Asn Pro Ser Thr Asn
               20                  25                  30
   Pro Trp His Asn Pro Arg Gln Gly Ser Ser Arg Glu Cys Arg Phe Asp
           35                  40                  45
   Arg Leu Gln Pro Phe Glu Pro Leu Arg Lys Val Arg Ser Glu Ala Gly
       50                  55                  60
   Val Thr Glu Tyr Phe Asp Glu Lys Asn Glu Leu Phe Gln Cys Thr Gly
   65                  70                  75                  80
   Thr Phe Val Ile Arg Arg Val Ile Gln Pro Gln Gly Leu Leu Val Pro
                   85                  90                  95
   Arg Tyr Thr Asn Ala Pro Gly Leu Val Tyr Ile Ile Gln Gly Arg Gly
               100                 105                 110
   Ser Ile Gly Leu Thr Phe Pro Gly Cys Pro Ala Thr Tyr Gln Gln Gln
           115                 120                 125
   Phe Gln Gln Phe Leu Pro Gln Glu Gln Ser Gln Ser Gln Lys Phe Arg
       130                 135                 140
   Asp Glu His Gln Lys Ile His Gln Phe Arg Gln Gly Asp Ile Val Ala
   145                 150                 155                 160
   Leu Pro Ala Gly Val Ala His Trp Phe Tyr Asn Asp Gly Asp Ala Pro
                   165                 170                 175
   Val Val Ala Val Tyr Val Tyr Asp Val Lys Asn Ser Ala Asn Gln Leu
               180                 185                 190
   Glu Pro Arg Gln Arg Glu Phe Leu Leu Gly Gly Asn Asn Met Arg Ala
           195                 200                 205
   Gln Gln Val Tyr Gly Ser Ser Ala Glu Gln His Ser Arg Gln Asn Ile
       210                 215                 220
   Phe Ser Gly Phe Gly Val Glu Ile Leu Ser Glu Ala Leu Gly Ile Ser
   225                 230                 235                 240
   Thr Val Thr Thr Lys Arg Leu Gln Ser Gln Asn Asp Gln Arg Gly Glu
                   245                 250                 255
   Ile Ile His Val Lys Asn Gly Leu Gln Phe Leu Lys Pro Thr Leu Thr
               260                 265                 270
   Gln Gln Gln Glu Gln Ala Gln Ala Gln Tyr Gln Glu Val Gln Tyr Ser
           275                 280                 285
   Glu Gln Gln Gln Thr Ser Ser Arg Trp Asn Gly Leu Asp Glu Asn Phe
       290                 295                 300
   Cys Thr Ile Lys Ala Arg Met Asn Ile Glu Asn Thr Ser Arg Ala Asp
   305                 310                 315                 320
   Thr Tyr Asn Pro Arg Ala Gly Arg Thr Thr Ser Leu Asn Ser Gln Lys
                   325                 330                 335
   Phe Pro Ile Leu Asn Leu Val Gln Met Ser Ala Thr Arg Val Asn Leu
               340                 345                 350
   Tyr Gln Asn Ala Ile Leu Ser Thr Phe Trp Asn Val Asn Ala His Ser
           355                 360                 365
   Leu Val Tyr Thr Ile Gln Gly Arg Ala Arg Val Gln Val Val Ser Asn
       370                 375                 380
   Phe Gly Lys Thr Val Phe Asp Gly Glu Leu Arg Pro Gly Gln Leu Leu
   385                 390                 395                 400
   Ile Ile Pro Gln His Tyr Val Val Leu Lys Lys Ala Gln Arg Glu Gly
                   405                 410                 415
   Phe Arg Tyr Ile Ala Ile Lys Thr Asn Ala Asn Ala Phe Val Ser Gln
               420                 425                 430
   Leu Val Gly Lys Asn Ser Val Phe Arg Ser Leu Pro Val Asp Val Ile
           435                 440                 445
   Ala Asn Val Tyr Arg Ile Ser Arg Glu Gln Ala Arg Ser Leu Lys Asn
       450                 455                 460
   Asn Arg Gly Glu Glu His Gly Ala Phe Ala Pro Arg Ser Gln Gln Gln
   465                 470                 475                 480
   Ser Tyr Pro Gly Phe Ser Asn Gln Ser Glu Ser Glu Thr Ser Glu
                   485                 490                 495
<210> 540
   <211> 494
   <212> PRT
   <213> Oryza brachyantha
<400> 540
   Met Ala Thr Thr Thr Phe Ser Arg Phe Ser Ile Tyr Phe Cys Val Leu
   1               5                   10                  15
   Leu Leu Cys His Gly Ser Met Ala Gln Leu Phe Ser Pro Thr Leu Asn
               20                  25                  30
   Pro Trp His Ser Ser Arg Arg Gly Gly Ser Arg Asp Cys Arg Phe Asp
           35                  40                  45
   Arg Leu Gln Ala Phe Glu Pro Leu Arg Arg Val Arg Ser Glu Ala Gly
       50                  55                  60
   Val Thr Glu Tyr Phe Asp Glu Arg Asn Glu Gln Phe Gln Cys Thr Gly
   65                  70                  75                  80
   Thr Phe Val Ile Arg Arg Val Ile Glu Pro Gln Gly Leu Leu Val Pro
                   85                  90                  95
   Arg Tyr Thr Asn Thr Pro Gly Val Val Tyr Ile Met Gln Gly Arg Gly
               100                 105                 110
   Ser Met Gly Leu Thr Phe Pro Gly Cys Pro Ala Thr Tyr Gln Gln Gln
           115                 120                 125
   Phe Gln Gln Phe Leu Pro Glu Gly Gln Ser Gln Ser Gln Lys Phe Arg
       130                 135                 140
   Asp Glu His Gln Lys Ile His Gln Phe Arg Gln Gly Asp Ile Val Ala
   145                 150                 155                 160
   Leu Pro Ala Gly Val Ala His Trp Phe Tyr Asn Glu Gly Asp Thr Pro
                   165                 170                 175
   Val Val Ala Leu Tyr Val Phe Asp Ile Asn Asn Ser Ala Asn Gln Leu
               180                 185                 190
   Glu Pro Arg Gln Lys Asp Phe Leu Leu Ala Gly Asn Asn Asn Arg Glu
           195                 200                 205
   Gln Gln Val Tyr Gly Arg Ser Ile Glu Lys His Ser Gly Gln Asn Ile
       210                 215                 220
   Phe Ser Gly Phe Asn His Glu Leu Leu Ser Glu Ala Leu Gly Ile Ser
   225                 230                 235                 240
   Thr Leu Ala Ala Lys Arg Leu Gln Gly Gln Asn Asp His Arg Gly Glu
   245 250 255
   Ile Ile Arg Val Arg Asn Gly Leu Gln Leu Leu Lys Pro Thr Phe Thr
   260 265 270
   Gln Gln Gln Glu Gln Ala Gln Ser Gln Tyr Gln Val Gln Tyr Ser Glu
   275 280 285
   Lys Gln Gln Glu Ser Thr Arg Cys Asn Gly Leu Asp Glu Asn Phe Cys
   290 295 300
   Thr Ile Asn Ala Arg Leu Asn Ile Glu Asn Pro Ser Arg Ala Asp Thr
   305 310 315 320
   Tyr Asn Pro Arg Ala Gly Arg Ile Thr His Leu Asn Asn Gln Lys Phe
   325 330 335
   Pro Ile Leu Asn Leu Val Gln Met Ser Ala Thr Arg Val Asn Leu Tyr
   340 345 350
   Gln Asn Ala Ile Leu Ser Pro Tyr Trp Asn Val Asn Ala His Ser Leu
   355 360 365
   Val Tyr Met Val Gln Gly His Ala Arg Val Gln Val Val Ser Asn Leu
   370 375 380
   Gly Lys Thr Val Phe Asn Ser Val Leu Arg Pro Gly Gln Leu Leu Ile
   385 390 395 400
   Ile Pro Gln His Tyr Val Val Leu Lys Lys Ala Glu Arg Glu Gly Cys
   405 410 415
   Gln Tyr Ile Ala Phe Lys Thr Asn Ala Asn Ser Ile Val Ser Gln Leu
   420 425 430
   Ala Gly Lys Asn Ser Ile Leu Arg Ala Met Pro Val Asp Val Val Ala
   435 440 445
   Asn Ala Tyr Arg Ile Ser Arg Glu Gln Ala Arg Asp Leu Lys Asn Asn
   450 455 460
   Arg Gly Glu Glu Leu Gly Ala Phe Thr Pro Lys Phe Glu Gln Gln Ser
   465 470 475 480
   Tyr Pro Gly Leu Ser Asn Glu Ser Glu Ser Glu Ala Ser Glu
   485 490
<210> 541
   <211> 500
   <212> PRT
   <213> Zizania latifolia
<400> 541
   Met Asn Met Ala Thr Ile Asn Gly Pro Thr Ile Phe Phe Thr Val Cys
   1               5                   10                  15
   Leu Phe Leu Leu Cys His Gly Ser Leu Ala Gln Leu Leu Gly Gln Ser
               20                  25                  30
   Thr Ser Gln Trp Gln Ser Ser His Arg Gly Ser Ser Arg Gln Cys Arg
           35                  40                  45
   Phe Asp Arg Leu Gln Ala Phe Glu Pro Val Arg Ser Val Arg Ser Gln
       50                  55                  60
   Ala Gly Thr Thr Glu Phe Phe Asp Ala Ser Asn Glu Leu Phe Gln Cys
   65                  70                  75                  80
   Ala Gly Val Ser Ile Val Arg Arg Ile Ile Glu Pro Arg Gly Leu Leu
                   85                  90                  95
   Leu Pro Gln Tyr Thr Asn Gly Ala Thr Ile Met Tyr Ile Ile Gln Gly
               100                 105                 110
   Arg Gly Ile Thr Gly Gln Thr Phe Pro Gly Cys Pro Glu Ser Tyr Gln
           115                 120                 125
   Gln Gln Phe Gln Gln Ser Met Gln Ala Gln Leu Thr Gly Ser Gln Ser
       130                 135                 140
   Gln Ser Gln Lys Phe Lys Asp Glu His Gln Lys Ile Asn Arg Phe Arg
   145                 150                 155                 160
   Gln Gly Asp Val Ile Ala Leu Pro Ala Gly Val Ala His Trp Cys Tyr
                   165                 170                 175
   Asn Asp Gly Glu Val Pro Val Val Ala Ile Tyr Val Ile Asp Ile Asn
               180                 185                 190
   Asn Ala Ala Asn Gln Leu Asp Pro Arg Gln Arg Asp Phe Leu Leu Ala
           195                 200                 205
   Gly Asn Met Arg Ser Pro Gln Ala Tyr Arg Arg Glu Val Glu Asn Gln
   210 215 220
   Ser Gln Asn Ile Phe Ser Gly Phe Ser Ala Glu Leu Leu Ser Glu Ala
   225 230 235 240
   Leu Gly Ile Ser Thr Gly Val Ala Arg Gln Leu Gln Cys Gln Asn Asp
   245 250 255
   Gln Arg Gly Glu Ile Val Arg Val Glu His Gly Leu Ser Leu Leu Gln
   260 265 270
   Pro Tyr Ala Ser Leu Gln Glu Gln Glu Gln Lys Gln Glu Gln Pro Arg
   275 280 285
   Glu Arg Tyr Gln Val Thr Gln His Gln Gln Ser Gln Tyr Gly Gly Gly
   290 295 300
   Cys Ser Asn Gly Leu Asp Glu Thr Phe Cys Ala Met Arg Ile Trp Gln
   305 310 315 320
   Asn Ile Asp Asn Pro Asn Leu Ala Asp Thr Tyr Asn Pro Arg Ala Gly
   325 330 335
   Arg Val Thr Asn Leu Asn Ser Gln Lys Phe Pro Ile Leu Asn Leu Ile
   340 345 350
   Gln Met Ser Ala Val Lys Val Asn Leu Tyr Gln Asn Ala Leu Leu Ser
   355 360 365
   Pro Phe Trp Asn Ile Asn Ser His Ser Val Val Tyr Val Thr Gln Gly
   370 375 380
   Cys Ala Arg Val Gln Val Val Asn Asn Asn Gly Lys Thr Val Phe Asn
   385 390 395 400
   Gly Glu Leu Arg Arg Gly Gln Leu Leu Ile Ile Pro Gln His Tyr Val
   405 410 415
   Val Val Lys Lys Ala Gln Arg Glu Gly Cys Ala Tyr Ile Ala Phe Lys
   420 425 430
   Thr Asn Pro Asn Ser Met Val Ser His Ile Val Gly Lys Ser Ser Ile
   435 440 445
   Phe Arg Ala Leu Pro Thr Asp Val Leu Ala Asn Ala Tyr Arg Ile Ser
   450 455 460
   Arg Glu Asp Ala Gln Arg Leu Lys His Asn Arg Gly Asp Glu Leu Gly
   465 470 475 480
   Ala Phe Thr Pro Leu Gln Tyr Lys Ser Tyr Gln Asp Val Ser Ser Val
   485 490 495
   Ala Ala Ser Ser
   500
<210> 542
   <211> 197
   <212> PRT
   <213> Zea mays
<400> 542
   Met Ala Lys Ile Ala Ala Ala Ala Ala Ala Ala Ala Ala Leu Cys Phe
   1 5 10 15
   Ala Ala Leu Val Ala Val Ala Val Cys Gln Gly Glu Val Glu Arg Gln
   20 25 30
   Arg Leu Arg Asp Leu Gln Cys Trp Gln Glu Val Gln Glu Ser Pro Leu
   35 40 45
   Asp Ala Cys Arg Gln Val Leu Asp Arg Gln Leu Thr Gly Gly Gly Val
   50 55 60
   Gly Gly Pro Phe Arg Trp Gly Thr Gly Leu Arg Met Arg Cys Cys Gln
   65 70 75 80
   Gln Leu Gln Asp Val Ser Arg Glu Cys Arg Cys Ala Ala Ile Arg Ser
   85 90 95
   Met Val Arg Gly Tyr Glu Glu Ala Met Pro Pro Leu Glu Lys Gly Trp
   100 105 110
   Trp Pro Trp Gly Arg Gln Gln Gln Pro Pro Pro Gln Gly Gly Gly Gly
   115 120 125
   Gly Gln Gly Gly Tyr Tyr Tyr Pro Cys Ser Arg Ala Gly Glu Gly Tyr
   130 135 140
   Gln Thr Gln Met Tyr Pro Pro Cys Arg Pro Gly Thr Thr Gly Pro Arg
   145 150 155 160
   Ile Gly Arg Val Arg Leu Thr Lys Ala Arg Glu Tyr Ala Ala Gly Leu
   165 170 175
   Pro Met Met Cys Arg Leu Ser Glu Pro Gln Glu Cys Ser Ile Phe Ser
   180 185 190
   Gly Gly Asp Gln Tyr
   195
<210> 543
   <211> 192
   <212> PRT
   <213> Sorghum bicolor
<400> 543
   Met Ala Lys Ile Ala Ala Val Ala Ala Thr Ala Ala Leu Cys Leu Ala
   1 5 10 15
   Ala Leu Val Ala Val Ala Val Gly Gln Gly Val Val Glu Arg Gln Arg
   20 25 30
   Leu Lys Asp Leu Gln Cys Trp Gln Glu Val Gln Glu Asn Pro Leu Gly
   35 40 45
   Ala Cys Arg Gln Val Leu Asp Arg Gln Leu Thr Gly Gly Met Arg Tyr
   50 55 60
   Gly Ile Gly Pro Phe Arg Trp Gly Thr Gly Leu Arg Met Arg Cys Cys
   65 70 75 80
   Gln Gln Leu Gln Asp Val Ser Arg Glu Cys Arg Cys Ala Ala Ile Arg
   85 90 95
   Ser Met Val Arg Gly Tyr Glu Glu Thr Met Pro Pro Leu Glu Lys Gly
   100 105 110
   Trp Trp Gly Gln Gln Pro Gln Pro Gly Tyr Asp Tyr Pro Cys Ser Gln
   115 120 125
   Ala Gly Glu Gly Tyr Gly Tyr Gly Glu Ser Gly Gln Gln Gln Met Tyr
   130 135 140
   Pro Pro Cys Arg Pro Gly Thr Gly Gln Lys Ile Ala Arg Val Lys Leu
   145 150 155 160
   Thr Lys Ala Arg Gln Tyr Ala Ala Gly Met Pro Met Met Cys Arg Leu
   165 170 175
   Ser Glu Pro Gln Glu Cys Ser Val Phe Ser Gly Gly Asp Gln Tyr Tyr
   180 185 190
<210> 544
   <211> 177
   <212> PRT
   <213> Setaria italica
<400> 544
   Met Ala Lys Phe Val Val Ala Ala Ala Thr Ala Ala Leu Cys Leu Ala
   1               5                   10                  15
   Ala Leu Val Ala Met Ala Ala Gly Gln Ser Gly Phe Glu Arg Gln Arg
               20                  25                  30
   Leu Arg Asp Leu Arg Cys Gln Arg Glu Val Glu Glu Asn Pro Leu Trp
           35                  40                  45
   Ala Cys Arg Gln Val Leu Asp Arg Gln Leu Thr Gly Gly Met Arg Tyr
       50                  55                  60
   Gly Val Gly Pro Phe Arg Trp Gly Thr Gly Leu Arg Met Arg Cys Cys
   65                  70                  75                  80
   Gln Gln Leu Gln Asp Val Ser Arg Glu Cys Arg Cys Ser Ala Val Arg
                   85                  90                  95
   Arg Met Val Arg Gly Tyr Glu Glu Ala Met Pro Pro Leu Glu Glu Gly
               100                 105                 110
   Pro Tyr Gly Tyr Gly Gly Glu Gln Gly Glu Gly Tyr Tyr Gly Gly Gly
           115                 120                 125
   Glu Gly Gly Glu Gly Tyr Leu Pro Phe Pro Pro Arg Arg Ile Gly Arg
       130                 135                 140
   Val Arg Leu Thr Lys Ala Arg Gln Tyr Ala Ala Gly Leu Pro Met Met
   145                 150                 155                 160
   Cys Arg Leu Glu Pro Gln Glu Cys Ser Val Phe Ser Gly Asp Gln Tyr
                   165                 170                 175
   Lys
<210> 545
   <211> 336
   <212> PRT
   <213> Staphylococcus aureus
<400> 545
   Met Lys Gly Lys Phe Leu Lys Val Ser Ser Leu Phe Val Ala Thr Leu
   1               5                   10                  15
   Thr Thr Ala Thr Leu Val Ser Ser Pro Ala Ala Asn Ala Leu Ser Ser
               20                  25                  30
   Lys Ala Met Asp Asn His Pro Gln Gln Ser Gln Ser Ser Lys Gln Gln
           35                  40                  45
   Thr Pro Lys Ile Gln Lys Gly Gly Asn Leu Lys Pro Leu Glu Gln Arg
       50                  55                  60
   Glu His Ala Asn Val Ile Leu Pro Asn Asn Asp Arg His Gln Ile Thr
   65                  70                  75                  80
   Asp Thr Thr Asn Gly His Tyr Ala Pro Val Thr Tyr Ile Gln Val Glu
                   85                  90                  95
   Ala Pro Thr Gly Thr Phe Ile Ala Ser Gly Val Val Val Gly Lys Asp
               100                 105                 110
   Thr Leu Leu Thr Asn Lys His Val Val Asp Ala Thr His Gly Asp Pro
           115                 120                 125
   His Ala Leu Lys Ala Phe Pro Ser Ala Ile Asn Gln Asp Asn Tyr Pro
       130                 135                 140
   Asn Gly Gly Phe Thr Ala Glu Gln Ile Thr Lys Tyr Ser Gly Glu Gly
   145                 150                 155                 160
   Asp Leu Ala Ile Val Lys Phe Ser Pro Asn Glu Gln Asn Lys His Ile
                   165                 170                 175
   Gly Glu Val Val Lys Pro Ala Thr Met Ser Asn Asn Ala Glu Thr Gln
               180                 185                 190
   Val Asn Gln Asn Ile Thr Val Thr Gly Tyr Pro Gly Asp Lys Pro Val
           195                 200                 205
   Ala Thr Met Trp Glu Ser Lys Gly Lys Ile Thr Tyr Leu Lys Gly Glu
       210                 215                 220
   Ala Met Gln Tyr Asp Leu Ser Thr Thr Gly Gly Asn Ser Gly Ser Pro
   225                 230                 235                 240
   Val Phe Asn Glu Lys Asn Glu Val Ile Gly Ile His Trp Gly Gly Val
                   245                 250                 255
   Pro Asn Glu Phe Asn Gly Ala Val Phe Ile Asn Glu Asn Val Arg Asn
   260 265 270
   Phe Leu Lys Gln Asn Ile Glu Asp Ile His Phe Ala Asn Asp Asp Gln
   275 280 285
   Pro Asn Asn Pro Asp Asn Pro Asp Asn Pro Asn Asn Pro Asp Asn Pro
   290 295 300
   Asn Asn Pro Asp Glu Pro Asn Asn Pro Asp Asn Pro Asn Asn Pro Asp
   305 310 315 320
   Asn Pro Asp Asn Gly Asp Asn Asn Asn Ser Asp Asn Pro Asp Ala Ala
   325 330 335
<210> 546
   <211> 11
   <212> PRT
   <213> Artificial Sequence
<220>
   <223> PEPTIDE
<400> 546
   Ser Arg Ala Ile Val Ile Val Thr Val Asn Glu
   1 5 10
<210> 547
   <211> 8
   <212> PRT
   <213> Artificial Sequence
<220>
   <223> PEPTIDE
<400> 547
   Ala Lys Leu Thr Pro Gly Asp Val
   1 5
<210> 548
   <211> 10
   <212> PRT
   <213> Artificial Sequence
<220>
   <223> PEPTIDE
<400> 548
   Ile Val Ile Val Thr Val Asn Glu Gly Lys
   1 5 10
<210> 549
   <211> 16
   <212> PRT
   <213> Artificial Sequence
<220>
   <223> PEPTIDE
<400> 549
   Leu Asp Ala Leu Glu Pro Asp Asn Arg Ile Glu Ser Glu Gly Gly Leu
   1 5 10 15
<210> 550
   <211> 10
   <212> PRT
   <213> Artificial Sequence
<220>
   <223> PEPTIDE
<400> 550
   Arg Pro Tyr Tyr Ser Asn Ala Pro Gln Glu
   1 5 10
<210> 551
   <211> 25
   <212> PRT
   <213> Artificial Sequence
<220>
   <223> PEPTIDE
<400> 551
   Leu Asp Ala Leu Glu Pro Asp Asn Arg Ile Glu Ser Glu Gly Gly Leu
   1 5 10 15
   Ile Glu Thr Trp Asn Pro Asn Asn Lys
   20 25
<210> 552
   <211> 11
   <212> PRT
   <213> Artificial Sequence
<220>
   <223> PEPTIDE
<400> 552
   Ala Ile Val Ile Val Thr Val Asn Glu Gly Lys
   1 5 10
<210> 553
   <211> 16
   <212> PRT
   <213> Artificial Sequence
<220>
   <223> PEPTIDE
<400> 553
   Leu Gln Val Val Asn Cys Asn Gly Asn Thr Val Phe Asp Gly Glu Leu
   1 5 10 15
<210> 554
   <211> 15
   <212> PRT
   <213> Artificial Sequence
<220>
   <223> PEPTIDE
<400> 554
   Gln Val Val Asn Cys Asn Gly Asn Thr Val Phe Asp Gly Glu Leu
   1 5 10 15
<210> 555
   <211> 10
   <212> PRT
   <213> Artificial Sequence
<220>
   <223> peptide
<400> 555
   Ile Ile Ala Val Pro Thr Gly Ile Val Phe
   1 5 10
<210> 556
   <211> 16
   <212> PRT
   <213> Artificial Sequence
<220>
   <223> PEPTIDE
<400> 556
   Gly Arg Arg Tyr Arg Asp Arg His Gln Lys Val Asn Arg Phe Arg Glu
   1 5 10 15
<210> 557
   <211> 11
   <212> PRT
   <213> Artificial Sequence
<220>
   <223> PEPTIDE
<400> 557
   Arg Pro Tyr Tyr Ser Asn Ala Pro Gln Glu Ile
   1 5 10
<210> 558
   <211> 12
   <212> PRT
   <213> Artificial Sequence
<220>
   <223> PEPTIDE
<400> 558
   Arg Leu Asp Ala Leu Glu Pro Asp Asn Arg Ile Glu
   1 5 10
<210> 559
   <211> 14
   <212> PRT
   <213> Artificial Sequence
<220>
   <223> PEPTIDE
<400> 559
   Arg Leu Asp Ala Leu Glu Pro Asp Asn Arg Ile Glu Ser Glu
   1 5 10
<210> 560
   <211> 20
   <212> PRT
   <213> Artificial Sequence
<220>
   <223> PEPTIDE
<400> 560
   Leu Asp Ala Leu Glu Pro Asp Asn Arg Ile Glu Ser Glu Gly Gly Leu
   1 5 10 15
   Ile Glu Thr Trp
   20
<210> 561
   <211> 11
   <212> PRT
   <213> Artificial Sequence
<220>
   <223> PEPTIDE
<400> 561
   Leu Asp Ala Leu Glu Pro Asp Asn Arg Ile Glu
   1 5 10
<210> 562
   <211> 18
   <212> PRT
   <213> Artificial Sequence
<220>
   <223> PEPTIDE
<400> 562
   Leu Asp Ala Leu Glu Pro Asp Asn Arg Ile Glu Ser Glu Gly Gly Leu
   1 5 10 15
   Ile Glu
<210> 563
   <211> 16
   <212> PRT
   <213> Artificial Sequence
<220>
   <223> PEPTIDE
<400> 563
   Leu Asp Ala Leu Glu Pro Asp Asn Arg Ile Glu Ser Glu Gly Gly Leu
   1 5 10 15
<210> 564
   <211> 10
   <212> PRT
   <213> Artificial Sequence
<220>
   <223> PEPTIDE
<400> 564
   Arg Pro Tyr Tyr Ser Asn Ala Pro Gln Glu
   1 5 10
<210> 565
   <211> 25
   <212> PRT
   <213> Artificial Sequence
<220>
   <223> PEPTIDE
<400> 565
   Leu Asp Ala Leu Glu Pro Asp Asn Arg Ile Glu Ser Glu Gly Gly Leu
   1               5                   10                  15
   Ile Glu Thr Trp Asn Pro Asn Asn Lys
               20                  25
<210> 566
   <211> 27
   <212> PRT
   <213> Artificial Sequence
<220>
   <223> PEPTIDE
<400> 566
   Val Glu His Gly Leu Ser Leu Leu Gln Pro Tyr Ala Ser Leu Gln Glu
   1               5                   10                  15
   Gln Glu Gln Gly Gln Val Gln Ser Arg Glu Arg
               20                  25
<210> 567
   <211> 9
   <212> PRT
   <213> Artificial Sequence
<220>
   <223> PEPTIDE
<400> 567
   Arg Ser Gln Asn Ile Phe Ser Gly Phe
   1               5
<210> 568
   <211> 14
   <212> PRT
   <213> Artificial Sequence
<220>
   <223> PEPTIDE
<400> 568
   Gly Ile Thr Gly Pro Thr Phe Pro Gly Cys Pro Glu Ser Tyr
   1               5                   10
<210> 569
   <211> 4
   <212> PRT
   <213> Artificial Sequence
<220>
   <223> PEPTIDE
<400> 569
   Cys Asn Gly Ser
   1
<210> 570
   <211> 5
   <212> PRT
   <213> Artificial Sequence
<220>
   <223> PEPTIDE
<400> 570
   Ser Pro Arg Glu Cys
   1 5
<210> 571
   <211> 4
   <212> PRT
   <213> Artificial Sequence
<220>
   <223> PEPTIDE
<400> 571
   Pro Arg Glu Cys
   1
<210> 572
   <211> 5
   <212> PRT
   <213> Artificial Sequence
<220>
   <223> PEPTIDE
<400> 572
   Pro Arg Glu Cys Arg
   1 5
<210> 573
   <211> 4
   <212> PRT
   <213> Artificial Sequence
<220>
   <223> PEPTIDE
<400> 573
   Cys Pro Glu Ser
   1
<210> 574
   <211> 4
   <212> PRT
   <213> Artificial Sequence
<220>
   <223> PEPTIDE
<400> 574
   Ser Gly Cys Ser
   1
<210> 575
   <211> 4
   <212> PRT
   <213> Artificial Sequence
<220>
   <223> PEPTIDE
<400> 575
   Cys Ser Asn Gly
   1
<210> 576
   <211> 12
   <212> PRT
   <213> Artificial Sequence
<220>
   <223> PEPTIDE
<400> 576
   Arg Ser Gln Asn Ile Phe Ser Gly Phe Ser Thr Glu
   1               5                   10
<210> 577
   <211> 14
   <212> PRT
   <213> Artificial Sequence
<220>
   <223> PEPTIDE
<400> 577
   Val Glu Glu Trp Ser Gln Asn Ile Phe Ser Gly Phe Ser Thr
   1               5                   10
<210> 578
   <211> 13
   <212> PRT
   <213> Artificial Sequence
<220>
   <223> PEPTIDE
<400> 578
   Trp Ser Gln Asn Ile Phe Ser Gly Phe Ser Thr Glu Leu
   1               5                   10
<210> 579
   <211> 12
   <212> PRT
   <213> Artificial Sequence
<220>
   <223> PEPTIDE
<400> 579
   Trp Ser Gln Asn Ile Phe Ser Gly Phe Ser Thr Glu
   1               5                   10
<210> 580
   <211> 10
   <212> PRT
   <213> Artificial Sequence
<220>
   <223> PEPTIDE
<400> 580
   Ser Thr Ser Gln Trp Gln Ser Ser Arg Arg
   1               5                   10
<210> 581
   <211> 4
   <212> PRT
   <213> Artificial Sequence
<220>
   <223> PEPTIDE
<400> 581
   Asn Arg Pro Ile
   1
<210> 582
   <211> 4
   <212> PRT
   <213> Artificial Sequence
<220>
   <223> PEPTIDE
<400> 582
   Cys Asp Gly Ser
   1
<210> 583
   <211> 4
   <212> PRT
   <213> Artificial Sequence
<220>
   <223> PEPTIDE
<400> 583
   Pro Arg Gly Cys
   1
<210> 584
   <211> 5
   <212> PRT
   <213> Artificial Sequence
<220>
   <223> PEPTIDE
<400> 584
   Pro Arg Gly Cys Arg
   1 5
<210> 585
   <211> 4
   <212> PRT
   <213> Artificial Sequence
<220>
   <223> PEPTIDE
<400> 585
   Arg Gly Cys Arg
   1
<210> 586
   <211> 4
   <212> PRT
   <213> Artificial Sequence
<220>
   <223> PEPTIDE
<400> 586
   Gly Cys Arg Phe
   1
<210> 587
   <211> 4
   <212> PRT
   <213> Artificial Sequence
<220>
   <223> PEPTIDE
<400> 587
   Pro Thr Phe Pro
   1
<210> 588
   <211> 5
   <212> PRT
   <213> Artificial Sequence
<220>
   <223> PEPTIDE
<400> 588
   Pro Gly Cys Pro Glu
   1 5
<210> 589
   <211> 4
   <212> PRT
   <213> Artificial Sequence
<220>
   <223> PEPTIDE
<400> 589
   Gly Cys Pro Glu
   1
<210> 590
   <211> 4
   <212> PRT
   <213> Artificial Sequence
<220>
   <223> PEPTIDE
<400> 590
   Cys Pro Glu Thr
   1
<210> 591
   <211> 4
   <212> PRT
   <213> Artificial Sequence
<220>
   <223> PEPTIDE
<400> 591
   Ala His Trp Cys
   1
<210> 592
   <211> 4
   <212> PRT
   <213> Artificial Sequence
<220>
   <223> PEPTIDE
<400> 592
   His Trp Cys Tyr
   1
<210> 593
   <211> 4
   <212> PRT
   <213> Artificial Sequence
<220>
   <223> PEPTIDE
<400> 593
   Ser Gly Cys Pro
   1
<210> 594
   <211> 5
   <212> PRT
   <213> Artificial Sequence
<220>
   <223> PEPTIDE
<400> 594
   Ser Gly Cys Pro Asn
   1               5
<210> 595
   <211> 4
   <212> PRT
   <213> Artificial Sequence
<220>
   <223> PEPTIDE
<400> 595
   Gly Cys Pro Asn
   1
<210> 596
   <211> 4
   <212> PRT
   <213> Artificial Sequence
<220>
   <223> PEPTIDE
<400> 596
   Cys Pro Asn Gly
   1
<210> 597
   <211> 5
   <212> PRT
   <213> Artificial Sequence
<220>
   <223> PEPTIDE
<400> 597
   Thr Phe Cys Thr Met
   1 5
<210> 598
   <211> 4
   <212> PRT
   <213> Artificial Sequence
<220>
   <223> PEPTIDE
<400> 598
   Phe Cys Thr Met
   1
<210> 599
   <211> 5
   <212> PRT
   <213> Artificial Sequence
<220>
   <223> PEPTIDE
<400> 599
   Phe Cys Thr Met Arg
   1 5
<210> 600
   <211> 4
   <212> PRT
   <213> Artificial Sequence
<220>
   <223> PEPTIDE
<400> 600
   Cys Thr Met Arg
   1
<210> 601
   <211> 4
   <212> PRT
   <213> Artificial Sequence
<220>
   <223> PEPTIDE
<400> 601
   Glu Gly Cys Ala
   1
<210> 602
   <211> 13
   <212> PRT
   <213> Artificial Sequence
<220>
   <223> PEPTIDE
<400> 602
   Ser Gln Asn Ile Phe Ser Gly Phe Ser Thr Glu Leu Leu
   1 5 10
<210> 603
   <211> 11
   <212> PRT
   <213> Artificial Sequence
<220>
   <223> PEPTIDE
<400> 603
   Ser Gln Asn Ile Phe Ser Gly Phe Ser Thr Glu
   1 5 10
<210> 604
   <211> 10
   <212> PRT
   <213> Artificial Sequence
<220>
   <223> PEPTIDE
<400> 604
   Gln Asn Asp Gln Arg Gly Glu Ile Val Arg
   1 5 10
<210> 605
   <211> 12
   <212> PRT
   <213> Artificial Sequence
<220>
   <223> PEPTIDE
<400> 605
   Ser Gln Asn Ile Phe Ser Gly Phe Ser Thr Glu Leu
   1               5                   10
<210> 606
   <211> 12
   <212> PRT
   <213> Artificial Sequence
<220>
   <223> PEPTIDE
<400> 606
   Gln Leu Gln Cys Gln Asn Asp Gln Arg Gly Glu Ile
   1               5                   10
<210> 607
   <211> 12
   <212> PRT
   <213> Artificial Sequence
<220>
   <223> PEPTIDE
<400> 607
   Leu Gly Gln Ser Thr Ser Gln Trp Gln Ser Ser Arg
   1               5                   10
<210> 608
   <211> 15
   <212> PRT
   <213> Artificial Sequence
<220>
   <223> PEPTIDE
<400> 608
   Gln Gln Leu Leu Gly Gln Ser Thr Ser Gln Trp Gln Ser Ser Arg
   1               5                   10                  15
<210> 609
   <211> 13
   <212> PRT
   <213> Artificial Sequence
<220>
   <223> PEPTIDE
<400> 609
   Leu Leu Gly Gln Ser Thr Ser Gln Trp Gln Ser Ser Arg
   1               5                   10
<210> 610
   <211> 9
   <212> PRT
   <213> Artificial Sequence
<220>
   <223> PEPTIDE
<400> 610
   Asn Asp Gln Arg Gly Glu Ile Val Arg
   1 5
<210> 611
   <211> 11
   <212> PRT
   <213> Artificial Sequence
<220>
   <223> PEPTIDE
<400> 611
   Gly Gln Ser Thr Ser Gln Trp Gln Ser Ser Arg
   1 5 10
<210> 612
   <211> 9
   <212> PRT
   <213> Artificial Sequence
<220>
   <223> PEPTIDE
<400> 612
   Ser Thr Ser Gln Trp Gln Ser Ser Arg
   1 5
<210> 613
   <211> 13
   <212> PRT
   <213> Artificial Sequence
<220>
   <223> PEPTIDE
<400> 613
   Gly Ile Thr Gly Pro Thr Phe Pro Gly Cys Pro Glu Thr
   1 5 10
<210> 614
   <211> 14
   <212> PRT
   <213> Artificial Sequence
<220>
   <223> PEPTIDE
<400> 614
   Gly Ile Thr Gly Pro Thr Phe Pro Gly Cys Pro Glu Thr Tyr
   1 5 10
<210> 615
   <211> 12
   <212> PRT
   <213> Artificial Sequence
<220>
   <223> PEPTIDE
<400> 615
   Ser Gln Asn Ile Phe Ser Gly Phe Ser Thr Glu Leu
   1 5 10
<210> 616
   <211> 12
   <212> PRT
   <213> Artificial Sequence
<220>
   <223> PEPTIDE
<400> 616
   Gln Leu Gln Cys Gln Asn Asp Gln Arg Gly Glu Ile
   1 5 10
<210> 617
   <211> 12
   <212> PRT
   <213> Artificial Sequence
<220>
   <223> PEPTIDE
<400> 617
   Leu Gly Gln Ser Thr Ser Gln Trp Gln Ser Ser Arg
   1 5 10
<210> 618
   <211> 15
   <212> PRT
   <213> Artificial Sequence
<220>
   <223> PEPTIDE
<400> 618
   Gln Gln Leu Leu Gly Gln Ser Thr Ser Gln Trp Gln Ser Ser Arg
   1 5 10 15
<210> 619
   <211> 13
   <212> PRT
   <213> Artificial Sequence
<220>
   <223> PEPTIDE
<400> 619
   Leu Leu Gly Gln Ser Thr Ser Gln Trp Gln Ser Ser Arg
   1               5                   10
<210> 620
   <211> 9
   <212> PRT
   <213> Artificial Sequence
<220>
   <223> PEPTIDE
<400> 620
   Ile Phe Phe Ala Asn Gln Thr Tyr Leu
   1               5
<210> 621
   <211> 14
   <212> PRT
   <213> Artificial Sequence
<220>
   <223> PEPTIDE
<400> 621
   Glu His Leu Glu Pro Asn Leu Glu Gly Leu Thr Val Glu Glu
   1               5                   10
<210> 622
   <211> 36
   <212> PRT
   <213> Artificial Sequence
<220>
   <223> PEPTIDE
<400> 622
   Ile Phe Phe Ala Asn Gln Thr Tyr Leu Pro Ser Glu Thr Pro Ala Pro
   1               5                   10                  15
   Leu Val His Tyr Arg Glu Glu Glu Leu Asn Asn Leu Arg Gly Asp Gly
               20                  25                  30
   Thr Gly Glu Arg
           35
<210> 623
   <211> 18
   <212> PRT
   <213> Artificial Sequence
<220>
   <223> PEPTIDE
<400> 623
   Ile His Phe Glu Trp Asp Asp Asp Met Gly Ile Pro Gly Ala Phe Tyr
   1               5                   10                  15
   Ile Lys
<210> 624
   <211> 29
   <212> PRT
   <213> Artificial Sequence
<220>
   <223> PEPTIDE
<400> 624
   Ile Phe Phe Ala Asn Gln Thr Tyr Leu Pro Ser Glu Thr Pro Ala Pro
   1               5                   10                  15
   Leu Val His Tyr Arg Glu Glu Glu Leu Asn Asn Leu Arg
               20                  25
<210> 625
   <211> 11
   <212> PRT
   <213> Artificial Sequence
<220>
   <223> PEPTIDE
<400> 625
   Thr Glu Gln Ala Leu Pro Ala Asp Leu Ile Lys
   1               5                   10
<210> 626
   <211> 20
   <212> PRT
   <213> Artificial Sequence
<220>
   <223> PEPTIDE
<400> 626
   Glu His Leu Glu Pro Asn Leu Glu Gly Leu Thr Val Glu Glu Ala Ile
   1               5                   10                  15
   Gln Asn Lys Lys
               20
<210> 627
   <211> 23
   <212> PRT
   <213> Artificial Sequence
<220>
   <223> PEPTIDE
<400> 627
   Ile Ser Lys Glu His Leu Glu Pro Asn Leu Glu Gly Leu Thr Val Glu
   1               5                   10                  15
   Glu Ala Ile Gln Asn Lys Lys
               20
<210> 628
   <211> 16
   <212> PRT
   <213> Artificial Sequence
<220>
   <223> PEPTIDE
<400> 628
   Leu Ser Leu Pro His Pro Gln Gly Asp Glu His Gly Ala Val Ser Tyr
   1               5                   10                  15
<210> 629
   <211> 22
   <212> PRT
   <213> Artificial Sequence
<220>
   <223> PEPTIDE
<400> 629
   Ile Ser Lys Glu His Leu Glu Pro Asn Leu Glu Gly Leu Thr Val Glu
   1               5                   10                  15
   Glu Ala Ile Gln Asn Lys
               20
<210> 630
   <211> 19
   <212> PRT
   <213> Artificial Sequence
<220>
   <223> PEPTIDE
<400> 630
   Glu His Leu Glu Pro Asn Leu Glu Gly Leu Thr Val Glu Glu Ala Ile
   1               5                   10                  15
   Gln Asn Lys
<210> 631
   <211> 18
   <212> PRT
   <213> Artificial Sequence
<220>
   <223> PEPTIDE
<400> 631
   Leu Ser Thr Thr Gly Gly Asn Ser Gly Ser Pro Val Phe Asn Glu Lys
   1               5                   10                  15
   Asn Glu
<210> 632
   <211> 17
   <212> PRT
   <213> Artificial Sequence
<220>
   <223> PEPTIDE
<400> 632
   Gln Ser Phe Leu Leu Ser Gly Asn Gln Asn Gln Gln Asn Tyr Leu Ser
   1               5                   10                  15
   Gly
<210> 633
   <211> 16
   <212> PRT
   <213> Artificial Sequence
<220>
   <223> PEPTIDE
<400> 633
   Val Leu Asp Leu Ala Ile Pro Val Asn Arg Pro Gly Gln Leu Gln Ser
   1               5                   10                  15
<210> 634
   <211> 18
   <212> PRT
   <213> Artificial Sequence
<220>
   <223> PEPTIDE
<400> 634
   Val Leu Asp Leu Ala Ile Pro Val Asn Arg Pro Gly Gln Leu Gln Ser
   1 5 10 15
   Phe Leu
<210> 635
   <211> 15
   <212> PRT
   <213> Artificial Sequence
<220>
   <223> PEPTIDE
<400> 635
   Phe Leu Leu Ser Gly Asn Gln Asn Gln Gln Asn Tyr Leu Ser Gly
   1 5 10 15
<210> 636
   <211> 18
   <212> PRT
   <213> Artificial Sequence
<220>
   <223> PEPTIDE
<400> 636
   Phe Leu Leu Ser Gly Asn Gln Asn Gln Gln Asn Tyr Leu Ser Gly Phe
   1 5 10 15
   Ser Lys
<210> 637
   <211> 18
   <212> PRT
   <213> Artificial Sequence
<220>
   <223> PEPTIDE
<400> 637
   Asp Pro Gln Asn Pro Phe Ile Phe Lys Ser Asn Lys Phe Gln Thr Leu
   1 5 10 15
   Phe Glu
<210> 638
   <211> 19
   <212> PRT
   <213> Artificial Sequence
<220>
   <223> PEPTIDE
<400> 638
   Glu Leu Ala Phe Pro Gly Ser Ala Gln Glu Val Asp Arg Ile Leu Glu
   1 5 10 15
   Asn Gln Lys
<210> 639
   <211> 11
   <212> PRT
   <213> Artificial Sequence
<220>
   <223> PEPTIDE
<400> 639
   Ala Ile Val Ile Val Thr Val Asn Glu Gly Lys
   1 5 10
<210> 640
   <211> 18
   <212> PRT
   <213> Artificial Sequence
<220>
   <223> PEPTIDE
<400> 640
   Ile Asn Ala Val Ala Ala Lys Arg Leu Gln Ser Gln Asn Asp Gln Arg
   1 5 10 15
   Gly Glu
<210> 641
   <211> 14
   <212> PRT
   <213> Artificial Sequence
<220>
   <223> PEPTIDE
<400> 641
   Asn Arg Ala Gln Gln Gln Gln Val Tyr Gly Ser Ser Ile Glu
   1               5                   10
<210> 642
   <211> 10
   <212> PRT
   <213> Artificial Sequence
<220>
   <223> PEPTIDE
<400> 642
   Pro Ser Thr Asn Pro Trp His Ser Pro Arg
   1               5                   10
<210> 643
   <211> 4
   <212> PRT
   <213> Artificial Sequence
<220>
   <223> PEPTIDE
<400> 643
   Cys His Gly Ser
   1
<210> 644
   <211> 5
   <212> PRT
   <213> Artificial Sequence
<220>
   <223> PEPTIDE
<400> 644
   Cys His Gly Ser Met
   1               5
<210> 645
   <211> 4
   <212> PRT
   <213> Artificial Sequence
<220>
   <223> PEPTIDE
<400> 645
   Pro Trp His Ser
   1
<210> 646
   <211> 4
   <212> PRT
   <213> Artificial Sequence
<220>
   <223> PEPTIDE
<400> 646
   Phe Arg Glu Cys
   1
<210> 647
   <211> 4
   <212> PRT
   <213> Artificial Sequence
<220>
   <223> PEPTIDE
<400> 647
   Arg Glu Cys Arg
   1
<210> 648
   <211> 4
   <212> PRT
   <213> Artificial Sequence
<220>
   <223> PEPTIDE
<400> 648
   Glu Cys Arg Phe
   1
<210> 649
   <211> 4
   <212> PRT
   <213> Artificial Sequence
<220>
   <223> PEPTIDE
<400> 649
   Cys Arg Phe Asp
   1
<210> 650
   <211> 5
   <212> PRT
   <213> Artificial Sequence
<220>
   <223> PEPTIDE
<400> 650
   Cys Arg Phe Asp Arg
   1 5
<210> 651
   <211> 4
   <212> PRT
   <213> Artificial Sequence
<220>
   <223> PEPTIDE
<400> 651
   Cys Thr Gly Thr
   1
<210> 652
   <211> 4
   <212> PRT
   <213> Artificial Sequence
<220>
   <223> PEPTIDE
<400> 652
   Phe Pro Gly Cys
   1
<210> 653
   <211> 5
   <212> PRT
   <213> Artificial Sequence
<220>
   <223> PEPTIDE
<400> 653
   Phe Pro Gly Cys Pro
   1 5
<210> 654
   <211> 4
   <212> PRT
   <213> Artificial Sequence
<220>
   <223> PEPTIDE
<400> 654
   Pro Gly Cys Pro
   1
<210> 655
   <211> 5
   <212> PRT
   <213> Artificial Sequence
<220>
   <223> PEPTIDE
<400> 655
   Pro Gly Cys Pro Ala
   1 5
<210> 656
   <211> 6
   <212> PRT
   <213> Artificial Sequence
<220>
   <223> PEPTIDE
<400> 656
   Pro Gly Cys Pro Ala Thr
   1 5
<210> 657
   <211> 4
   <212> PRT
   <213> Artificial Sequence
<220>
   <223> PEPTIDE
<400> 657
   Gly Cys Pro Ala
   1
<210> 658
   <211> 4
   <212> PRT
   <213> Artificial Sequence
<220>
   <223> PEPTIDE
<400> 658
   Cys Pro Ala Thr
   1
<210> 659
   <211> 4
   <212> PRT
   <213> Artificial Sequence
<220>
   <223> PEPTIDE
<400> 659
   Glu Asn Phe Cys
   1
<210> 660
   <211> 4
   <212> PRT
   <213> Artificial Sequence
<220>
   <223> PEPTIDE
<400> 660
   Asn Phe Cys Thr
   1
<210> 661
   <211> 5
   <212> PRT
   <213> Artificial Sequence
<220>
   <223> PEPTIDE
<400> 661
   Asn Phe Cys Thr Ile
   1 5
<210> 662
   <211> 4
   <212> PRT
   <213> Artificial Sequence
<220>
   <223> PEPTIDE
<400> 662
   Phe Cys Thr Ile
   1
<210> 663
   <211> 14
   <212> PRT
   <213> Artificial Sequence
<220>
   <223> PEPTIDE
<400> 663
   Ala Gln Gln Gln Gln Val Tyr Gly Ser Ser Ile Glu Gln His
   1 5 10
<210> 664
   <211> 23
   <212> PRT
   <213> Artificial Sequence
<220>
   <223> PEPTIDE
<400> 664
   Ala Gln Gln Gln Gln Val Tyr Gly Ser Ser Ile Glu Gln His Ser Gly
   1               5                   10                  15
   Gln Asn Ile Phe Ser Gly Phe
               20
<210> 665
   <211> 14
   <212> PRT
   <213> Artificial Sequence
<220>
   <223> PEPTIDE
<400> 665
   Ala Ala Lys Arg Leu Gln Ser Gln Asn Asp Gln Arg Gly Glu
   1               5                   10
<210> 666
   <211> 12
   <212> PRT
   <213> Artificial Sequence
<220>
   <223> PEPTIDE
<400> 666
   Gln Ala Arg Ser Leu Lys Asn Asn Arg Gly Glu Glu
   1               5                   10
<210> 667
   <211> 15
   <212> PRT
   <213> Artificial Sequence
<220>
   <223> PEPTIDE
<400> 667
   Phe Asn Pro Ser Thr Asn Pro Trp His Ser Pro Arg Gln Gly Ser
   1               5                   10                  15
<210> 668
   <211> 12
   <212> PRT
   <213> Artificial Sequence
<220>
   <223> PEPTIDE
<400> 668
   Gln Ala Arg Ser Leu Lys Asn Asn Arg Gly Glu Glu
   1 5 10
<210> 669
   <211> 26
   <212> PRT
   <213> Artificial Sequence
<220>
   <223> PEPTIDE
<400> 669
   Ala Ala Ala Ser Leu Pro Ala Phe Cys Asn Val Asp Ile Pro Asn Gly
   1 5 10 15
   Gly Gly Gly Val Cys Tyr Trp Leu Ala Arg
   20 25
<210> 670
   <211> 18
   <212> PRT
   <213> Artificial Sequence
<220>
   <223> PEPTIDE
<400> 670
   Val Ala Gly Ser Ala Pro Gly Ile Glu Gly Asp Glu Ile Ala Pro Leu
   1 5 10 15
   Ala Lys
<210> 671
   <211> 22
   <212> PRT
   <213> Artificial Sequence
<220>
   <223> PEPTIDE
<400> 671
   Leu Gly Val Ala Gly Ser Ala Pro Gly Ile Glu Gly Asp Glu Ile Ala
   1 5 10 15
   Pro Leu Ala Lys Glu Asn
   20
<210> 672
   <211> 17
   <212> PRT
   <213> Artificial Sequence
<220>
   <223> PEPTIDE
<400> 672
   Gly Ser Ala Pro Gly Ile Glu Gly Asp Glu Ile Ala Pro Leu Ala Lys
   1               5                   10                  15
   Glu
<210> 673
   <211> 15
   <212> PRT
   <213> Artificial Sequence
<220>
   <223> PEPTIDE
<400> 673
   Val Ala Gly Ser Ala Pro Gly Ile Glu Gly Asp Glu Ile Ala Pro
   1               5                   10                  15
<210> 674
   <211> 19
   <212> PRT
   <213> Artificial Sequence
<220>
   <223> PEPTIDE
<400> 674
   Gly Val Ala Gly Ser Ala Pro Gly Ile Glu Gly Asp Glu Ile Ala Pro
   1               5                   10                  15
   Leu Ala Lys
<210> 675
   <211> 21
   <212> PRT
   <213> Artificial Sequence
<220>
   <223> PEPTIDE
<400> 675
   Gly Val Ala Gly Ser Ala Pro Gly Ile Glu Gly Asp Glu Ile Ala Pro
   1 5 10 15
   Leu Ala Lys Glu Asn
   20
<210> 676
   <211> 20
   <212> PRT
   <213> Artificial Sequence
<220>
   <223> PEPTIDE
<400> 676
   Val Ala Gly Ser Ala Pro Gly Ile Glu Gly Asp Glu Ile Ala Pro Leu
   1 5 10 15
   Ala Lys Glu Asn
   20
<210> 677
   <211> 15
   <212> PRT
   <213> Artificial Sequence
<220>
   <223> PEPTIDE
<400> 677
   Ser Ala Pro Gly Ile Glu Gly Asp Glu Ile Ala Pro Leu Ala Lys
   1 5 10 15
<210> 678
   <211> 16
   <212> PRT
   <213> Artificial Sequence
<220>
   <223> PEPTIDE
<400> 678
   Gly Ser Ala Pro Gly Ile Glu Gly Asp Glu Ile Ala Pro Leu Ala Lys
   1 5 10 15
<210> 679
   <211> 17
   <212> PRT
   <213> Artificial Sequence
<220>
   <223> PEPTIDE
<400> 679
   Ser Ala Pro Gly Ile Glu Gly Asp Glu Ile Ala Pro Leu Ala Lys Glu
   1               5                   10                  15
   Asn
<210> 680
   <211> 15
   <212> PRT
   <213> Artificial Sequence
<220>
   <223> PEPTIDE
<400> 680
   Phe Asn Pro Ser Thr Asn Pro Trp His Ser Pro Arg Gln Gly Ser
   1               5                   10                  15
<210> 681
   <211> 12
   <212> PRT
   <213> Artificial Sequence
<220>
   <223> PEPTIDE
<400> 681
   Val Asp Leu Val Ile Pro Val Asn Gly Pro Gly Lys
   1               5                   10
<210> 682
   <211> 13
   <212> PRT
   <213> Artificial Sequence
<220>
   <223> PEPTIDE
<400> 682
   Leu Val Asp Leu Val Ile Pro Val Asn Gly Pro Gly Lys
   1               5                   10
<210> 683
   <211> 10
   <212> PRT
   <213> Artificial Sequence
<220>
   <223> PEPTIDE
<400> 683
   Ile Lys Leu Pro Ala Gly Thr Thr Ser Tyr
   1 5 10
<210> 684
   <211> 11
   <212> PRT
   <213> Artificial Sequence
<220>
   <223> PEPTIDE
<400> 684
   Ile Lys Leu Pro Ala Gly Thr Thr Ser Tyr Leu
   1 5 10
<210> 685
   <211> 12
   <212> PRT
   <213> Artificial Sequence
<220>
   <223> PEPTIDE
<400> 685
   Arg Arg Asn Pro Phe Leu Phe Lys Ser Asn Lys Phe
   1 5 10
<210> 686
   <211> 19
   <212> PRT
   <213> Artificial Sequence
<220>
   <223> PEPTIDE
<400> 686
   Ile Glu Asn Pro Val Lys Glu Leu Thr Phe Pro Gly Ser Val Gln Glu
   1 5 10 15
   Ile Asn Arg
<210> 687
   <211> 14
   <212> PRT
   <213> Artificial Sequence
<220>
   <223> PEPTIDE
<400> 687
   Arg Arg Asn Pro Phe Leu Phe Lys Ser Asn Lys Phe Leu Thr
   1 5 10
<210> 688
   <211> 14
   <212> PRT
   <213> Artificial Sequence
<220>
   <223> PEPTIDE
<400> 688
   Ala Lys Pro His Thr Ile Phe Leu Pro Gln His Ile Asp Ala
   1 5 10
<210> 689
   <211> 15
   <212> PRT
   <213> Artificial Sequence
<220>
   <223> PEPTIDE
<400> 689
   Ala Lys Pro His Thr Ile Phe Leu Pro Gln His Ile Asp Ala Asp
   1 5 10 15
<210> 690
   <211> 9
   <212> PRT
   <213> Artificial Sequence
<220>
   <223> PEPTIDE
<400> 690
   Lys Gln Lys Tyr Arg Tyr Gln Arg Glu
   1 5
<210> 691
   <211> 9
   <212> PRT
   <213> Artificial Sequence
<220>
   <223> PEPTIDE
<400> 691
   Lys Gln Lys Tyr Gln Tyr Gln Arg Glu
   1 5
<210> 692
   <211> 4
   <212> PRT
   <213> Artificial Sequence
<220>
   <223> PEPTIDE
<400> 692
   Met Leu Pro His
   1
<210> 693
   <211> 19
   <212> PRT
   <213> Artificial Sequence
<220>
   <223> PEPTIDE
<400> 693
   Arg Arg Asn Pro Phe Leu Phe Lys Ser Asn Lys Phe Leu Thr Leu Phe
   1 5 10 15
   Glu Asn Glu
<210> 694
   <211> 14
   <212> PRT
   <213> Artificial Sequence
<220>
   <223> PEPTIDE
<400> 694
   Pro Phe Leu Phe Lys Ser Asn Lys Phe Leu Thr Leu Phe Glu
   1 5 10
<210> 695
   <211> 20
   <212> PRT
   <213> Artificial Sequence
<220>
   <223> PEPTIDE
<400> 695
   Ser Gln Glu Arg Arg Asn Pro Phe Leu Phe Lys Ser Asn Lys Phe Leu
   1 5 10 15
   Thr Leu Phe Glu
   20
<210> 696
   <211> 17
   <212> PRT
   <213> Artificial Sequence
<220>
   <223> PEPTIDE
<400> 696
   Arg Arg Asn Pro Phe Leu Phe Lys Ser Asn Lys Phe Leu Thr Leu Phe
   1               5                   10                  15
   Glu
<210> 697
   <211> 22
   <212> PRT
   <213> Artificial Sequence
<220>
   <223> PEPTIDE
<400> 697
   Ser Gln Glu Arg Arg Asn Pro Phe Leu Phe Lys Ser Asn Lys Phe Leu
   1               5                   10                  15
   Thr Leu Phe Glu Asn Glu
               20
<210> 698
   <211> 9
   <212> PRT
   <213> Artificial Sequence
<220>
   <223> PEPTIDE
<400> 698
   Leu Thr Phe Pro Gly Ser Val Gln Glu
   1               5
<210> 699
   <211> 13
   <212> PRT
   <213> Artificial Sequence
<220>
   <223> PEPTIDE
<400> 699
   Glu Leu Thr Phe Pro Gly Ser Val Gln Glu Ile Asn Arg
   1               5                   10
<210> 700
   <211> 18
   <212> PRT
   <213> Artificial Sequence
<220>
   <223> PEPTIDE
<400> 700
   Lys Asn Pro Gln Leu Gln Asp Leu Asp Ile Phe Val Asn Tyr Val Glu
   1               5                   10                  15
   Ile Lys
<210> 701
   <211> 24
   <212> PRT
   <213> Artificial Sequence
<220>
   <223> PEPTIDE
<400> 701
   Pro Gly Gln Leu Gln Ser Phe Leu Leu Ser Gly Asn Gln Asn Gln Gln
   1               5                   10                  15
   Asn Tyr Leu Ser Gly Phe Ser Lys
               20
<210> 702
   <211> 14
   <212> PRT
   <213> Artificial Sequence
<220>
   <223> PEPTIDE
<400> 702
   Arg Gly Pro Gln Gln Tyr Ala Glu Trp Gln Ile Asn Glu Lys
   1               5                   10
<210> 703
   <211> 14
   <212> PRT
   <213> Artificial Sequence
<220>
   <223> PEPTIDE
<400> 703
   Val Leu Asp Leu Ala Ile Pro Val Asn Arg Pro Gly Gln Leu
   1               5                   10
<210> 704
   <211> 24
   <212> PRT
   <213> Artificial Sequence
<220>
   <223> PEPTIDE
<400> 704
   Gly Tyr Val Gly Leu Thr Phe Pro Gly Cys Pro Ala Thr His Gln Gln
   1               5                   10                  15
   Gln Phe Gln Leu Phe Glu Gln Arg
               20
<210> 705
   <211> 820
   <212> PRT
   <213> Oryza sativa
<400> 705
   Met Leu Cys Leu Thr Ser Ser Ser Ser Ser Ala Pro Ala Pro Leu Leu
   1               5                   10                  15
   Pro Ser Leu Ala Asp Arg Pro Ser Pro Gly Ile Ala Gly Gly Gly Gly
               20                  25                  30
   Asn Val Arg Leu Ser Val Val Ser Ser Pro Arg Arg Ser Trp Pro Gly
           35                  40                  45
   Lys Val Lys Thr Asn Phe Ser Val Pro Ala Thr Ala Arg Lys Asn Lys
       50                  55                  60
   Thr Met Val Thr Val Val Glu Glu Val Asp His Leu Pro Ile Tyr Asp
   65                  70                  75                  80
   Leu Asp Pro Lys Leu Glu Glu Phe Lys Asp His Phe Asn Tyr Arg Ile
                   85                  90                  95
   Lys Arg Tyr Leu Asp Gln Lys Cys Leu Ile Glu Lys His Glu Gly Gly
               100                 105                 110
   Leu Glu Glu Phe Ser Lys Gly Tyr Leu Lys Phe Gly Ile Asn Thr Val
           115                 120                 125
   Asp Gly Ala Thr Ile Tyr Arg Glu Trp Ala Pro Ala Ala Gln Glu Ala
       130                 135                 140
   Gln Leu Ile Gly Glu Phe Asn Asn Trp Asn Gly Ala Lys His Lys Met
   145                 150                 155                 160
   Glu Lys Asp Lys Phe Gly Ile Trp Ser Ile Lys Ile Ser His Val Asn
                   165                 170                 175
   Gly Lys Pro Ala Ile Pro His Asn Ser Lys Val Lys Phe Arg Phe Arg
               180                 185                 190
   His Gly Gly Gly Ala Trp Val Asp Arg Ile Pro Ala Trp Ile Arg Tyr
           195                 200                 205
   Ala Thr Phe Asp Ala Ser Lys Phe Gly Ala Pro Tyr Asp Gly Val His
       210                 215                 220
   Trp Asp Pro Pro Ala Cys Glu Arg Tyr Val Phe Lys His Pro Arg Pro
   225 230 235 240
   Pro Lys Pro Asp Ala Pro Arg Ile Tyr Glu Ala His Val Gly Met Ser
   245 250 255
   Gly Glu Glu Pro Glu Val Ser Thr Tyr Arg Glu Phe Ala Asp Asn Val
   260 265 270
   Leu Pro Arg Ile Arg Ala Asn Asn Tyr Asn Thr Val Gln Leu Met Ala
   275 280 285
   Ile Met Glu His Ser Tyr Tyr Ala Ser Phe Gly Tyr His Val Thr Asn
   290 295 300
   Phe Phe Ala Val Ser Ser Arg Ser Gly Thr Pro Glu Asp Leu Lys Tyr
   305 310 315 320
   Leu Val Asp Lys Ala His Ser Leu Gly Leu Arg Val Leu Met Asp Val
   325 330 335
   Val His Ser His Ala Ser Asn Asn Val Thr Asp Gly Leu Asn Gly Tyr
   340 345 350
   Asp Val Gly Gln Asn Thr His Glu Ser Tyr Phe His Thr Gly Asp Arg
   355 360 365
   Gly Tyr His Lys Leu Trp Asp Ser Arg Leu Phe Asn Tyr Ala Asn Trp
   370 375 380
   Glu Val Leu Arg Phe Leu Leu Ser Asn Leu Arg Tyr Trp Met Asp Glu
   385 390 395 400
   Phe Met Phe Asp Gly Phe Arg Phe Asp Gly Val Thr Ser Met Leu Tyr
   405 410 415
   His His His Gly Ile Asn Lys Gly Phe Thr Gly Asn Tyr Lys Glu Tyr
   420 425 430
   Phe Ser Leu Asp Thr Asp Val Asp Ala Ile Val Tyr Met Met Leu Ala
   435 440 445
   Asn His Leu Met His Lys Leu Leu Pro Glu Ala Thr Ile Val Ala Glu
   450 455 460
   Asp Val Ser Gly Met Pro Val Leu Cys Arg Pro Val Asp Glu Gly Gly
   465 470 475 480
   Val Gly Phe Asp Phe Arg Leu Ala Met Ala Ile Pro Asp Arg Trp Ile
                   485                 490                 495
   Asp Tyr Leu Lys Asn Lys Glu Asp Arg Lys Trp Ser Met Ser Glu Ile
               500                 505                 510
   Val Gln Thr Leu Thr Asn Arg Arg Tyr Thr Glu Lys Cys Ile Ala Tyr
           515                 520                 525
   Ala Glu Ser His Asp Gln Ser Ile Val Gly Asp Lys Thr Ile Ala Phe
       530                 535                 540
   Leu Leu Met Asp Lys Glu Met Tyr Thr Gly Met Ser Asp Leu Gln Pro
   545                 550                 555                 560
   Ala Ser Pro Thr Ile Asn Arg Gly Ile Ala Leu Gln Lys Met Ile His
                   565                 570                 575
   Phe Ile Thr Met Ala Leu Gly Gly Asp Gly Tyr Leu Asn Phe Met Gly
               580                 585                 590
   Asn Glu Phe Gly His Pro Glu Trp Ile Asp Phe Pro Arg Glu Gly Asn
           595                 600                 605
   Asn Trp Ser Tyr Asp Lys Cys Arg Arg Gln Trp Ser Leu Val Asp Thr
       610                 615                 620
   Asp His Leu Arg Tyr Lys Tyr Met Asn Ala Phe Asp Gln Ala Met Asn
   625                 630                 635                 640
   Ala Leu Glu Glu Glu Phe Ser Phe Leu Ser Ser Ser Lys Gln Ile Val
                   645                 650                 655
   Ser Asp Met Asn Glu Lys Asp Lys Val Ile Val Phe Glu Arg Gly Asp
               660                 665                 670
   Leu Val Phe Val Phe Asn Phe His Pro Asn Lys Thr Tyr Lys Gly Tyr
           675                 680                 685
   Lys Val Gly Cys Asp Leu Pro Gly Lys Tyr Arg Val Ala Leu Asp Ser
       690                 695                 700
   Asp Ala Leu Val Phe Gly Gly His Gly Arg Val Gly His Asp Val Asp
   705                 710                 715                 720
   His Phe Thr Ser Pro Glu Gly Met Pro Gly Val Pro Glu Thr Asn Phe
                   725                 730                 735
   Asn Asn Arg Pro Asn Ser Phe Lys Val Leu Ser Pro Pro Arg Thr Cys
   740 745 750
   Val Ala Tyr Tyr Arg Val Asp Glu Asp Arg Glu Glu Leu Arg Arg Gly
   755 760 765
   Gly Ala Val Ala Ser Gly Lys Ile Val Thr Glu Tyr Ile Asp Val Glu
   770 775 780
   Ala Thr Ser Gly Glu Thr Ile Ser Gly Gly Trp Lys Gly Ser Glu Lys
   785 790 795 800
   Asp Asp Cys Gly Lys Lys Gly Met Lys Phe Val Phe Arg Ser Ser Asp
   805 810 815
   Glu Asp Cys Lys
   820
<210> 706
   <211> 296
   <212> PRT
   <213> Oryza sativa
<400> 706
   Met Asp Arg Tyr Gln Arg Val Glu Arg Pro Arg Pro Glu Ser Ala Ile
   1 5 10 15
   Glu Glu Asn Glu Ile Arg Ile Thr Ala Gln Gly Leu Ile Arg Asn Tyr
   20 25 30
   Val Ser Tyr Ala Thr Ser Leu Leu Gln Asp Arg Arg Ile Lys Glu Ile
   35 40 45
   Val Leu Lys Ala Met Gly Gln Ala Ile Ser Lys Ser Val Ala Val Ala
   50 55 60
   Glu Ile Ile Lys Lys Arg Val Pro Gly Leu Tyr Gln Asp Thr Asn Ile
   65 70 75 80
   Ser Ser Val Ser Ile Thr Asp Val Trp Glu Pro Ile Glu Glu Gly Leu
   85 90 95
   Val Pro Leu Glu Met Thr Arg His Val Ser Met Ile Ser Ile Thr Leu
   100 105 110
   Ser Pro Arg Asp Leu Asp Lys Asn Ser Pro Gly Tyr Gln Thr Pro Val
   115 120 125
   Tyr Val Glu Gln Pro Arg Gln Gln Pro Arg Leu Gln Gln Ala Pro Pro
   130 135 140
   Pro Pro Gln Arg Gln Val Arg Gln Pro Pro Pro Asp Tyr Glu Asp Ser
   145 150 155 160
   Tyr Val Arg Gly Arg Gly Arg Gly Arg Gly Arg Gly Arg Gly Arg Gly
   165 170 175
   Trp Gly Arg Gly Gly Tyr Gly Gly Tyr Gly Gly Tyr Gly Asn Asn Gln
   180 185 190
   Gly Gly Tyr Asn Gln Gly Gly Gly Tyr Tyr Asp Asn Gln Gly Gly Tyr
   195 200 205
   Gly Gly Tyr Asp Asn Gln Gly Gly Tyr Gly Gly Tyr Asp Asn Gln Gly
   210 215 220
   Gly Tyr Gly Gly Gly Gly Tyr Gly Tyr Asn Gln Gly Arg Tyr Gly Asn
   225 230 235 240
   Tyr Gln Glu Asn Gly Gly Tyr Asn Arg Gly Arg Gly Gly Met Arg Gly
   245 250 255
   Arg Gly Asn Trp Asn Tyr Arg Gly Gly Tyr Glu Arg Gly Arg Gly Gly
   260 265 270
   Gly Phe Pro Gly Gly Arg Gly Tyr Gly Gly Arg Gly Arg Gly Arg Met
   275 280 285
   Gly Gly Arg Gly Gly Arg Gly Asn
   290 295
<210> 707
   <211> 184
   <212> PRT
   <213> Oryza sativa
<400> 707
   Met Ala Leu Glu His Leu Gly Arg Arg Asn Val Ala Gly Ser Leu Leu
   1 5 10 15
   Leu Leu Asn Leu Leu Met Tyr Val Phe Leu Leu Gly Phe Ala Gly Trp
   20 25 30
   Ala Leu Asn Ser Ser Ile Lys Asn Ala Gly Ala Asp Val Gly Val Gly
   35 40 45
   Trp Gly Glu Gln Pro Trp Ser Pro Tyr Tyr Arg Gln Ser Ala Trp Phe
   50 55 60
   Ala Ser Arg Phe His Leu Ala Thr Phe Ala Ala Leu Ala Gly Ala Leu
   65 70 75 80
   Gly Val Ala Ala Lys Ala Ser Ala Ala Tyr His Gly Gly Arg Ser Gly
   85 90 95
   Ala Ser Trp Arg Pro Gln Gly Leu Ala Ala Ala Ala Ser Leu Gly Thr
   100 105 110
   Ala Ala Trp Ala Ala Thr Ala Leu Ala Phe Gly Val Ala Cys Arg Glu
   115 120 125
   Ile His Asp Ala Ala Ala Ala Gly Pro Ala Gly Ala Ala Arg Gly Trp
   130 135 140
   Arg Met Arg Ala Leu Glu Gly Leu Thr Val Thr Leu Ala Phe Thr Gln
   145 150 155 160
   Leu Leu Tyr Val Leu Leu Leu His Ala Ala Val Ala Gly Glu Arg Cys
   165 170 175
   Gly Leu Ala Cys Ala Ala Asp Ala
   180
<210> 708
   <211> 483
   <212> PRT
   <213> Pisum sativum
<400> 708
   Met Ala Ile Lys Thr Lys Leu Ser Leu Thr Ile Phe Leu Phe Phe Leu
   1 5 10 15
   Leu Ala Leu Leu Cys Ser Asn Leu Ala Val Gly Arg Lys Glu Lys Asp
   20 25 30
   Pro Glu Leu Thr Thr Cys Lys Asp Gln Cys Asp Met Gln Arg Gln Tyr
   35 40 45
   Asp Glu Glu Asp Lys Arg Ile Cys Met Glu Arg Cys Asp Asp Tyr Ile
   50 55 60
   Lys Lys Lys Gln Glu Arg Gln Lys His Lys Glu His Glu Glu Glu Glu
   65 70 75 80
   Glu Gln Glu Gln Glu Glu Asp Glu Asn Pro Tyr Val Phe Glu Asp Asn
   85 90 95
   Asp Phe Glu Thr Lys Ile Asp Thr Lys Asp Gly Arg Val Leu Ile Leu
   100 105 110
   Asn Lys Phe Asn Glu Lys Ser Lys Leu Leu Lys Asn Ile Glu Asn Tyr
   115 120 125
   Gly Leu Ala Val Leu Glu Ile Lys Ala Asn Ala Phe Leu Ser Pro His
   130 135 140
   His Tyr Asp Ser Glu Ala Ile Leu Phe Asn Ile Lys Gly Arg Gly Ile
   145 150 155 160
   Ile Gly Leu Val Ala Glu Asp Arg Thr Glu Arg Phe Asn Leu Glu Glu
   165 170 175
   Gly Asp Ile Met Arg Val Pro Ala Gly Thr Pro Met Tyr Leu Val Asn
   180 185 190
   Arg Asp Glu Asn Glu Lys Leu Tyr Ile Ala Ala Phe His Met Pro Pro
   195 200 205
   Ser Ser Gly Ser Ala Pro Val Asn Leu Glu Pro Phe Phe Glu Ser Ala
   210 215 220
   Gly Arg Lys Pro Glu Ser Val Leu Asn Thr Phe Ser Ser Lys Val Leu
   225 230 235 240
   Gln Ala Ala Leu Lys Ser Ser Lys Gly Glu Leu Glu Thr Val Leu Asp
   245 250 255
   Glu Gln Lys Lys Gly Arg Ile Phe Lys Ile Glu Lys Glu Asp Val Arg
   260 265 270
   Gly Leu Ala Pro Lys Lys Ser Leu Trp Pro Phe Gly Gly Pro Phe Lys
   275 280 285
   Ser Pro Phe Asn Ile Phe Ser Asn Asn Pro Ala Phe Ser Asn Lys Phe
   290 295 300
   Gly Ser Leu Phe Glu Val Gly Pro Ser Gln Glu Lys Ser Gly Leu Glu
   305 310 315 320
   Gly Leu Asn Leu Met Leu Thr Leu Ala Asn Ile Thr Lys Gly Ser Met
   325 330 335
   Ser Thr Ile His Tyr Asn Thr Asn Ala Asn Lys Ile Ala Leu Val Ile
   340 345 350
   Asp Gly Glu Gly Glu Leu Glu Met Ala Cys Pro His Met Pro Ser Ser
   355 360 365
   Ser Ser Asn Ser Arg Gln Lys Lys Ser Ser Ile Ser Tyr His Asn Ile
   370 375 380
   Asn Ala Lys Leu Arg Pro Gly Val Met Phe Val Val Pro Ala Gly His
   385 390 395 400
   Pro Phe Val Asn Ile Ala Ser Lys Lys Lys Asn Leu Ile Val Val Cys
   405 410 415
   Phe Glu Val Asn Ala Gln Arg Asn Lys Lys Leu Ala Leu Ala Gly Lys
   420 425 430
   Lys Asn Ile Val Ser Ala Leu Asp Lys Ala Ala Lys Glu Val Ala Phe
   435 440 445
   Asp Ile Ala Ala Glu Lys Val Asp Glu Val Phe Glu Arg Lys Glu Glu
   450 455 460
   Phe Phe Phe Pro Tyr Asp Asn Glu Glu Arg Lys Glu Glu His Gly Arg
   465 470 475 480
   Ala Val Val
<210> 709
   <211> 197
   <212> PRT
   <213> Pisum sativum
<400> 709
   Met Phe Arg Arg Ala Thr Ser Thr Phe Leu Ser Arg Ala Ser Ala Thr
   1               5                   10                  15
   Arg Arg Phe Ser Thr Asp Val Ala Thr Pro Ala Thr Asn Ser Ser Phe
               20                  25                  30
   Val Glu Ala Trp Arg Lys Val Ser Pro Asn Ile Asp Pro Pro Lys Thr
           35                  40                  45
   Pro Leu Glu Phe Leu Lys Thr Arg Pro Pro Val Pro Ser Thr Ile Pro
       50                  55                  60
   Thr Lys Leu Thr Val Asn Phe Val Leu Pro Tyr Ser Ser Gln Leu Ala
   65                  70                  75                  80
   Ala Lys Glu Val Asp Ser Val Ile Ile Pro Ala Thr Thr Gly Glu Met
                   85                  90                  95
   Gly Val Leu Pro Gly His Val Ala Thr Ile Ala Glu Leu Lys Pro Gly
               100                 105                 110
   Val Leu Thr Val Gln Glu Gly Thr Asp Thr Thr Lys Tyr Phe Val Ser
           115                 120                 125
   Ser Gly Phe Arg Phe Ile His Ala Asn Ser Val Ala Asp Ile Ile Ala
       130                 135                 140
   Val Glu Ala Val Pro Val Asn Gln Leu Asp Arg Asp Leu Val Gln Lys
   145                 150                 155                 160
   Gly Leu Gln Glu Phe Thr Gln Lys Leu Asn Ser Ala Thr Thr Asp Leu
                   165                 170                 175
   Glu Lys Arg Glu Ala Gln Ile Gly Ile Asp Val Asp Ser Ala Leu Asn
               180                 185                 190
   Ser Ala Leu Thr Gly
           195
<210> 710
   <211> 156
   <212> PRT
   <213> Oryza sativa
<400> 710
   Met Lys Ile Ile Phe Phe Phe Ala Leu Leu Ala Ile Ala Ala Cys Ser
   1               5                   10                  15
   Ala Ser Ala Gln Phe Asp Ala Val Thr Gln Val Tyr Arg Gln Tyr Gln
               20                  25                  30
   Leu Gln Pro His Leu Met Leu Gln Gln Gln Met Leu Ser Pro Cys Gly
           35                  40                  45
   Glu Phe Val Arg Gln Gln Cys Ser Thr Val Ala Thr Pro Phe Phe Gln
       50                  55                  60
   Ser Pro Val Phe Gln Leu Arg Asn Cys Gln Val Met Gln Gln Gln Cys
   65                  70                  75                  80
   Cys Gln Gln Leu Arg Met Ile Ala Gln Gln Ser His Cys Gln Ala Ile
                   85                  90                  95
   Ser Ser Val Gln Ala Ile Val Gln Gln Leu Arg Leu Gln Gln Phe Ala
               100                 105                 110
   Ser Val Tyr Phe Asp Gln Ser Gln Ala Gln Ala Gln Ala Met Leu Ala
           115                 120                 125
   Leu Asn Met Pro Ser Ile Cys Gly Ile Tyr Pro Ser Tyr Asn Thr Ala
       130                 135                 140
   Pro Cys Ser Ile Pro Thr Val Gly Gly Ile Trp Tyr
   145                 150                 155
<210> 711
   <211> 704
   <212> PRT
   <213> Pisum sativum
<400> 711
   Met Gln Asp Ser Ser Thr Met Lys Phe Ser Pro Leu Asp Leu Met Thr
   1               5                   10                  15
   Ala Ile Ile Lys Gly Lys Phe Asn Pro Ser Asn Asp Ser Ser Gln Ala
               20                  25                  30
   Pro Ala Ser Ile Ile Phe Glu Asn Arg Glu Phe Val Met Ile Leu Thr
           35                  40                  45
   Thr Ser Ile Ala Val Leu Ile Gly Cys Val Val Val Leu Ile Trp Arg
       50                  55                  60
   Arg Ser Asn Ser Asn Lys Ser Lys Gln Ile Glu Val Pro Lys Leu Val
   65                  70                  75                  80
   Ile Lys Lys Leu Pro Glu Leu Asp Val Asp Asp Gly Lys Lys Lys Val
                   85                  90                  95
   Thr Val Phe Phe Gly Thr Gln Thr Gly Thr Ala Glu Gly Phe Ala Lys
               100                 105                 110
   Ala Ile Ala Glu Glu Ala Lys Ala Arg Tyr Glu Lys Ala Lys Phe Arg
           115                 120                 125
   Val Val Asp Met Asp Asp Tyr Ala Ala Asp Asp Asp Glu Tyr Leu Glu
   130 135 140
   Lys Leu Lys Arg Glu Thr Met Ala Leu Phe Phe Leu Ala Thr Tyr Gly
   145 150 155 160
   Asp Gly Glu Pro Thr Asp Asn Ala Ala Arg Phe Tyr Lys Trp Phe Thr
   165 170 175
   Glu Glu Tyr Glu Gly Glu Glu Asp Ser Phe Lys Asn Leu Ser Tyr Gly
   180 185 190
   Val Phe Gly Leu Gly Asn Arg Gln Tyr Glu His Phe Asn Lys Val Ala
   195 200 205
   Lys Val Val Asp Asp Lys Leu Leu Glu Gln Gly Gly Lys Arg Leu Val
   210 215 220
   Pro Val Gly Leu Gly Asp Asp Asp Gln Cys Ile Glu Asp Asp Phe Thr
   225 230 235 240
   Ala Trp Lys Glu Glu Leu Trp Pro Ala Leu Asp Gln Leu Leu Arg Asp
   245 250 255
   Glu Asp Asp Thr Pro Val Ala Thr Pro Tyr Thr Ala Ala Val Ser Glu
   260 265 270
   Tyr Arg Val Val Ile His Asp Pro Leu Asp Ala Thr Val Asp Glu Lys
   275 280 285
   Lys Arg His Asn Val Asn Gly His Ala Val Val Asp Ala Gln His Pro
   290 295 300
   Val Arg Ala Asn Val Ala Val Arg Arg Glu Leu His Thr Pro Ala Ser
   305 310 315 320
   Asp Arg Ser Cys Thr His Leu Glu Phe Asp Ile Ser Gly Thr Gly Val
   325 330 335
   Val Tyr Glu Thr Gly Asp His Val Gly Val Tyr Cys Glu Asn Leu Ser
   340 345 350
   Asp Thr Val Glu Glu Ala Glu Arg Ile Leu Gly Leu Ser Pro Asp Thr
   355 360 365
   Tyr Leu Ser Ile His Thr Asp Asp Glu Glu Gly Lys Pro Leu Gly Gly
   370 375 380
   Ser Ser Leu Pro Pro Pro Phe Pro Pro Cys Thr Leu Arg Thr Ala Leu
   385 390 395 400
   Thr Lys Tyr Ala Asp Leu Leu Ser Ser Pro Lys Lys Ser Ala Leu Val
   405 410 415
   Ala Leu Ala Ala His Ala Ser Asp Pro Ser Glu Ala Asp Arg Leu Arg
   420 425 430
   His Leu Ala Ser Pro Ala Gly Lys Asp Glu Tyr Ala Glu Trp Val Ile
   435 440 445
   Ser Ser Gln Arg Ser Leu Leu Glu Val Met Ala Glu Phe Ser Ser Ala
   450 455 460
   Lys Pro Pro Ile Gly Val Phe Phe Ala Ala Val Ala Pro Arg Leu Gln
   465 470 475 480
   Pro Arg Tyr Tyr Ser Ile Ser Ser Ser Pro Arg Met Ala Pro Ser Arg
   485 490 495
   Ile His Val Thr Cys Ala Leu Val His Asp Lys Met Pro Thr Gly Arg
   500 505 510
   Ile His Lys Gly Val Cys Ser Thr Trp Met Lys Asn Ser Val Pro Leu
   515 520 525
   Glu Lys Asn Gln Asp Cys Ser Trp Ala Pro Ile Phe Val Arg Gln Ser
   530 535 540
   Asn Phe Arg Leu Pro Ala Asp Asn Lys Val Pro Val Ile Met Ile Gly
   545 550 555 560
   Pro Gly Thr Gly Leu Ala Pro Phe Arg Gly Phe Leu Gln Glu Arg Leu
   565 570 575
   Ala Leu Lys Glu Asp Gly Ala Glu Leu Gly Pro Ser Val Leu Phe Phe
   580 585 590
   Gly Cys Arg Asn Arg Gln Val Asp Tyr Ile Tyr Glu Asp Glu Leu Asn
   595 600 605
   His Phe Val Asn Gly Gly Ala Leu Ser Glu Leu Ile Val Ala Phe Ser
   610 615 620
   Arg Asp Gly Pro Thr Lys Glu Tyr Val Gln His Lys Met Met Glu Lys
   625 630 635 640
   Ala Ser Asp Ile Trp Asn Met Ile Ser Gln Gly Ala Tyr Val Tyr Val
   645 650 655
   Cys Gly Asp Ala Lys Gly Met Ala Arg Asp Val His Arg Thr Leu His
   660 665 670
   Thr Ile Leu Gln Glu Gln Gly Ser Leu Asp Ser Ser Lys Thr Glu Ser
   675 680 685
   Met Val Lys Asn Leu Gln Met Thr Gly Arg Tyr Leu Arg Asp Val Trp
   690 695 700
<210> 712
   <211> 358
   <212> PRT
   <213> Oryza sativa
<400> 712
   Met Ser Ala Tyr Cys Gly Lys Tyr Lys Asp Glu Leu Ile Lys Asn Ala
   1 5 10 15
   Ala Tyr Ile Gly Thr Pro Gly Lys Gly Ile Leu Ala Ala Asp Glu Ser
   20 25 30
   Thr Gly Thr Ile Gly Lys Arg Phe Ala Ser Ile Asn Val Glu Asn Val
   35 40 45
   Glu Glu Asn Arg Arg Ser Leu Arg Glu Leu Leu Phe Thr Thr Pro Gly
   50 55 60
   Ala Leu Gln His Leu Ser Gly Val Ile Leu Phe Glu Glu Thr Leu Tyr
   65 70 75 80
   Gln Lys Thr Lys Asp Gly Lys Pro Phe Val Asp Val Leu Lys Glu Gly
   85 90 95
   Gly Val Leu Pro Gly Ile Lys Val Asp Lys Gly Thr Val Glu Val Ala
   100 105 110
   Gly Thr Asn Lys Glu Thr Thr Thr Gln Gly His Asp Asp Leu Gly Lys
   115 120 125
   Arg Cys Ala Lys Tyr Tyr Glu Ala Gly Ala Arg Phe Ala Lys Trp Arg
   130 135 140
   Ala Val Leu Lys Ile Gly Pro Asn Glu Pro Ser Gln Leu Ser Ile Asp
   145 150 155 160
   Leu Asn Ala Gln Gly Leu Ala Arg Tyr Ala Ile Ile Cys Gln Glu Asn
   165 170 175
   Gly Leu Val Pro Ile Val Glu Pro Glu Ile Leu Val Asp Gly Ser His
   180 185 190
   Asp Ile Glu Arg Cys Ala Tyr Val Thr Glu Lys Val Leu Ala Ala Cys
   195 200 205
   Tyr Lys Ala Leu Asn Glu His His Val Leu Leu Glu Gly Ser Leu Leu
   210 215 220
   Lys Pro Asn Met Val Thr Pro Gly Ser Glu Ser Lys Lys Val Ser Pro
   225 230 235 240
   Gln Leu Ile Ala Glu Tyr Thr Val Arg Ala Leu Gln Arg Thr Val Pro
   245 250 255
   Ala Ala Val Pro Ala Ile Val Phe Leu Ser Gly Gly Gln Ser Glu Glu
   260 265 270
   Glu Ala Thr Val Asn Leu Asn Ala Met Asn Lys Leu Ser Thr Lys Lys
   275 280 285
   Pro Trp Ala Leu Ser Phe Ser Phe Gly Arg Ala Leu Gln Gln Ser Thr
   290 295 300
   Leu Lys Ala Trp Gly Gly Lys Thr Glu Asn Val Val Lys Ala Gln Lys
   305 310 315 320
   Ala Phe Ile Thr Arg Cys Lys Ala Asn Ser Glu Ala Thr Leu Gly Thr
   325 330 335
   Tyr Gln Gly Asp Ala Val Leu Gly Glu Gly Ala Ser Glu Ser Leu His
   340 345 350
   Val Lys Asp Tyr Lys Tyr
   355
<210> 713
   <211> 275
   <212> PRT
   <213> Pisum sativum
<400> 713
   Met Ala Ser Leu Gln Thr Gln Met Ile Ser Phe Tyr Ala Ile Phe Leu
   1 5 10 15
   Ser Ile Leu Leu Thr Thr Ile Leu Phe Phe Lys Val Asn Ser Thr Glu
               20                  25                  30
   Thr Thr Ser Phe Leu Ile Thr Lys Phe Ser Pro Asp Gln Gln Asn Leu
           35                  40                  45
   Ile Phe Gln Gly Asp Gly Tyr Thr Thr Lys Glu Lys Leu Thr Leu Thr
       50                  55                  60
   Lys Ala Val Lys Asn Thr Val Gly Arg Ala Leu Tyr Ser Ser Pro Ile
   65                  70                  75                  80
   His Ile Trp Asp Arg Glu Thr Gly Asn Val Ala Asn Phe Val Thr Ser
                   85                  90                  95
   Phe Thr Phe Val Ile Asn Ala Pro Asn Ser Tyr Asn Val Ala Asp Gly
               100                 105                 110
   Phe Thr Phe Phe Ile Ala Pro Val Asp Thr Lys Pro Gln Thr Gly Gly
           115                 120                 125
   Gly Tyr Leu Gly Val Phe Asn Ser Ala Glu Tyr Asp Lys Thr Thr Gln
       130                 135                 140
   Thr Val Ala Val Glu Phe Asp Thr Phe Tyr Asn Ala Ala Trp Asp Pro
   145                 150                 155                 160
   Ser Asn Arg Asp Arg His Ile Gly Ile Asp Val Asn Ser Ile Lys Ser
                   165                 170                 175
   Val Asn Thr Lys Ser Trp Lys Leu Gln Asn Gly Glu Glu Ala Asn Val
               180                 185                 190
   Val Ile Ala Phe Asn Ala Ala Thr Asn Val Leu Thr Val Ser Leu Thr
           195                 200                 205
   Tyr Pro Asn Ser Leu Glu Glu Glu Asn Val Thr Ser Tyr Thr Leu Ser
       210                 215                 220
   Asp Val Val Ser Leu Lys Asp Val Val Pro Glu Trp Val Arg Ile Gly
   225                 230                 235                 240
   Phe Ser Ala Thr Thr Gly Ala Glu Tyr Ala Ala His Glu Val Leu Ser
                   245                 250                 255
   Trp Ser Phe His Ser Glu Leu Ser Gly Thr Ser Ser Ser Lys Gln Ala
               260                 265                 270
   Ala Asp Ala
   275
<210> 714
   <211> 484
   <212> PRT
   <213> Oryza sativa
<400> 714
   Met Ala Thr Thr Thr Ser Leu Leu Ser Ser Cys Leu Cys Ala Leu Leu
   1 5 10 15
   Leu Ala Pro Leu Phe Ser Gln Gly Val Asp Ala Trp Glu Ser Arg Gln
   20 25 30
   Gly Ala Ser Arg Gln Cys Arg Phe Asp Arg Leu Gln Ala Phe Glu Pro
   35 40 45
   Leu Arg Lys Val Arg Ser Glu Ala Gly Asp Thr Glu Tyr Phe Asp Glu
   50 55 60
   Arg Asn Glu Gln Phe Arg Cys Ala Gly Val Phe Val Ile Arg Arg Val
   65 70 75 80
   Ile Glu Pro Gln Gly Leu Val Val Pro Arg Tyr Ser Asn Thr Pro Ala
   85 90 95
   Leu Ala Tyr Ile Ile Gln Gly Lys Gly Tyr Val Gly Leu Thr Phe Pro
   100 105 110
   Gly Cys Pro Ala Thr His Gln Gln Gln Phe Gln Leu Phe Glu Gln Arg
   115 120 125
   Gln Ser Asp Gln Ala His Lys Phe Arg Asp Glu His Gln Lys Ile His
   130 135 140
   Glu Phe Arg Gln Gly Asp Val Val Ala Leu Pro Ala Ser Val Ala His
   145 150 155 160
   Trp Phe Tyr Asn Gly Gly Asp Thr Pro Ala Val Val Val Tyr Val Tyr
   165 170 175
   Asp Ile Lys Ser Phe Ala Asn Gln Leu Glu Pro Arg Gln Lys Glu Phe
   180 185 190
   Leu Leu Ala Gly Asn Asn Gln Arg Gly Gln Gln Ile Phe Glu His Ser
   195 200 205
   Ile Phe Gln His Ser Gly Gln Asn Ile Phe Ser Gly Phe Asn Thr Glu
       210                 215                 220
   Val Leu Ser Glu Ala Leu Gly Ile Asn Thr Glu Ala Ser Lys Arg Leu
   225                 230                 235                 240
   Gln Ser Gln Asn Asp Gln Arg Gly Asp Ile Ile Arg Val Lys His Gly
                   245                 250                 255
   Leu Gln Leu Leu Lys Pro Thr Leu Thr Gln Arg Gln Glu Glu His Arg
               260                 265                 270
   Gln Tyr Gln Gln Val Gln Tyr Arg Glu Gly Gln Tyr Asn Gly Leu Asp
           275                 280                 285
   Glu Asn Phe Cys Thr Ile Lys Ala Arg Val Asn Ile Glu Asn Pro Ser
       290                 295                 300
   Arg Ala Asp Tyr Tyr Asn Pro Arg Ala Gly Arg Ile Thr Leu Leu Asn
   305                 310                 315                 320
   Asn Gln Lys Phe Pro Ile Leu Asn Leu Ile Gly Met Gly Ala Ala Arg
                   325                 330                 335
   Val Asn Leu Tyr Gln Asn Ala Leu Leu Ser Pro Phe Trp Asn Ile Asn
               340                 345                 350
   Ala His Ser Val Val Tyr Ile Ile Gln Gly Ser Val Arg Val Gln Val
           355                 360                 365
   Ala Asn Asn Gln Gly Arg Ser Val Phe Asn Gly Val Leu His Gln Gly
       370                 375                 380
   Gln Leu Leu Ile Ile Pro Gln Asn His Ala Val Ile Lys Lys Ala Glu
   385                 390                 395                 400
   His Asn Gly Cys Gln Tyr Val Ala Ile Lys Thr Ile Ser Asp Pro Thr
                   405                 410                 415
   Val Ser Trp Val Ala Gly Lys Asn Ser Ile Leu Arg Ala Leu Pro Val
               420                 425                 430
   Asp Val Ile Ala Asn Ala Tyr Arg Ile Ser Arg Asp Glu Ala Arg Arg
           435                 440                 445
   Leu Lys Asn Asn Arg Ala Asp Glu Ile Gly Pro Phe Thr Pro Arg Phe
   450 455 460
   Pro Gln Lys Ser Gln Arg Gly Tyr Gln Phe Leu Thr Glu Gly Leu Ser
   465 470 475 480
   Leu Ile Gly Met
<210> 715
   <211> 123
   <212> PRT
   <213> Pisum sativum
<400> 715
   Gln Lys Ala Pro Arg Lys Gln Leu Ala Thr Lys Ala Ala Arg Lys Ser
   1 5 10 15
   Ala Pro Ala Thr Gly Gly Val Lys Lys Pro His Arg Phe Arg Pro Gly
   20 25 30
   Thr Val Ala Leu Arg Glu Ile Arg Lys Tyr Gln Lys Ser Thr Glu Leu
   35 40 45
   Leu Ile Arg Lys Leu Pro Phe Gln Arg Leu Val Arg Glu Ile Ala Gln
   50 55 60
   Asp Phe Lys Thr Asp Leu Arg Phe Gln Ser Ser Ala Val Ser Ala Leu
   65 70 75 80
   Gln Glu Ala Ala Glu Ala Tyr Leu Val Gly Leu Phe Glu Asp Thr Asn
   85 90 95
   Leu Cys Ala Ile His Ala Lys Arg Val Thr Ile Met Pro Lys Asp Ile
   100 105 110
   Gln Leu Ala Arg Arg Ile Arg Gly Glu Arg Ala
   115 120
<210> 716
   <211> 232
   <212> PRT
   <213> Pisum sativum
<400> 716
   Met Ala Gln Ser Val Ser Leu Ser Thr Ile Ala Ser Pro Ile Leu Ser
   1               5                   10                  15
   Gln Lys Pro Gly Ser Ser Val Lys Ser Thr Pro Pro Cys Met Ala Ser
               20                  25                  30
   Phe Pro Leu Arg Arg Gln Leu Pro Arg Leu Gly Leu Arg Asn Val Arg
           35                  40                  45
   Ala Gln Ala Gly Gly Asp Gly Asp Asn Lys Asp Asn Ser Val Glu Val
       50                  55                  60
   His Arg Val Asn Lys Asp Asp Gln Gly Thr Ala Val Glu Arg Lys Pro
   65                  70                  75                  80
   Arg Arg Ser Ser Ile Asp Ile Ser Pro Phe Gly Leu Leu Asp Pro Trp
                   85                  90                  95
   Ser Pro Met Arg Ser Met Arg Gln Met Leu Asp Thr Met Asp Arg Ile
               100                 105                 110
   Phe Glu Asp Ala Ile Thr Ile Pro Gly Arg Asn Ile Gly Gly Gly Glu
           115                 120                 125
   Ile Arg Val Pro Trp Glu Ile Lys Asp Glu Glu His Glu Ile Arg Met
       130                 135                 140
   Arg Phe Asp Met Pro Gly Val Ser Lys Glu Asp Val Lys Val Ser Val
   145                 150                 155                 160
   Glu Asp Asp Val Leu Val Ile Lys Ser Asp His Arg Glu Glu Asn Gly
                   165                 170                 175
   Gly Glu Asp Cys Trp Ser Arg Lys Ser Tyr Ser Cys Tyr Asp Thr Arg
               180                 185                 190
   Leu Lys Leu Pro Asp Asn Cys Glu Lys Glu Lys Val Lys Ala Glu Leu
           195                 200                 205
   Lys Asp Gly Val Leu Tyr Ile Thr Ile Pro Lys Thr Lys Ile Glu Arg
       210                 215                 220
   Thr Val Ile Asp Val Gln Ile Gln
   225                 230
<210> 717
   <211> 10
   <212> PRT
   <213> Artificial Sequence
<220>
   <223> peptide
<400> 717
   Leu Asp Val Thr Pro Leu Ser Leu Gly Leu
   1 5 10
<210> 718
   <211> 12
   <212> PRT
   <213> Artificial Sequence
<220>
   <223> PEPTIDE
<400> 718
   Glu Glu Gly Ile Gln Leu Val Ala Glu Ala Ile Arg
   1 5 10
<210> 719
   <211> 9
   <212> PRT
   <213> Artificial Sequence
<220>
   <223> PEPTIDE
<400> 719
   Tyr Ser Leu Lys Pro Leu Val Pro Arg
   1 5
<210> 720
   <211> 4
   <212> PRT
   <213> Artificial Sequence
<220>
   <223> PEPTIDE
<400> 720
   Trp His Thr Ala
   1
<210> 721
   <211> 4
   <212> PRT
   <213> Artificial Sequence
<220>
   <223> PEPTIDE
<400> 721
   Trp His Asn Ala
   1
<210> 722
   <211> 4
   <212> PRT
   <213> Artificial Sequence
<220>
   <223> PEPTIDE
<400> 722
   Asn Asn Pro Phe
   1
<210> 723
   <211> 4
   <212> PRT
   <213> Artificial Sequence
<220>
   <223> PEPTIDE
<400> 723
   Met Arg Phe Arg
   1
<210> 724
   <211> 5
   <212> PRT
   <213> Artificial Sequence
<220>
   <223> PEPTIDE
<400> 724
   Met Pro Pro Ser Ser
   1 5
<210> 725
   <211> 4
   <212> PRT
   <213> Artificial Sequence
<220>
   <223> PEPTIDE
<400> 725
   His Met Pro Ser
   1
<210> 726
   <211> 5
   <212> PRT
   <213> Artificial Sequence
<220>
   <223> PEPTIDE
<400> 726
   His Met Pro Pro Ser
   1 5
<210> 727
   <211> 4
   <212> PRT
   <213> Artificial Sequence
<220>
   <223> PEPTIDE
<400> 727
   Gly His Pro Met
   1
<210> 728
   <211> 4
   <212> PRT
   <213> Artificial Sequence
<220>
   <223> PEPTIDE
<400> 728
   Phe Trp Asn Ala
   1
<210> 729
   <211> 4
   <212> PRT
   <213> Artificial Sequence
<220>
   <223> PEPTIDE
<400> 729
   Phe His Met Pro
   1
<210> 730
   <211> 11
   <212> PRT
   <213> Artificial Sequence
<220>
   <223> PEPTIDE
<400> 730
   Trp Thr Ile Val Gln Gly Leu Pro Ile Asp Glu
   1 5 10
<210> 731
   <211> 9
   <212> PRT
   <213> Artificial Sequence
<220>
   <223> PEPTIDE
<400> 731
   Gly Tyr Pro Met Tyr Pro Leu Pro Arg
   1 5
<210> 732
   <211> 9
   <212> PRT
   <213> Artificial Sequence
<220>
   <223> PEPTIDE
<400> 732
   His Gly Gly Glu Gly Gly Arg Pro Tyr
   1 5
<210> 733
   <211> 6
   <212> PRT
   <213> Artificial Sequence
<220>
   <223> PEPTIDE
<400> 733
   Leu Arg Gly Phe Ser Lys
   1 5
<210> 734
   <211> 9
   <212> PRT
   <213> Artificial Sequence
<220>
   <223> PEPTIDE
<400> 734
   Gly Ala Leu Met Leu Pro His Tyr Asn
   1 5
<210> 735
   <211> 11
   <212> PRT
   <213> Artificial Sequence
<220>
   <223> PEPTIDE
<400> 735
   Gly Ala Leu Met Leu Pro His Tyr Asn Ser Arg
   1 5 10
<210> 736
   <211> 9
   <212> PRT
   <213> Artificial Sequence
<220>
   <223> PEPTIDE
<400> 736
   Val Phe Asp Gly Val Leu Arg Pro Gly
   1 5
<210> 737
   <211> 6
   <212> PRT
   <213> Artificial Sequence
<220>
   <223> PEPTIDE
<400> 737
   Leu Gln Ser Gln Asn Asp
   1 5
<210> 738
   <211> 11
   <212> PRT
   <213> Artificial Sequence
<220>
   <223> PEPTIDE
<400> 738
   Leu Gln Ser Gln Asn Asp Gln Arg Gly Glu Ile
   1 5 10
<210> 739
   <211> 14
   <212> PRT
   <213> Artificial Sequence
<220>
   <223> PEPTIDE
<400> 739
   Gln Ser Gln Asn Asp Gln Arg Gly Glu Ile Ile His Val Lys
   1 5 10
<210> 740
   <211> 8
   <212> PRT
   <213> Artificial Sequence
<220>
   <223> PEPTIDE
<400> 740
   Arg Gly Glu Ile Ile His Val Lys
   1 5
<210> 741
   <211> 8
   <212> PRT
   <213> Artificial Sequence
<220>
   <223> PEPTIDE
<400> 741
   Arg Leu Gln Ser Gln Asn Asp Gln
   1 5
<210> 742
   <211> 10
   <212> PRT
   <213> Artificial Sequence
<220>
   <223> PEPTIDE
<400> 742
   Arg Leu Gln Ser Gln Asn Asp Gln Arg Gly
   1 5 10
<210> 743
   <211> 14
   <212> PRT
   <213> Artificial Sequence
<220>
   <223> PEPTIDE
<400> 743
   Arg Leu Gln Ser Gln Asn Asp Gln Arg Gly Glu Ile Ile His
   1 5 10
<210> 744
   <211> 4
   <212> PRT
   <213> Artificial Sequence
<220>
   <223> PEPTIDE
<400> 744
   Met Pro Met Pro
   1
<210> 745
   <211> 4
   <212> PRT
   <213> Artificial Sequence
<220>
   <223> PEPTIDE
<400> 745
   Pro Met Pro Leu
   1
<210> 746
   <211> 6
   <212> PRT
   <213> Artificial Sequence
<220>
   <223> PEPTIDE
<400> 746
   Leu Glu Pro Asp Asn Arg
   1 5
<210> 747
   <211> 13
   <212> PRT
   <213> Artificial Sequence
<220>
   <223> PEPTIDE
<400> 747
   Gly Ile Ala Arg Leu Ala Gly Thr Ser Ser Val Ile Asn
   1 5 10
<210> 748
   <211> 6
   <212> PRT
   <213> Artificial Sequence
<220>
   <223> PEPTIDE
<400> 748
   Arg Ser Gln Asn Ile Phe
   1 5
<210> 749
   <211> 4
   <212> PRT
   <213> Artificial Sequence
<220>
   <223> PEPTIDE
<400> 749
   Pro Asn Ser Met
   1
<210> 750
   <211> 4
   <212> PRT
   <213> Artificial Sequence
<220>
   <223> PEPTIDE
<400> 750
   Gly His Pro Met
   1
<210> 751
   <211> 4
   <212> PRT
   <213> Artificial Sequence
<220>
   <223> PEPTIDE
<400> 751
   His Pro Met Ser
   1
<210> 752
   <211> 7
   <212> PRT
   <213> Artificial Sequence
<220>
   <223> PEPTIDE
<400> 752
   Phe Leu Pro Gln His Thr Asp
   1 5
<210> 753
   <211> 7
   <212> PRT
   <213> Artificial Sequence
<220>
   <223> PEPTIDE
<400> 753
   Glu Trp Gln Ile Asn Glu Lys
   1 5
<210> 754
   <211> 13
   <212> PRT
   <213> Artificial Sequence
<220>
   <223> PEPTIDE
<400> 754
   Gly Pro Gln Gln Tyr Ala Glu Trp Gln Ile Asn Glu Lys
   1 5 10
<210> 755
   <211> 8
   <212> PRT
   <213> Artificial Sequence
<220>
   <223> PEPTIDE
<400> 755
   Pro Gln Gln Tyr Ala Glu Trp Gln
   1 5
<210> 756
   <211> 7
   <212> PRT
   <213> Artificial Sequence
<220>
   <223> PEPTIDE
<400> 756
   Arg Gly Pro Gln Gln Tyr Ala
   1 5
<210> 757
   <211> 4
   <212> PRT
   <213> Artificial Sequence
<220>
   <223> PEPTIDE
<400> 757
   His Asn Pro Arg
   1
<210> 758
   <211> 4
   <212> PRT
   <213> Artificial Sequence
<220>
   <223> PEPTIDE
<400> 758
   Trp His Asn Arg
   1
<210> 759
   <211> 4
   <212> PRT
   <213> Artificial Sequence
<220>
   <223> PEPTIDE
<400> 759
   Trp Asp Pro Ala
   1
<210> 760
   <211> 4
   <212> PRT
   <213> Artificial Sequence
<220>
   <223> PEPTIDE
<400> 760
   His Pro Ser Phe
   1
<210> 761
   <211> 22
   <212> PRT
   <213> Artificial Sequence
<220>
   <223> PEPTIDE
<400> 761
   Pro Gly Gln Leu Gln Ser Phe Leu Leu Ser Gly Asn Gln Asn Gln Gln
   1               5                   10                  15
   Asn Tyr Leu Ser Gly Phe
               20
<210> 762
   <211> 22
   <212> PRT
   <213> Artificial Sequence
<220>
   <223> PEPTIDE
<400> 762
   Gln Leu Gln Ser Phe Leu Leu Ser Gly Asn Gln Asn Gln Gln Asn Tyr
   1 5 10 15
   Leu Ser Gly Phe Ser Lys
   20
<210> 763
   <211> 12
   <212> PRT
   <213> Artificial Sequence
<220>
   <223> PEPTIDE
<400> 763
   Gln Ser Phe Leu Leu Ser Gly Asn Gln Asn Gln Gln
   1 5 10
<210> 764
   <211> 12
   <212> PRT
   <213> Artificial Sequence
<220>
   <223> PEPTIDE
<400> 764
   Pro Gly Gln Leu Gln Ser Phe Leu Leu Ser Gly Asn
   1 5 10
<210> 765
   <211> 9
   <212> PRT
   <213> Artificial Sequence
<220>
   <223> PEPTIDE
<400> 765
   Gln Ser Phe Leu Leu Ser Gly Asn Gln
   1 5
<210> 766
   <211> 12
   <212> PRT
   <213> Artificial Sequence
<220>
   <223> PEPTIDE
<400> 766
   Gln Asn Gln Gln Asn Tyr Leu Ser Gly Phe Ser Lys
   1 5 10
<210> 767
   <211> 6
   <212> PRT
   <213> Artificial Sequence
<220>
   <223> PEPTIDE
<400> 767
   Tyr Leu Arg Gly Phe Ser
   1 5
<210> 768
   <211> 11
   <212> PRT
   <213> Artificial Sequence
<220>
   <223> PEPTIDE
<400> 768
   Pro Val Glu Met Pro Thr Leu Leu Tyr Pro Ser
   1 5 10
<210> 769
   <211> 13
   <212> PRT
   <213> Artificial Sequence
<220>
   <223> PEPTIDE
<400> 769
   Arg Gly Pro Gln Gln Tyr Ala Glu Trp Gln Ile Asn Glu
   1 5 10
<210> 770
   <211> 24
   <212> PRT
   <213> Artificial Sequence
<220>
   <223> PEPTIDE
<400> 770
   Gly Tyr Val Gly Leu Thr Phe Pro Gly Cys Pro Ala Thr His Gln Gln
   1 5 10 15
   Gln Phe Gln Leu Phe Glu Gln Arg
   20
<210> 771
   <211> 18
   <212> PRT
   <213> Artificial Sequence
<220>
   <223> PEPTIDE
<400> 771
   Lys Asn Pro Gln Leu Gln Asp Leu Asp Ile Phe Val Asn Tyr Val Glu
   1 5 10 15
   Ile Lys
<210> 772
   <211> 24
   <212> PRT
   <213> Artificial Sequence
<220>
   <223> PEPTIDE
<400> 772
   Pro Gly Gln Leu Gln Ser Phe Leu Leu Ser Gly Asn Gln Asn Gln Gln
   1 5 10 15
   Asn Tyr Leu Ser Gly Phe Ser Lys
   20
<210> 773
   <211> 14
   <212> PRT
   <213> Artificial Sequence
<220>
   <223> PEPTIDE
<400> 773
   Arg Gly Pro Gln Gln Tyr Ala Glu Trp Gln Ile Asn Glu Lys
   1 5 10
<210> 774
   <211> 14
   <212> PRT
   <213> Artificial Sequence
<220>
   <223> PEPTIDE
<400> 774
   Val Leu Asp Leu Ala Ile Pro Val Asn Arg Pro Gly Gln Leu
   1               5                   10
<210> 775
   <211> 14
   <212> PRT
   <213> Artificial Sequence
<220>
   <223> PEPTIDE
<400> 775
   Arg Gly Pro Gln Gln Tyr Ala Glu Trp Gln Ile Asn Glu Lys
   1               5                   10